# EUROPEAN PATENT APPLICATION

(11) **EP 1 710 248 A1**
(43) Date of publication of application: **11.10.2006**
(21) Application number: 05703988.5
(22) Date of filing: 21.01.2005
(51) Int. Cl.: C07H 15/00

(54) **NOVEL AMINOGLYCOSIDE ANTIBIOTIC EFFECTIVE AGAINST METHICILLIN RESISTANT STAPHYLOCOCCUS AUREUS (MRSA)**

(30) Priority: 21.01.2004 JP 2004013642
(71) Applicant: Meiji Seika Kaisha, Ltd., Tokyo 104-8002 (JP); Microbial Chemistry Research Foundation, Tokyo (JP)
(72) Inventor: MINOWA, Nobuto;, uhoku-ku, Yokohama-shi, Kanagawa 2228567 (JP); USUI, Takayuki;, uhoku-ku, Yokohama-shi, Kanagawa 2228567 (JP); AKIYAMA, Yoshihisa;, uhoku-ku, Yokohama-shi, Kanagawa 2228567 (JP); HIRAIWA, Yukiko;, uhoku-ku, Yokohama-shi, Kanagawa 2228567 (JP); YONEDA, Toshio;, uhoku-ku, Yokohama-shi, Kanagawa 2228567 (JP); HASEGAWA, Toshifumi;, uhoku-ku, Yokohama-shi, Kanagawa 2228567 (JP); MAEBASHI, Kazunori;, uhoku-ku, Yokohama-shi, Kanagawa 2228567 (JP); IDA, Takashi;, uhoku-ku, Yokohama-shi, Kanagawa 2228567 (JP); KATSUMATA, Kazuko;, uhoku-ku, Yokohama-shi, Kanagawa 2228567 (JP); OTSUKA, Keiko;, uhoku-ku, Yokohama-shi, Kanagawa 2228567 (JP); IKEDA, Daishiro, Tokyo 1510053 (JP)
(74) Representative: Texier, Christian
(86) International application number: PCT/JP2005/000767
(87) International publication number: WO 2005/070945

(57) **Abstract**

Disclosed are compounds represented by general formula (I) or pharmacologically acceptable salts or solvates thereof having excellent antimicrobial activity against bacteria causative of severe infections such as pneumonia and septicemia, particularly against MRSA, and also antimicrobial agents comprising these compounds, and pharmaceutical compositions comprising these compounds as an active component.

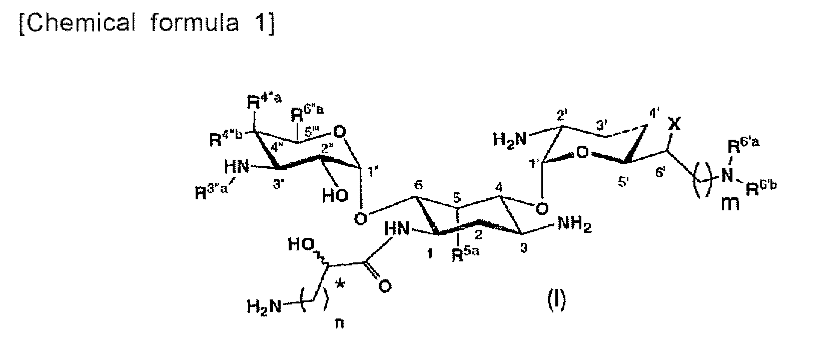

## Description

### CROSS-REFERENCE TO RELATED APPLICATIONS

This patent application claims priority to the prior Japanese Patent Application No. 13642/2004 filed January 21, 2004, and the description of the specification of the prior patent application is incorporated herein by reference.

### BACKGROUND OF THE INVENTION

Field of the Invention
The present invention relates to novel aminoglycoside antibiotics effective against bacteria causative of clinically severe infections, particularly against methicillin resistant Staphylococcus aureus (MRSAs).

Background Art
MRSAs have recently become regarded as problematic bacteria because they rapidly propagate through in-hospital infection and cause clinically severe infections, and, thus, studies have been made on therapeutic agents for the infections.

For example, the Journal of Antibiotics, vol. 24, 1971, p. 485 discloses that various derivatives of kanamycin, which is an amino glycoside antibiotic substance, have been synthesized, and 3',4'-deoxykanamycin B (dibekacin) has been found from the kanamycin derivatives. Dibekacin has been widely used as a chemotherapeutic agent effective against resistant bacteria since 1975.

The Journal of Antibiotics, vol. 26, 1973, p. 412 discloses (S)-1-N-(4-amino-2-hydroxybutyroyl)dibekacin (arbekacin) in which the amino group at the 1-position of dibekacin has been acylated with aminohydroxybutyryl acid (HABA), and arbekacin has been used as a specific medicine for MRSA infections since the end of 1990.

Journal of the JAPANESE SOCIETY OF CHEMOTHERAPY, vol. 50, 2002, p. 494 discloses that, despite the elapse of 10 years or more since arbekacin become used as a therapeutic agent for MRSA infections, bacteria highly resistant to arbekacin hardly appears clinically yet although the presence of low-resistant MRSAs has been found.

Japanese Patent No. 3215759 discloses that 5-substituted 2"-deoxy-2"-amino derivatives as a 5-substituted derivative of arbekacin are effective against resistant bacteria.

U.S Patent No. 4000261 and U.S Patent No. 4000262 disclose 5-epi derivatives in which the steric configuration of hydroxyl at the 5-position of aminoglycoside antibiotics has been reversed. These publications, however, neither suggest nor disclose the anti-MRSA activity of 5-epi derivatives.

Japanese Patent Publication No. 10719/1988 discloses a process for producing 1-N-(L-4-amino-2-hydroxybutyryl)-3',4'-dideoxykanamycin B having antimicrobial activity effective against various resistant bacteria.

The Journal of Antibiotics (1981), 34 (12), 1635-40 discloses a method for chemically modifying 5-deoxyarbekacin and further discloses 5-deoxyarbekacin and the like.

Carbohydrate Research 276 (1995), 75-89 discloses a process for producing 5-deoxy-5-epifluoroarbekacin and the like and the antimicrobial activity thereof.

The Journal of Antibiotics (1998), 51 (8), 735-42 discloses a process for producing 3"-N-acetylarbekacin and the like and the antimicrobial activity thereof.

The Journal of Antibiotics(1975), 28(4), 340-343 discloses a process for producing a 1-acylated derivative of 3',4'-dideoxy-6'-N-methyl-kanamycin B and the antimicrobial activity thereof.

### SUMMARY OF THE INVENTION

The present inventors have now found a group of compounds in aminoglycoside antibiotics, particularly arbekacin derivatives, characterized by having such a structure that the steric configuration of the 5-position of arbekacin has been reversed and various substituents have been introduced, and further found that this group of compounds have significant antimicrobial activity against bacteria causative of infectious diseases, particularly against MRSAs. The present inventors have further found a group of compounds characterized by having such a structure that two substituents have been introduced into the 5-position of arbekacin, and found that this group of compounds have significant antimicrobial activity against bacterial causative of infectious diseases, particularly against MRSAs. The present inventors have further found a group of compounds of which the 6'-, 3"-, 4"-, and 6"-positions corresponding to arbekacin have been derivatized, and found that this group of compounds have significant antimicrobial activity against bacterial causative of infectious diseases, particularly against MRSAs. The present invention has been found based on these finding.

Accordingly, an object of the present invention is to provide novel aminoglycoside antibiotics having significant antimicrobial activity against bacterial causative of severe infectious diseases, particularly against MRSAs.

According to a first aspect of the present invention, there are provided compounds represented by general formula (I) or pharmacologically acceptable salts or solvates thereof: 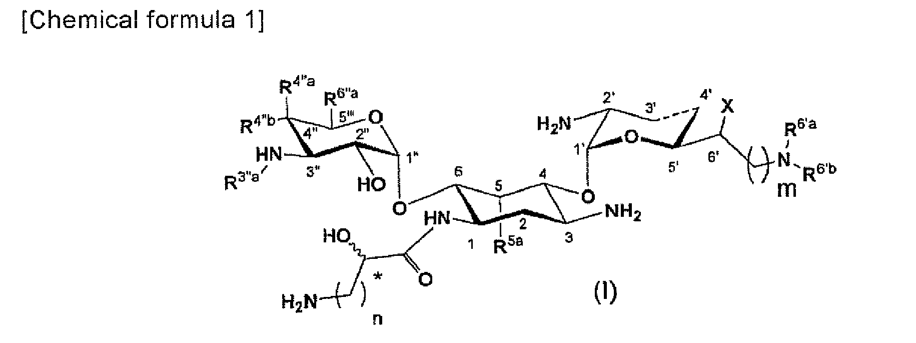 wherein
R^{4"a} and R^{4"b}, which may be the same or different, represent a hydrogen atom or hydroxyl,
R^{5a} represents a halogen atom,
hydroxyl,
amino,
azide,
C₁₋₆ alkanoyloxy,
C₁₋₆ alkylsulfonyloxy,
C₁₋₆ alkanoylamino,
arylcarbonylamino,
di-C₁₋₆ alkylamino, or
C₁₋₆ alkylamino wherein one or more hydrogen atoms in the alkyl group are optionally substituted by hydroxyl, phenyl, vinyl, amino, or hydroxymethyl,
R^{6"a} represents C₁₋₆ alkyl wherein one or more hydrogen atoms in the alkyl group are optionally substituted by hydroxyl, a halogen atom, or amino,
R^{6'a} and R^{6'b}, which may be the same or different, represent a hydrogen atom or C₁₋₆ alkyl,
R^{3"a} represents a hydrogen atom or C₁₋₆ alkyl,
the dashed line represents a single bond or a double bond,
m represents an integer of 0 to 2,
X represents a hydrogen atom or hydroxyl,
n represents an integer of 1 to 3, and
* represents an R or S configuration, provided that
R^{5a} represents a group as defined above other than a fluorine atom when R^{4"a} represents a hydrogen atom, R^{4"b} represents hydroxyl, and, the dashed line represents a single bond; and R^{5a} represents a group as defined above other than hydroxyl, amino, and azide when R^{3"a} represents a hydrogen atom, R^{4"a} represents a hydrogen atom, R^{4"b} represents hydroxyl, R^{6"a} represents hydroxymethyl, both R^{6'a} and R^{6'b} represent a hydrogen atom, X represents a hydrogen atom, and, the dashed line represents a single bond.

Further, according to the first aspect of the present invention, there is provided an antimicrobial agent comprising a compound according to the first aspect of the present invention or a pharmacologically acceptable salt or solvate thereof.

Furthermore, according to the first aspect of the present invention, there is provided an anti-MRSA agent comprising a compound represented by general formula (la) or a pharmacologically acceptable salt or solvate thereof: 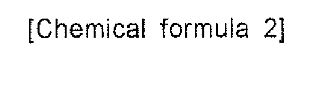 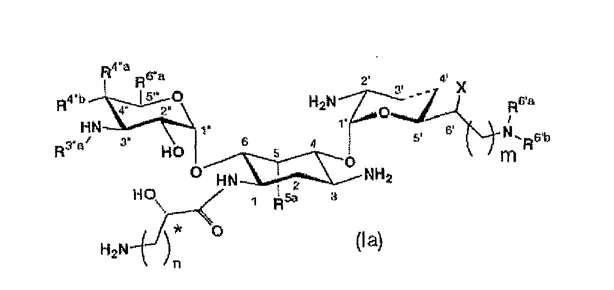 wherein
R^{4"a} and R^{4"b}, which may be the same or different, represent a hydrogen atom or hydroxyl,
R^{5a} represents a halogen atom,
hydroxyl,
amino,
azide,
C₁₋₆ alkanoyloxy,
C₁₋₆ alkylsulfonyloxy,
C₁₋₆ alkanoylamino,
arylcarbonylamino,
di-C₁₋₆ alkylamino, or
C₁₋₆ alkylamino wherein one or more hydrogen atoms in the alkyl group are optionally substituted by hydroxyl, phenyl, vinyl, amino, or hydroxymethyl,
R^{6"a} represents C₁₋₆ alkyl wherein one or more hydrogen atoms in the alkyl group are optionally substituted by hydroxyl, a halogen atom, or amino,
R^{6'a} and R^{6'b}, which may be the same or different, represent a hydrogen atom or C₁₋₆ alkyl,
R^{3"a} represents a hydrogen atom or C₁₋₆ alkyl,
the dashed line represents a single bond or a double bond, m represents an integer of 0 to 2,
X represents a hydrogen atom or hydroxyl,
n represents an integer of 1 to 3, and
* represents an R or S configuration, provided that
R^{5a} represents a group as defined above other than a fluorine atom when R^{4"a} represents a hydrogen atom, R^{4"b} represents hydroxyl, and the dashed line represents a single bond.

According to a second aspect of the present invention, there are provided compounds represented by general formula (II) or pharmacologically acceptable salts or solvates thereof: 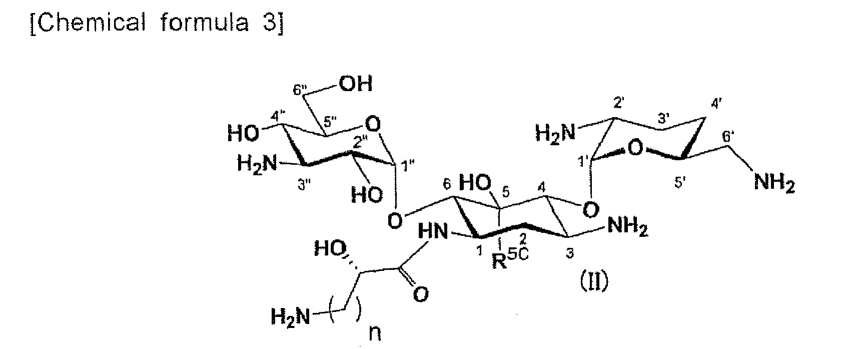 wherein
R^{5C} represents C₁₋₆ alkyl wherein one or more hydrogen atoms in the alkyl group are optionally substituted by C₁₋₆ alkoxy,
C₂₋₆ alkenyl, or
amino C₁₋₆ alkyl wherein one or more hydrogen atoms in the amino group are optionally substituted by C₁₋₆ alkyl wherein one or more hydrogen atoms in the alkyl group are optionally substituted by amino, hydroxyl, or heteroaryl, and
n represents an integer of 1 to 3.

According to the second aspect of the present invention, there is provided an antimicrobial agent comprising a compound according to the second aspect of the present invention or a pharmacologically acceptable salt or solvate thereof.

Further, according to the second aspect of the present invention, there is provided an anti-MRSA agent comprising a compound according to the second aspect of the present invention or a pharmacologically acceptable salt or solvate thereof.

According to a third aspect of the present invention, there are provided compounds represented by general formula (III) or pharmacologically acceptable salts or solvates thereof: 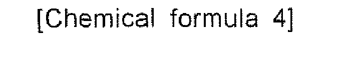 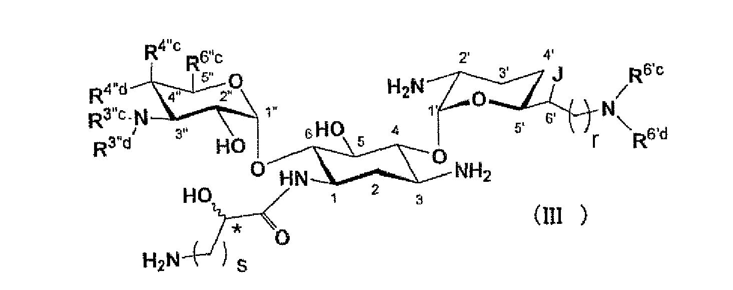 wherein
R^{4"c} represents a hydrogen atom or hydroxyl,
R^{4"d} represents a hydrogen atom or hydroxyl wherein, when
R^{4"c} represents hydroxyl, R^{4"d} represents a hydrogen atom,
R^{6"c} represents C₁₋₆ alkyl wherein one or more hydrogen atoms in the alkyl group are optionally substituted by hydroxyl, amino, or azide; or a groupof the formula: 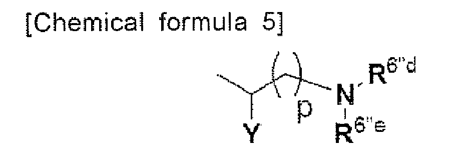 wherein R^{6"d} and R^{6"e}, which may be the same or different, represent a hydrogen atom or amino C₁₋₆ alkyl, or R^{6"d} and R^{6"e} together may represent a six-membered cyclic group containing 1 to 4 heteroatom, Y represents a hydrogen atom or hydroxyl, and p represents an integer of 0 or 1,
R^{3"c} and R^{3"d}, which may be the same or different, represent
a hydrogen atom,
C₁₋₁₀ alkyl wherein one or more hydrogen atoms in the alkyl group are optionally substituted by hydroxyl or aryl optionally substituted by hydroxyl or amino,
formimidoyl, or
amidino,
R^{6'c} and R^{6'd}, which may be the same or different, represent
a hydrogen atom,
amino C₁₋₆ alkyl,
formimidoyl,
amidino, or
benzyl optionally substituted by hydroxyl,
r represents an integer of 0 (zero) to 2,
J represents a hydrogen atom or hydroxyl,
s represents an integer of 1 to 3, and
* represents an R or S configuration,
excluding compounds wherein
R^{4"c} , R^{3"c}, R^{3"d}, R^{6'c}, and R^{6'd} simultaneously represent a hydrogen atom, R^{4"d} represents hydroxyl, R^{6"c} represents hydroxymethyl, r represents 0, X represents a hydrogen atom, and s represents 2.

According to the third aspect of the present invention, there is provided an antimicrobial agent comprising a compound according to the third aspect of the present invention or a pharmacologically acceptable salt or solvate thereof.

Further, according to the third aspect of the present invention, there is provided an anti-MRSA agent comprising a compound according to the third aspect of the present invention or a pharmacologically acceptable salt or solvate thereof.

The present invention provides novel aminoglycoside antibiotics that have excellent antimicrobial activity even against arbekacin resistant bacteria which are clinically obtained only in rare cases. The novel aminoglycoside antibiotics according to the present invention also have significant antimicrobial activity against bacteria causative of infectious diseases, for example, escherichia coli and Pseudomonas aeruginosa.

### DETAILED DESCRIPTION OF THE INVENTION

The term "alkyl," "alkoxy," or "alkenyl" as used herein as a group or a part of a group respectively mean straight chain, branched chain or cyclic alkyl, alkoxy, and alkenyl unless otherwise specified. The term "aryl" as used herein means phenyl or naphthyl unless otherwise specified, and the term "heteroaryl" as used herein means five- or six-membered heteroaryl (five- or six-membered cyclic aromatic heterocyclic group) containing 1 to 3 nitrogen, oxygen, or sulfur atoms unless otherwise specified.

5-Epiarbekacin analogues
In the first aspect of the present invention, halogen atoms represented by R^{5a} include, for example, fluorine, chlorine, bromine, and iodine atoms. More preferred are fluorine and chlorine atoms.

In the first aspect of the present invention, C₁₋₆ alkanoyloxy represented by R^{5a} is preferably C₁₋₃ alkanoyloxy, and specific examples thereof include formyloxy, acetyloxy, propionyloxy, butyryloxy, and isobutyryloxy. Among them, acetyloxy is more preferred.

In the first aspect of the present invention, C₁₋₆ alkylsulfonyloxy represented by R^{5a} is preferably C₁₋₃ alkylsulfonyloxy, and specific examples thereof include methylsulfonyloxy, ethylsulfonyloxy, propylsulfonyloxy, isopropylsulfonyloxy, and butylsulfonyloxy. Methylsulfonyloxy is more preferred.

In the first aspect of the present invention, C₁₋₆ alkanoylamino represented by R^{5a} is preferably C₁₋₃ alkanoylamino, and specific examples thereof include formylamino, acetylamino, propionylamino, butyrylamino, and isobutyrylamino. Among them, acetylamino is more preferred.

In the first aspect of the present invention, arylcarbonylamino represented by R^{5a} is preferably C₆₋₁₀ arylcarbonylamino, and specific examples thereof include phenylcarbonylamino and naphthylcarbonylamino. Phenylcarbonylamino is more preferred.

In the first aspect of the present invention, di-C₁₋₆ alkylamino represented by R^{5a} is preferably di-C₁₋₃ alkylamino, and specific examples thereof include dimethylamino, diethylamino, and methylethylamino. Dimethylamino is more preferred.

In the first aspect of the present invention, C₁₋₆ alkylamino represented by R^{5a} is preferably C₁₋₃ alkylamino, and specific examples thereof include methylamino, ethylamino, propylamino, isopropylamino, butylamino, tert-butylamino. Methyl amino is more preferred.

Further, one or more hydrogen atoms in the C₁₋₆ alkylamino group represented by R^{5a} are optionally substituted by hydroxyl, phenyl, vinyl, amino, or hydroxymethyl. Specific examples of substituted alkylamino include hydroxymethylamino, 2-hydraxyethylamino, 3-hydroxypropylamino, benzylamino, phenethylamino, 3-phenylpropyl, 4-phenylbutyl, arylamino, aminomethylamino, (2-aminoethyl)amino, and (2-hydroxy-1-hydroxymethylethyl)amino.

In the first aspect of the present invention, C₁₋₆ alkyl represented by R^{6"a} is preferably C₁₋₃ alkyl, and specific examples thereof include straight chain or branched chain C₁₋₆ alkyl, for example, methyl, ethyl, propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, and 2-ethylbutyl. Methyl or ethyl is more preferred.

Further, one or more hydrogen atoms in the C₁₋₆ alkyl group represented by R^{6"a} are optionally substituted by hydroxyl, a halogen atom, or amino. Specific examples of substituted C₁₋₆ alkyl include 2-aminol-hydroxyethyl, hydroxymethyl, hydroxyethyl, and fluoromethyl.

In the first aspect of the present invention, C₁₋₆ alkyl represented by R^{6'a}, R^{6'b}, and R^{3"a} is preferably C₁₋₃ alkyl.

In a preferred embodiment of the present invention, compounds represented by formula (I) are those
wherein
R^{5a} represents a halogen atom, hydroxyl, amino, azide, C₁₋₃ alkanoyloxy, C₁₋₃ alkylsulfonyloxy, C₁₋₃ alkanoylamino, phenylcarbonylamino, naphthylcarbonylamino, di-C₁₋₃ alkylamino, or C₁₋₃ alkylamino wherein one or more hydrogen atoms in the alkyl group are optionally substituted by hydroxyl, phenyl, vinyl, amino, or hydroxymethyl,
R^{6"a} represents C₁₋₃ alkyl wherein one or more hydrogen atoms in the alkyl group are optionally substituted by hydroxyl, a halogen atom, or amino,
R^{6'a} and R^{6'b}, which may be the same or different, represent a hydrogen atom or C₁₋₃ alkyl, and
R^{3"a} represents a hydrogen atom or C₁₋₃ alkyl.

In another preferred embodiment of the present invention, compounds represented by formula (I) are those
wherein
R^{4"a} represents a hydrogen atom or hydroxyl,
R^{4"b} represents a hydrogen atom,
R^{6"a} represents hydroxymethyl,
either one of R^{6'a} and R^{6'b} represents a hydrogen atom,
the dashed line represents a single bond,
m represents 0,
X represents a hydrogen atom, and
n represents an integer of 1 to 3.

In still another preferred embodiment of the present invention, compounds represented by formula (I) are those wherein
R^{5a} represents a chlorine atom, hydroxyl, amino, azide, C₁₋₆alkanoyloxy, C₁₋₆alkylsulfonyloxy, C₁₋₆alkanoylamino, arylcarbonylamino, di-C₁₋₆ alkylamino, or, C₁₋₆ alkylamino wherein one or more hydrogen atoms in the alkyl group are optionally substituted by hydroxyl, phenyl, vinyl, amino, or hydroxymethyl,
R^{6"a} represents hydroxymethyl,
either one of R^{6'a} and R^{6'b} represents a hydrogen atom,
the dashed line represents a single bond,
m represents 0,
X represents a hydrogen atom, and
n represents 2.

In a further preferred embodiment of the present invention, compounds represented by formula (I) are those
wherein
R^{6"a} represents hydroxymethyl or fluoromethyl,
both R^{6'a} and R^{6'b} represent a hydrogen atom,
R^{3"a} represents a hydrogen atom,
the dashed line represents a double bond,
m represents 0,
X represents a hydrogen atom, and
n represents 1 or 2.

5-Disubstituted arbekacin analogues
In the second aspect of the present invention, C₁₋₆ alkyl represented by R^{5c} is preferably C₁₋₃ alkyl.

One or more hydrogen atoms in the C₁₋₆ alkyl group (preferably C₁₋₃ alkyl group) represented by R^{5c} are optionally substituted by C₁₋₆ alkoxy, preferably C₁₋₃ alkoxy, and specific examples of substituted alkyl include methoxymethyl, and ethoxymethyl. Methoxymethyl is more preferred.

In the second aspect of the present invention, C₂₋₆ alkenyl represented by R^{5c} is preferably C₂₋₄ alkenyl, and specific examples thereof include vinyl, 2-propenyl, 1-methyl-2-propenyl, 2-methyl-2-propenyl, 2-ethyl-2-propenyl, 2-butenyl, 1-methyl-2-butenyl, 2-methyl-2-butenyl, 1-ethyl-2-butenyl, 3-butenyl, 1-methyl-3-butenyl, 2-methyl-3-butenyl, and 1-ethyl-3-butenyl.

In the second aspect of the present invention, amino C₁₋₆ alkyl represented by R^{5c} is preferably amino C₁₋₃ alkyl, and specific examples thereof include aminomethyl, 2-aminoethyl, and 3-aminopropyl. Aminomethyl is more preferred.

Further, one or more hydrogen atoms in the amino group in the amino C₁₋₆ alkyl group (preferably amino C₁₋₃ alkyl group) represented by R^{5c} are optionally substituted by C₁₋₆ alkyl, preferably C₁₋₃ alkyl. Methylaminomethyl is more preferred.

Further, one or more hydrogen atoms in the C₁₋₆ alkyl group (preferably C₁₋₃ alkyl group) as a substituent for one or more hydrogen atoms in the amino group in the amino C₁₋₆ alkyl group (preferably amino C₁₋₃ alkyl group) represented by R^{5c} are optionally substituted by amino, hydroxyl, or heteroaryl (preferably pyrrolyl or pyridyl), and examples of such R^{5c} include (2-aminoethyl)aminomethyl, (3-aminopropyl)aminamethyl, (3-amino-2-hydroxypropyl)aminomethyl, (2-hydroxyethyl)aminomethyl, arylaminomethyl, and (2-pyridylmethyl)amino. More preferred are (2-aminoethyl)aminomethyl, (3-amino-2-hydroxypropyl)aminomethyl, (2-hydroxyethyl)aminomethyl, and (2-pyridylmethyl)amino.

In another preferred embodiment of the present invention, compounds represented by formula (II) are those wherein R^{5c} represents C₁₋₃ alkyl wherein one or more hydrogen atoms in the alkyl group are optionally substituted by C₁₋₆ alkoxy; C₂₋₄ alkenyl; or amino C₁₋₃ alkyl wherein one or more hydrogen atoms in the amino group are optionally substituted by C₁₋₆ alkyl wherein one or more hydrogen atoms in the alkyl group are optionally substituted by amino, hydroxyl or heteroaryl.

In still another preferred embodiment of the present invention, compounds represented by formula (II) are those wherein R^{5C} represents C₁₋₆ alkyl wherein one or more hydrogen atoms in the alkyl group are optionally substituted by C₁₋₃ alkoxy; C₂₋₆ alkenyl; or amino C₁₋₆ alkyl wherein one or more hydrogen atoms in the amino group are optionally substituted by C₁₋₃ alkyl wherein one or more hydrogen atoms in the alkyl group are optionally substituted by amino, hydroxyl, pyrrolyl, or pyridyl.

In a further preferred embodiment of the present invention, compounds represented by formula (II) are those wherein R^{5C} represents C₁₋₃ alkyl wherein one or more hydrogen atoms in the alkyl group are optionally substituted by C₁₋₃ alkoxy; C₂₋₄ alkenyl; or amino C₁₋₃ alkyl wherein one or more hydrogen atoms in the amino group are optionally substituted by C₁₋₃ alkyl wherein one or more hydrogen atoms in the alkyl group are optionally substituted by amino, hydroxyl, pyrrolyl, or pyridyl.

Arbekacin derivatives wherein derivatization occurs at 6'-3"-, 4"-, and 6"-positions
In the third aspect of the present invention, C₁₋₆ alkyl represented by R^{6"c} is preferably C₁₋₃ alkyl.

Further, one or more hydrogen atoms in the C₁₋₆ alkyl group (preferably C₁₋₃ alkyl group) represented by R^{6"c} are optionally substituted by hydroxyl, amino, or azide. Examples of substituted alkyl include hydroxymethyl, aminomethyl, aminoethyl, azidomethyl, and azidoethyl. More preferred are hydroxymethyl, aminomethyl, and azidomethyl.

In the third aspect of the present invention, amino C₁₋₆ alkyl represented by R^{6"d} and R^{6"e} is preferably amino C₁₋₃ alkyl, and aminoethyl is more preferred.

In the third aspect of the present invention, the six-membered cyclic group containing 1 to 4 heteroatoms which R^{6"d} and R^{6"e} together represent includes six-membered heterocyclic saturated rings containing one or two heteroatoms selected from N and O. Specific examples thereof include morpholinyl, piperazyl, and piperidyl. Morpholinyl is more preferred.

In the third aspect of the present invention, C₁₋₁₀ alkyl represented by R^{3"c} and R^{3"d} is preferably C₁₋₆ alkyl. Specific examples thereof include straight chain or branched chain C₁₋₁₀ alkyl, for example, methyl, ethyl, propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, n-hexyl, n-heptyl, n-octyl, and n-nonyl. Preferred are methyl, ethyl, propyl, n-butyl, n-pentyl, and n-hexyl. Methyl or ethyl is more preferred.

Further, one or more hydrogen atoms in the C₁₋₁₀ alkyl group (preferably C₁₋₆ alkyl) represented by R^{3"c} and R^{3"d} are optionally substituted by hydroxyl or aryl optionally substituted by hydroxyl or amino. Specific examples of substituted alkyl include hydroxymethyl, hydroxyethyl, benzyl, phenethyl, (m-hydroxy)benzyl, (p-hydroxy)benzyl, and (m-amino)benzyl. Preferred are hydroxyethyl, hydroxyethyl, benzyl, phenethyl, (m-hydroxy)benzyl, (p-hydroxy)benzyl, and (m-amino)benzyl.

In the third aspect of the present invention, amino C₁₋₆ alkyl represented by R^{6'c} and R^{6'd} is preferably amino C₁₋₃ alkyl. Aminoethyl is more preferred.

In the third aspect of the present invention, specific examples of benzyl, which is optionally substituted by hydroxyl, represented by R^{6'c} and R^{6'd} include (o-hydroxy)benzyl, (m-hydroxy)benzyl, and (p-hydroxy)benzyl. (o-Hydroxy)benzyl is more preferred.

In a preferred embodiment of the present invention, compounds represented by formula (III) are those wherein R^{6"c} represents C₁₋₃ alkyl wherein one or more hydrogen atoms in the alkyl group are optionally substituted by hydroxyl, amino, or azide; or a group of formula: 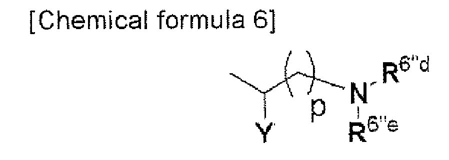 wherein R^{6"d} and R^{6"e}, which may be the same or different, represent a hydrogen atom or amino C₁₋₃alkyl, or R^{6"d} and R^{6"e} together may represent a six-membered cyclic group containing 1 to 4 heteroatom, Y represents a hydrogen atom or hydroxyl, and p represents an integer of 0 or 1,
R^{3"c} and R^{3"d}, which may be the same or different, represent
a hydrogen atom,
C₁₋₆ alkyl wherein one or more hydrogen atoms in the alkyl group are optionally substituted by hydroxyl; phenyl optionally substituted by hydroxyl or amino; or naphthyl optionally substituted by hydroxyl or amino,
formimidoyl, or
amidino,
R^{6'c} and R^{6'd}, which may be the same or different, represent
a hydrogen atom,
amino C₁₋₃alkyl,
formimidoyl,
amidino, or
benzyl optionally substituted by hydroxyl.

In another preferred embodiment of the present invention, compounds represented by formula (III) are those wherein R^{4"c} represents a hydrogen atom, R^{4"d} represents hydroxyl, both R^{6'c} and R^{6'd} represent a hydrogen atom, both R^{3"c} and R^{3"d} represent a hydrogen atom, r represents 0, J represents a hydrogen atom, and s represents 2.

In still another preferred embodiment of the present invention, compounds represented by formula (III) are those wherein R^{4"c} represents a hydrogen atom, R^{4"d} represents hydroxyl, both R^{6'c} and R^{6'd} represent a hydrogen atom, R^{6"c} represents hydroxymethyl, either one of R^{3"c} and R^{3"d} represent a hydrogen atom, r is 0 (zero), J represents a hydrogen atom, and s is 2.

In a further preferred embodiment of the present invention, compounds represented by formula (III) are those wherein R^{4"c} represents a hydrogen atom, R^{4"d} represents hydroxyl, R^{6"c} represents hydroxymethyl, both R^{3"c} and R^{3"d} represent a hydrogen atom, and s represents 2.

Further, according to the present invention, there are provided 5,4"-Diepiarbekacin, 5-deoxy-4"-epi-5-epifluoroarbekacin, 5-deoxy-4"-epi-5-epichloroarbekacin, 5-deoxy-4"-epi-5-epiaminoarbekacin, 4"-deoxy-5-epiarbekacin, 1-N-[(S)-(3-amino-2-hydroxypropanoyl)]-5,4"-diepidibekacin, 5,4"-diepi-3"-N-methylarbekacin, 5,4"-diepi-6'-N-methylarbekacin, 5-epiarbekacin, 5-deoxy-5-epichloroarbekacin, 5-deoxy-5-epiaminoarbekacin, 5-deoxy-5-epi(2-aminoethyl)aminoarbekacin, 5-epi-3"-N-methylarbekacin, 6"-aminomethyl-5-epiarbekacin, 3',4'-didehydro-5-epiarbekacin, 5-deoxy-3',4'-didehydro-5-epifluoroarbekacin, 5-deoxy-3',4'-didehydro-5-epiaminoarbekacin, 1-N-[(S)-(3-amino-2-hydroxypropanoyl)]-3',4'-didehydro-5-epidibekacin, 3',4'-didehydro-5,4"-diepiarbekacin, 5-deoxy-3',4'-didehydro-4"-epi-5-epifluoroarbekacin, 5-deoxy-3',4'-didehydro-4"-epi-5-epiaminoarbekacin, 4"-deoxy-3',4'-didehydro-5-epiarbekacin, and 6"-aminomethylarbekacin.

Production process
The compounds according to the first aspect of the present invention can be produced by the following processes A to Q.

### Process A

In process A, compounds represented by general formula (A9) are produced by introducing substituent R^{5a} into the 5-position of compound (A1) in an axial configuration and then introducing substituent R^{4"a} into the 4"-position in an axial configuration. Process A comprises the following steps. The compound represented by formula (A1) as a starting compound may be produced by the method described in Japanese Patent Laid-Open Nos. 62442/1974, 81897/1980, 164696/1980, and 10719/1988.

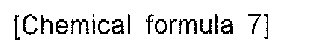

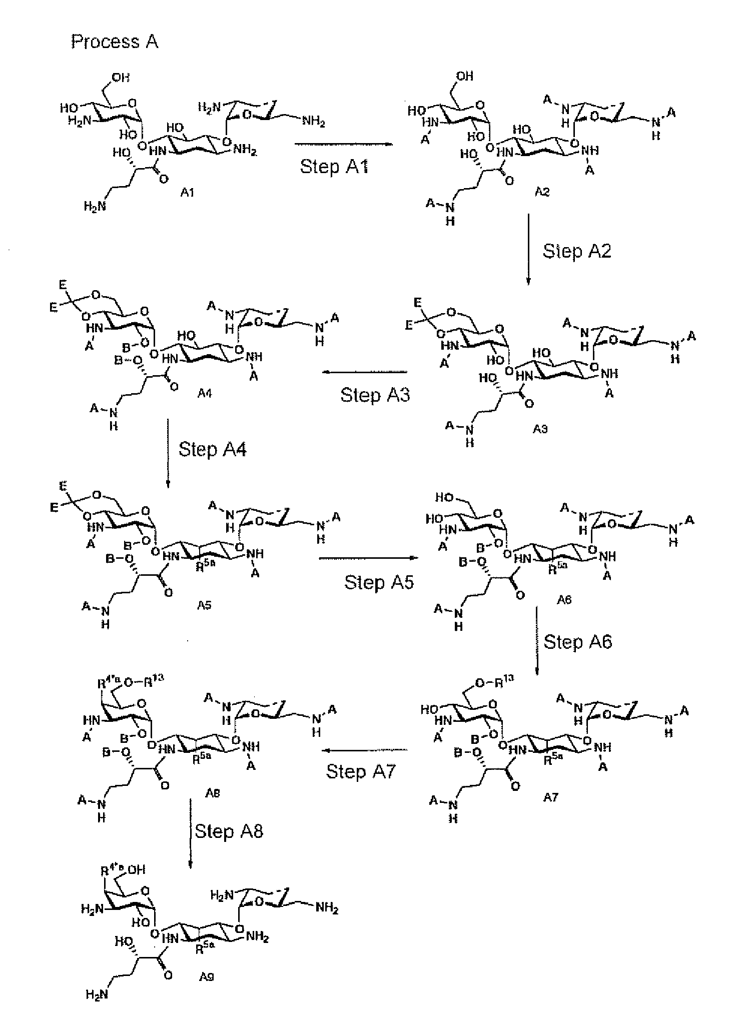

Step A1
In step A1, a compound of general formula (A2) is produced by introducing protective group (A) into five amino groups in the compound of formula (A1). Step A1 is achieved by reacting the comound of formula (A1) with A₂O or ACl in the presence of a base,
wherein A represents tert-butoxycarbonyl (Boc), benzyloxycarbonyl (group Z), p-methoxybenzyloxycarbonyl, or p-nitrobenzyloxycarbonyl.

Solvents usable in step A1 include water, N,N-dimethylformamide, tetrahydrofuran, dioxane, and mixed solvents thereof. The solvent is preferably a mixed solvent composed of water and N,N-dimethylformamide. Bases usable herein include sodium hydroxide, potassium carbonate, sodium carbonate, triethylamine, pyridine, and 4-dimethylaminopyridine. The base is preferably triethylamine. The reaction temperature is 0°C to 40°C, and the reaction time is 1 to 24 hr.

Process A2
In step A2, a compound of general formula (A3) is produced by introducing a protective group into hydroxyl at the 4"-position and 6"-position of the compound of general formula (A2). This step is achieved by reacting the compound of general formula (A2) with E₂CO or E₂C(OMe)₂ in the presence of an acid, wherein E represents a hydrogen atom, methyl, or phenyl or, as E₂C, cyclohexyl.

Solvents usable in this step include, for example, N,N-dimethylformamide, methylene chloride, chloroform, 1,2-dichloroethane, and ethyl acetate. The solvent is preferably N,N-dimethylformamide. Acids usable herein include, for example, p-toluenesulfonic acid, pyridinium p-toluenesulfonate, camphorsulfonic acid, and hydrochloric acid. The acid is preferably p-toluenesulfonic acid. The reaction temperature is 20°C to 50°C. The reaction time is 1 to 8 hr.

Step A3
In step A3, a compound of general formula (A4) is produced by introducing a protective group into hydroxyl at the 2"-position and 2"'-position of the compound of general formula (A3). This step is achieved by reacting the compound of general formula (A3) with B₂O or BCl wherein B represents acetyl or benzoyl in the presence of a base.

Solvents usable in this step include pyridine, N,N-dimethylformamide, methylene chloride, chloroform, and 1,2-dichloroethane. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step A4
In this step A4, a compound of general formula (A5) is produced by introducing a leaving group into hydroxyl at the 5-position of the compound of general formula (A4) and then subjecting the compound to a substitution reaction. This step is achieved by reacting the compound of general formula (A4) with WSO₂Cl, wherein W represents methyl, phenyl, or p-tolyl, in the presence of a base to synthesize a comound having substituted sulfonyloxy at the 5-position, and then reacting the resultant compound with R^{5a}M wherein R^{5a} represents acetoxy, azide, a chlorine atom, or C₁₋₆ alkylamino wherein one or more hydrogen atoms in the alkyl group are optionally substituted by hydroxyl, phenyl, vinyl, amino, or hydroxymethyl, and M represents lithium, sodium, cesium, or a hydrogen atom.

Solvents usable in the step of introducing a leaving group include, for example, methylene chloride, chloroform, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Solvents usable in the step of a substitution reaciotn include tetrahydrofuran, dioxane, 1,2-dimethoxyethane, N,N-dimethylformamide, and dimethylsulfoxide. The solvent is preferably N,N-dimethylformamide. The reaction temperature is 60°C to 90°C. The reaction time is 1 to 12 hr.

Step A5
In step A5, the protective group at the 4"-positon and 6"-position of the compound of general formula (A5) is removed. This step is achieved by reacting the compound of general formula (A5) with an acid.

Solvents usable in this step include tetrahydrofuran, diethyl ether, dioxane, methanol, methylene chloride, chloroform, water, and mixed solvents thereof. The solvent is preferably a mixed solvent composed of methylene chloride and methanol. Acids usable herein include acetic acid, trifluoroacetic acid, hydrochloric acid, sulfuric acid, p-toluenesulfonic acid, and boron trichloride. The acid is preferably trifluoroacetic acid. The reaction temperature is 0°C to 30°C. The reaction time is 0.1 to 8 hr.

In the compound of general formula (A5), when one of substituents E₂C is a hydrogen atom while the other substituent is phenyl, the protective group can also be removed by reacting the compound of general formula (A5) with hydrogen and a catalyst for catalytic hydrogen reduction. Catalysts for catalytic hydrogen reduction usable herein include palladium-carbon, palladium black, palladium hydroxide, and platinum oxide. The catalyst for catalytic hydrogen reduction is preferably a palladium-carbon catalyst. Any solvent may be used without particular limitation so far as the solvent is inert to this reaction. Preferred are methanol, ethanol, tetrahydrofuran, dioxane, and a mixed solvent composed of an organic solvent and water. The reaction temperature is 10°C to 30°C. The reaction time is generally 1 to 8 hr.

Step A6
In step A6, a compound of general formula (A7) is produced by introducing a protective group into hydroxyl at the 6"-position of the compound of general formula (A6). This step is achieved by reacting the compound of general formula (A6) with R¹³Cl wherein R¹³ represents triphenylmethyl, tert-butyldimethylsilyl, triisopropylsilyl, tert-butyldiphenylsilyl, or benzoyl, in the presence of a base.

Solvents usable in the step of introducing triphenylmethyl include methylene chloride, acetonitrile, and pyridine. The solvent is preferably pyridine. Bases usable herein include triethylamine, pyridine, and 4-dimethylaminopyridine. The base is preferably pyridine. The reaction temperature is 20°C to 80°C. The reaction time is generally 2 to 10 hr.

Preferred solvents usable in the step of introducing silyl include methylene chloride, chloroform, dimethylformamide, acetonitrile, and pyridine. Bases usable herein include 4-dimethylaminopyridine, triethylamine, imidazole, and diisopropylethylamine. The base is preferably 4-dimethylaminopyridine. The reaction temperature is 0°C to 30°C. The reaction time is 1 to 12 hr.

Preferred solvents usable in the step of introducing benzoyl include acetonitrile, pyridine, N,N-dimethylformamide, and tetrahydrofuran. Preferred bases usable herein include triethylamine, tetramethylethylendiamine, and pyridine. The reaction temperature is 0°C to 30°C. The reaction time is 1 to 8 hr. In this step, in addition to benzoyl chloride, for example, benzoic anhydride, benzoyl cyanide, or combinations of benzoic acid, diisopropyl azodicarboxylate, and triphenylphosphine may be used as the benzoylation reagent.

Step A7
In step A7, a compound of general formula (A8) is produced by introducing a leaving group into hydroxyl at the 4"-position of the compound of general formula (A7) and then subjecting the compound to a substitution reaction. This step is achieved by reacting the compound of general formula (A7) with trifluoromethanesulfonyl chloride or trifluoromethanesulfonic anhydride in the presence of a base to synthesize a compound having trifluoromethanesulfonyloxy at the 4"-position, and then reacting the resultant compound with R^{4"a}M wherein R^{4"a} represents C₁₋₆alkanoyloxy, or benzoyloxy and M represents lithium, sodium, or cesium.

Solvents usable in the step of introducing a leaving group include methylene chloride, chloroform, tetrahydrofuran, and ethyl acetate. The solvent is preferably methylene chloride. Bases usable herein include pyridine, lutidine, collidine, triethylamine, and diisopropylethylamine. The base is preferably pyridine. The reaction temperature is -30°C to 20°C. The reaction time is 1 to 6 hr.

Solvents usable in the step of a substitution reaction include tetrahydrofuran, dioxane, methylene chloride, and N,N-dimethylformamide. The solvent is preferably N,N-dimethylformamide. The reaction temperature is 0°C to 50°C. The reaction time is 1 to 6 hr.

Step A8
In step A8, a compound of general formula (A9) is produced by removing the protective group in the compound of general formula (A8). This step is achieved by reacting the compound of general formula (A8) with a base to remove the protective group of hydroxyl except for the protective group of hydroxyl at the 6"-position, and then reacting the resultant compound with an acid to remove the protective group of amino and hydroxyl at the 6"-position.

Solvents usable in the step of removing the protective group of hydroxyl except for the protective group of hydroxyl at the 6"-position include methanol, ethanol, isopropyl alcohol, tert-butylalcohol, methylene chloride, chloroform, and mixed solvents thereof. The solvent is preferably a mixed solvent composed of methanol and methylene chloride. Bases usable herein include potassium carbonate, sodium carbonate, potassium hydroxide, sodium hydroxide, sodium methoxide, sodium ethoxide, and tert-BuOK. The base is preferably sodium methoxide. The reaction temperature is 0°C to 60°C. The reaction time is 1 to 8 hr.

Solvents suitable in the step of removing the protective group of amino and hydroxyl at the 6"-position include ethyl acetate, methylene chloride, acetonitrile, acetone, and water. The solvent is preferably water. Acids usable herein include p-toluenesulfonic acid, methanesulfonic acid, acetic acid, and trifluoroacetic acid. The acid is preferably trifluoroacetic acid. The reaction temperature is generally 0°C to 30°C. The reaction time is 1 to 12 hr. When protective group A in the compound of general formula (A8) is benzyloxycarbonyl, p-methoxybenzyloxycarbonyl, or p-nitrobenzyloxycarbonyl and, at the same time, the dashed line represents a single bond, the protective group may also be removed by reacting the compound with hydrogen and a catalytic hydrogen reduction catalyst. Catalytic hydrogen reduction catalysts usable herein include palladium-carbon, palladium black, palladium hydroxide, and platinum oxide. The catalyst is preferably palladium-carbon. The solvent is not particularly limited so far as the solvent is inert to this reaction. Preferred are methanol, ethanol, tetrahydrofuran, dioxane, and a mixed solvent composed of these organic solvent and water. The reaction temperature is 10°C to 30°C. The reaction time is generally 1 to 8 hr.

Process B
In process B, compounds of general formula (B6) are produced by introducing substituent R^{4"a} into the 4"-position of a compound of general formula (B1) in an axial configuration and then introducing substituent R^{5a} into the 5-position in an axial configuration. Process B comprises the following steps. The compound of general formula (B1) as a starting compound may be produced in the same manner as in steps A1 to A3 in the above-described process A.

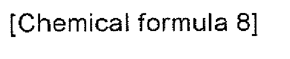

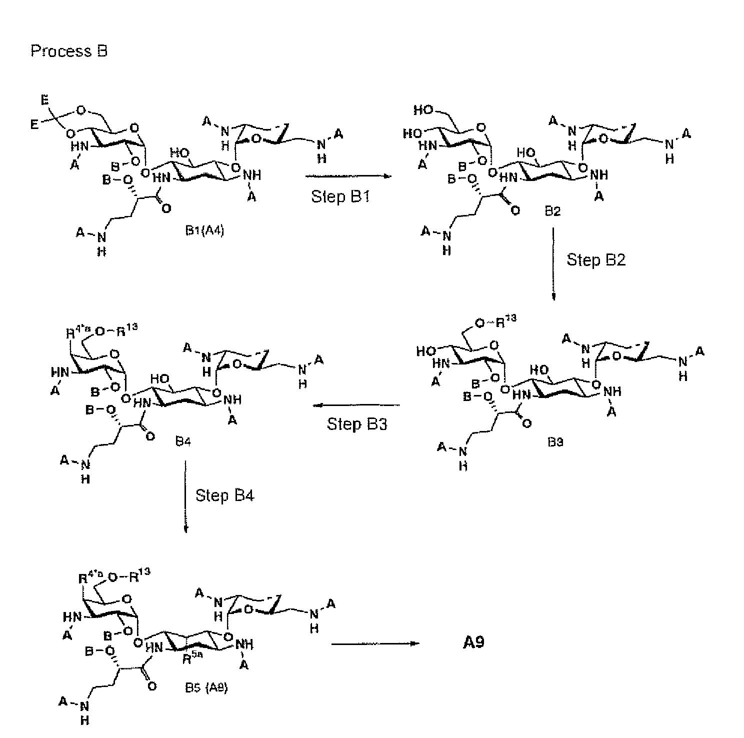

Step B1
In step (B1), the protective group at the 4"-positon and the 6"-position of the compound of general formula (B1) is removed. This step is achieved by reacting the compound of general formula (B1) with an acid.

Solvents usable in this step include tetrahydrofuran, diethyl ether, dioxane, methanol, methylene chloride, chloroform, water, and mixed solvents thereof. The solvent is preferably a mixed solvent composed of methylene chloride and methanol. Acids usable herein include acetic acid, trifluoroacetic acid, hydrochloric acid, sulfuric acid, p-toluenesulfonic acid, and boron trichloride. The acid is preferably trifluoroacetic acid. The reaction temperature is 0°C to 30°C. The reaction time is 0.1 to 8 hr.

In the compound of general formula (B1), when one of substituents of E₂C is a hydrogen atom while the other substituent is phenyl, the protective group can also be removed by reacting this compound with hydrogen and a catalyst for catalytic hydrogen reduction. Catalysts for catalytic hydrogen reduction usable herein include palladium-carbon, palladium black, palladium hydroxide, and platinum oxide. The catalyst for catalytic hydrogen reduction is preferably palladium-carbon. Any solvent may be used without particular limitation so far as the solvent is inert to this reaction. Preferred are methanol, ethanol, tetrahydrofuran, dioxane, and a mixed solvent composed of these organic solvent and water. The reaction temperature is 10°C to 30°C. The reaction time is generally 1 to 8 hr.

Process B2
In process B2, a protective group is introduced into hydroxyl at the 6"-position of the compound of general formula (B2). This step is achieved by reacting the compound of general formula (B2) with R¹³Cl wherein R¹³ represents triphenylmethyl, triisopropylsilyl, tert-butyldimethylsilyl, tert-butyldiphenylsilyl, or benzoyl, in the presence of a base.

Solvents usable in the step of introducing triphenylmethyl include methylene chloride, acetonitrile, and pyridine. The solvent is preferably pyridine. Bases usable herein include triethylamine, pyridine, and 4-dimethylaminopyridin. The base is preferably pyridine. The reaction temperature is 20°C to 80°C. The reaction time is generally 2 to 10 hr.

Preferred solvents usable in the step of introducing silyl include methylene chloride, chloroform, N,N-dimethylformamide, acetonitrile, and pyridine. Bases usable herein include 4-dimethylaminopyridine, triethylamine, imidazole, and diisopropylethylamine. The base is preferably 4-dimethylaminopyridine. The reaction temperature is 0°C to 30°C. The reaction time is 1 to 12 hr.

Preferred solvents usable in the step of introducing benzoyl include acetonitrile, pyridine, N,N-dimethylformamide, and tetrahydrofuran. Preferred bases usable herein include triethylamine, tetramethylethylendiamine, and pyridine. The reaction temperature is 0°C to 30°C. The reaction time is 1 to 8 hr. In this step, in addition to benzoyl chloride, for example, benzoic anhydride, benzoyl cyanide, or combinations of benzoic acid, diisopropyl azodicarboxylate, and triphenylphosphine may be used as the benzoylation reagent.

Step B3
In step B3, a compound of general formula (B4) is produced by introducing a leaving group into hydroxyl at the 4"-position of the compound of general formula (B3) and then subjecting the resultant compound to a substitution reaction. This step is achieved by reacting the compound of general formula (B3) with trifluoromethanesulfonyl chloride or trifluoromethanesulfonic anhydride in the presence of a base to synthesize a compound having trifluoromethanesulfonyloxy at the 4"-position, and then reacting the resultant compound with R^{4"a}M wherein R^{4"a} represents C₁-C₆ alkanoyl, or benzoyloxy and M represents lithium, sodium, or cesium.

Solvents usable in the step of introducing a leaving group include methylene chloride, chloroform, tetrahydrofuran, and ethyl acetate. The solvent is preferably methylene chloride. Bases usable herein include pyridine, lutidine, collidine, triethylamine, and diisopropylethylamine. The base is preferably pyridine. The reaction temperature is -30°C to 20°C. The reaction time is 1 to 6 hr.

Solvents usable in the step of a substitution reaction include tetrahydrofuran, dioxane, methylene chloride, and N,N-dimethylformamide. The solvent is preferably N,N-dimethylformamide. The reaction temperature is 0°C to 50°C. The reaction time is 1 to 6 hr.

Step B4
In step B4, a compound of general formula (B5) is produced by introducing a leaving group into hydroxyl at the 5-position of the compound of general formula (B4), and then subjecting the resultant compound to a substitution reaction. This step is achieved by reacting the compound of general formula (B4) with WSO₂Cl, wherein W represents methyl, phenyl, or p-tolyl, in the presence of a base to synthesize of a compound having substituted sulfonyloxy at the 5-position, and then reacting the resultant compound with R^{5a}M wherein R^{5a} represents acetoxy, azide, a chlorine atom, a bromine atom, or C₁₋₆ alkylamino wherein one or more hydrogen atoms in the alkyl group are optionally substituted by hydroxyl, phenyl, vinyl, amino, or hydroxymethyl, and M represents lithium, sodium, cesium, or a hydrogen atom.

Solvents usable in the step of introducing a leaving group include methylene chloride, chloroform, 1,2-dichloroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Solvents usable in the step of a substitution reaction include tetrahydrofuran, dioxane, 1,2-dimethoxyethane, and N,N-dimethylformamide. The solvent is preferably N,N-dimethylformamide. The reaction temperature is 20°C to 120°C, preferably 80°C to 120°C. The reaction time is 1 to 12 hr. A compound of general formula (A9) may be produced by deprotecting the compound of general formula (B5) according to step A8 in process A.

Process C
In process C, a compound of general formula (C3) is produced by introducing fluoro into the 5-position of a compound of general formula (C1) in an axial configuration. The process C comprises the following steps. Each step constituting process C will be described in detail. The compound of general formula (C1) as a starting compound in process C can be produced according to steps B1 to B3 in the above-described process B.

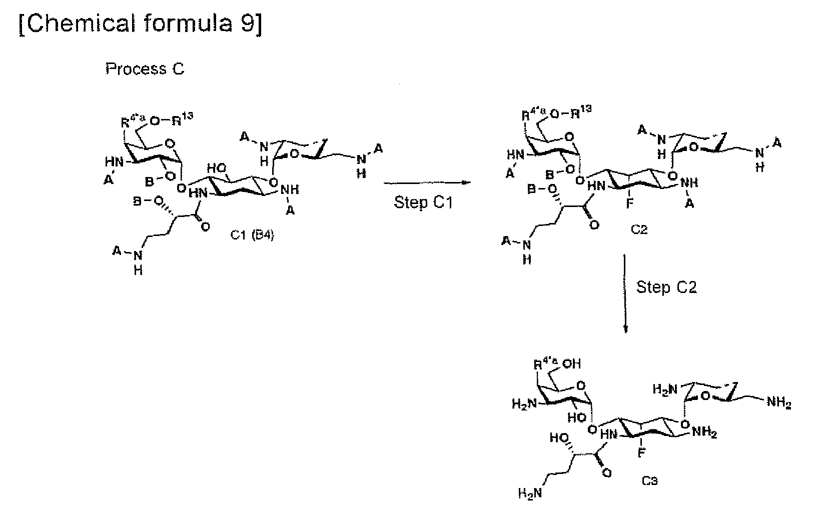

Step C1
In step C1, fluoro is introduced into the 5-position of the compound of general formula (C1) in an axial configuration. This step is achieved by reacting the compound of general formula (C1) with a fluorinating agent.

Fluorinating agents usable in this step include diethylamino sulfur trifluoride (DAST) and morpholino sulfur trifluoride. Solvents usable herein include tetrahydrofuran, dimethoxyethane, methylene chloride, and chloroform. The solvent is preferably methylene chloride. The reaction temperature is -40°C to 30°C. The reaction time is 1 to 8 hr.

Step C2
In step C2, a compound of general formula (C3) is produced by removing the protective group in the compound of general formula (C2). The protective group can be removed in the same manner as in step A8.

Process D
In process D, a compound of general formula (D2) is produced by reducing azide at the 5-position of a compound of general formula (D1) to amino. Process D comprises the following step. The compound of general formula (D1) as a starting compound may be produced according to processes A and B described above and processes E to H, J, and M to Q which will be described later.

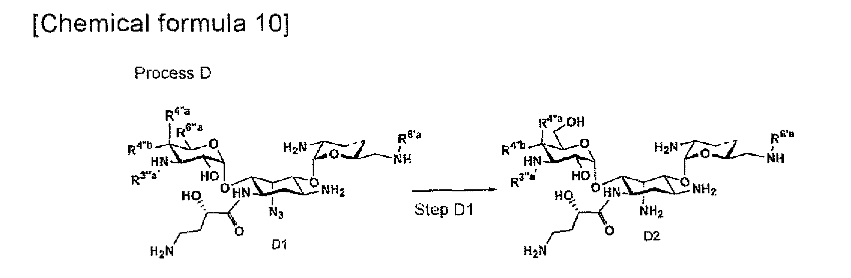

Step D1
In step D1, azide at the 5-position of a compound of general formula (D1) is reduced to amino. This step is achieved by reacting the compound of general formula (D1) with a reducing agent.

Reducing agents usable in this step include trimethylphosphine, tributylphosphine, triphenylphosphine, hydrogen and catalysts for catalytic hydrogen reduction such as palladium-carbon, palladium black, palladium hydroxide, and platinum oxide. When the dashed line in the compound of general formula (D1) represents a double bond, tributylphosphine is preferred. On the other hand, when the dashed line represents a single bond, hydrogen and palladium-carbon catalysts are suitable. Solvents usable herein include methanol, ethanol, tetrahydrofuran, dioxane, N,N-dimethylformamide, water, or mixed solvents composed of water and these organic solvents. The reaction temperature is 10°C to 30°C. The reaction time is generally 1 to 8 hr.

Process E
In process E, a compound of general formula (E5) is produced by reducing the 4"-position of a compound of general formula (E1) and then introducing substituent R^{5a} into the 5-position in an axial configuration. This process comprises the following steps. The compound of general formula (E1) as a starting compound can be produced according to steps B1 to B2 in process B.

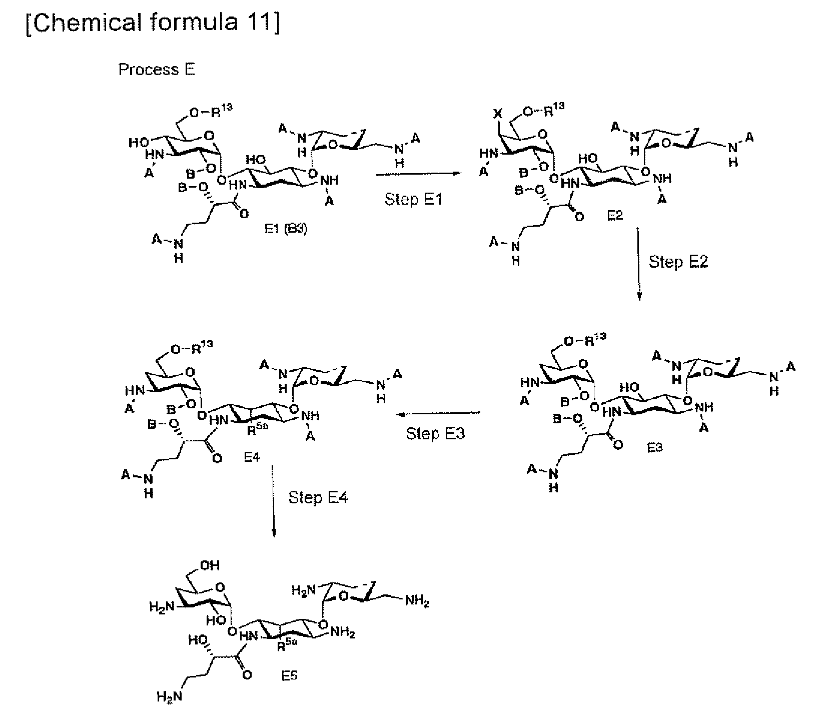

Step E1
In step E1, a compound of general formula (E2) is produced by introducing a leaving group into hydroxyl at the 4"-position of a compound of general formula (E1) and then subjecting the resultant compound to a substitution reaction. This step is achieved by reacting the compound of general formula (E1) with trifluoromethanesulfonyl chloride or trifluoromethanesulfonic anhydride in the presence of a base to synthesize a compound having trifluoromethanesulfonyloxy at the 4"-position and then reacting the resultant compound with MX wherein X represents a chlorine atom or a bromine atom, and M represents lithium or sodium.

Solvents usable in the step of introducing a leaving group include methylene chloride, chloroform, 1,2-dichloroethane, tetrahydrofuran, and ethyl acetate. The solvent is preferably methylene chloride. Bases usable herein include pyridine, lutidine, collidine, triethylamine, and diisopropylethylamine. The base is preferably pyridine. The reaction temperature is -30°C to 20°C. The reaction time is 1 to 6 hr.

Solvents usable in the step of a substitution reaction include tetrahydrofuran, dioxane, 1,2-dimetoxyethane, methylene chloride, and N,N-dimethylformamide. The solvent is preferably N,N-dimethylformamide. The reaction temperature is 0°C to 50°C. The reaction time is 1 to 12 hr.

Step E2
In step E2, a compound of general formula (E3) is produced by reducing a halogen group at the 4"-position of the compound of general formula (E2). This step is achieved by reacting the compound of general formula (E2) with a reducing agent in the presence of a free radical initiator.

Reducing agents usable in this step include, for example, tri-n-butyltin hydride, di-n-butyltin hydride, triethyltin hydride, and triphenyltin hydride. The reducing agent is preferably tri-n-butyltin hydride. Free radical initiators usable herein include azobisisobutyronitrile. Solvents usable herein include tetrahydrofuran, dioxane, benzene, and toluene. The solvent is preferably dioxane. The reaction temperature is 20°C to 120°C. The reaction time is 1 to 8 hr.

Step E3
In step E3, a compound of general formula (E4) is produced by introducing a leaving group into hydroxyl at the 5-position of the compound of general formula (E3) and then subjecting the resultant compound to a substitution reaction. This step is achieved by reacting the compound of general formula (E3) with WSO₂Cl wherein W represents methyl, phenyl, or p-tolyl in the presence of a base to synthesize a compound having substituted sulfonyloxy at the 5-position and then reacting the resultant compound with R^{5a}M wherein R^{5a} represents acetoxy, azide, a chlorine atom, a bromine atom, or C₁₋₆ alkylamino wherein one or more hydrogen atoms in the alkyl group are optionally substituted by hydroxyl, phenyl, vinyl, amino, or hydroxymethyl, and M represents lithium, sodium, cesium, or a hydrogen atom.

Solvents usable in the step of introducing a leaving group include, for example, methylene chloride, chloroform, 1,2-dichloroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Solvents usable in the step of a substitution reaction include tetrahydrofuran, dioxane, 1,2-dimethoxyethane, N,N-dimethylformamide, and dimethylsulfoxide. The solvent is preferably N,N-dimethylformamide. The reaction temperature is 60°C to 90°C. The reaction time is 1 to 12 hr.

Step E4
In step E4, a compound of general formula (E5) is produced by removing the protective group in the compound of general formula (E4). The protective group can be removed in the same manner as in step A8.

Process F
In process F, a compound of general formula (F11) is produced by introducing substituent R^{4"a} into the 4"-position of a compound of formula (F1) in an axial configuration, then introducing substituent R^{5a} into the 5-position in an axial configuration, and further introducing a side chain into amino at the 1-position. Process F comprises the following steps. The compound of formula (F1) as a starting compound can be produced according to the method described in Tetrahedron Lett., 4951-4954 (1979), and J. Med. Chem., 34, 1483-1492 (1991).

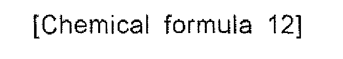

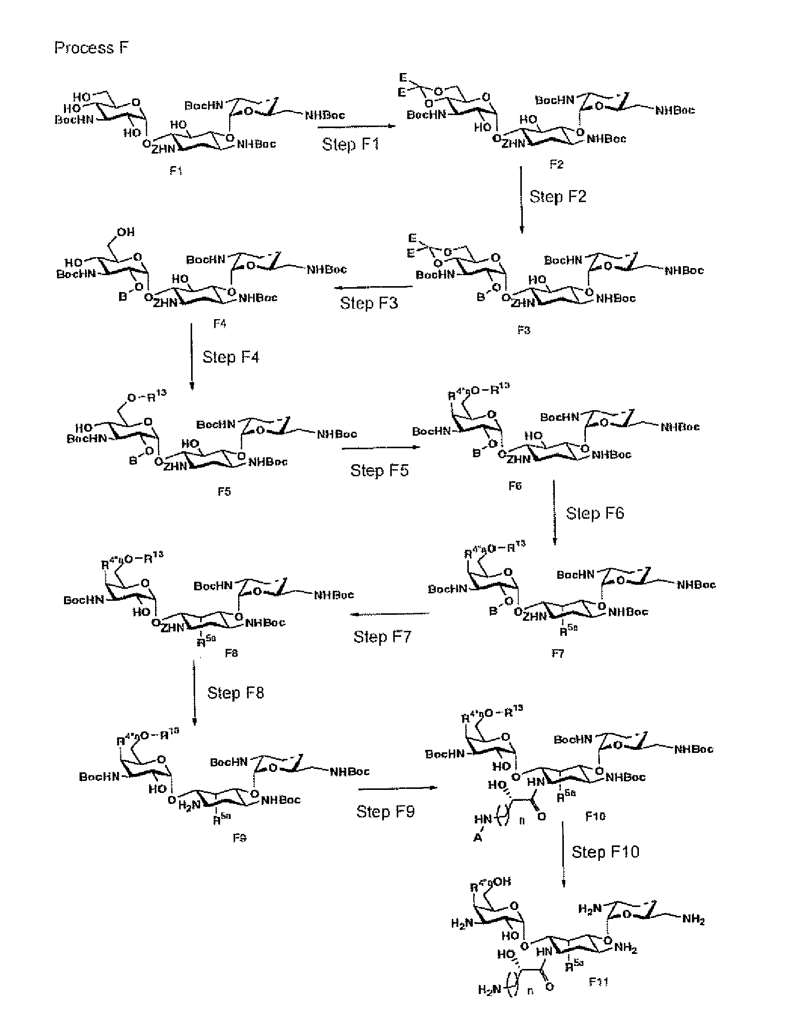

Step F1
In step F1, a compound of general formula (F2) is produced by introducing a protective group into hydroxyl at the 4"-position and 6"-position of the compound of formula (F1). This step is achieved by reacting the compound of formula (F1) with E₂CO or E₂C(OMe)₂ in the presence of an acid, wherein E represents a hydrogen atom, methyl, or phenyl, or, as E₂C, cyclohexyl.

Solvents usable in this step include, for example, N,N-dimethylformamide, methylene chloride, and ethyl acetate. The solvent is preferably N,N-dimethylformamide. Acids usable herein include p-toluenesulfonic acid, pyridinium p-toluenesulfonate, camphorsulfonic acid, and hydrochloric acid. The acid is preferably p-toluenesulfonic acid. The reaction temperature is 20°C to 50°C. The reaction time is 1 to 8 hr.

Step F2
In step F2, a compound of general formula (F3) is produced by introducing a protective group into hydroxyl at the 2"-position of the compound of general formula (F2). This step is achieved by reacting the compound of general formula (F2) with B₂O or BCI wherein B represents acetyl or benzoyl in the presence of a base.

Solvents usable in this step include pyridine, N,N-dimethylformamide, methylene chloride, and chloroform. The solvent is preferably pyridine. Bases usable herein include triethylamine, pyridine, 4-dimethylaminopyridine. The base is preferably pyridine. The reaction temperature is 0°C to 30°C. The reaction time is 1 to 8 hr.

Step F3
In step F3, the compound of general formula (F3) is converted to a compound of general formula (F4). This step is achieved by reacting the compound of general formula (F3) with an acid.

Solvents usable in this step include tetrahydrofuran, diethyl ether, dioxane, methanol, methylene chloride, chloroform, water, and mixed solvents thereof. The solvent is preferably a mixed solvent composed of methylene chloride and methanol. Acids usable herein include acetic acid, trifluoroacetic acid, hydrochloric acid, sulfuric acid, p-toluenesulfonic acid, and boron trichloride. The acid is preferably trifluoroacetic acid. The reaction temperature is 0°C to 30°C. The reaction time is 0.1 to 8 hr.

Sep F4
In step F4, a compound of general formula (F5) is produced by introducing a protective group into hydroxyl at the 6"-position of the compound of general formula (F4). This step is achieved by reacting the compound of general formula (F4) with R¹³Cl wherein R¹³ represents triphenylmethyl, tert-butyldimethylsilyl, triisopropylsilyl, or tert-butyldiphenylsilyl, in the presence of a base.

Solvents usable in the step of introducing triphenylmethyl include methylene chloride, acetonitrile, and pyridine. The solvent is preferably pyridine. Bases usable herein include triethylamine, pyridine, and 4-dimethylaminopyridine. The base is preferably pyridine. The reaction temperature is 20°C to 80°C. The reaction time is generally 2 to 10 hr.

Preferred solvents usable in the step of introducing silyl include methylene chloride, chloroform, dimethylformamide, acetonitrile, and pyridine. Bases usable herein include 4-dimethylaminopyridine, triethylamine, imidazole, and diisopropylethylamine. The base is preferably 4-dimethylaminopyridine. The reaction temperature is 0°C to 30°C. The reaction time is 1 to 12 hr.

Step F5
In step F5, a compound of general formula (F6) is produced by introducing a leaving group into hydroxyl at the 4"-position of the compound of general formula (F5) and then subjecting the resultant compound to a substitution reaction. This step is achieved by reacting the compound of general formula (F5) with trifluoromethanesulfonyl chloride or trifluoromethanesulfonic anhydride in the presence of a base to synthesize a compound having trifluoromethanesulfonyloxy at the 4"-position and then reacting the resultant compound with R^{4"a}M wherein R^{4"a} represents C₁-C₆ alkanoyloxy, or benzoyloxy, and M represents lithium, sodium, or cesium.

Solvents usable in the step of introducing a leaving group include methylene chloride, chloroform, tetrahydrofuran, and ethyl acetate. The solvent is preferably methylene chloride. Bases usable herein include pyridine, lutidine, collidine, triethylamine, and diisopropylethylamine. The base is preferably pyridine. The reaction temperature is -30°C to 20°C. The reaction time is 1 to 6 hr.

Solvents usable in the step of a substitution reaction include tetrahydrofuran, dioxane, methylene chloride, and N,N-dimethylformamide. The solvent is preferably N,N-dimethylformamide. The reaction temperature is 0°C to 50°C. The reaction time is 1 to 6 hr.

Step F6
In step F6, a compound of general formula (F7) is produced by introducing a leaving group into hydroxyl at the 5-position of the compound of general formula (F6) and then subjecting the resultant compound to a substitution reaction. This step is achieved by reacting the compound of general formula (F6) with WSO₂Cl wherein W represents methyl, phenyl, or p-tolyl, in the presence of a base to synthesize a compound having substituted sulfonyloxy at the 5-position and then reacting, in the presence of a base, the resultant compound with R^{5a}M wherein R^{5a} represents acetoxy, azide, a chlorine atom, a bromine atom, or C₁₋₆ alkylamino wherein one or more hydrogen atoms in the alkyl group are optionally substituted by hydroxyl, phenyl, vinyl, amino, or hydroxymethyl, and M represents lithium, sodium, cesium, or a hydrogen atom.

Solvents usable in the step of introducing a leaving group include, for example, methylene chloride, chloroform, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Solvents usable in the step of a substitution reaction include tetrahydrofuran, dioxane, N,N-dimethylformamide, and dimethylsulfoxide. The solvent is preferably N,N-dimethylfiormamide. The reaction temperature is 60°C to 90°C. The reaction time is 1 to 12 hr.

Step F7
In step F7, a compound of general formula (F8) is produced by removing the protective group of hydroxyl except for the protective group at the 6"-position of the compound of general formula (F7), This step is achieved by reacting the compound of general formula (F7) with a base.

Solvents usable in this step include methanol, ethanol, isopropyl alcohol, t-butyl alcohol, methylene chloride, chloroform, and mixed solvents thereof. The solvent is preferably a mixed solvent composed of methanol and methylene chloride. Bases usable herein include potassium carbonate, sodium carbonate, potassium hydroxide, sodium hydroxide, sodium methoxide, sodium ethoxide, and tert-BuOK. The base is preferably sodium methoxide. The reaction temperature is 0°C to 60°C, The reaction time is 1 to 8 hr.

Step F8
In step F8, a compound of general formula (F9) is produced by removing the protective group in the compound of general formula (F8). This step is achieved by reacting the compound of general formula (F8) with a reducing agent.

Reducing agents usable in this step include catalysts for catalytic hydrogen reduction used together with hydrogen, for example, palladium-carbon, palladium black, palladium hydroxide, and platinum oxide, metallic sodium and metallic lithium. When the dashed line in the compound of general formula (F8) represents a double bond, metallic sodium is preferred. On the other hand, when the dashed line represents a single bond, hydrogen and palladium-carbon catalysts are preferred. Solvents usable herein include, in the case of catalytic hydrogen reduction, methanol, ethanol, tetrahydrofuran, dioxane, N,N-dimethylformamide, water, or mixed solvents composed of water and these organic solvents. When metallic sodium is used, liquid ammonia is preferred. The reaction temperature is -60°C to 30°C. The reaction time is generally 1 to 8 hr.

Step F9
In step F9, a compound of general formula (F10), wherein A represents tert-butoxycarbonyl, benzyloxycarbonyl, p-methoxybenzyloxycarbonyl, or p-nitrobenzyloxycarbonyl, is produced by introducing a side chain into amino at the 1-position of the compound of general formula (F9). This step is achieved by condensing, in the presence of a condensing agent, the compound of general formula (F9) with a carbocylic acid ANH(CH₂)ₙCH(OH)COOH, wherein A is as defined above and n represents an integer of 1 to 3, or by reacting the compound of general formula (F9) with a derivative of a carboxylic acid ANH(CH₂)ₙCH(OH)COOH, wherein A is as defined above, in the absence of a condensing agent.

Condensing agents usable in this step include, for example, carbodiimides such as dicyclohexylcarbodiimide, diisopropylcarbodiimide, and N-ethyl-N'-3-dimethylaminopropylcarbodiimide, or these condensing agents to which, for example, 1-oxobenzotriazole or 3-hydroxy-4-oxo-3,4-dihydro-1,2,3-benzotriazine has been added as an additive. Carboxylic acid derivatives usable herein include N-hydroxyphthalimide esters, N-hydroxysuccinimide esters, p-nitrophenyl esters, and pentafluorophenyl esters. The carboxylic acid derivative is preferably an N-hydroxysuccinimide ester. Solvents usable herein include tetrahydrofuran, dioxane, methylene chloride, chloroform, and N,N-dimethylformamide. The solvent is preferably N,N-dimethylformamide. The reaction temperature is 0°C to 30°C. The reaction time is 1 to 12 hr.

Step F10
In step F10, a compound of general formula (F11) is produced by removing the protective group in the compound of general formula (F10). The protective group can be removed in the same manner as in step A8.

Process G
In process G, a compound of general formula (G6) is produced by introducing substituent R^{6'a} into amino at the 6'-position of the compound of formula (G1). Process G comprises the following steps. A compound of general formula (G7) can be produced from the compound of general formula (G6) according to steps A3 to A8 in process A described above or steps B1 to B6 in process B described above, or process J which will be described later.

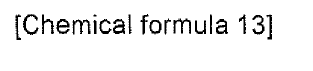

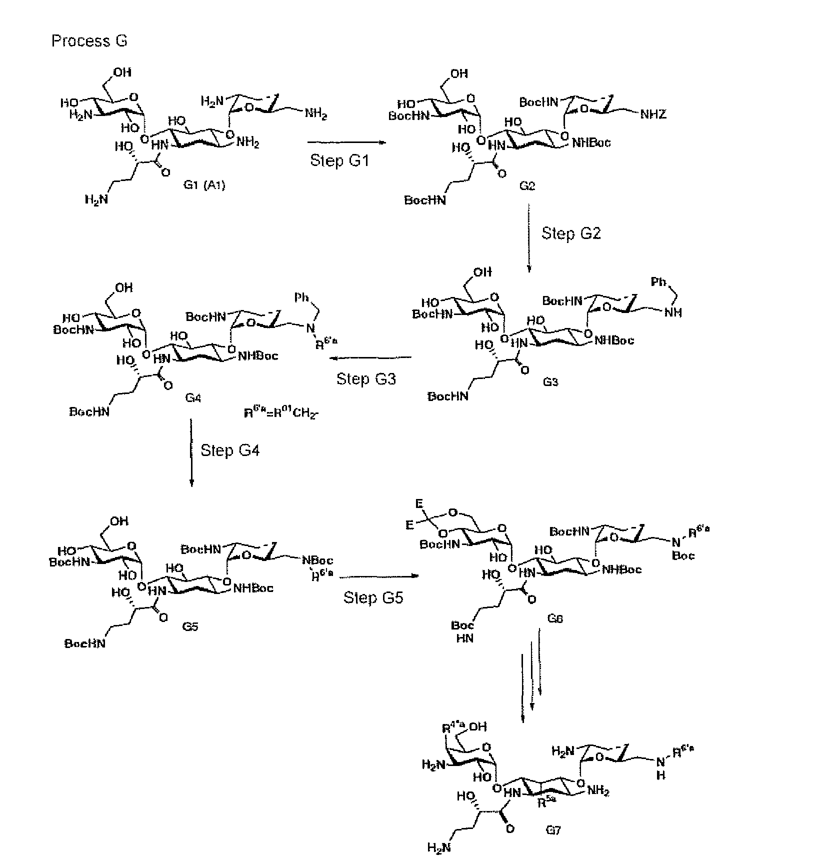

### Step G1

In step G1, a compound of formula (G2) is produced by protecting amino in the compound of formula (G1). This step is achieved by first reacting the compound of formula (G1) with N-benzyloxycarbonyloxysuccinimide in the presence of zinc acetate to give the compound in which the amino group at the 6'-position has been protected by benzyloxycarbonyl (group Z), and then reacting the amino groups in the 3-position, 2'-position, 3"-position, 4'''-position of the resultant compound with di-tert-butyl dicarbonate in the presence of a base to protect the amino groups with tert-butoxycarbonyl (group Boc).

Solvents usable in the step of protecting the 6'-position include tetrahydrofuran, dioxane, water, N,N-dimethylformamide, or mixed solvents thereof. The solvent is preferably a mixed solvent composed of water and N,N-dimethylformamide. The reaction temperature is 0°C to 30°C. The reaction time is 1 to 8 hr.

Solvents usable in the step of protecting the 3-position, 2'-position, 3"-position, and 4"'-position include water, N,N-dimethylformamide, tetrahydrofuran, dioxane, and mixed solvents thereof. The solvent is preferably a mixed solvent composed of water and dioxane. Bases usable herein include sodium hydroxide, potassium carbonate, sodium carbonate, triethylamine, pyridine, and 4-dimethylaminopyridine. The base is preferably triethylamine. The reaction temperature is 0°C to 40°C. The reaction time is 1 to 24 hr.

### Step G2

In step G2, a compound of formula (G3) is produced by removing the protective group in the compound of formula (G2) and then protecting the compound with benzyl, This step is achieved by subjecting the compound of formula (G2) to catalytic hydrogen reduction with hydrogen and then reacting the resultant compound having amino at the 6'-position with benzaldehyde in the presence of a reducing agent.

Reducing agents usable in the step of deprotection include hydrogen and catalysts for catalytic hydrogen reduction, such as palladium-carbon, palladium black, palladium hydroxide, and platinum oxide, metallic sodium and metallic lithium. When the dashed line in the compound represented by general formula (G2) represents a double bond, metallic sodium is preferable. On the other hand, when the dashed line represents a single bond, hydrogen and palladium-carbon catalysts are preferable. Solvents usable herein include, in the case of catalytic hydrogen reduction, methanol, ethanol, tetrahydrofuran, dioxane, N,N-dimethylformamide, water, or mixed solvents composed of water and these organic solvents. When metallic sodium is used, liquid ammonia is preferable. The reaction temperature is -60°C to 30°C. The reaction time is generally 1 to 8 hr.

Reducing agents usable in the step of benzylation include sodium borohydride, sodium cyanoborohydride, and lithium cyanoborohydride. The reducing agent is preferably sodium borohydride. Solvents usable herein include methanol, ethanol, isopropyl alcohol, dioxane, water, or mixed solvents thereof. The solvent is preferably a mixed solvent composed of methanol and dioxane. The reaction temperature is 0°C to 30°C. The reaction time is 1 to 8 hr. This step may also be achieved by a reaction with benzyl bromide in the presence of a base.

### Step G3

In step G3, a compound of general formula (G4) is produced by introducing a substituent into amino at the 6'-position of the compound of formula (G3). This step is achieved by reacting the compound of formula (G3) with R⁰¹CHO wherein R⁰¹ represents a hydrogen atom or C₁₋₅ alkyl in the presence of a reducing agent.

Reducing agents usable in this step include sodium borohydride, sodium cyanoborohydride, and lithium cyanoborohydride. The reducing agent is preferably sodium borohydride. Solvents usable herein include methanol, ethanol, isopropyl alcohol, dioxane, water, or mixed solvents thereof. The solvent is preferably a mixed solvent composed of methanol and dioxane. The reaction temperature is 0°C to 30°C. The reaction time is to 8 hr.

### Step G4

In step G4, the benzyl group at the 6'-position of the compound of general formula (G4) is converted to tert-butoxycarbonyl. This step is achieved by reacting the compound of general formula (G4) with di-tert-butyl dicarbonate and a reducing agent.

Solvents usable in this step include, for example, methanol, ethanol, tetrahydrofuran, dioxane, tetrahydrofuran, or mixed solvents composed of these organic solvents and water. The solvent is preferably a mixed solvent composed of water and tetrahydrofuran. Reducing agents usable herein include hydrogen and catalysts for catalytic hydrogen reduction such as palladium-carbon, palladium black, palladium hydroxide, and platinum oxide. The reducing agent is preferably, hydrogen and palladium-carbon. The reaction temperature is 10°C to 30°C. The reaction time is generally 1 to 8 hr.

### Step G5

In step G5, a compound of general formula (G6) is produced by introducing a protective group into hydroxyl at the 4"-position and 6"-position of the compound of general formula (G5). This step is achieved by reacting the compound of general formula (G5) with E₂CO or E₂C(OMe)₂ wherein E represents a hydrogen atom, methyl, or phenyl or, as E₂C, cyclohexyl in the presence of an acid.

Solvents usable in this step include, for example, N,N-dimethylformamide, methylene chloride, and ethyl acetate. The solvent is preferably N,N-dimethylformamide. Acids usable herein include p-toluenesulfonic acid, pyridinium p-toluenesulfonate, camphorsulfonic acid, and hydrochloric acid. The acid is preferably p-toluenesulfonic acid. The reaction temperature is 20°C to 50°C. The reaction time is 1 to 8 hr.

Process H
In process H, a compound of general formula (G6) is produced by introducing substituents R^{5a} and R^{4"a} into the 5-position and 4"-position of the compound of formula (H1) in an axial configuration and then introducing substituent R^{3"a} into amino at the 3"-position. Process H comprises the following steps. The compound of formula (H1) as a starting compound can be produced by the method described in Japanese Patent Laid-Open No. 1319/1988, Japanese Patent Laid-Open No. 82290/1995, and U.S Patent No. 4297485.

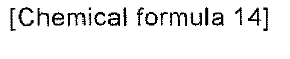

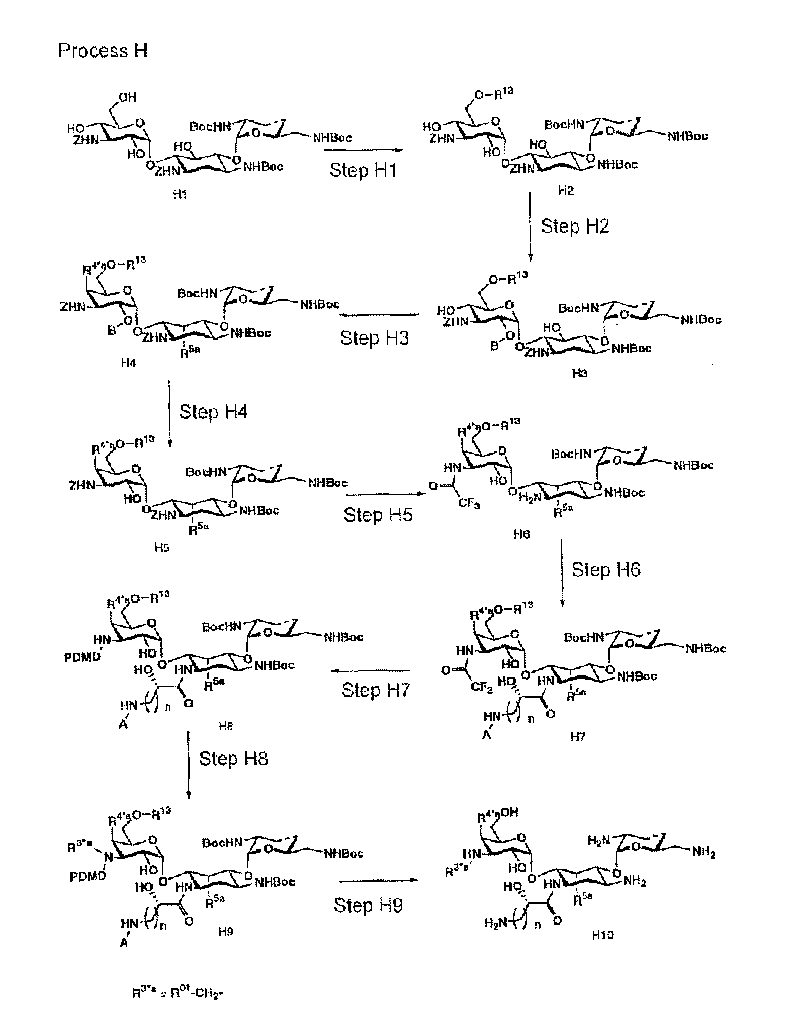

Step H1
In step H1, a compound of formula (H2) is produced by selectively protecting hydroxyl at the 6"-position of the compound of formula (H1). This step is achieved by reacting the compound of formula (H1) with R¹³Cl wherein R¹³ represents triphenylmethyl, tert-butyldimethylsilyl, triisopropylsilyl, or tert-butyldiphenylsilyl in the presence of a base.

Solvents usable in the step of introducing triphenylmethyl include methylene chloride, acetonitrile, and pyridine. The solvent is preferably pyridine. Bases usable herein include triethylamine, pyridine, and 4-dimethylaminopyridine. The base is preferably pyridine. The reaction temperature is 20°C to 80°C. The reaction time is generally 2 to 10 hr.

Preferred solvents usable in the step of introducing silyl include methylene chloride, chloroform, dimethylformamide, acetonitrile, and pyridine. Bases usable herein include 4-dimethylaminopyridine, triethylamine, imidazole, and diisopropylethylamine. The base is preferably 4-dimethylaminopyridine. The reaction temperature is 0°C to 30°C. The reaction time is 1 to 12 hr.

Step H2
In step H2, a compound of general formula (H3) is produced by introducing a protective group into hydroxyl at the 2"-position of the compound of formula (H2). This step is achieved by reacting the compound of formula (H2) with B₂O or BCI wherein B represents acetyl or benzoyl in the presence of a base.

Solvents usable in this step include pyridine, N,N-dimethylformamide, methylene chloride, and chloroform. The solvent is preferably pyridine. Bases usable herein include triethylamine, pyridine, and 4-dimethylaminopyridine, The base is preferably pyridine. The reaction temperature is 0°C to 30°C. The reaction time is 1 to 8 hr.

Step H3
In step H3, a compound of general formula (H4) is produced by introducing a leaving group into hydroxyl at the 5-position and 4"-position of the compound of general formula (H3) and then subjecting the resultant compound to a substitution reaction. This step is achieved by reacting the compound of general formula (H3) with WSO₂Cl wherein W represents methyl, phenyl, or p-tolyl in the presence of a base to synthesize a compound having substituted sulfonyloxy at the 5-position and 4"-position and then reacting the resultant compound with R^{4"a}M wherein R^{4"a} represents C₁-C₆ alkanoyloxy or benzoyloxy, and M represents lithium, sodium, or cesium. Substituent R^{5a} at the 5-position of the compound of general formula (H4) is as defined in R^{4"a}.

Solvents usable in the step of introducing a leaving group include, for example, methylene chloride, chloroform, 1,2-dichloroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Solvents usable in the step of a substitution reaction include tetrahydrofuran, 1,2-dimethoxyethane, dioxane, N,N-dimethylformamide, and dimethylsulfoxide. The solvent is preferably N,N-dimethylformamide. The reaction temperature is 60°C to 90°C. The reaction time is 1 to 12 hr.

Step H4
In step H4, a compound of general formula (H5) is produced by removing the protective group of hydroxyl except for the protective group at the 6"-position of the compound of general formula (H4). This step is achieved by reacting the compound of general formula (H4) with a base.

Solvents usable in this step include methanol, ethanol, isopropyl alcohol, tert-butyl alcohol, methylene chloride, chloroform, and mixed solvents thereof. The solvent is preferably a mixed solvent composed of methanol and methylene chloride. Bases usable herein include potassium carbonate, sodium carbonate, potassium hydroxide, sodium hydroxide, sodium methoxide, sodium ethoxide, and tert-BuOK. The base is preferably sodium methoxide. The reaction temperature is 0°C to 60°C. The reaction time is 1 to 8 hr.

Step H5
In step H5, a compound of general formula (H6) is produced by removing the protective group of amino at the 1-position and 3"-position of the compound of general formula (H5) and then selectively protecting amino at the 3"-position. This step is achieved by reacting the compound of general formula (H5) with a reducing agent and then reacting the resultant compound having amino at the 1- and 3"-positions with trifluoroethyl acetate in the presence of a base.

Reducing agents usable in the step of deprotection include catalysts for catalytic hydrogen reduction used together with hydrogen, for example, palladium-carbon, palladium black, palladium hydroxide, and platinum oxide, or metallic sodium and metallic lithium. When the dashed line in the compound of general formula (H5) represents a double bond, metallic sodium is preferred. On the other hand, when the dashed line represents a single bond, hydrogen and a palladium-carbon catalyst are preferred. Solvents usable herein include, in the case of catalytic hydrogen reduction, methanol, ethanol, tetrahydrofuran, dioxane, N,N-dimethylformamide, water, or mixed solvents composed of water and these organic solvents, When metallic sodium is used, liquid ammonia is preferred. The reaction temperature is -60°C to 30°C. The reaction time is generally 1 to 8 hr.

Solvents usable in the step of proteciton include tetrahydrofuran, dioxane, methylene chloride, chloroform, and N,N-dimethylformamide. The solvent is preferably N,N-dimethylformamide. Bases usable herein include triethylamine, diisopropylethylamine, pyridine, and 4-dimethylaminopyridine. The base is preferably pyridine. The reaction temperature is 0°C to 30°C. The reaction time is 1 to 8 hr.

Step H6
In step H6, a compound of general formula (H7), wherein A represents tert-butoxycarbonyl, benzyloxycarbonyl, p-methoxybenzyloxycarbonyl, or p-nitrobenzyloxycarbonyl, is produced by introducing a side chain into amino at the 1-position of the compound of general formula (H6). This step is achieved by reacting the compound of general formula (H6) with a carboxylic acid ANH(CH₂)ₙCH(OH)COOH, wherein A is as defined above and n represents an integer of 1 to 3, in the presence of a condensing agent, or by reacting the compound of general formula (H6) with a derivative of a carboxylic acid ANH(CH₂)nCH(OH)COOH, wherein A is as defined above, in the absence of a condensing agent.

Condensing agents usable in this step include, for example, carbodiimides such as dicyclohexylcarbodiimide, diisopropylcarbodiimide and N-ethyl-N'-3-dimethylaminopropylcarbodiimide, or these condensing agents to which, for example, 1-oxobenzotriazole or 3-hydroxy-4-oxo-3,4-dihydro-1,2,3-benzotriazine has been added as an additive. Carboxylic acid derivatives usable herein include N-hydroxyphthalimide esters, N-hydroxysuccinimide esters, p-nitrophenyl esters, and pentafluorophenyl esters. The carboxylic acid derivative is preferably an N-hydroxysuccinimide ester. Solvents usable herein include tetrahydrofuran, dioxane, methylene chloride, chloroform, and N,N-dimethylformamide. The solvent is preferably N,N-dimethylformamide. The reaction temperature is 0°C to 30°C. The reaction time is 1 to 12 hr.

Step H7
In step H7, a compound of general formula (H8) is produced by converting the protective group at the 3"-position of the compound of general formula (H7) from trifluoroacetyl to di(4-methoxyphenyl)methyl (group PDMD). This step is achieved by reacting the compound of general formula (H7) with a base and then reacting the resultant compound having amino at the 3"-position with di(4-methoxyphenyl)methyl chloride in the presence of a base.

Bases usable in the step of deprotection include potassium carbonate, sodium carbonate, barium hydroxide, and ammonium hydroxide. Among them, ammonium hydroxide is preferred. Solvents usable herein include methanol, ethanol, isopropyl alcohol, tetrahydrofuran, dioxane, methylene chloride, chloroform, water, or mixed solvents thereof. The solvent is preferably a mixed solvent composed of tetrahydrofuran and ethanol. The reaction temperature is 0°C to 50°C. The reaction time is 1 to 8 hr.

Bases usable in the step of protection include triethylamine, diisopropylethylamine, pyridine, and 4-dimethylaminopyridine. The base is preferably triethylamine. The reaction temperature is 0°C to 30°C. The reaction time is 1 to 24 hr.

Step H8
In Step H8, a compound of general formula (H9) is produced by introducing a substituent into amino at the 3"-position of the compound of general formula (H8). This step is achieved by reacting the compound of general formula (H8) with R⁰¹CHO wherein R⁰¹ represents a hydrogen atom or C₁₋₅ alkyl in the presence of a reducing agent.

Reducing agents usable in this step include sodium borohydride, sodium cyanoborohydride, and lithium cyanoborohydride. The reducing agent is preferably sodium borohydride. Solvents usable herein include methanol, ethanol, isopropyl alcohol, dioxane, water, or mixed solvents thereof. The solvent is preferably a mixed solvent composed of methanol and dioxane. The reaction temperature is 0°C to 30°C. The reaction time is 1 to 8 hr.

Step H9
In step H9, a compound of general formula (H10) is produced by removing the protective group in the compound of general formula (H9). The protective group can be removed in the same manner as in step A8.

Process I
In process I, a compound of general formula (18) is produced by introducing a side chain into the 1-position of the compound of formula (I1) and then introducing fluoro into the 5-position and 6"-position. Process I comprises the following steps. Each step in process I will be described in detail.

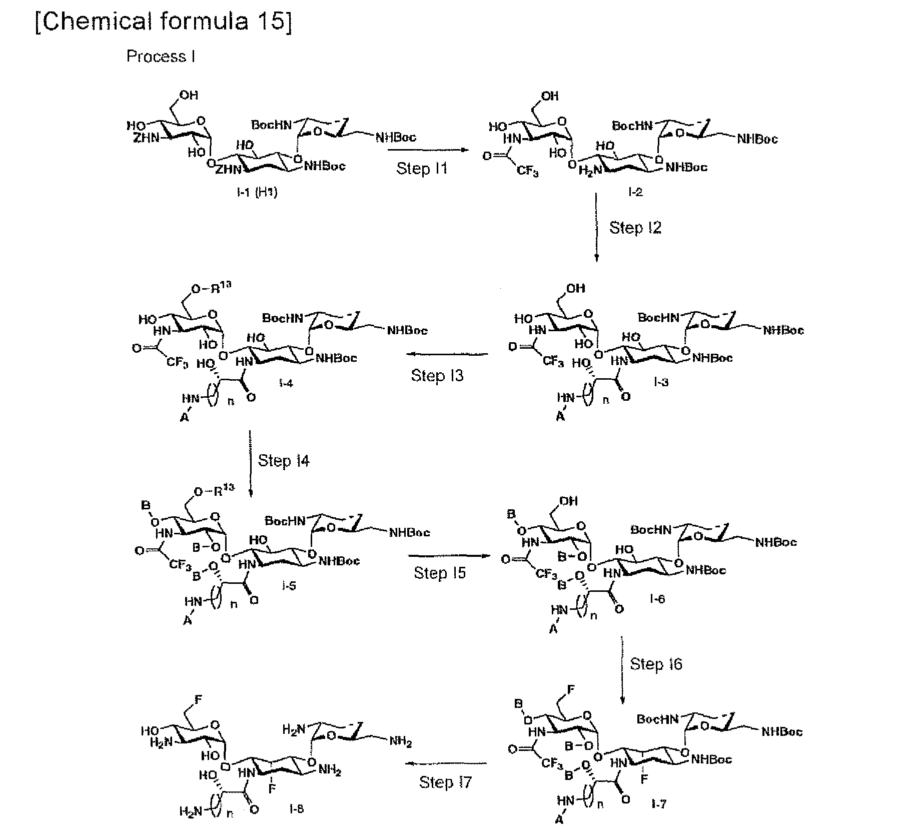

Step I1
In step I1, a compound of formula (I2) is produced by removing the protective group of amino at the 1-position and 3"-position of the compound of formula (I1) and then selectively protecting amino at the 3"-position. This step is achieved by reacting the compound of formula (I1) with a reducing agent and then reacting the resultant compound having amino at the 1,3"-position with trifluoroethyl acetate in the presence of a base.

Reducing agents usable in the step of deprotection include catalysts for catalytic hydrogen reduction used together with hydrogen, for example, palladium-carbon, palladium black, palladium hydroxide, and platinum oxide, or metallic sodium and metallic lithium. When the dashed line in the compound of general formula (I1) represents a double bond, metallic sodium is preferred. On the other hand, when the dashed line represents a single bond, hydrogen and palladium-carbon catalysts are preferred. Solvents usable herein include, in the case of catalytic hydrogen reduction, methanol, ethanol, tetrahydrofuran, dioxane, N,N-dimethylformamide, water, or mixed solvents composed of water and these organic solvents. When metallic sodium is used, liquid ammonia is preferred. The reaction temperature is -60°C to 30°C. The reaction time is generally 1 to 8 hr.

Solvents usable in the step of protection include tetrahydrofuran, dioxane, methylene chloride, chloroform, and N,N-dimethylformamide. The solvent is preferably N,N-dimethylformamide. Bases usable herein include triethylamine, diisopropylethylamine, pyridine, and 4-dimethylaminopyridine. The base is preferably pyridine. The reaction temperature is 0°C to 30°C. The reaction time is 1 to 8 hr.

Step 12
In step 12, a compound of general formula (I3) wherein A represents tert-butoxycarbonyl, benzyloxycarbonyl, p-methoxybenzyloxycarbonyl, or p-nitrobenzyloxycarbonyl, is produced by introducing a side chain into amino at the 1-position of the compound of formula (I2). This step is achieved by reacting the compound of formula (I2) with a carboxylic acid ANH(CH₂)ₙCH(OH)COOH wherein A is as defined above in the presence of a condensing agent, or by reacting the compound of formula (12) with a derivative of a carboxylic acid ANH(CH₂)nCH(OH)COOH wherein A is as defined above and n represents an integer of 1 to 3 in the absence of a condensing agent.

Condensing agents usable in this step include, for example, carbodiimides such as dicyclohexylcarbodiimide, diisopropylcarbodiimide and N-ethyl-N'-3-dimethylaminopropylcarbodiimide, or these condensing agents to which, for example, 1-oxobenzotriazole or 3-hydroxy-4-oxo-3,4-dihydro-1,2,3-benzotriazine has been added as an additive. Carboxylic acid derivatives usable herein include N-hydroxyphthalimide esters, N-hydroxysuccinimide esters, p-nitrophenyl esters, and pentafluorophenyl esters. The carboxylic acid derivative is preferably an N-hydroxysuccinimide ester. Solvents usable herein include tetrahydrofuran, dioxane, methylene chloride, chloroform, and N,N-dimethylformamide. The solvent is preferably N,N-dimethylformamide. The reaction temperature is 0°C to 30°C. The reaction time is 1 to 12 hr.

Step 13
In step I3, a compound of general formula (I4) is produced by introducing a protective group into hydroxyl at the 6"-position of the compound of general formula (13). This step is achieved by reacting the compound of general formula (I3) with R¹³Cl wherein R¹³ represents triphenylmethyl, tert-butyldimethylsilyl, triisopropylsilyl, or tert-butyldiphenylsilyl in the presence of a base.

Solvents usable in the step of introducing triphenylmethyl include methylene chloride, acetonitrile, and pyridine. The solvent is preferably pyridine. Bases usable herein include triethylamine, pyridine, and 4-dimethylaminopyridine. The base is preferably pyridine. The reaction temperature is 20°C to 80°C. The reaction time is generally 2 to 10 hr.

Preferred solvents usable in the step of introducing silyl include methylene chloride, chloroform, dimethylformamide, acetonitrile, and pyridine. Bases usable herein include 4-dimethylaminopyridine, triethylamine, imidazole, and diisopropylethylamine. The base is preferably 4-dimethylaminopyridine. The reaction temperature is 0°C to 30°C. The reaction time is 1 to 12 hr.

Step 14
In step 14, a compound of general formula (15) is produced by introducing a protective group into hydroxyl at the 2"-position, 4"-position, and 2'''-position of the compound of general formula (14). This step is achieved by reacting the compound of general formula (14) with B₂O or BCI wherein B represents acetyl or benzoyl in the presence of a base.

Solvents usable in this step include pyridine, N,N-dimethylformamide, methylene chloride, and chloroform. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step 15
In step 15, a compound of general formula (16) is produced by removing triphenylmethyl or silyl as the protective group of hydroxyl at the 6"-position of the compound of general formula (15). This step is achieved by reacting the compound of general formula (15) with an acid or a base.

Solvents usable in the step of deprotection of triphenylmethyl group include diethyl ether, tetrahydrofuran, dimethoxyethane, and water. The solvent is preferably diethyl ether. Acids usable herein include formic acid and acetic acid. The acid is preferably formic acid. The reaction temperature is 0°C to 30°C. The reaction time is 1 to 8 hr.

Preferred solvents usable in the step of deprotection of silyl group include acetonitrile, tetrahydrofuran, and methylene chloride. Reagents usable in the deprotection include tetrabutylammonium fluoride, hydrogen fluoride-pyridine, hydrogen fluoride-triethylamine, and hydrogen fluoride. The reaction temperature is 0°C to 30°C. The reaction time is 1 to 12 hr.

Step 16
In step 16, a compound of general formula (17) is produced by fluorinating the 5-position and 6"-position of the compound of general formula (16). This step is achieved by reacting the compound of general formula (I6) with a fluorinating reagent.

Fluorinating reagents usable in this step include diethylaminosulfur trifluoride (DAST) and marpholinasulfur trifluoride. Solvents usable herein include tetrahydrofuran, dimethoxyethane, methylene chloride, and chloroform. The solvent is preferably methylene chloride. The reaction temperature is -40°C to 30°C. The reaction time is 1 to 8 hr.

Step 17
In step 17, a compound of general formula (18) is produced by removing the protective group in the compound of general formula (17). The protective group can be removed in the same manner as in step A8.

Process J
In process J, a compound of general formula (J4) is produced by introducing substituent R^{5a} into the 5-position of the compound of formula (J1) in an axial configuration. Process J comprises the following steps. Each step in process J will be described.

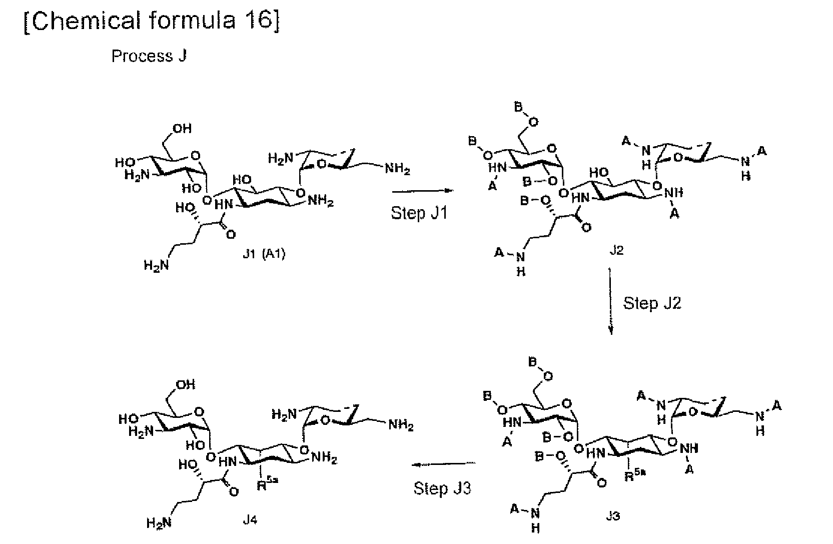

Step J1
In step J1, a protective group is introduced into all hydroxyl and amino except for the 5-position of the compound of formula (J1). This step is achieved by first reacting a compound of formula (J1) with A₂O or ACl wherein A represents tert-butoxycarbonyl, benzyloxycarbonyl, p-methoxybenzyloxycarbonyl, or p-nitrobenzyloxycarbonyl in the presence of a base to introduce a protective group into amino and then reacting the resultant compound with B₂O or BCl wherein B represents acetyl or benzoyl in the presence of a base to introduce a protective group into hydroxyl.

Solvents usable in the step of protecting amino include water, N,N-dimethylformamide, tetrahydrofuran, dioxane, and mixed solvents thereof. The solvent is preferably a mixed solvent composed of water and N,N-dimethylformamide. Bases usable herein include sodium hydroxide, potassium carbonate, sodium carbonate, triethylamine, pyridine, and 4-dimethylaminopyridine. The base is preferably triethylamine. The reaction temperature is 0°C to 40°C. The reaction time is 1 to 24 hr.

Solvents usable in the step of protecting hydroxyl include pyridine, N,N-dimethylformamide, methylene chloride, and chloroform. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step J2
In step J2, a compound of general formula (J3) is produced by introducing a leaving group into hydroxyl at the 5-position of the compound of general formula (J2) and then subjecting the resultant compound to a substitution reaction. This step is achieved by reacting the compound of general formula (J2) with WSO₂Cl wherein W represents methyl, phenyl, or p-tolyl in the presence of a base to synthesize a compound having substituted sulfonyloxy at the 5-position and then reacting the resultant compound with R^{5a}M wherein R^{5a} represents acetoxy, azide, a chlorine atom, a bromine atom, or C₁₋₆ alkylamino wherein one or more hydrogen atoms in the alkyl group are optionally substituted by hydroxyl, phenyl, vinyl, amino, or hydroxymethyl, and M represens lithium, sodium, cesium, or a hydrogen atom.

Solvents usable in the step of introducing a leaving group include, for example, methylene chloride, chloroform, 1,2-dichloroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Solvents usable in the step of a substitution reaction include tetrahydrofuran, 1,2-dimetnoxyethane, dioxane, N,N-dimethylformamide, and dimethylsulfoxide. The solvent is preferably N,N-dimethylformamide. The reaction temperature is 60°C to 90°C. The reaction time is 1 to 12 hr.

Step J3
In step J3, a compound of general formula (J4) is produced by removing the protective group in the compound of general formula (J3). The protective group can be removed in the same manner as in step A8.

Process K
In process K, a compound of general formula (K4) is produced by reducing azide in an axial configuration at the 5-position of the compound of general formula (K1) to amino and introducing a substituent into the amino group. Process K comprises the following steps. The compound of general formula (K1) as a starting compound can be produced according to steps J1 and J2 in the above process J.

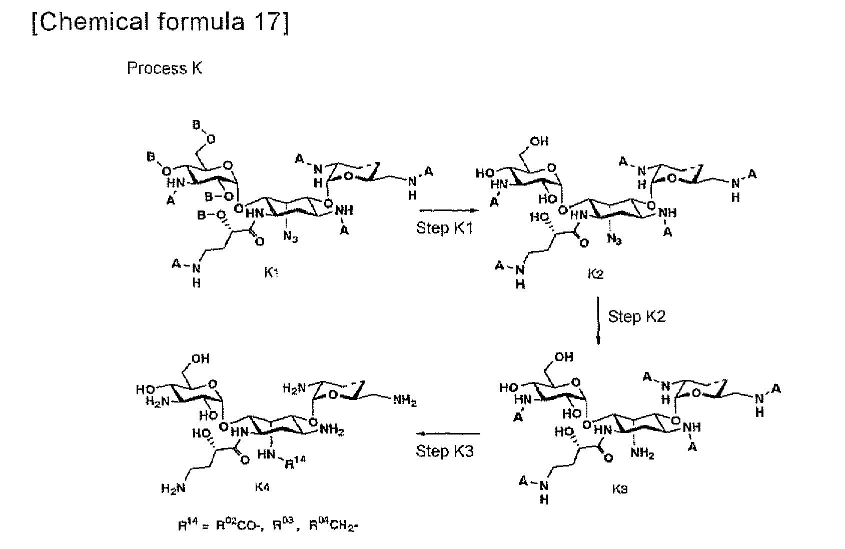

Step K1
In step K1, a compound of general formula (K2) is produced by removing the protective group of hydroxyl in the compound of general formula (K1). This step is achieved by reacting the compound of general formula (K1) with a base.

Solvents usable in this step include methanol, ethanol, isopropyl alcohol, tert-butyl alcohol, methylene chloride, chloroform, and mixed solvents thereof. The solvent is preferably a mixed solvent composed of methanol and methylene chloride. Bases usable herein include potassium carbonate, sodium carbonate, potassium hydroxide, sodium hydroxide, sodium methoxide, sodium ethoxide, and tert-BuOK. The base is preferably sodium methoxide. The reaction temperature is 0°C to 60°C. The reaction time is 1 to 8 hr.

Step K2
In step K2, azide at the 5-position of the compound of general formula (K2) is reduced to amino. This step is achieved by reacting the compound of general formula (K2) with a reducing agent.

Reducing agents usable in this step include trimethylphosphine, tributylphosphine, triphenylphosphine, and catalysts for catalytic hydrogen reduction used together with hydrogen, for example, palladium-carbon, palladium black, palladium hydroxide, and platinum oxide. When the dashed line in the compound of general formula (K2) represents a double bond, tributylphosphine is preferred. When the dashed line represents a single bond, hydrogen and palladium-carbon catalysts are preferred. Solvents usable herein include methanol, ethanol, tetrahydrofuran, dioxane, N,N-dimethylformamide, water, or mixed solvents composed of water and these organic solvents. The reaction temperature is 10°C to 30°C. The reaction time is generally 1 to 8 hr.

Step K3
In step K3, a compound of general formula (K4) is produced by introducing a substituent into amino at the 5-position of the compound of general formula (K3) and then removing the protective group. This step is achieved by either reacting, in the presence of a base, the compound of general formula (K3) with R⁰²COCl wherein R⁰² represents C₁₋₅ alkyl or aryl, or R⁰³X wherein R⁰³ represents C₁₋₆ alkyl or aralkyl, and X represents a halogen, or reacting the compound of general formula (K3) with R⁰⁴CHO wherein R represents a hydrogen atom, C₁₋₅ alkyl, or aryl, and then reacting the resultant compound with a base or an acid in the presence of a reducing agent, and then removing the protective group.

Solvents usable in the step of the reaction with R⁰²COCl include methylene chloride, chloroform, 1,2-dichloroethane, and pyridine. The solvent is preferably pyridine. Bases usable herein include triethylamine, diisopropylethylamine, pyridine, and 4-dimethylaminopyridine. The base is preferably pyridine. The reaction temperature is 0°C to 30°C. The reaction time is 1 to 8 hr.

Solvents usable in the step of the reaction with R⁰³X include tetrahydrofuran, 1,2-dimethoxyethane, dioxane, N,N-dimethylformamide, methylene chloride, chloroform, 1,2-dichloroethane, methanol, ethanol, acetonitrile, and water. The solvent is preferably N,N-dimethylformamide. Bases usable herein include potassium carbonate, sodium carbonate, triethylamine, and 4-dimethylaminopyridine. The base is preferably potassium carbonate. The reaction temperature is 20°C to 60°C. The reaction time is 1 to 12 hr.

Solvents usable in the step of the reaction with R⁰⁴CHO include, methanol, ethanol, isopropyl alcohol, dioxane, water, acetic acid, or mixed solvents thereof. The solvent is preferably a mixed solvent composed of methanol, dioxane, and acetic acid. Reducing agents usable herein include sodium borohydride, sodium cyanoborohydride, lithium cyanoborohydride, and sodium triacetoxy borohydride. The reducing agent is preferably sodium triacetoxy borohydride. The reaction temperature is 0°C to 30°C. The reaction time is 1 to 8 hr.

Solvents usable in the step of deprotection with a base include methanol, ethanol, isopropyl alcohol, tert-butyl alcohol, methylene chloride, chloroform, and mixed solvents thereof. The solvent is preferably a mixed solvent composed of methanol and methylene chloride. Bases usable herein include potassium carbonate, sodium carbonate, potassium hydroxide, sodium hydroxide, sodium methoxide, sodium ethoxide, and tert-BuOK. The base is preferably sodium methoxide. The reaction temperature is 0°C to 60°C. The reaction time is 1 to 8 hr.

Solvents usable in the step of deprotection with an acid include ethyl acetate, methylene chloride, acetonitrile, acetone, and water. The solvent is preferably water. Acids usable herein include p-toluenesulfonic acid, methanesulfonic acid, acetic acid, and trifluoroacetic acid. The acid is preferably trifluoroacetic acid. The reaction temperature is generally 0°C to 30°C. The reaction time is 1 to 12 hr. When the protective group A in the compound of general formula (K3) is benzyloxycarbonyl, p-methoxybenzyloxycarbonyl, or p-nitrobenzyloxycarbonyl, the protective group can also be removed by reacting this compound with hydrogen and a catalyst for catalytic hydrogen reduction. Catalysts for catalytic hydrogen reduction usabe herein include palladium-carbon and platinum oxide. Any solvent may be used without particular limitation so far as the solvent is inert to this reaction. Preferred solvents are methanol, ethanol, tetrahydrofuran, dioxane, and a mixed solvent composed of these organic solvent and water. The reaction temperature is 10°C to 30°C. The reaction time is generally 1 to 8 hr.

Process L
In Process L, a compound of general formula (L5) is produced by introducing substituted sulfonyloxy into the 5-position of the compound of general formula (L1), wherein R^{5a} represents C₁₋₆ alkanoyloxy, in an axial configuration. Process L comprises the following steps. The compound of general formula (L1) as a starting compound can be produced according to the steps J1 and J2 in the above process J.

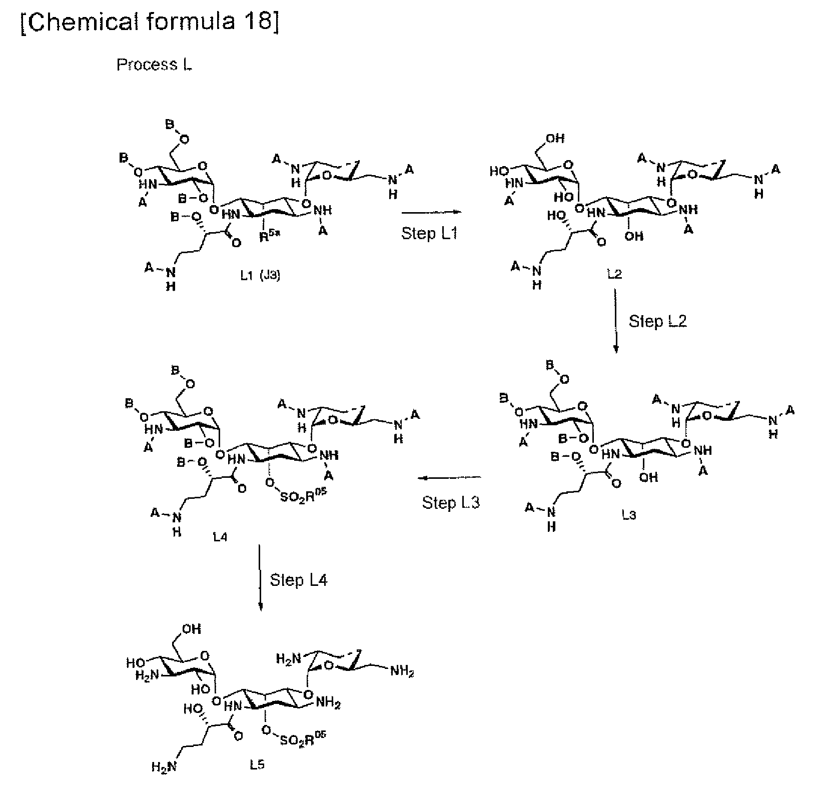

Step L1
In step L1, a compound of general formula (L2) is produced by removing the protective group of hydroxyl in the compound of general formula (L1). This step is achieved by reacting the compound of general formula (L1) with a base.

Solvents usable in this step include methanol, ethanol, isopropyl alcohol, tert-butyl alcohol, methylene chloride, chloroform, and mixed solvents thereof. The solvent is preferably a mixed solvent composed of methanol and methylene chloride. Bases usable herein include potassium carbonate, sodium carbonate, potassium hydroxide, sodium hydroxide, sodium methoxide, sodium ethoxide, and tert-BuOK. The base is preferably sodium methoxide. The reaction temperature is 0°C to 60°C. The reaction time is 1 to 8 hr.

Step L2
In step L2, a compound of general formula (L3) is produced by introducing a protective group into hydroxyl in the compound of general formula (L2) except for hydroxyl at the 5-position. This step is achieved by reacting the compound of general formula (L2) with B₂O or BCl wherein B represents acetyl or benzoyl in the presence of a base.

Solvents usable in this step include pyridine, N,N-dimethylformamide, methylene chloride, and chloroform. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step L3
In step L3, hydroxyl at the 5-position of the compound of general formula (L3) is sulfonated. This step is achieved by reacting the compound of general formula (L3) with R⁰⁵SO₂Cl wherein R⁰⁵ represents C₁₋₆ alkyl in the presence of a base.

Solvents usable in this step include, for example, methylene chloride, chloroform, 1,2-dichloroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Step L4
In step L4, a compound of general formula (L5) is produced by removing the protective group in the compound of general formula (L4). The protective group can be removed in the same manner as in step A8.

Process M
In process M, a compound of general formula (M6) is produced by introducing substituent R^{5a} into the 5-position of the compound of general formula (M1) in an axial configuration and then introducing a side chain into the 1-position. Process M comprises the following steps. The compound of general formula (M1) as a starting compound can be produced according to step 11 in the above proess I.

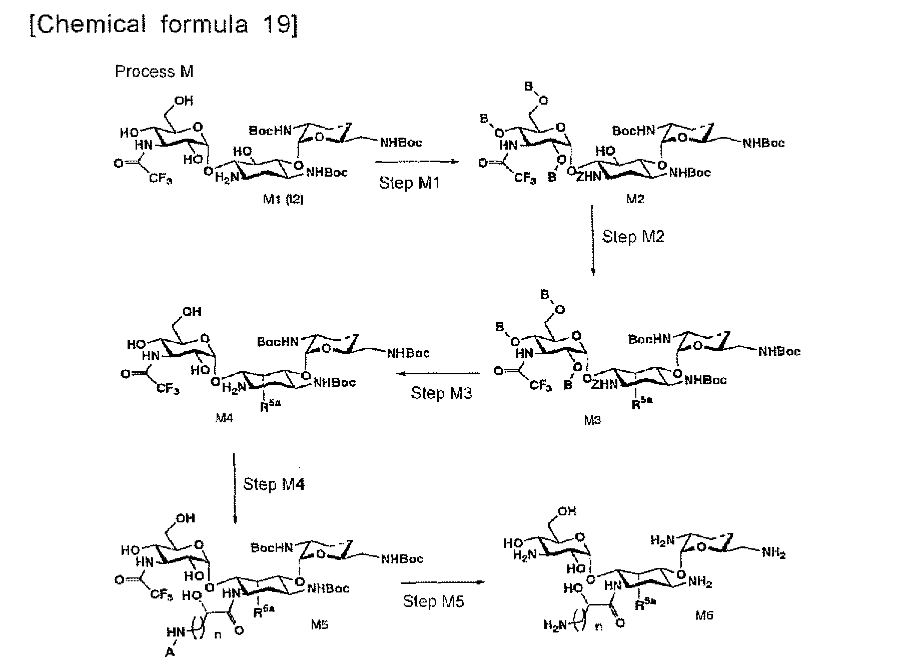

Process M1
In step M1, a compound of general formula (M2) is produced by introducing a protective group into hydroxyl in the compound of general formula (M1) except for hydroxyl at the 5-position. This step is achieved by reacting the compound of general formula (M1) with B₂O or BCI wherein B represents acetyl or benzoyl in the presence of a base.

Solvents usable in this step include pyridine, N,N-dimethylformamide, methylene chloride, and chloroform. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step M2
In step M2, a compound of general formula (M3) is produced by introducing a leaving group into hydroxyl at the 5-position of the compound of general formula (M2) and then subjecting the resultant compound to a substitution reaction. This step is achieved by reacting the compound of general formula (M2) with WSO₂Cl wherein W represents methyl, phenyl, or p-tolyl in the presence of a base to synthesize a compound having substituted sulfonyloxy at the 5-position and then reacting the resultant compound with R^{5a}M wherein R^{5a} represents acetoxy, azide, a chlorine atom, or C₁₋₆ alkylamino wherein one or more hydrogen atoms in the alkyl group are optionally substituted by hydroxyl, phenyl, vinyl, amino, or hydroxymethyl, and M represents lithium, sodium, cesium, or a hydrogen atom.

Solvents usable in the step of introducing a leaving group include, for example, methylene chloride, chloroform, 1,2-dichloroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Solvents usable in the step of a substitution reaction include tetrahydrofuran, 1,2-dimethoxyethane, dioxane, N,N-dimethylformamide, and dimethylsulfoxide. The solvent is preferably N,N-dimethylformamide. The reaction temperature is 60°C to 90°C. The reaction time is 1 to 12 hr.

Step M3
In step M3, the protective group of hydroxyl in the compound of general formula (M3) is removed, and the protective group of amino in the 1-position is then removed. This step is achieved by reacting the compound of general formula (M3) with a base and then reacting the resultant compound with a reducing agent.

Solvents usable in the step of removing the protective group of hydroxyl include methanol, ethanol, isopropyl alcohol, tert-butyl alcohol, methylene chloride, chloroform, and mixed solvents thereof. The solvent is preferably a mixed solvent composed of methanol and methylene chloride. Bases usable herein include potassium carbonate, sodium carbonate, potassium hydroxide, sodium hydroxide, sodium methoxide, sodium ethoxide, and tert-BuOK. The base is preferably sodium methoxide. The reaction temperature is 0°C to 60°C. The reaction time is 1 to 8 hr.

Reducing agents usable in the step of removing the protective group of amino include hydrogen and catalysts for catalytic hydrogen reduction such as palladium-carbon, palladium black, palladium hydroxide, and platinum oxide, or metallic sodium and metallic lithium. When the dashed line in the compound of general formula (M3) represents a double bond, metallic sodium is preferred. On the other hand, when the dashed line represents a single bond, hydrogen and palladium-carbon catalysts are preferred. Solvents usable herein include, in the case of catalytic hydrogen reduction, methanol, ethanol, tetrahydrofuran, dioxane, N,N-dimethylformamide, water, or mixed solvents composed of water and these organic solvents. When metallic sodium is used, liquid ammonia is preferred. The reaction temperature is -60°C to 30°C. The reaction time is generally 1 to 8 hr.

Step M4
In step M4, a compound of general formula (M5) wherein A represents tert-butoxycarbonyl, benzyloxycarbonyl, p-methoxybenzyloxycarbonyl, or p-nitrobenzyloxycarbonyl, is produced by introducing a side chain into amino at the 1-position of the compound of general formula (M4). This step is achieved by condensing the compound of general formula (M4) with a carboxylic acid ANH(CH₂)ₙCH(OH)COOH wherein A is as defined above and n represents an integer of 1 to 3 in the presence of a condensing agent, or by reacting the compound of general formula (M4) with a derivative of a carboxylic acid ANH(CH₂)ₙCH(OH)COOH wherein A is as defined above in the absence of a condensing agent.

Condensing agents usable in this step include, for example, carbodiimides such as dicyclohexylcarbodiimide, diisopropylcarbodiimide and N-ethyl-N'-3-dimethylaminopropylcarbodiimide, or these condensing agents to which, for example, 1-oxobenzotriazole or 3-hydroxy-4-oxo-3,4-dihydro-1,2,3-benzotriazine has been added as an additive. Carboxylic acid derivatives usable herein include N-hydroxyphthalimide esters, N-hydroxysuccinimide esters, p-nitrophenyl esters, and pentafluorophenyl esters. The carboxylic acid derivative is preferably an N-hydroxysuccinimide ester. Solvents usable herein include tetrahydrofuran, dioxane, methylene chloride, chloroform, and N,N-dimethylformamide. The solvent is preferably N,N-dimethylformamide. The reaction temperature is 0°C to 30°C. The reaction time is 1 to 12 hr.

Step M5
In step M5, a compound of general formula (M6) is produced by removing the protective group in the compound of general formula (M5). The protective group can be removed in the same manner as in step A8.

Process N
In process N, a compound of general formula (N6) is produced by introducing substituent R^{3"a} into the 3"-position in the compound of general formula (N1) and then introducing substituent R^{5a} into the 5-position in an axial configuration. Process N comprises the following steps. The compound of formula (N1) as a starting compound can be produced by the method described in Japanese Patent Laid-Open No. 164696/1980.

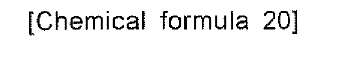

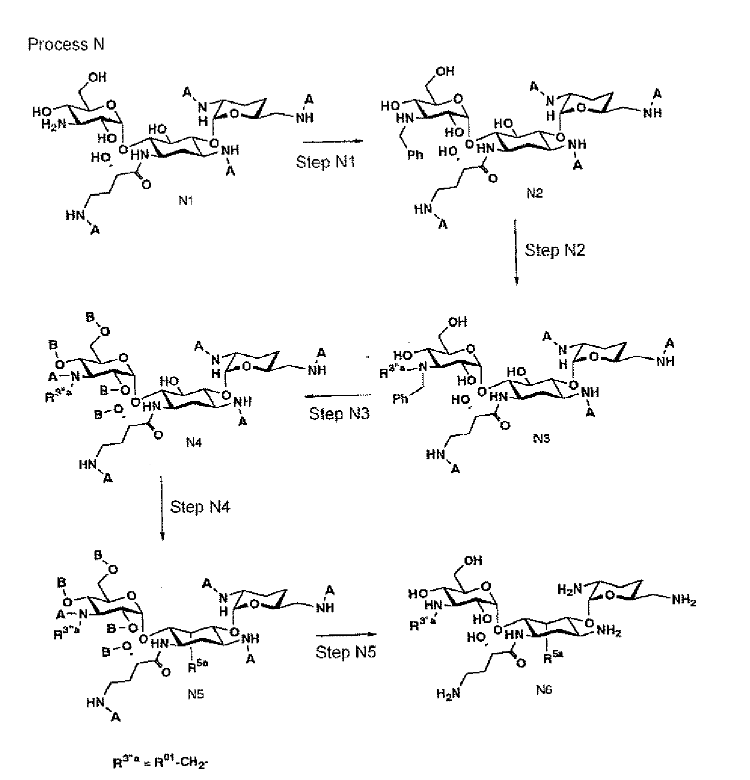

Step N1
In step N1, benzyl is introduced into amino at the 3"-position of the compound of general formula (N1). This step is achieved by reacting the compound of general formula (N1) with benzyl bromide in the presence of a base. This step can also be achieved by reacting the compound of general formula (N1) with benzaldehyde in the presence of a reducing agent.

Solvents usable in this step include tetrahydrofuran, dioxane, 1,2-dimethoxyethane, N,N-dimethylformamide, dimethylsulfoxide, and methylene chloride. The solvent is preferably N,N-dimethylformamide. Bases usable herein include potassium carbonate, sodium carbonate, triethylamine, and 4-dimethylaminopyridine. The base is preferably potassium carbonate. The reaction temperature is 20°C to 60°C. The reaction time is 1 to 12 hr.

Step N2
In step N2, a compound of general formula (N3) is produced by introducing a substituent into amino at the 3"-position of the compound of general formula (N2). This step is achieved by reacting the compound of general formula (N2) with R⁰¹CHO wherein R⁰¹ represents a hydrogen atom or C₁₋₅ alkyl in the presence of a reducing agent.

Reducing agents usable in this step include sodium borohydride, sodium cyanoborohydride, lithium cyanoborohydride, and sodium triacetoxyborohydride. The reducing agent is preferably sodium borohydride. Solvents usable herein include methanol, ethanol, isopropyl alcohol, dioxane, water, or mixed solvents thereof. The solvent is preferably a mixed solvent composed of methanol and dioxane. The reaction temperature is 0°C to 30°C. The reaction time is 1 to 8 hr.

Step N3
In step N3, benzyl at the 3"-position of the compound of general formula (N3) is converted to tert-butoxycarbonyl. This step is achieved by reacting the compound of general formula (N3) with di-tert-butyl dicarbonate and a reducing agent.

Solvents usable in this step include, for example, methanol, ethanol, tetrahydrofuran, dioxane, tetrahydrofuran, or mixed solvents composed of these organic solvents and water. The solvent is preferably a mixed solvent composed of water and tetrahydrofuran. Reducing agents usable herein include catalysts for catalytic hydrogen reduction used together with hydrogen, for example, palladium-carbon, palladium black, palladium hydroxide, and platinum oxide. The reducing agent is preferably hydrogen and palladium-carbon. The reaction temperature is 10°C to 30°C. The reaction time is generally 1 to 8 hr.

Step N4
In step N4, a compound of general formula (N5) is produced by introducing a leaving group into hydroxyl at the 5-position of the compound of general formula (N4) and then subjecting the resultant compound to a substitution reaction. This step is achieved by reacting the compound of general formula (N4) with WSO₂Cl wherein W represents methyl, phenyl, or p-tolyl in the presence of a base to synthesize a compound having substituted sulfonyloxy at the 5-position and then reacting the resultant compound with R^{5a}M wherein R^{5a} represents acetoxy, azide, a chlorine atom, a bromine atom, or C₁₋₆ alkylamino wherein one or more hydrogen atoms in the alkyl group are optionally substituted by hydroxyl, phenyl, vinyl, amino, or hydroxymethyl, and M represents lithium, sodium, cesium, or a hydrogen atom.

Solvents usable in the step of introducing a leaving group include, for example, methylene chloride, chloroform, 1,2-dichloroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine, The reaction temperature is generally 0°C to 30°C. The reaction time is 1 to 24 hr.

Solvents usable in the step of a substitution reaction include tetrahydrofuran, 1,2-dimethoxyethane, dioxane, N,N-dimethylformamide, and dimethylsulfoxide. The solvent is preferably N,N-dimethylformamide. The reaction temperature is 60°C to 90°C. The reaction time is 1 to 12 hr.

Step N5
In step N5, a compound of general formula (N6) is produced by removing the protective group in the compound of general formula (N5). The protective group can be removed in the same manner as in step A8.

Process O
In process O, a compound of general formula (09) is produced by introducing a side chain into the 6'-position of the compound of general formula (O1) and then introducing a substituent R^{5a} into the 5-position in an axial configuration. Process O comprises the following steps. The compound of general formula (O1) as a starting compound can be produced according to step G1 in the above process G.

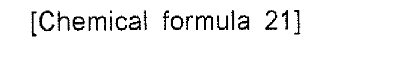

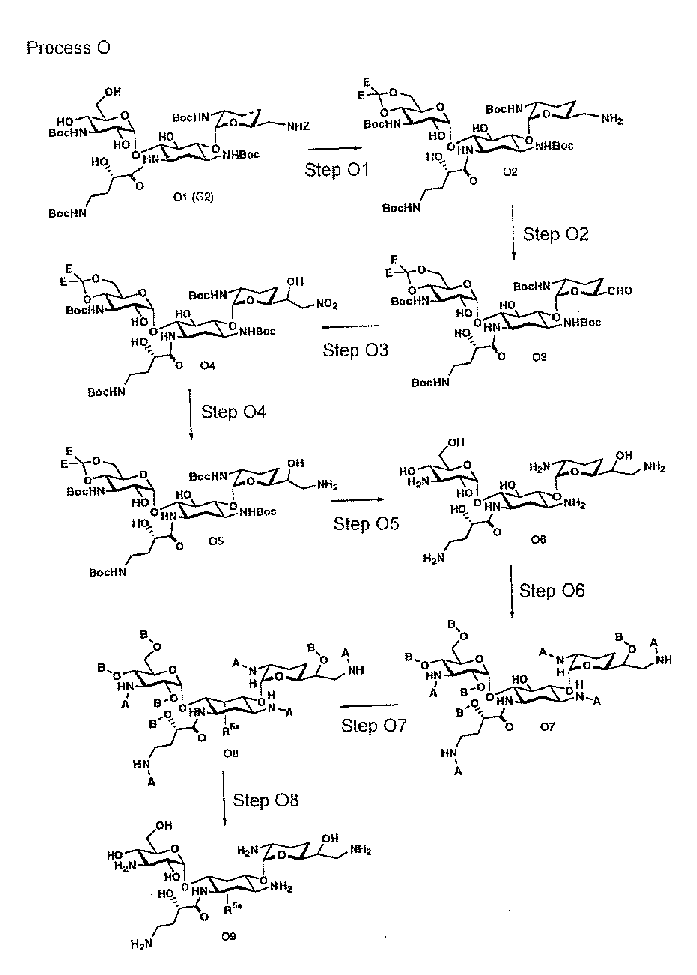

Step O1
In step O1, a protective group is introduced into the 4"-position and 6"-position of the compound of general formula (O1), and the protective group at the 6'-position is then removed. This step is achieved by reacting the compound of general formula (O1) with E₂CO or E₂C(OMe)₂ wherein E represents a hydrogen atom, methyl, or phenyl or, as E₂C, cyclohexyl in the presence of an acid and then reacting the resultant compound with a reducing agent.

Solvents usable in the step of protection include, for example, N,N-dimethylformamide, methylene chloride, and ethyl acetate. The solvent is preferably N,N-dimethylformamide. Acids usable herein include p-toluenesulfonic acid, pyridinium p-toluenesulfonate, camphorsulfonic acid, and hydrochloric acid. The acid is preferably p-toluenesulfonic acid. The reaction temperature is 20°C to 50°C. The reaction time is 1 to 8 hr.

Reducing agents usable in the step of deprotection include catalysts for catalytic hydrogen reduction used together with hydrogen, for example, palladium-carbon, palladium black, palladium hydroxide, and platinum oxide. The reducing agent is preferably hydrogen and palladium-carbon. Any solvent may be used without particular limitation so far as the solvent is inert to this reaction. Preferred solvents are methanol, ethanol, tetrahydrofuran, dioxane, and mixed solvents composed of these organic solvents and water. The reaction temperature is 10°C to 30°C. The reaction time is generally 1 to 8 hr.

Step O2
In step O2, amino at the 6'-position of the compound of general formula (O2) is oxidized to aldehyde. This step is achieved by reacting the compound of general formula (02) with an oxidizing agent in the presence of a base.

A mixed solvent composed of water and chloroform is preferred as a solvent used in this step. The oxidizing agent is preferably ninhydrin. Bases usable herein include sodium hydrogencarbonate. The reaction temperature is 0°C to 30°C. The reaction time is 12 to 48 hr.

Step O3
In step O3, a compound of general formula (O4) is produced. This step is achieved by reacting the compound of general formula (O3) with nitromethane in the presence of a base. Solvents usable in this step include methanol, ethanol, tert-butyl alcohol, methylene chloride, dichloroethane, and mixed solvents thereof. The solvent is preferably a mixed solvent composed of methanol and methylene chloride. Bases usable herein include sodium methoxide, sodium ethoxide, and tert-BuOK. The base is preferably sodium methoxide. The reaction temperature is 0°C to 30°C. The reaction time is 1 to 6 hr.

Step O4
In step O4, a compound of general formula (O5) is produced. This step is achieved by reacting the compound of general formula (O4) with a reducing agent. Reducing agents usable in this step include hydrogen and catalysts for catalytic hydrogen reduction such as palladium-carbon, palladium black, palladium hydroxide, and platinum oxide. Preferred are hydrogen and platinum oxide. Any solvent may be used without particular limitation so far as the solvent is inert to this reaction. Preferred are methanol, ethanol, tetrahydrofuran, dioxane, and mixed solvents composed of these organic solvents and water. The reaction temperature is 10°C to 30°C. The reaction time is generally 1 to 8 hr.

Step O5
In step O5, a compound of formula (O6) is produced by removing the protective group in general formula (O5). The protective group can be removed in the same manner as in step A8.

Step O6
In step O6, a protective group is introduced into all hydroxyl and amino in the compound of formula (O6) except for the 5-position of the compound. This step is achieved by first reacting the compound of formula (O6) with A₂O or ACl wherein A represents tert-butoxycarbonyl, benzyloxycarbonyl, p-methoxybenzyloxycarbonyl, or p-nitrobenzyloxycarbonyl in the presence of a base to introduce a protective group into amino and then reacting the resultant compound with B₂O or BCl wherein B represents acetyl or benzoyl in the presence of a base to introduce a protective group into hydroxyl.

Solvents usable in the step of protecting amino include water, N,N-dimethylformamide, tetrahydrofuran, dioxane, and mixed solvents thereof. The solvent is preferably a mixed solvent composed of water and N,N-dimethylformamide. Bases usable herein include sodium hydroxide, potassium carbonate, sodium carbonate, triethylamine, pyridine, and 4-dimethylaminopyridine. The base is preferably triethylamine. The reaction temperature is 0°C to 40°C. The reaction time is 1 to 24 hr.

Solvents usable in the step of protecting hydroxyl include pyridine, N,N-dimethylformamide, methylene chloride, and chloroform. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step O7
In step O7, a compound of general formula (O8) is produced by introducing a leaving group into hydroxyl at the 5-position of the compound of general formula (O7) and then subjecting the resultant compound to a substitution reaction. This step is achieved by reacting the compound of general formula (O7) with WSO₂Cl wherein W represents methyl, phenyl, or p-tolyl in the presence of a base to synthesize a compound having substituted sulfonyloxy at the 5-position and then reacting the resultant compound with R^{5a}M wherein R^{5a} represents acetoxy, azide, a chlorine atom, a bromine atom, or C₁₋₆ alkylamino wherein one or more hydrogen atoms in the alkyl group are optionally substituted by hydroxyl, phenyl, vinyl, amino, or hydroxymethyl, and M represents lithium, sodium, cesium, or a hydrogen atom.

Solvents usable in the step of introducing a leaving group include, for example, methylene chloride, chloroform, 1,2-dichloroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Solvents usable in the step of a substitution reaction include tetrahydrofuran, 1,2-dimethoxyethane, dioxane, N,N-dimethylformamide, and dimethylsulfoxide. The solvent is preferably N,N-dimethylformamide. The reaction temperature is 60°C to 90°C. The reaction time is 1 to 12 hr.

Step O8
In step O8, a compound of general formula (O9) is produced by removing the protective group in the compound of general formula (O8). The protective group can be removed in the same manner as in step A8.

Process P
In process P, a compound of general formula (P6) is produced by reducing hydroxyl at the 6"-position of the compound of general formula (P1) and then introducing substituent R^{5a} into the 5-position in an axial configuration. This process comprises the following steps. The compound of general formula (P1) as a starting compound can be produced according to step A1 in the above process A.

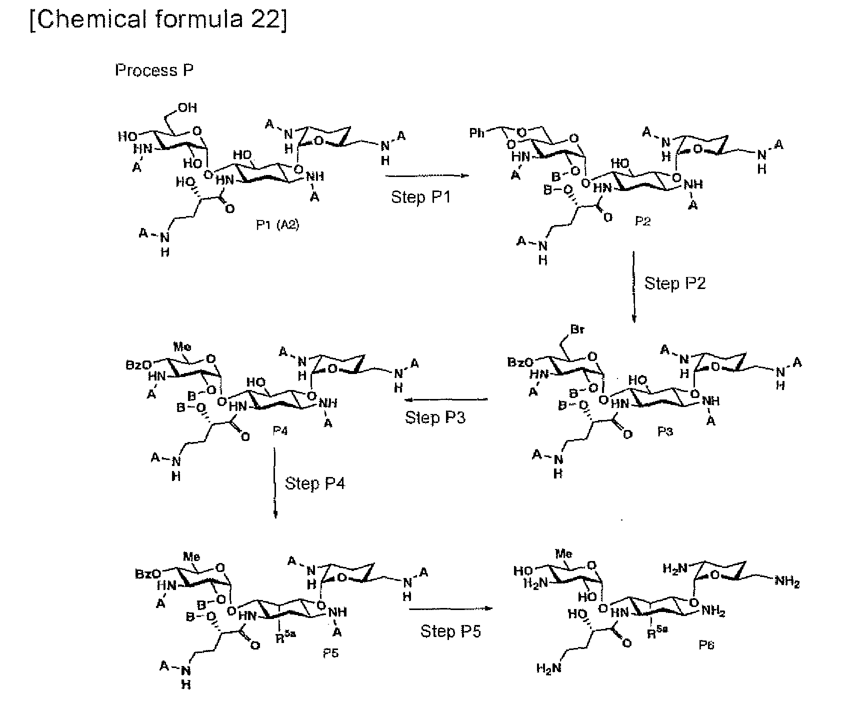

Step P1
In step P1, a protective group is introduced into hydroxyl in the compound of general formula (P1) except for hydroxyl at the 5-position of the compound. This step is achieved by reacting the compound of general formula (P1) with PhCH(OR⁰⁶)₂ wherein R⁰⁶ represents methyl or ethyl in the presence of an acid to protect the 4"-position and 6"-position and then reacting the resultant compound with B₂O or BCI wherein B represents acetyl or benzoyl in the presence of a base to protect the 2"-position and 2"'-position.

Solvents usable in the step of protecting 4"-position and 6"-position include, for example, N,N-dimethylformamide, methylene chloride, and ethyl acetate. The solvent is preferably N,N-dimethylformamide. Acids usable herein include p-toluenesulfonic acid, pyridinium p-toluenesulfonate, camphorsulfonic acid, and hydrochloric acid. The acid is preferably p-toluenesulfonic acid. The reaction temperature is 0°C to 10°C. The reaction time is 1 to 8 hr.

Solvents usable in the step of protecting the 2"-position and 2"'-position include pyridine, N,N-dimethylformamide, methylene chloride, and chloroform. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step P2
In step P2, a compound of general formula (P3) is produced. This step is achieved by reacting the compound of general formula (P2) with a halogenating agent. Carbon tetrachloride is a preferred solvent usable in this step. Halogenating agents usable herein include N-bromosuccinimide. The reaction temperature is 20°C to 60°C. The reaction time is 1 to 12 hr.

Step P3
In step P3, a compound of general formula (P4) is produced. This step is achieved by reacting the compound of general formula (P3) with a reducing agent in the presence of a free radical initiator, or by catalytic hydrogen reduction of the compound of general formula (P3).

Reducing agents usable in this step include, for example, tri-n-butyltin hydride, di-n-butyltin hydride, triethyltin hydride, and triphenyltin hydride. The reducing agent is preferably tri-n-butyltin hydride. Azobisisobutylnitrile may be mentioned as the free radical initiator. Solvents usable herein include tetrahydrofuran, dioxane, and benzene, and toluene. The solvent is preferably dioxane. The reaction temperature is 20°C to 120°C. The reaction time is 1 to 8 hr.

Catalysts usable in the catalytic hydrogen reduction include palladium-carbon, palladium black, palladium hydroxide, and platinum oxide. The catalyst is preferably palladium-carbon. Any solvent may be used without particular limitation so far as the solvent is inert to this reaction. Preferred are methanol, ethanol, tetrahydrofuran, dioxane, and mixed solvents composed of these organic solvents and water. The reaction temperature is 10°C to 30°C. The reaction time is generally 1 to 8 hr.

Step P4
In step P4, a compound of general formula (P5) is produced by introducing a leaving group into hydroxyl at the 5-position of the compound of general formula (P4) and then subjecting the resultant compound to a substitution reaction. This step is achieved by reacting the compound of general formula (P4) with WSO₂Cl wherein W represents methyl, phenyl, or p-tolyl in the presence of a base to synthesize a compound having substituted sulfonyloxy at the 5-position and then reacting the resultant compound with R^{5a}M wherein R^{5a} represents acetoxy, azide, a chlorine atom, a bromine atom, or C₁₋₆ alkylamino wherein one or more hydrogen atoms in the alkyl group are optionally substituted by hydroxyl, phenyl, vinyl, amino, or hydroxymethyl, and M represents lithium, sodium, cesium, or a hydrogen atom.

Solvents usable in the step of introducing a leaving group include, for example, methylene chloride, chloroform, 1,2-dichioroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Solvents usable in the step of a substitution reaction include tetrahydrofuran, 1,2-dimethoxyethane, dioxane, N,N-dimethylformamide, and dimethylsulfoxide. The solvent is preferably N,N-dimethylformamide. The reaction temperature is 60°C to 90°C. The reaction time is 1 to 12 hr.

Step P5
In step P5, a compound of general formula (P6) is produced by removing the protective group in the compound of general formula (P5). The protective group can be removed in the same manner as in step A8.

Process Q
In process Q, a compound of general formula (Q7) is produced by introducing a side chain R^{6"a} wherein R^{6"a} represents 2-amino-1-hydroxyethyl into the 6"-position of the compound of general formula (Q1). Process Q comprises the following steps. The compound of general formula (Q1) as a starting compound can be produced according to steps J1 and J2 in the above process J.

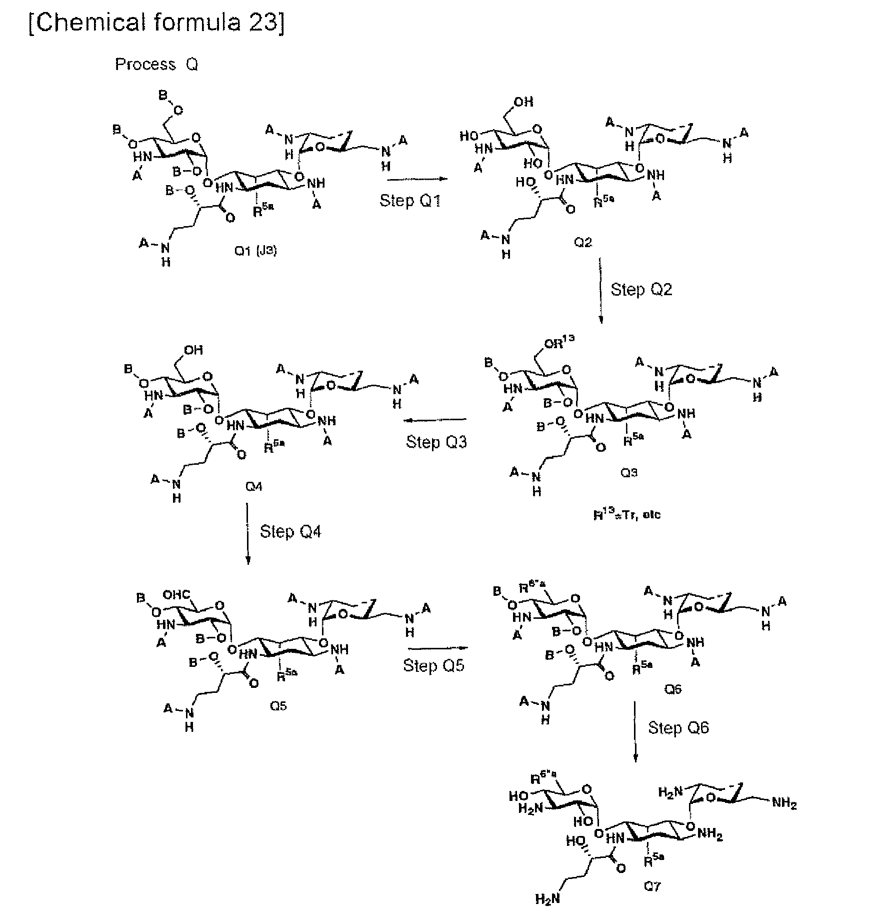

Process Q1
In process Q1, the protective group of hydroxyl in the compound of general formula (Q1) is removed. This step is achieved by reacting the compound of general formula (Q1) with a base. Solvents usable in this step include methanol, ethanol, isopropyl alcohol, tert-butyl alcohol, methylene chloride, chloroform, and mixed solvents thereof. The solvent is preferably a mixed solvent composed of methanol and methylene chloride. Bases usable herein include potassium carbonate, sodium carbonate, potassium hydroxide, sodium hydroxide, sodium methoxide, sodium ethoxide, and tert-BuOK. The base is preferably sodium methoxide. The reaction temperature is 0°C to 60°C. The reaction time is 1 to 8 hr.

Step Q2
A compound of general formula (Q3) is produced by protecting hydroxyl at the 6"-position of the compound of general formula (Q2) by triphenylmethyl or silyl and then protecting the remaining hydroxyl in the compound by acyl. This step is achieved by reacting the compound of general formula (Q2) with R¹³Cl wherein R¹³ represents triphenylmethyl, tert-butyldimethylsilyl, triisopropylsilyl, or tert-butyldiphenylsilyl in the presence of a base and then reacting the resultant compound with B₂O or BCI wherein B represents acetyl or benzoyl in the presence of a base.

Solvents usable in the step of introducing triphenylmethyl include methylene chloride, acetonitrile, and pyridine. The solvent is preferably pyridine. Bases usable herein include triethylamine, pyridine, and 4-dimethylaminopyridine. The base is preferably pyridine. The reaction temperature is 20°C to 80°C. The reaction time is generally 2 to 10 hr.

Preferred solvents usable in the step of introducing silyl include methylene chloride, chloroform, dimethylformamide, acetonitrile, and pyridine. Bases usable herein include 4-dimethylaminopyridine, triethylamine, imidazole, and diisopropylethylamine, The base is preferably 4-dimethylaminopyridine. The reaction temperature is 0°C to 30°C. The reaction time is 1 to 12 hr.

Solvents usable in the step of introducing acyl include pyridine, N,N-dimethylformamide, methylene chloride, and chloroform. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step Q3
In step Q3, a compound of general formula (Q4) is produced by removing the protective groups of hydroxyl, i.e., triphenylmethyl or silyl, at the 6"-position of the compound of general formula (Q3). This step is achieved by reacting the compound of general formula (Q3) with an acid or a base.

Solvents usable in the step of deprotection of triphenylmethyl group include diethyl ether, tetrahydrofuran, 1,2-dimethoxyethane, and water. The solvent is preferably diethyl ether. Acids usable herein include formic acid and acetic acid. The acid is preferably formic acid. The reaction temperature is 0°C to 30°C. The reaction time is 1 to 8 hr.

Preferred solvents usable in the step of deprotection of silyl group include acetonitrile, tetrahydrofuran, and methylene chloride. Reagents usable in the deprotection include tetrabutylammonium fluoride, hydrogen fluoride-pyridiene, hydrogen fluoride-triethylamine, and hydrogen fluoride. The reaction temperature is 0°C to 30°C. The reaction time is 1 to 12 hr.

Step Q4
In step Q4, hydroxyl at the 6"-position of the compound of general formula (Q4) is oxidized to aldehyde. This step is achieved by reacting the compound of general formula (Q3) with an oxidizing agent.

Pyridine may be mentioned as a preferred solvent used in this step. Oxidizing agents usable in this step include dimethylsulfoxide, dicyclohexylcarbodiimide, and hydrogen donating compounds. Hydrogen donating compounds include phosphoric acid and trifluoroacetic acid. The hydrogen donating compound is preferably trifluoroacetic acid. The reaction temperature is 0°C to 30°C. The reaction time is 6 to 24 hr.

Step Q5
In step Q5, a compound of general formula (Q6) is produced. This step is achieved by reacting the compound of general formula (Q5) with nitromethane in the presence of a base and then reacting the resultant nitro compound with a reducing agent.

Solvents usable in the step of the reaction with nitromethane include methanol, ethanol, tert-butyl alcohol, methylene chloride, 1,2-dichloroethane, and mixed solvents thereof. The solvent is preferably a mixed solvent composed of methanol and methylene chloride. Bases usable herein include sodium methoxide, sodium ethoxide, and tert-BuOK. The base is preferably sodium methoxide. The reaction temperature is 0°C to 30°C. The reaction time is 1 to 6 hr.

Reducing agents usable in the step of reduction include catalysts for catalytic hydrogen reduction used together with hydrogen, for example, palladium-carbon, palladium black, palladium hydroxide, and platinum oxide, or iron. When the dashed line in the compound of general formula (Q5) represents a single bond, hydrogen and platinum oxide are preferred. On the other hand, when the dashed line represents a double bond, iron is preferred. Any solvent may be used without particular limitation so far as the solvent is inert to this reaction. Preferred solvents include methanol, ethanol, tetrahydrofuran, dioxane, acetic acid, or mixed solvents composed of these organic solvents and water. The reaction temperature is 0°C to 30°C. The reaction time is generally 1 to 8 hr.

Step Q6
In step Q6, a compound of general formula (Q7) is produced by removing the protective group in the compound of general formula (Q6). The protective group can be removed in the same manner as in step A8.

The compounds of general formula (II) according to the second aspect of the present invention can be produced by the following process R.

### Process R

In process R, a compound of general formula (R4) is produced by introducing a substituent into the 5-position of a compound of general foemula (R1) in an axial configuration. Process R comprises the following steps. The compound of general formula (R1) as a starting compound can be produced according to step J1 in the above process J.

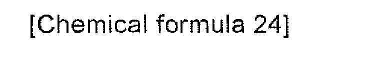

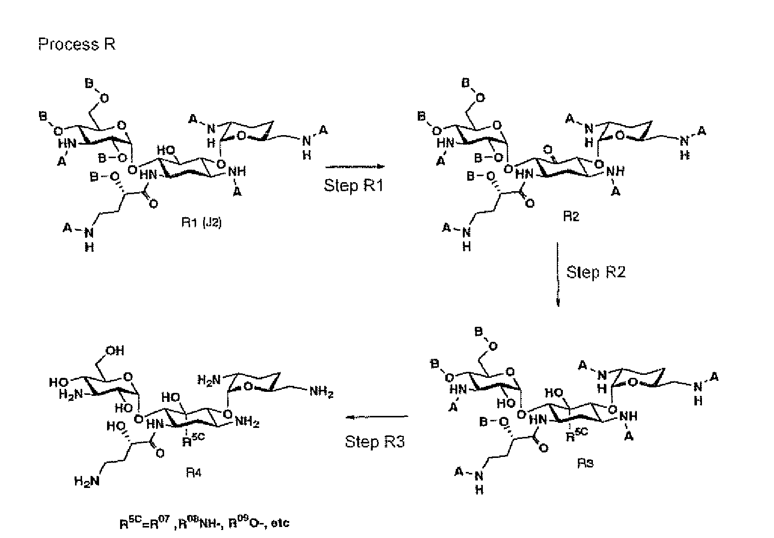

Step R1 In step R1, a compound of general formula (R2) is produced. This step is achieved by reacting the compound of general formula (R1) with an oxidizing agent. The oxidizing agents is preferably a combination of dimethylsulfoxide with acetic anhydride. The reaction temperature is 0°C to 30°C. The reaction time is 48 to 72 hr.

Step R2 In step R2, a compound of general formula (R3) is produced. This step is achieved by either reacting the compound of general formula (R2) with R⁰⁷MgX wherein R⁰⁷ represents C₁₋₆ alkyl or C₂₋₆ alkenyl, and X represents a halogen, or reacting the compound of general formula (R2) with diazomethane and then reacting the resultant epoxy compound with NaN₃; R⁰⁸NH₂ wherein R⁰⁸ represents C₁₋₆ alkyl, in which one or more hydrogen atoms in the alkyl group are optionally substituted by amino or hydroxyl, or aralkyl; or R⁰⁹ONa wherein R⁰⁹ represents C₁₋₆ alkyl. The azide compound produced by the reaction with NaN₃ can be converted to an amino compound by a reaction with a reducing agent.

Solvents usable in the step of the reaction with R⁰⁷MgX include diethyl ether, tetrahydrofuran, dimethoxyethane, dioxane, benzene, and toluene. The solvent is preferably tetrahydrofuran. The reaction temperature is -40°C to 30°C. The reaction time is 1 to 8 hr.

Solvents usable in the step of the reaction with diazomethane include methanol, ethanol, methylene chloride, and dichloroethane. The solvent is preferably methanol. The reaction temperature is 0°C to 30°C. The reaction time is 1 to 4 hr.

Solvents usable in the step of the reaction with NaN₃, R⁰⁸NH₂, and R⁰⁹ONa include tetrahydrofuran, 1,2-dimethoxyethane, dioxane, N,N-dimethylformamide, dimethylsulfoxide, methanol, and ethanol. The solvent is preferably N,N-dimethylformamide. The reaction temperature is 0°C to 80°C. The reaction time is 1 to 12 hr.

Reducing agents usable in the step of reducing the azide compound include catalysts for catalytic hydrogen reduction used together with hydrogen, for example, palladium-carbon, palladium black, palladium hydroxide, and platinum oxide. Preferred are hydrogen and palladium-carbon. The solvent is not particularly limited so far as it is inert to this reaction. Preferred solvents include methanol, ethanol, tetrahydrofuran, dioxane, and mixed solvents composed of these organic solvents and water. The reaction temperature is 10°C to 30°C. The reaction time is generally 1 to 8 hr.

Step R3
In step R3, a compound of general formula (R4) is produced by removing the protective group in the compound of general formula (R3). The protective group can be removed in the same manner as in step A8.

The compounds of general formula (III) according to the third aspect of the present invention can be produced by the following processes S to X.

Process S
In process S, a compound of general formula (S3) is produced by introducing substituent R^{3"c} into the 3"-position of the compound of general formula (S1). Process S comprises the following steps. Each step constituting this process will be described in detail. The compound of general formula (S3) can also be produced by removing the protective group in the compound of general formula (N4).

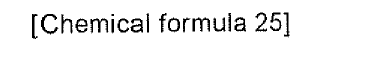

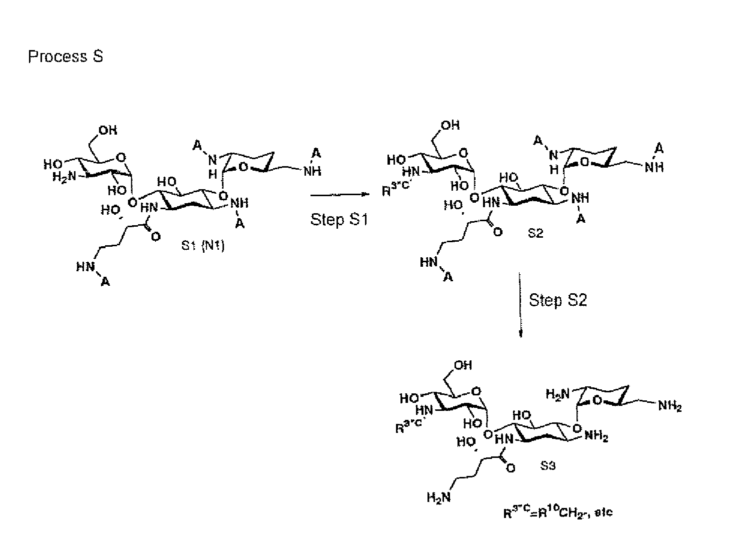

Step S1
In step S1, a compound of general formula (S2) is produced by introducing substituent R^{3"c} into amino at the 3"-position of the compound of general formula (S1). This step is achieved by reacting, in the presence of a reducing agent, the compound of general formula (S1) with R¹⁰CHO wherein R¹⁰ represents C₁₋₉ alkyl in which one or more hydrogen atoms in the alkyl group are optionally substituted by hydroxyl; aryl; or aralkyl. This step can also be achieved by reacting , in the presence of a base, the compound of general formula (S1) with R¹⁴X wherein R¹⁴ represents C₁₋₁₀ alkyl in which one or more hydrogen atoms in the alkyl group are optionally substituted by hydroxyl; or OR¹⁵ in which R¹⁵ represents triphenylmethyl, tert-butyldimethylsilyl, triisopropylsilyl, tert-butyldiphenylsilyl, or tetrahydropyranyl; or aralkyl. Further, this step can be achieved by reacting the compound of general formula (S1) with an imidoylating agent or an amidinoylating agent for introducing formimidoyl or amidino.

Reducing agents usable in the step of the reaction with R¹⁰CHO include sodium borohydride, sodium cyanoborohydride, lithium cyanoborohydride, and sodium triacetoxyborohydride. The reducing agent is preferably sodium borohydride. Solvents usable herein include methanol, ethanol, isopropyl alcohol, dioxane, water, or mixed solvents thereof. The solvent is preferably a mixed solvent composed of methanol and dioxane. The reaction temperature is 0°C to 30°C. The reaction time is 1 to 8 hr.

Solvents usable in the step of the reaction with R¹⁴X include diethyl ether, tetrahydrofuran, 1,2-dimethoxyethane, dioxane, N,N-dimethylformamide, water, or mixed solvents thereof. The solvent is preferably a mixed solvent composed of N,N-dimethylformamide and water. Bases usable herein include sodium carbonate, potassium carbonate, sodium hydroxide, and potassium hydroxide. The base is preferably potassium carbonate. The reaction temperature is 20°C to 80°C. The reaction time is 1 to 16 hr.

Preferred imidoylating agents usable in the step of introducing formimidoyl include imidate hydrochlorides and EtOCH=NH·HCl. Solvents usable herein include methylene chloride, dichloroethane, acetonitrile, methanol, ethanol, and tetrahydrofuran. The solvent is preferably methanol. The reaction temperature is 0°C to 30°C. The reaction time is 1 to 12 hr.

Preferred amidinoylating agents usable in the step of introducing amidino include 1,3-bis(tert-butoxycarbonyl)-2-methyl-2-thiopseudourea. Solvents usable herein include tetrahydrofuran, dioxane, and N,N-dimethylformamide. The solvent is preferably N,N-dimethylformamide. Bases usable herein include triethylamine. Mercury chloride may be included as an additive. The reaction temperature is 0°C to 30°C. The reaction time is 1 to 6 hr.

Step S2
In step S2, a compound of general formula (S3) is produced by removing the protective group in the compound of general formula (S2). The protective group can be removed in the same manner as in step A8.

Process T
A compound of general formula (T4) is produced by introducing substituent R^{6'c} into the 6'-position of the compound of general formula (T1). This process comprises the following steps. The compound of general formula (T1) can be produced according to step G1 in the above process G.

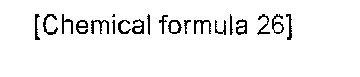

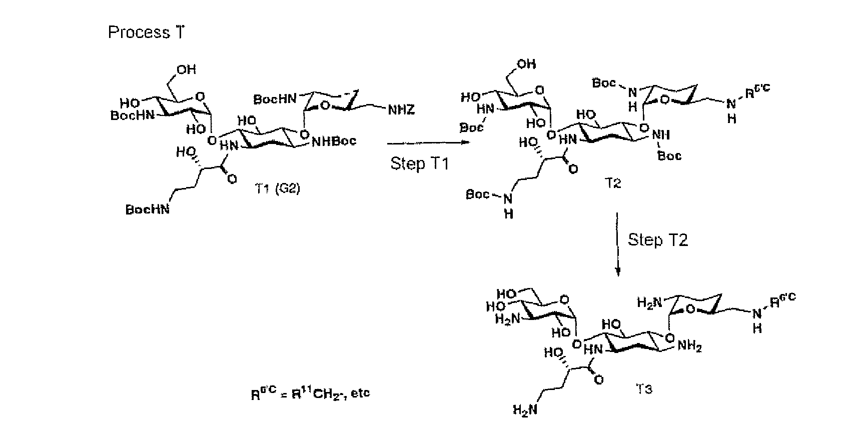

Step T1
In step T1, a compound of general formula (T2) is produced. This step is achieved by first removing the protective group at the 6'-position and then reacting the compound of general formula (T1) with R¹¹CHO wherein R¹¹ represents C₁₋₅ alkyl in which one or more hydrogen atoms in the alkyl group are optionally substituted by amino protected by tert-butoxycarbonyl, p-methoxybenzyloxycarbonyl or the like; or aryl, in the presence of a reducing agent, or by reacting the compound of general formula (T1) with a formimidoylating agent or an amidinoylating agent when the introduction of formimidayl and amidino is contemplated.

Solvents usable in the step of the reaction with R¹¹CHO include methanol, ethanol, isopropyl alcohol, dioxane, water, or mixed solvents thereof. The solvent is preferably a mixed solvent composed of methanol and dioxane. Reducing agents usable herein include sodium borohydride, sodium cyanoborohydride, lithium cyanoborohydride, and sodium triacetoxyborohydride. The reducing agent is preferably sodium borohydride. The reaction temperature is 0°C to 30°C. The reaction time is 1 to 8 hr.

Preferred imidoylating agents usable in the step of introducing formimidoyl include imidate hydrochlorides and EtOCH=NH·HCl. Solvents usable herein include methylene chloride, dichloroethane, acetonitrile, methanol, ethanol, and tetrahydrofuran. The solvent is preferably methanol. The reaction temperature is 0°C to 30°C. The reaction time is 1 to 12 hr.

Preferred amidinoylating agents usable in the step of introducing amidino include 1,3-bis(tert-butoxycarbonyl)-2-methyl-2-thiopseudourea. Solvents usable herein include tetrahydrofuran, dioxane, and N,N-dimethylformamide. The solvent is preferably N,N-dimethylformamide. Bases usable herein include triethylamine. Mercury chloride may be included as an additive. The reaction temperature is 0°C to 30°C. The reaction time is 1 to 6 hr.

Step T2
In step T2, a compound of general formula (T3) is produced by removing the protective group in the compound of general formula (T2). The protective group can be removed in the same manner as in step A8.

Process U
In process U, a compound of general formula (U4) is produced by introducing a side chain into the 6'-position of the compound of general formula (U1). This process comprises the following steps. The compound of general formula (U1) as a starting compound can be produced according to steps O1 and O2 in the above process O.

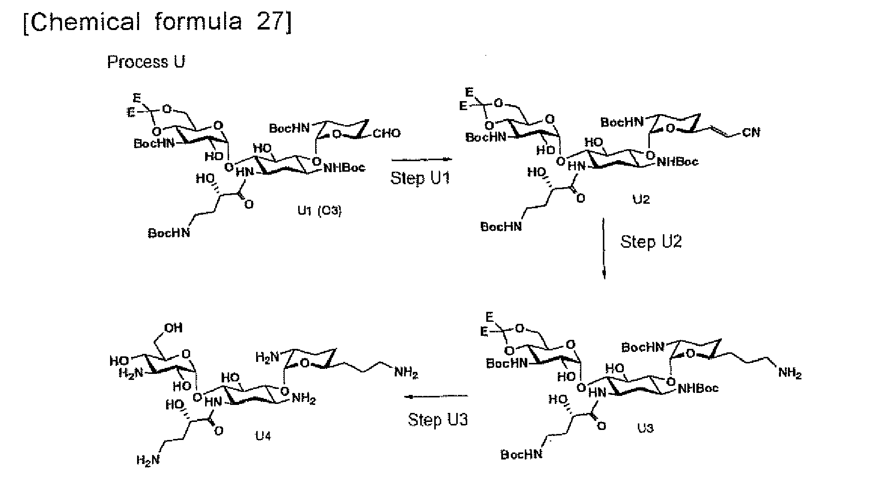

Step U1
In step U1, a compound of general formula (U2) is produced. This step is achieved by reacting the compound of general formula (U1) with Ph₃P=CHCN, or by reacting the compound of general formula (U1) with (EtO)₂P(O)CH₂CN in the presence of a base.

Solvents usable in the step of the reaction with Ph₃P=CHCN include methylene chloride, 1,2-dichloroethane, chloroform, benzene, and toluene. The solvent is preferably chloroform. The reaction temperature is 20°C to 60°C. The reaction time is 1 to 24 hr. Solvents usable in the step of the reaction with (EtO)₂P(O)CH₂CN include diethyl ether, tetrahydrofuran, 1,2-dimethoxyethane, dioxane, and N.N-dimethylformamide. The solvent is preferably tetrahydrofuran. Bases usable herein include sodium hydride, potassium hydride, and tert-BuOK. The base is preferably sodium hydride. The reaction temperature is 0°C to 30°C. The reaction time is 1 to 8 hr.

Step U2
In step U2, a compound of general formula (U3) is produced. This step is achieved by reacting the compound of general formula (U2) with a reducing agent. Reducing agents usable in this step include catalysts for catalytic hydrogen reduction used together with hydrogen, for example, palladium-carbon, palladium black, palladium hydroxide, and platinum oxide. Preferred are hydrogen and platinum oxide. Any solvent may be used without particular limitation so far as the solvent is inert to this reaction. Preferred solvents include methanol, ethanol, tetrahydrofuran, dioxane, and mixed solvents composed of these organic solvents and water. The reaction temperature is 10°C to 30°C. The reaction time is generally 1 to 8 hr.

Step U3
In step U3, a compound of general formula (U4) is produced by removing the protective group in the compound of general formula (U3). The protective group can be removed in the same manner as in step A8.

Process V
In process V, a compound of general formula (V6) is produced by introducing a side chain into the 6"-position of the compound of general formula (V1). This process comprises the following steps. The compound of general formula (V1) as a starting compound can be produced according to step A1 in the above process A.

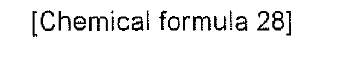

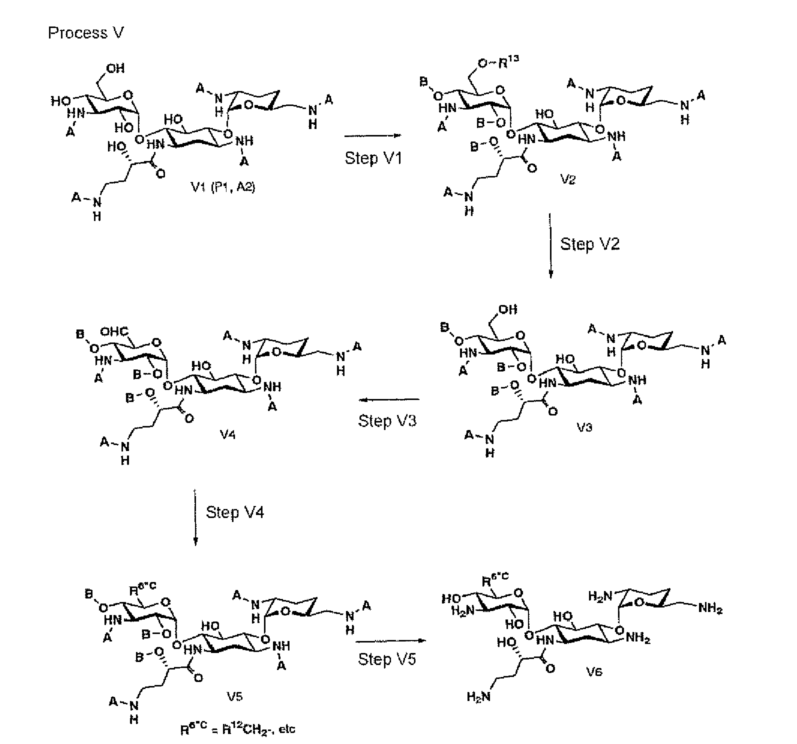

Step V1
In step V1, a compound of general formula (V2) is produced by protecting hydroxyl at the 6"-position of the compound of general formula (V1) by triphenylmethyl or silyl and then protecting the remaining hydroxyl in the compound by acyl. This step is achieved by reacting the compound of general formula (V1) with R¹³Cl wherein R¹³ represents triphenylmethyl, tert-butyldimethylsilyl, triisopropylsilyl, or tert-butyldiphenylsilyl in the presence of a base, and then reacting the resultant compound with B₂O or BCl wherein B represents acetyl or benzoyl in the presence of a base.

Solvents usable in the step of introducing triphenylmethyl include methylene chloride, acetonitrile, and pyridine. The solvent is preferably pyridine. Bases usable herein include triethylamine, pyridine, and 4-dimethylaminopyridine. The base is preferably pyridine. The reaction temperature is 20°C to 80°C. The reaction time is generally 2 to 10 hr.

Preferred solvents usable in the step of introducing silyl include methylene chloride, chloroform, dimethylformamide, acetonitrile, and pyridine. Bases usable herein include 4-dimethylaminopyridine, triethylamine, imidazole, and diisopropylethylamine. The base is preferably 4-dimethylaminopyridine. The reaction temperature is 0°C to 30°C. The reaction time is 1 to 12 hr.

Solvents usable in the step of introducing acyl include pyridine, N,N-dimethylformamide, methylene chloride, and chloroform. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step V2
In step V2, a compound of general formula (V3) is produced by removing the protective group of hydroxyl, that is, triphenylmethyl or silyl, at the 6"-position of the compound of general formula (V2). This step is achieved by reacting the compound of general formula (V2) with an acid or a base.

Solvents usable in the step of deprotection of triphenylmethyl group include diethyl ether, tetrahydrofuran, and water. The solvent is preferably diethyl ether. Acids usable herein include formic acid and acetic acid. The acid is preferably formic acid. The reaction temperature is 0°C to 30°C. The reaction time is 1 to 8 hr.

Preferred solvents usable in the step of deprotection of silyl group include acetonitrile, tetrahydrofuran, and methylene chloride. Reagents usable in the deprotection include tetrabutylammonium fluoride, hydrogen fluoride-pyridine, hydrogen fluoride-triethylamine, and hydrogen fluoride. The reaction temperature is 0°C to 30°C. The reaction time is 1 to 12 hr.

Step V3
In step V3, hydroxyl at the 6"-position of the compound of general formula (V3) is oxidized to aldehyde. This step is achieved by reacting the compound of general formula (V3) with an oxidizing agent. The solvent used in this step is preferably pyridine. Dimethylsulfoxide, dicyclohexylcarbodiimide, and a hydrogen donating compound may be used in combination as the oxidizing agent. Hydrogen donating compounds usable herein include phosphoric acid and trifluoroacetic acid. The hydrogen donating compound is preferably trifluoroacetic acid. The reaction temperature is 0°C to 30°C. The reaction time is 6 to 24 hr.

Step V4
In step V4, a compound of general formula (V5) is produced. This step can be achieved by reacting the compound of general formula (V4) with nitromethane in the presence of a base and then reacting the resultant nitro compound with a reducing agent. This step can also be achieved by reacting, in the presence of a reducing agent, the compound of general formula (V4) with R¹²NH₂ wherein R¹² represents C₁₋₆ alkyl, wherein one or more hydrogen atoms in the alkyl group are optionally substituted by amino protected by tert-butoxycarbonyl, p-methoxybenzyloxycarbonyl or the like, or by reactiong, in the presence of a reducing agent, the compound of general formula (V4) with morpholine.

Solvents usable in the step of the reaction with nitromethane include methanol, ethanol, tert-butyl alcohol, methylene chloride, dichloroethane, and mixed solvents thereof. The solvent is preferably a mixed solvent composed of methanol and methylene chloride. Bases usable herein include sodium methoxide, sodium ethoxide, and tert-BuOK. The base is preferably sodium methoxide. The reaction temperature is 0°C to 30°C. The reaction time is 1 to 6 hr. Reducing agents usable in the next step of reduction include hydrogen and catalysts for catalytic hydrogen reduction such as palladium-carbon, palladium black, palladium hydroxide, and platinum oxide. Preferred are hydrogen and platinum oxide. Any solvent may be used without particular limitation so far as the solvent is inert to this reaction. Preferred are methanol, ethanol, tetrahydrofuran, dioxane, and mixed solvent composed of these organic solvents and water. The reaction temperature is 10°C to 30°C. The reaction time is generally 1 to 8 hr.

Reducing agents usable in the step of the reaction with the amino compound include sodium borohydride, sodium cyanoborohydride, lithium cyanoborohydride, and sodium triacetoxyborohydride. The reducing agent is preferably sodium borohydride. Solvents usable herein include methanol, ethanol, isopropyl alcohol, dioxane, water, or mixed solvents thereof. The solvent is preferably a mixed solvent composed of methanol and dioxane. The reaction temperature is 0°C to 30°C. The reaction time is 1 to 8 hr.

Step V5
In step V5, a compound of general formula (V6) is produced by removing the protective group in the compound of general formula (V5). The protective group can be removed in the same manner as in step A8.

Process W
In process W, a compound of general formula (W5)
wherein R^{6"c} represents azidomethyl or aminomethyl, is produced by introducing a side chain into the 6"-position of the compound of general formula (W1). This process comprises the following steps. The compound of the general formula (W1) as a starting compound can be produced according to step A1 in the above process A.

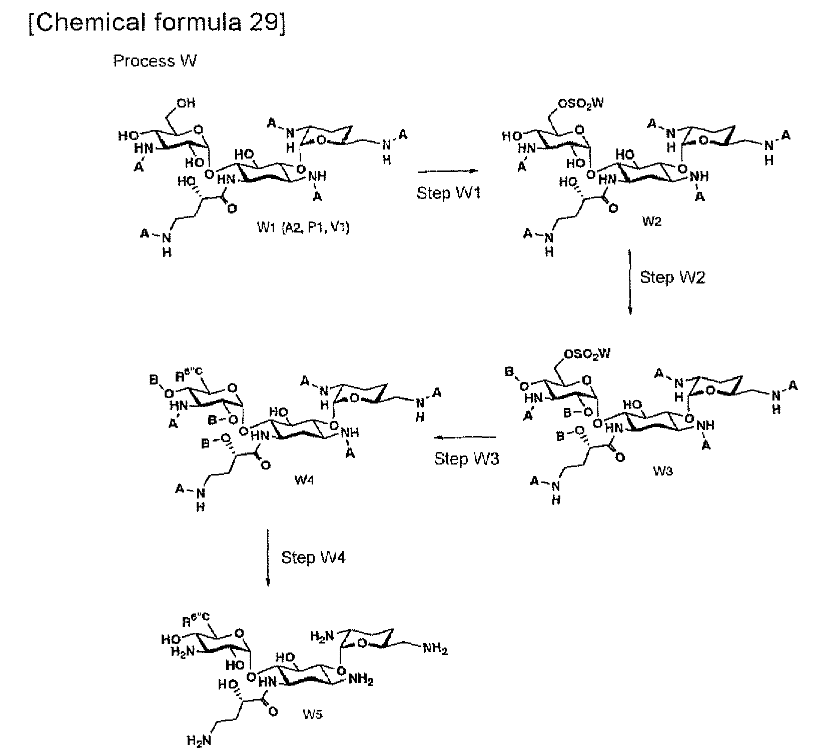

Step W1
In step W1, a compound of general formula (W2) is produced. This step is achieved by reacting the compound of general formula (W1) with WSO₂Cl wherein W represents methyl, phenyl, or p-tolyl.

Solvents usable in this step include, for example, methylene chloride, chloroform, 1,2-dichloroethane, tetrahydrofuran, acetonitrile, and ethyl acetate. The solvent is preferably methylene chloride. Bases usable herein include pyridine, triethylamine, diisopropylethylamine, and 4-dimethylaminopyridine. The base is preferably 4-dimethylaminopyridine. The reaction temperature is generally 0°C to 30°C. The reaction time is 1 to 24 hr.

Step W2
In step W2, a compound of general formula (W3) is produced by introducing a protective group into hydroxyl at the 2"-position, 4"-position, and 2"'-position of the compound of general formula (W2). This step is achieved by reacting the compound of general formula (W2) with B₂O or BCl wherein B represents acetyl or benzoyl in the presence of a base.

Solvents usable in this step include pyridine, N,N-dimethylformamide, methylene chloride, and chloroform. The solvent is preferably pyridine. Bases usable herein include triethylamine, pyridine, and 4-dimethylaminopyridine. The base is preferably pyridine. The reaction temperature is 0°C to 30°C. The reaction time is 1 to 8 hr.

Step W3
In step W3, a compound of general formula (W4) is produced. This step is achieved by reacting the compound of general formula (W3) with sodium azide and then reacting the resultant azide compound with a reducing agent.

Solvents usable in the step of azidation include tetrahydrofuran, dioxane, 1,2dimethoxyethane, N,N-dimethylformamide, and dimethylsulfoxide. The solvent is preferably N,N-dimethylformamide. The reaction temperature is 60°C to 90°C. The reaction time is 1 to 12 hr.

Reducing agents usable in the step of reduction include catalysts for catalytic hydrogen reduction used together with hydrogen, for example, palladium-carbon, palladium black, palladium hydroxide, and platinum oxide. Preferred are hydrogen and palladium-carbon. Any solvent may be used without particular limitation so far as the solvent is inert to this reaction. Preferred solvents include methanol, ethanol, tetrahydrofuran, dioxane, and mixed solvents composed of these organic solvents and water. The reaction temperature is 10°C to 30°C. The reaction time is generally 1 to 8 hr.

Step W4
In step W4, a compound of general formula (W5) is produced by removing the protective group in the compound of general formula (W4). The protective group can be removed in the same manner as in step A8.

Salt
The compounds according to the present invention may form salts. Such salts include pharmaceutically acceptable nontoxic salts. Specific examples thereof include hydrohalogenic acid salts such as hydrofluoric acid salt, hydrochloric acid salts, hydrobromic acid salts, and hydroiodic acid salts, inorganic acid salts such as sulfuric acid salts, nitric acid salts, phosphoric acid salts, perchloric acid salts, and carbonic acid salts, carboxylic acid salts such as acetic acid salts, trichloroacetic acid salts, trifluoroacetic acid salts, hydroxyacetic acid salts, lactic acid salts, citric acid salts, tartaric acid salts, oxalic acid salts, benzoic acid salts, mandelic acid salts, butyric acid salts, maleic acid salts, propionic acid salts, forming acid salts, and malic acid salts, amino acid salts such as alginic acid salts, aspartic acid salts, and glutamic acid salts, and organic acid salts such as methanesulfonic acid salts and p-toluenesulfonic acid salts. Preferred are inorganic acid salts such as sulfuric acid salts.

Solvate
The compounds according to the present invention may form solvates. Preferred solvates include, for example, hydrates and ethanolates,

Antimicrobial activity
The compounds according to the present invention or pharmacologically acceptable salts or solvates thereof have potent antimicrobial activity against bacteria causative of infectious diseases, for example, MRSAs, Staphylococcus aureus, Escherichia coli, and Pseudomonas aeruginosa and thus can be used as antimicrobial agents, especially anti-MRSA agents. Thus, according to another aspect of the present invention, there is provided use of a compound according to the present invention or a pharmacologically acceptable salt or solvate thereof, for the manufacture of an antimicrobial agent.

Medicament
The compounds according to the present invention or pharmacologically acceptable salts or solvates thereof may also be utilized as medicaments. Specifically, the compounds according to the present invention or pharmacologically acceptable salts or solvates thereof may be used for preventing or treating infectious diseases. Such infectious diseases include, for example, septicemia, infectious endocarditis, dermatological infections, surgical infections, orthopaedic infections, respiratory tract infections, urinary-tract infections, enteric infections, peritonitis, meningitis, ophthalmologic infections, and otologic infections. More specific examples of infectious diseases include skin suppuration, burn/operation wound secondary infections, pneumonia, endobronchial infections, tuberculosis, pyelonephritis, enteritis including food poisoning, conjunctivitis, and tympanitis. Thus, according to another aspect of the present invention, there is provided a composition, especially a pharmaceutical composition, comprising a compound according to the present invention or a pharmacologically acceptable salt or solvate thereof. Further, according to still another aspect of the present invention, there is provided a method for treating or preventing an infectious disease, comprising the step of administering a compound according to the present invention or a pharmacologically acceptable salt or solvate thereof to an animal including a human. According to a further aspect of the present invention, there is provided use of a compound according to the present invention or a pharmacologically acceptable salt or solvate thereof, for the manufacture of a pharmaceutical composition.

Pharmaceutical compositions comprising compounds of the present invention or pharmacologically acceptable salts thereof as an active ingredient can be administered to all mammals including humans orally or parenterally by administration routes, for example, intravenous administration, intramuscular administration, subcutaneous administration, rectal administration, percutaneous administration, ocular topical administration, or pulmonic administration. Therefore, the pharmaceutical composition comprising a compound according to the present invention as an active ingredient may be formulated into suitable dosage forms according to the administration routes. Specifically, the pharmaceutical composition may be mainly formulated into any of injections such as intravenous injections and intramuscular injections, oral preparations such as capsules, tablets, granules, powders, pills, fine subtles, and troches, ointments, eye drops, dry powders, or atomized aerosol formulations. These preparations may be prepared by conventional methods, for example, with commonly used additives for preparations, such as excipients, extenders, binders, wetting agents, disintegrants, surfactants, lubricants, dispersants, buffering agents, preservatives, solubilizers, antiseptics, corrigents, deodorants, soothing agents, stabilizers, tonicity adjusting agents, and pH adjustors. Nontoxic additives usable herein include, for example, lactose, D-mannitol, fructose, glucose, starches, gelatin, methylcellulose or its salts, gum arabics, polyethylene glycols, syrup, vaseline, lanoline, glycerin, ethanol, propylene glycol, citric acid or its salts, sodium chloride, sodium sulfite, benzalconium chloride, sodium phosphate, methyl p-oxybenzoate, propyl p-oxybenzoate, β-cyclodextrin, hydroxypropyl-β-cyclodextrin, Tween 80, sodium hydroxide, and sulfuric acid. The dose may be appropriately determined in consideration of particular conditions, for example, dose regimen, the age, sex, and severity of condition of patients.

### EXAMPLES

The present invention is further illustrated by the following Examples that are not intended as a limitation of the invention.
In the following Examples, arbekacin, 3,2',6'-tri-N-t-butoxycarbonyl-4"'-N-p-methoxybenzyloxycarbonylarbekacin, and 3,2',6'-tri-N-t-butoxycarbonyl-3"-trifluoroacetyl-dibekacin were synthesized according to the method described in Japanese Patent Laid-Open No. 164696/1980. 2',3,6'-Tri-N-(t-butoxycarbonyl)-3"-N-trifluoroacetyl-3',4'-didehydrodibekacin was synthesized using 3',4'-didehydrodibekacin according to the method described in Japanese Patent Laid-Open No. 164696/1980. 2',3,6'-Tri-N-(t-butoxycarbonyl)-3"-N-trifluoroacetyl-3',4'-didehydrodibekacin was synthesized, by using 3',4'-didehydradibekacin, according to the method described in Japanese Patent Laid-Open No. 164696/1980. 3',4'-Didehydroarbekacin was synthesized according to the method described in Japanese Patent Publication No. 10719/1988. 3,2',6',3"-Tetra-N-t-butoxycarbonyl-3',4'-didehydro-4"'-p-methoxybenzyl-oxycarbonylarbekacin was synthesized according to the method described in Japanese Patent Laid-Open No. 81897/1980.

Example 1

### 5,4"-Diepiarbekacin

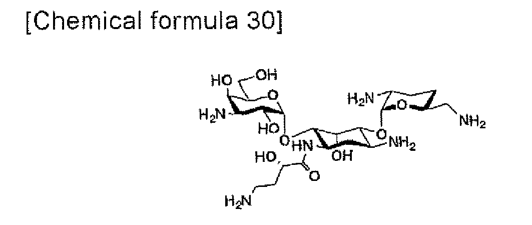

Production step 1-(a)
N,N-Dimethylformamide (900 mL) was added to a solution of 100 g of arbekacin dissolved in 450 mL of water. Di-t-butyl dicarbonate (250 g) was added thereto under an ice bath, and the mixture was stirred at room temperature overnight. Ethyl acetate was added to the reaction solution, and the mixture was washed with a saturated aqueous sodium hydrogencarbonate solution and a saturated aqueous sodium chloride solution in that order and was dried over anhydrous magnesium sulfate. This solution was concentrated to dryness to give 188 g of the following compound as a solid.

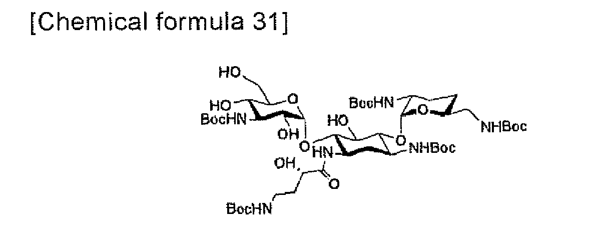

Production step 1-(b)
The compound (40 g) prepared in production step 1-(a) was dissolved in 360 mL of N,N-dimethylformamide, 11.6 mL of 1,1-dimethoxycyclohexane and 1.3 g of p-toluenesulfonic acid monohydrate were added to the solution, and the mixture was allowed to react under conditions of 50°C and 46 to 48 mbar for 5 hr. Ethyl acetate was added thereto, and the mixture was washed with water and was concentrated to dryness to give 45 g of the following compound. 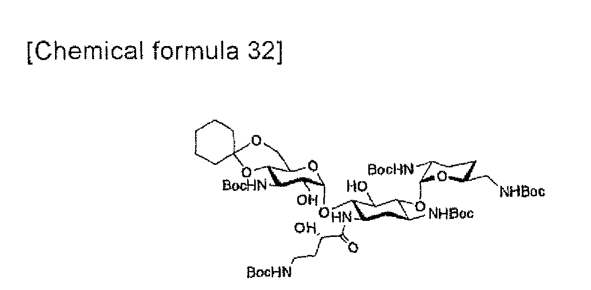 FABMS: m/z 1155 [M+Na]⁺, 1171 [M+Na]⁺.

Production step 1-(c)
The compound (25 g) prepared in production step 1-(b) was dissolved in 500 mL of pyridine, and benzoyl chloride was added dropwise to the solution at an internal temperature of 4 to 6°C over a period of 20 min. This solution was stirred for 2 hr while maintaining the internal temperature of the solution at 4 to 6°C, and the temperature was raised to room temperature, followed by stirring for 1 hr. Water (0.75 mL) was added to this solution, and the mixture was concentrated under the reduced pressure. Ethyl acetate was added to the residue, and the mixture was washed with water, a 10% aqueous potassium hydrogensulfate solution, a saturated aqueous sodium hydrogencarbonate solution, and a saturated aqueous sodium chloride solution in that order, dried over anhydrous magnesium sulfate, and then concentrated to dryness to give 30 g of the following compound. 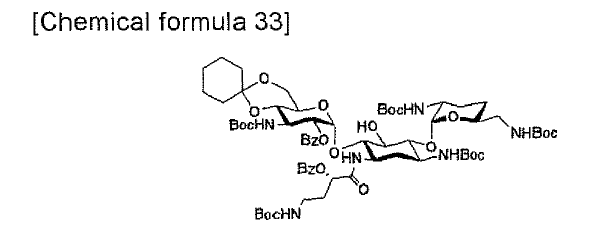 FABMS: m/z 1363 [M+Na]⁺, 1379 [M+K]⁺.

Production step 1-(d)
The compound (11.6 g) prepared in production step 1-(c) was dissolved in 150 mL of methylene chloride. 4-Dimethylaminopyridine (18 g) was added to the solution at room temperature, 4.5 mL of mesyl chloride was added thereto under an ice bath, and the mixture was stirred under an ice bath for 1 hr. The temperature was then raised to room temperature before stirring for 4 hr. Water (100 mL) was added to this reaction solution under an ice bath, and 240 mL of methylene chloride was added thereto. This solution was washed with a 10% aqueous potassium hydrogensulfate solution, a saturated aqueous sodium hydrogencarbonate solution, and a saturated aqueous sodium chloride solution in that order and was dried over anhydrous magnesium sulfate. This solution was concentrated under the reduced pressure, and the residue was purified by column chromatography on silica gel (hexane : ethyl acetate = 1 : 1 to 1 : 2) to give 7.4 g of the following compound.

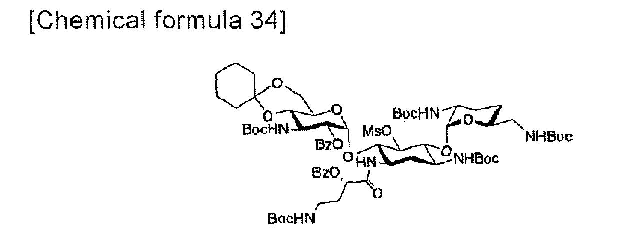

Production step 1-(e)
The compound (6.0 g) prepared in production step 1-(d) was dissolved in 60 mL of N,N-dimethylformamide. Cesium acetate (6.4 g) dried at 120°C in a sample dryer for 2 hr was added to the solution, and a reaction was allowed to proceed at 100°C for 2 hr. This solution was cooled to room temperature and was concentrated under the reduced pressure. Methylene chloride (300 mL) was added to the residue, and the mixture was washed with water and was dried over anhydrous magnesium sulfate. The solution was then concentrated under the reduced pressure, and the residue was purified by column chromatography on silica gel (hexane : ethyl acetate = 1 : 1 to 2 : 3) to give 4.6 g of the following compound. 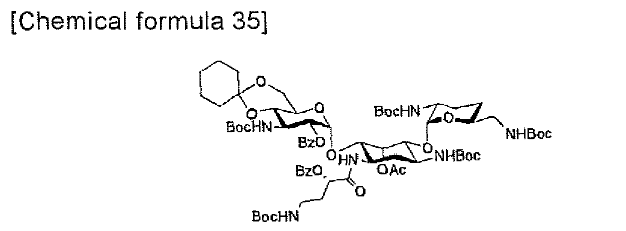 FABMS: m/z 1405 [M+Na]⁺, 1421 [M+K]⁺.

Production step 1-(f)
The compound (1.13 g) prepared in production step 1-(e) was dissolved in a mixed solution composed of 40 mL of methylene chloride and 4 mL of methanol. A 90% aqueous trifluoroacetic acid solution (4 mL) was added to the solution, and the mixture was stirred for 1 hr. Water (20 mL) was added to the reaction solution, and the organic layer was washed with a saturated aqueous sodium hydrogencarbonate solution, dried over anhydrous magnesium sulfate, and then concentrated under the reduced pressure to give 1.03 g of the following compound.

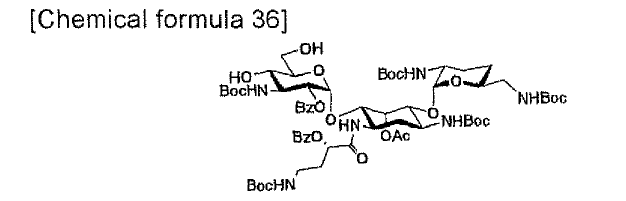

Production step 1-(g)
The compound (1.03 g) prepared in production step 1-(f) was dissolved in 8 mL of pyridine. Triphenylmethyl chloride (0.56 g) was added to the solution, and the mixture was stirred at 60°C overnight. After the completion of this reaction, 0.15 mL of methanol was added thereto, and the mixture was stirred for 1 hr. This reaction solution was extracted with ethyl acetate, dried over anhydrous magnesium sulfate, and then concentrated under the reduced pressure to give 0.99 g of the following compound. 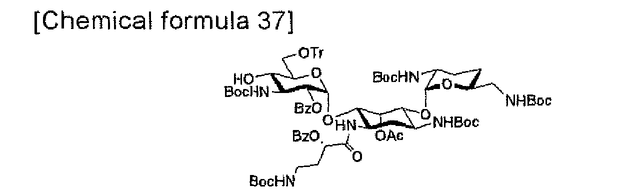 FABMS: m/z 1567 [M+Na]⁺.

Production step 1-(h)
The compound (0.84 g) prepared in production step 1-(g) was dissolved in 8 mL of methylene chloride, and 0.54 mL of pyridine was added to the solution. The mixture was cooled to -18°C, 0.24 mL of trifiluoramethanesulfonic anhydride was added thereto, and the mixture was stirred at -5°C for 2 hr. After six drops of methanol were added to this reaction solution, the mixture was extracted with ethyl acetate. The organic layer was washed with ice water, a saturated aqueous sodium hydrogencarbonate solution, and a 10% aqueous potassium hydrogensulfate solution, dried over anhydrous magnesium sulfate, and then concentrated under the reduced pressure. Further, toluene was added to the residue, and the mixture was concentrated under the reduced pressure. The residue was dissolved in 9 mL of N,N-dimethylformamide. Cesium acetate (0.59 g) dried at 120°C for 2 hr in a sample dryer was added to this solution, and the mixture was allowed to react at room temperature for 2 hr. This reaction solution was extracted with ethyl acetate, and the extract was dried over anhydrous magnesium sulfate to give 0.93 g of the following compound.

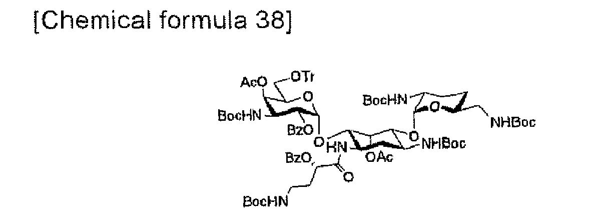

Production step 1-(i)
The compound (0.92 g) prepared in production step 1-(h) was dissolved in 15 mL of methylene chloride, 5 mL of a 0.5 M sodium methoxide-methanol solution was added to the solution, and the mixture was stirred at room temperature for 3 hr. Dry ice was added to the reaction solution, and the mixture was stirred for 30 min and concentrated under the reduced pressure to give the following compound (0.72 g) as a crude product.

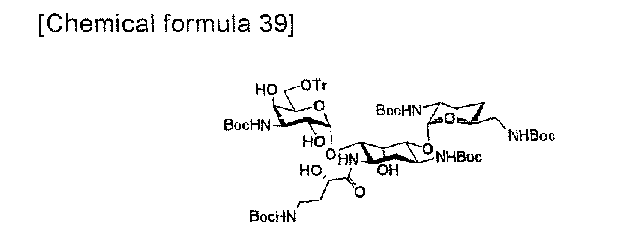

Production step 1-(j)
A 90% aqueous trifluoroacetic acid solution (15 mL) was added to 0.72 g of the crude product prepared in production step 1-(i), and the mixture was stirred at room temperature overnight. Water (10 mL) was added to the reaction solution, and the aqueous layer was washed three times with 5 mL of diethyl ether. The aqueous layer was neutralized and adjusted to pH 7 by the addition of aqueous ammonia and was purified by Bio-Rex70(NH₄⁺, 110 mL, 100-200 mesh) to give the title compound: 5,4"-diepiarbekacin (0.16 g).
TSPMS: m/z 553 [M+H]⁺;
¹H-NMR (D₂O+ND₃) δ: 1.54 (1 H, m), 1.60 (1 H, m), 1.93 (4H, m), 2.11 (1H, m), 2.22 (1 H, ddd, J = 4.4, 4.6, 12.9 Hz), 2.85 (2H, m), 2.94 (2H, m), 3.02 (1 H, m), 3.17 (1 H, dd, J = 2.9, 10.7 Hz), 3.41 (1 H, m), 3.67 (1H, dd, 2.4, 10.0 Hz), 3.76 (1 H, dd, J = 3.9, 10.7 Hz), 3.92 (2H, m), 4.00 (1 H, dd, J = 2.6, 10.5 Hz), 4.02 (1 H, m), 4.07 (1 H, brd), 4.27 (1 H, m), 4.38 (1 H, dd, J = 3.6, 9.2 Hz), 4.46 (1 H, m), 4.75 (1H, dd, J = 2.3 Hz), 5.14 (1 H, d, J = 3.4 Hz), 5.27 (1 H, d, J = 3.9 Hz).

Example 2

### 5-Deoxy-4"-epi-5-epifluoroarbekacin

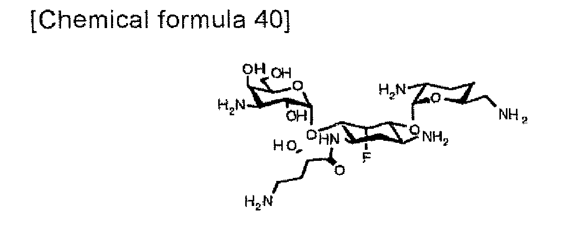

Production step 2-(a)
The compound (2.5 g) prepared in production step 1-(b) of Example 1 was dissolved in 7.0 mL of pyridine, 3.0 mL of acetic anhydride was added to the solution, and the mixture was stirred at room temperature overnight. The reaction solution was extracted with ethyl acetate, dried over anhydrous magnesium sulfate, and was then concentrated under the reduced pressure. The residue was purified by column chromatography on silica gel (hexane : ethyl acetate = 1 : 2) to give 1.9 g of the following compound. 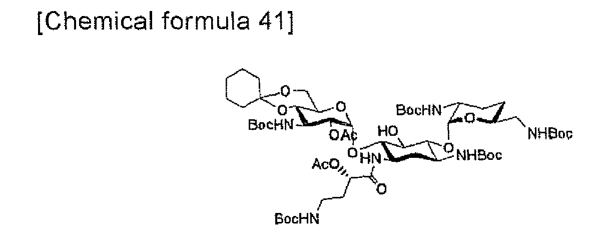 ESIMS: m/z 1217 [M+H]⁺.

Production step 2-(b)
1.0 g of the compound prepared in production step 2-(a) was used in the same manner as in production step 1-(f) in Example 1 to give the following compound (0.91 g). 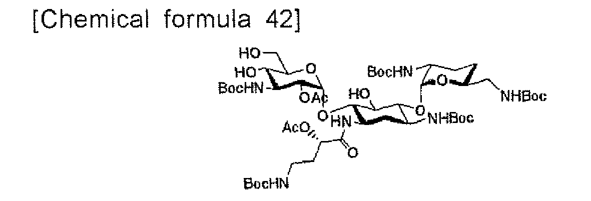 FABMS: m/z 1159 [M+Na]⁺

Production step 2-(c)
2.4 g of the compound prepared in production step 2-(b) was used in the same manner as in production step 1-(g) to give the following compound (2.2 g). 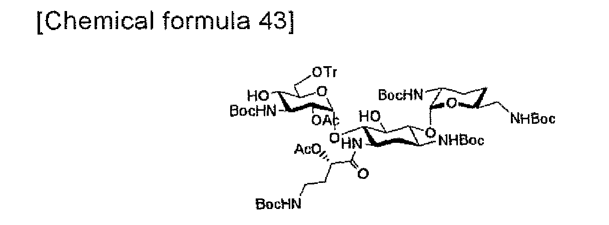 FABMS: m/z 1379 [M+H]⁺.

Production step 2-(d)
0.74 g of the compound prepared in production step 2-(c) was used in the same manner as in production step 1-(h) in Example 1 to give the following compound (0.25 g). 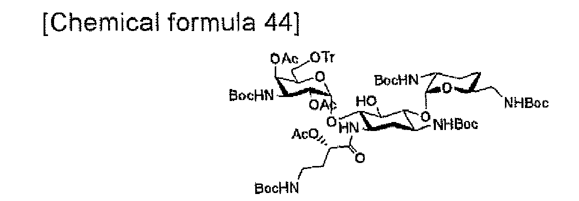 FABMS: m/z 1443 [M+Na]⁺.

Production step 2-(e)
The compound (0.18 g) prepared in production step 2-(d) was dissolved in 5.0 mL of methylene chloride. Under cooling at -50°C, 0.058 mL of diethylamino sulfur trifluoride (DAST) was added to the solution, and the mixture was stirred at room temperature for 3 hr. Thereafter, under ice cooling, 5 mL of saturated aqueous sodium hydrogencarbonate solution was added thereto, and the mixture was stirred for 5 min and was extracted with ethyl acetate. The extract was dried over anhydrous magnesium sulfate and was then concentrated under the reduced pressure to give the following compound as a crude product. The crude product as such was used in the next step, production step 2-(f), without isolation and purification.

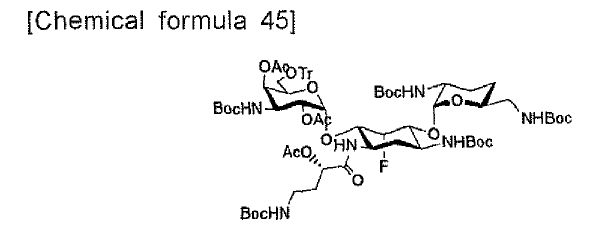

Production step 2-(f)
170 mg of the crude product prepared in production step 2-(e) was used in the same manner as in production steps 1-(i) and (j) in Example 1 to give the title compound: 5-deoxy-4"-epi-5-epifluoroarbekacin (7.3 mg).
FABMS: m/z 555 [M+H]⁺;
¹H-NMR (D₂O+ND₃) δ: 1.88 (2H, m), 2.12 (4H, m), 2.20 (3H, m), 2.37 (1H, m), 2.52 (1H, m), 3.12 (2H, m), 3.23 (3H, m), 3.40 (1 H, dd, J = 3.0, 10.7 Hz), 3.66 (1 H, m), 4.03 (1H, dd, J = 3.9, 10.7 Hz), 4.03 (1H, dd, J = 10.3, 27.1 Hz), 4.18 (2H, m), 4.28 (1 H, m), 4.32 (1 H, m), 4.38 (1 H, dd, J = 11.0, 27.3 Hz), 4.52 (1H, m), 4.63 (1H, dd, J = 3.6, 9.3 Hz), 4.67 (1H, ddd, J = 4.7, 11.0 Hz), 5.43 (1 H, d, J = 3.2 Hz), 5.52 (1 H, d, J = 3.6 Hz), 5.88 (1 H, d, J = 52.2 Hz).

Example 3

### 5-Deoxy-4"-epi-5-epichloroarbekacin

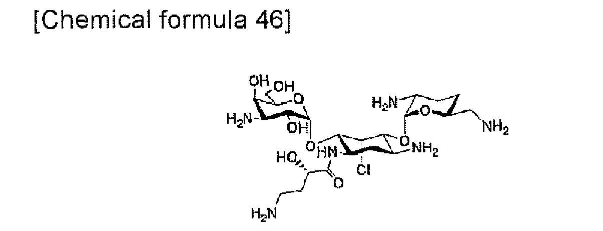

### Production step 3-(a)

0.28 g of the compound prepared in production step 2-(d) was used in the same manner as in production step 1-(d) to give the following compound (0.21 g).

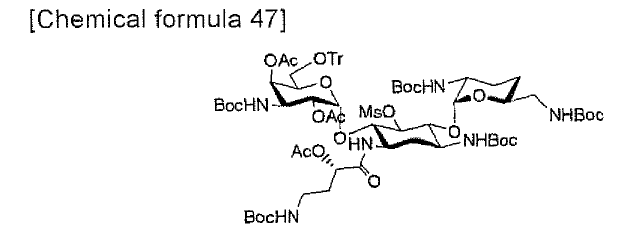

Production step 3-(b)
0.21 g of the compound prepared in production step 3-(a) was used in the same manner as in production step 1-(e) in Example 1 except that lithium chloride was used instead of cesium acetate, to give the following compound (0.13 g). 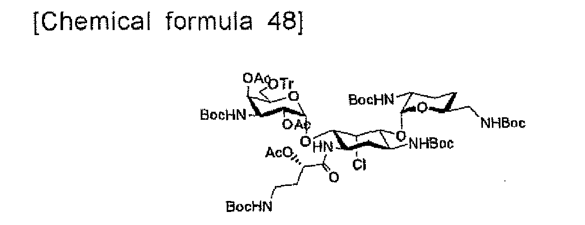 FABMS: m/z 1461 [M+Na]⁺, 1477 [M+K]⁺.

Production step 3-(c)
130 mg of the compound prepared in production step 3-(b) was used in the same manner as in production steps 1-(i) and 1-(j) to give the title compound: 5-deoxy-4"-epi-5-epichloroarbekacin (14.0 mg) FABMS: m/z 571 [M+H]⁺
¹H-NMR (D₂O+ND₃) δ: 1.60 (2H, m), 1.92 (4H, m), 2.11 (1 H, m), 2.26 (1 H, m), 2.85 (2H, m), 2.98 (3H, m), 3.16 (1 H, dd, J = 2.9, 10.7 Hz), 3.52 (1 H, m), 3.77 (1 H, dd, J = 3.9, 10.7 Hz), 3.92 (3H, m), 4.02 (1 H, m), 4.07 (1H, brd, J = 2.9 Hz), 4.24 (1 H, dd, J = 3.1, 10.5 Hz), 4.33 (1 H, m), 4.38 (1 H, dd, J = 3.6, 9.2 Hz), 4.53 (1 H, m), 5.10 (1 H, d, J = 3.4 Hz), 5.23 (1 H, brs, J = 3.7 Hz), 5.27 (1 H, d, J = 3.9 Hz).

Example 4

### 5-Deoxy-4"-epi-5-epiazidoarbekacin

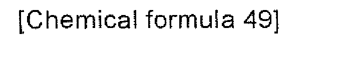

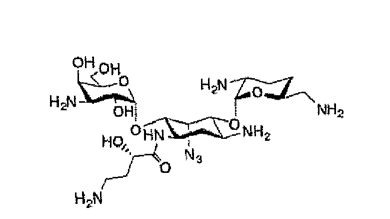

### Production step 4-(a)

100 mg of the compound prepared in production step 3-(a) was used in the same manner as in production step 1-(e) except that sodium azide was used instead of cesium acetate, to give the following compound (57 mg). 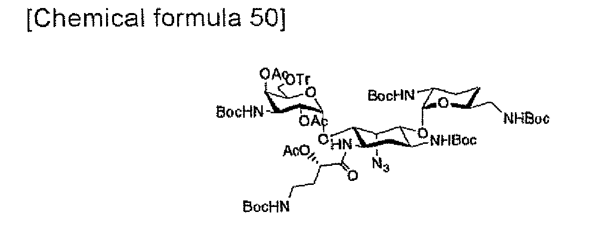 FABMS: m/z 1446 [M+H]⁺.

Production step 4-(b)
The compound (52 mg) prepared in step 4-(a) was used in the same manner as in production steps 1-(i) and 1-(j) to give the title compound: 5-deoxy-4"-epi-5-epiazidoarbekacin (6.3 mg).
FABMS: m/z 578 [M+H]⁺
¹H-NMR (D₂O+ND₃) δ: 1.54 (1H, m), 1.60 (1H, m), 1.92 (4H, m), 2. 12 (1 H, m), 2.19 (1H, ddd, J = 4.5, 12.9 Hz), 2.86 (2H, m), 2.95 ( 2H, m), 3.04 (1H, m), 3.14 (1H, dd, J = 3.0, 10.7 Hz), 3.34 (1H, m), 3.78 (2H, m), 3.95 (2H, m), 4.00 (1H, m), 4.09 (2H, brd), 4.24 (2H, m), 4.37 (1H, dd, J = 3.6, 9.3 Hz), 4.46 (1H, ddd, J = 3.6, 1 1.5 Hz), 4.61 (1 H, brs), 5.16 (1 H, d, J = 3.4 Hz), 5.30 (1 H, d, J = 3.9 Hz).

Example 5

### 5-Deoxy-4"-epi-5-epiaminoarbekacin

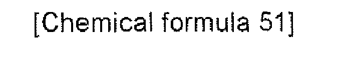

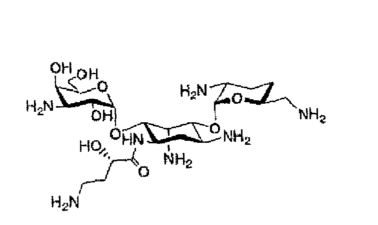

### Production step 5-(a)

The compound produced in Example 4: 5-deoxy-4"-epi-5-epiazidoarbekacin (8.3 mg) was dissolved in 5.0 mL of water. Under an argon gas stream, 10°/o Pd-C (8.0 mg) was added thereto. The air in the system was then replaced by hydrogen and the mixture was stirred at room temperature for 5 hr. The reaction solution was filtered through Celite and was then purified by CM-Sephadex (NH₄⁺) to give the title compound: 5-deoxy-4"-epi-5-epiaminoarbekacin (5.7 mg).
FABMS: m/z 552 [M+H]⁺
¹H-NMR (D₂O+ND₃): δ 1.57 (1H, m), 1.62 (1H, m), 1.97 (4H, m), 2.15 (1 H, m), 2.23 (1 H, m), 2.89 (2H, m), 2.98 (2H, m), 3.05 (1 H, m), 3.20 (1 H, dd, J = 3.2, 11.0 Hz), 3.47 (1H, ddd, J = 4.4, 11.7 Hz), 3.74 (1 H, dd, J = 3.4, 10.3 Hz), 3.79 (1H, dd, J = 3.9, 11.0 Hz), 3.94 (2H, m), 4.04 (2H, m), 4.12 (2H, m), 4.22 (1 H, dd, J = 6.1 Hz), 4.41 (1 H, dd, J = 3.4, 9.3 Hz), 4.52 (1H, ddd, J = 3.6, 7.3 Hz), 5.16 (1H, d, J = 3.2 Hz), 5.30 (1 H, d, J = 3.9 Hz).

Example 6

### 5-Deoxy-4"-epi-5-epi(2-hydroxyethyl)aminoarbekacin

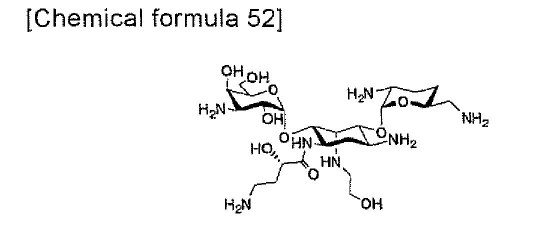

### Production step 6-(a)

The compound (100 mg) produced in production step 3-(a) was used in the same manner as in production step 1-(e) except that 2-aminoethanol instead of cesium acetate, to give the following compound as a crude product. This crude product was used in production step 6-(b) without isolation and purification.

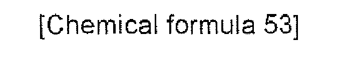

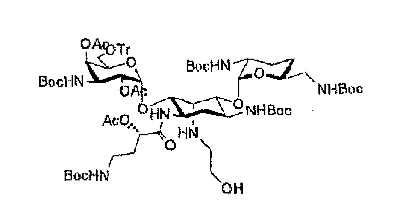

Production step 6-(b)
72 mg of the compound produced in production step 6-(a) was used in the same manner as in production steps 1-(i) and 1-(j) to give the title compound: 5-deoxy-4"-epi-5-epi(2-hydroxyethyl)aminoarbekacin (13 mg).
FABMS: m/z 596 [M+H]⁺
¹H-NMR (D₂O+ND₃): δ 1.56 (1H, m), 1.65 (1H, m), 1.99 (4H, m), 2.17 (1H, m), 2.26 (1H, ddd, J = 4.9, 13.2 Hz), 2.90 (2H, m), 3.00 (2H, m), 3.08 (2H, m), 3.17 (1 H, m), 3.27 (1 H, m), 3.46 (1 H, m), 3.80 (1 H, m), 4.00 (5H, m), 4.13 (1 H, m), 4.15 (1 H, dd, J = 3.2, 11.0 Hz), 4.27 (1 H, dd, J = 5.8, 5.9 Hz), 4.41 (1 H, dd, J = 3.4, 9.2 Hz), 4.65 (1H, ddd, J = 4.2, 11.7 Hz), 5.17 (1H, d, J = 3.2 Hz), 5.30 (1 H, d, J = 3.9 Hz).

Example 7

### 4"-Epiarbekacin

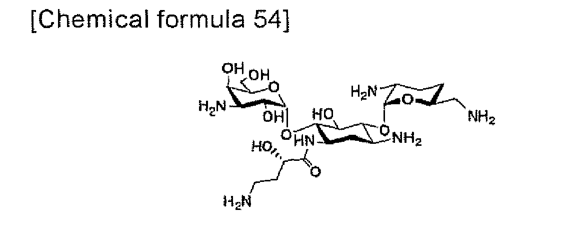

### Production step 7-(a)

75 mg of the compound produced in production step 2-(d) was used in the same manner as in production steps 1-(i) and (j) to give the title compound: 4"-epiarbekacin (24 mg).

FABMS: m/z 553 [M+H]⁺
¹H-NMR (D₂O+ND₃): δ 1.70 (2H, m), 1.90 (1H, m), 2.00 (3H, m), 2. 19 (2H, m), 2.92 (2H, m), 3.02 (2H, m), 3.12 (1H, ddd, J = 3.7, 1 2.4 Hz), 3.18 (1H, m), 3.20 (1H, m), 3.61 (1H, dd, J = 9.1 Hz), 3. 85 (1H, dd, J = 3.8, 10.7 Hz), 3.97 (4H, m), 4.13 (1 H, m), 4.16 (1 H, brd), 4.22 (1 H, ddd, J = 4.3, 9.7, 13.4 Hz), 4.44 (1H, dd, J = 3 .6, 9.2 Hz), 4.51 (1 H, m), 5.39 (1 H, d, J = 3.8 Hz), 5.41 (1 H, d, J = 3.9 Hz).

Example 8

### 4"-Deoxy-5-epiarbekacin

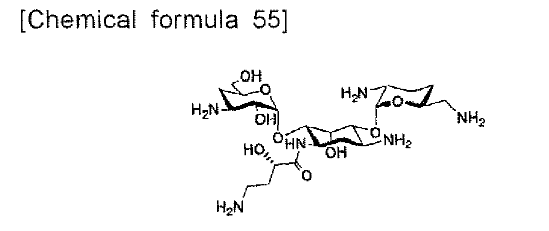

### Production step 8-(a)

560 mg of the compound produced in production step 2-(c) was used in the same manner as in production step 2-(d) except that lithium chloride was used instead of cesium acetate, to give the following compound (284 mg) 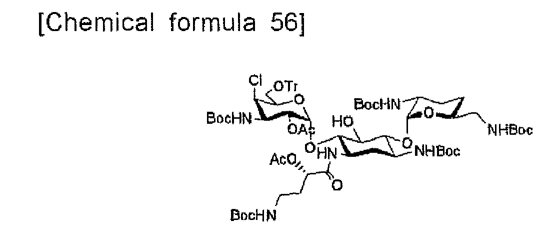 FABMS: m/z 1435 [M+K]⁺.

Production step 8-(b)
The compound (140 mg) prepared in production step 8-(a) was dissolved in 7.0 mL of dioxane. Tri-n-butyltin hydride (0.43 mL) and 15 mg of azobisisobutylnitrile were added to the solution, and the mixture was stirred at 80°C overnight. The reaction solution was concentrated under the reduced pressure, and the residue was washed with hexane and was purified by preparative TLC (hexane : ethyl acetate = 5 : 7) to give 109 mg of the following compound. 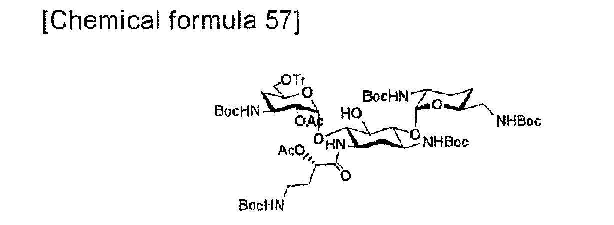 FABMS: m/z 1385 [M+Na]⁺, 1401 [M+K]⁺

Production step 8-(c)
95 mg of the compound prepared in production step 8-(b) was used in the same manner as in production step 1-(d) to give the following compound as a crude product. This compound as such was used in the next step without isolating and purifying the compound.

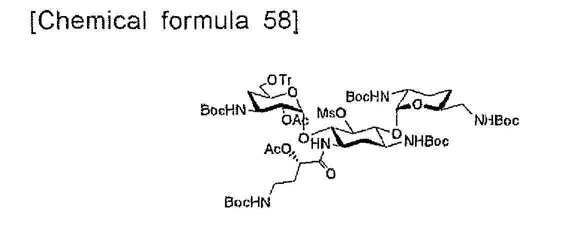

Production step 8-(d)
The compound produced in production step 8-(c) was used in the same manner as in production step 1-(e) to give the following compound (25 mg). 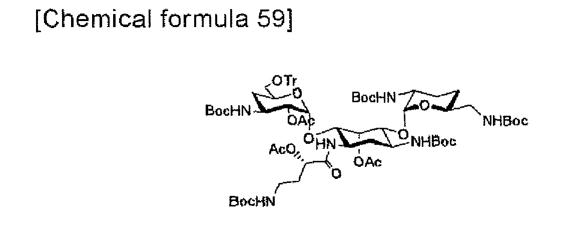 FABMS: m/z 1427 [M+Na]⁺.

Production step 8-(e)
25 mg of the compound produced in production step 8-(d) was used in the same manner as in production steps 1-(i) and 1-(j) to give the title compound: 4"-deoxy-5-epiarbekacin (10 mg).
FABMS: m/z 537 [M+H]⁺
¹H-NMR (D₂O+ND₃): δ 1.30 (1H, m), 1.40 (1H, m), 1.47 (1H, m), 1.78 (4H, m), 1.91 (1 H, m), 2.00 (1 H, m), 2.08 (1 H, m), 2.72 (2H, m), 2.83 (2H, m), 2.89 (1 H, m), 3.16 (1 H, m), 3.28 (1 H, m), 3.33 (1 H, dd, J = 3.8, 10.2 Hz), 3.52 (1 H, dd, J = 2.2, 10.3 Hz), 3.60 (1 H, dd, J = 7.6, 12.1 Hz), 3.70 (1 H, dd, J = 2.7, 12.1 Hz), 3.84 (1 H, m), 3.92 (1 H, m), 4.16 (1H, m), 4.25 (1H, dd, J = 3.6, 9.5 Hz), 4.31 (1 H, ddd, J = 4.6, 12.2 Hz), 4.57 (1 H, brs), 5.01 (1H, d, J = 3.4 Hz), 5.12 (1H, d, J = 3.8 Hz).

Example 9

### 1-N-[(S)-(3-amino-2-hydroxypropanoyl)]-5,4"-diepidibekacin

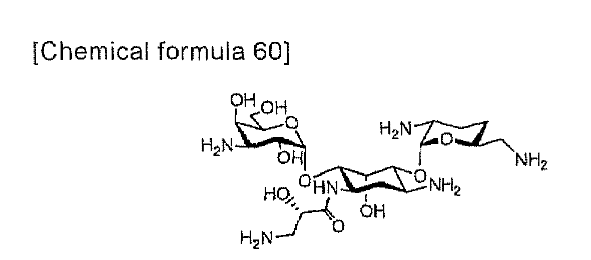

### Production step 9-(a)

3,2',6'-Tri-N-t-butoxycarbonyl-3"-trifluoroacetyl-dibekacin (5 g) was dissolved in 100 mL of 1,4-dioxane and 50 mL of water. Triethylamine (0.44 mL) and 2 g of benzyloxycarbonyloxysuccinimide were added to the solution, and the mixture was stirred for one hr. The reaction solution was concentrated under the reduced pressure, and the residue was washed with hexane, diisopropyl ether, and diethyl ether and was dried to give the following compound.

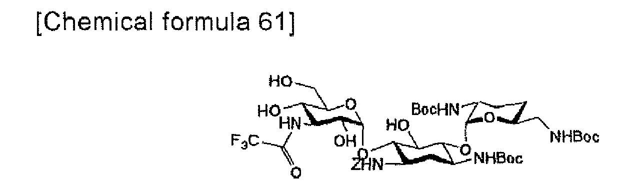

Production step 9-(b)
The compound produced in production step 9-(a) was dissolved in 150 mL of methanol. 150 mL of aqueous ammonia was added to the solution, and the mixture was stirred at room temperature overnight. This reaction solution was concentrated under the reduced pressure to give the following compound.

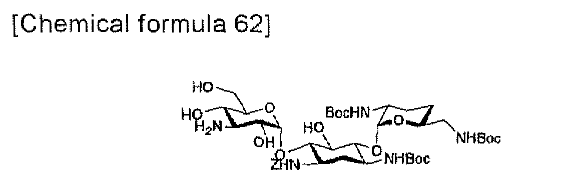

Production step 9-(c)
The compound produced in production step 9-(b) was dissolved in 100 mL of tetrahydrofuran and 50 mL of water. Triethylamine (0.66 mL) and 1.9 mL of t-butyl dicarbonate were added to the solution, and the mixture was stirred overnight. The reaction solution was concentrated under the reduced pressure, and the residue was purified by column chromatography on silica gel (methylene chloride : methanol : aqueous ammonia = 300 : 10 : 1) to give 2.8 g of the following compound.

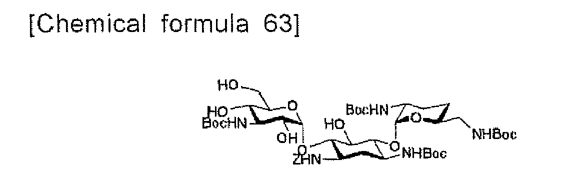

Production step 9-(d)
The compound produced in production step 9-(c) was used in the same manner as in production step 1-(b) to give the following compound.

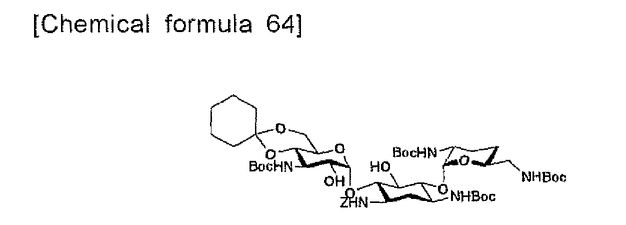

Production step 9-(e)
The compound produced in production step 9-(d) was used in the same manner as in production step 2-(a) to give the following compound.

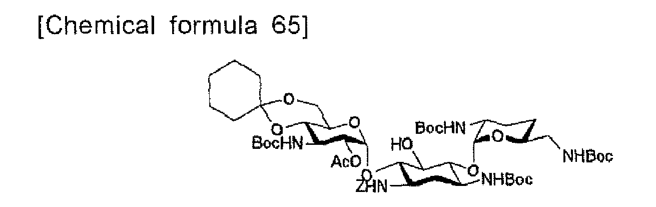

Production step 9-(f)
The compound produced in production step 9-(e) was used in the same manner as in production step 1-(f) to give the following compound.

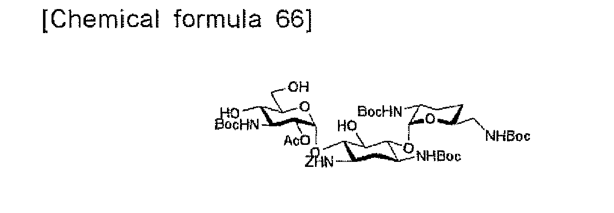

Production step 9-(g)
3 g of the compound produced in production step 9-(f) was used in the same manner as in production step 1-(g) to give the following compound (1.2 g).

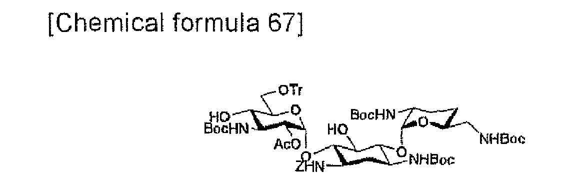

Production step 9-(h)
1.2 g of the compound produced in production step 9-(g) was used in the same manner as in production step 1-(h) to give the following compound (1.2 g).

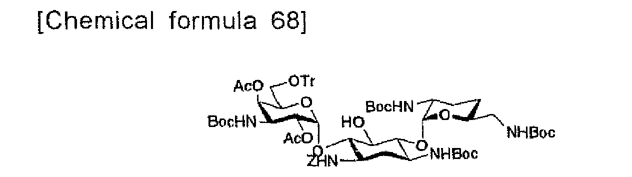

Production step 9-(i)
1.2 g of the compound produced in production step 9-(h) was used in the same manner as in production step 1-(d) to give the following compound (0.35 g).

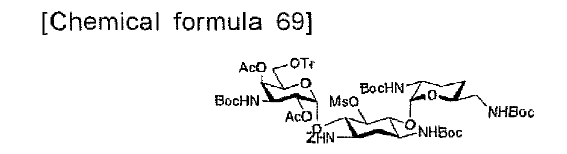

Production step 9-(j)
0.35 g of the compound produced in production step 9-(i) was used in the same manner as in production step 1-(e) to give the following compound (0.20 g).

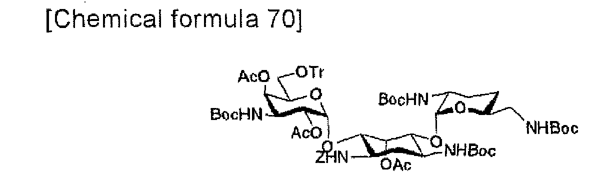

Production step 9-(k)
0.20 g of the compound produced in production step 9-(j) was used in the same manner as in production step 1-(i) to give the following compound (0.17 g).

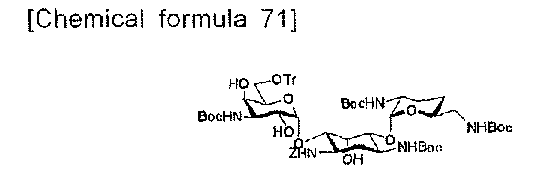

Production step 9-(I)
The compound (75 mg) produced in production step 9-(k) was dissolved in methanol. 0.23 mL of formic acid and 145 mg of palladium-carbon were added to the solution, and the mixture was stirred for 5 hr. The reaction solution was filtered through Celite, and the filtrate was concentrated under the reduced pressure. Methylene chloride was added to the residue, and the mixture was washed with a 5% aqueous sodium hydrogencarbonate solution and was dried over magnesium sulfate. This solution was concentrated to dryness to give 46 mg of the following compound.

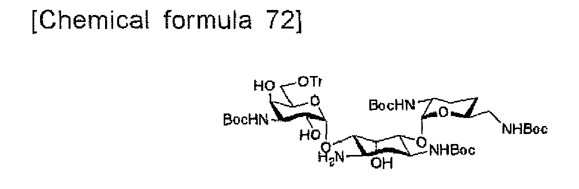

Production step 9-(m)
(s)-3-Benzyloxycarbonylamino-2-hydroxypropionic acid (30 mg) was dissolved in 1 mL of tetrahydrofuran. DCC (25 mg) and 14 mg of hydroxysuccinimide were added to the solution, and the mixture was stirred for 5 hr to give an active ester solution. The compound (67 mg) produced in production step 9-(I) was dissolved in 1 mL of a tetrahydrofuran/water solution (tetrahydrofuran : water = 3 : 1), and 13 mg of sodium carbonate was added to the solution. The active ester solution prepared above was added to this solution, and the mixture was stirred for one hr. Thereafter, 80 mL of methylene chloride was added to the reaction solution, and the mixture was washed with saturated brine and dried over anhydrous magnesium sulfate. This solution was concentrated under the reduced pressure, and the residue was purified by column chromatography on silica gel (methylene chloride : methanol: aqueous ammonia = 100 : 10 : 1) to give 20 mg of the following compound.

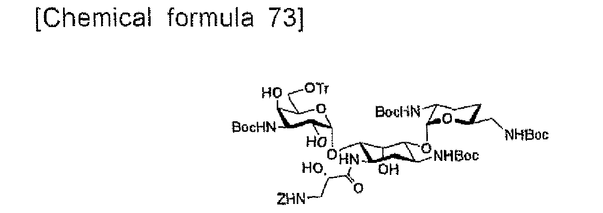

Production step 9-(n)
The compound (20 mg) produced in production step 9-(m) was dissolved in 2 mL of 90% aqueous trifluoroacetic acid solution, and the mixture was stirred for 4 hr. The reaction solution was concentrated under the reduced pressure. Thereafter, the concentrate was dissolved in 2 mL of water, 20 mg of palladium-carbon was added to the solution, and the mixture was stirred in a hydrogen atmosphere for 4 hr. This solution was filtered through Celite and was concentrated under the reduced pressure. The residue was purified by CM-Sephadex(NH₄⁺) to give the title compound: 1-N-[(S)-(3-amino-2-hydroxypropanoyl)]-5,4"-diepidibekacin (10 mg).
FABMS: m/z 539 [M+H]⁺;
¹H-NMR (D₂O+ND₃) δ: 1.33 - 1.54 (2H, m), 1.75 - 1.88 (3H, m), 2. 05 - 2.13 (1H, m), 2.69 - 2.83 (2H, m), 2.86 - 2.95 (2H, m), 3.00 - 3.09 (2H, m), 3.20 - 3.33(1H, m), 3.54 (1 H, dd, J = 2.5), 3.64 ( 1 H, dd, J = 4.0, 10.8 Hz), 3.79 - 4.00 (3H, m), 3.86 (1 H, dd, J = 2.3, 10.8 Hz), 3.96 (1H, d, J = 1.9 Hz), 4.12 - 4.18 (1H, m), 4.22 (1 H, dd, J = 3.9, 6.9 Hz), 4.30 - 4.38 (1 H, m), 4.56 (1 H, s), 5.02 ( 1H, d, J = 3.2 Hz), 5.14 (1H, d, J = 4.1 Hz).

Example 10

### 1-N-[(S)-(5-Amino-2-hydroxypentanoyl)]-5,4"-diepidibekacin

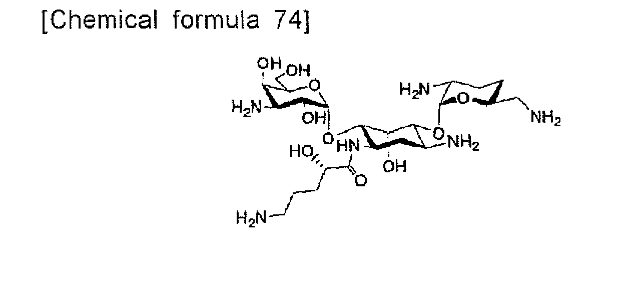

Production step 10-(a)
(s)-5-benzyloxycarbonylamino-2-hydroxypentanoic acid was used in the same manner as in Example 9 to give the title compound: 1-N-[(S)-(5-amino-2-hydroxypentanoyl)]-5,4"-diepidibekacin.
FABMS: m/z 567 [M+H]⁺.

Example 11

### 5,4"-Diepi-3"-N-methylarbekacin

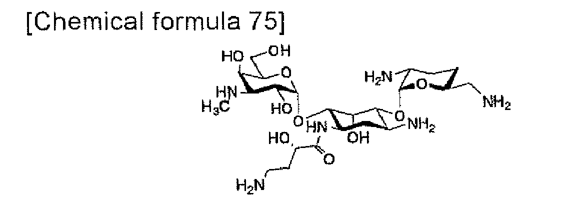

### Production step 11-(a)

3,2',6'-Tri-N-t-butoxycarbonyl-4"'-N-p-methoxybenzyloxycarbonyl-arbekacin (3.0 g) was dissolved in 30 mL of a methanol/dioxane solution (methanol : dioxane = 1 : 1) 0.84 mL of triethylamine and 0.46 mL of benzaldehyde were added to the solution, and the mixture was stirred at room temperature for 2 hr. The reaction solution was concentrated under the reduced pressure, followed by washing with diisopropyl ether. The residue was dissolved in 30 mL of a methanol/dioxane solution (methanol : dioxane = 1 : 1). Sodium borohydride (113 mg) was added to the solution, and the mixture was stirred at room temperature for 1 hr. The reaction solution was concentrated under the reduced pressure, the concentrate was extracted with methylene chloride, and the extract was dried over anhydrous magnesium sulfate and was then concentrated under the reduced pressure. The residue was purified by column chromatography on silica gel (methylene chloride : methanol = 9 : 1) to give 0.87 g of the following compound. 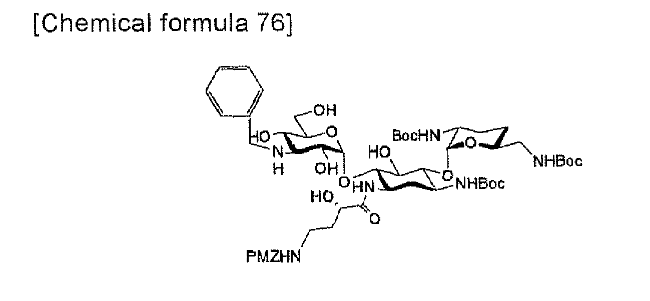 FABMS: m/z 1107 [M+H]⁺.

Production step 11-(b)
1.40 g of the compound produced in production step 1 1-(a) was used in the same manner as in production step 11-(a) except that formaldehyde was used instead of benzaldehyde, to give the following compound (0.99 g). 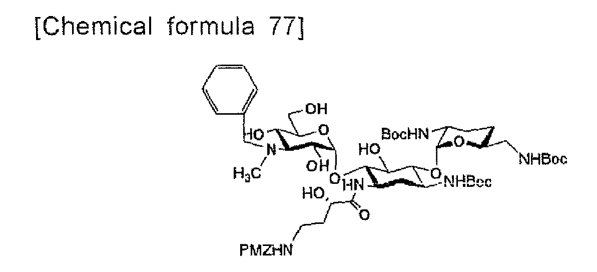 FABMS: m/z 1121 [M+H]⁺.

Production step 11-(c)
A reaction was allowed to proceed in the same manner as in production steps 1-(b) to 1-(j), except that the compound produced in the above step (b) was used. The compound thus obtained was dissolved in water. 1 M hydrochloric acid was added to the solution, and 10%Pd-C was added thereto under an argon gas stream. The air in the system was then replaced by hydrogen, followed by stirring at room temperature overnight. The reaction solution was filtered through Celite and was then purified by CM-Sephadex(NH₄⁺) to give the title compound: 5,4"-diepi-3"-N-methylarbekacin.
FABMS: m/z 567 [M+H]⁺.

### Example 12

### 5,4"-Diepi-6'-N-methylarbekacin

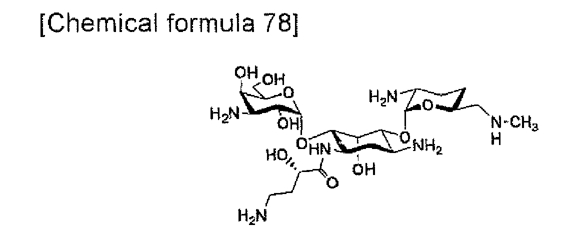

### Production step 12-(a)

Arbekacin (22 g) was dissolved in 116 mL of water, and 772 mL of N,N-dimethylformamide was added to the solution. Zinc acetate (14.7 g) was added thereto, and the mixture was stirred for 1 hr. Benzyloxycarbonyloxysuccinimide was added thereto, and the mixture was stirred for 5 hr. The reaction solution was concentrated under the reduced pressure, and the residue was purified by Amberlite CG-50(NH₄⁺) to give 4.9 g of the following compound. 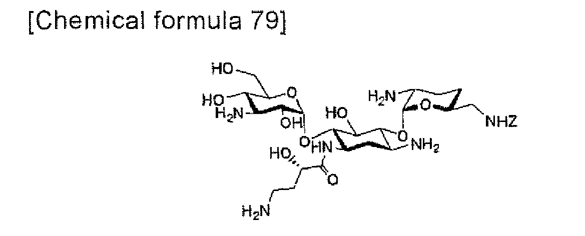 FABMS: m/z 687 [M+H]⁺.

Production step 12-(b)
The compound (4.9 g) produced in production step 12-(a) was dissolved in 100 mL of water. Methanol (150 mL) and 25 mL of 1,4-dioxane were added to the solution, 0.8 mL of triethylamine and 9.5 mL of di-t-butyl dicarbonate were further added thereto, and the mixture was stirred overnight. The reaction solution was concentrated under the reduced pressure. The residue was washed with hexane, diisopropyl ether and diethyl ether, and concentrated to dryness to give 8.0 g of the following compound.

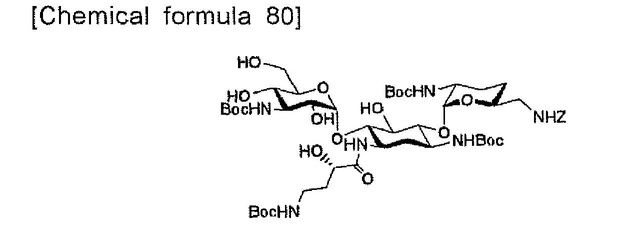

Production step 12-(c)
The compound produced in production step 12-(b) was dissolved in 80 mL of 1,4-dioxane, 16 mL of water was added to the solution, 0.5 g of 10%Pd-C was added thereto, and a catalytic hydrogen reduction reaction was carried out under a hydrogen atmosphere. After filtration through Celite, the filtrate was concentrated to dryness to give 6.0 g of the following compound.

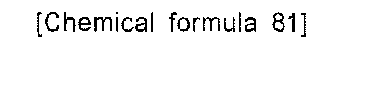

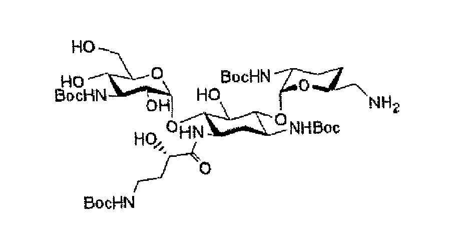

Production step 12-(d)
The compound (6.0 g) produced in production step 12-(c) was dissolved in 100 mL of 1,4-dioxane. Methanol (100 mL) was added to the solution, 1.8 mL of triethylamine and 1.0 mL of benzaldehyde were added thereto, and the mixture was stirred for 2 hr. The reaction solution was concentrated under the reduced pressure. The residue was washed with diisoproyl ether and was then concentrated to dryness. The residue was dissolved in 100 mL of 1,4-dioxane. Methanol (100 mL) was added to the solution, 0.24 g of sodium borohydride was added thereto, and the mixture was stirred for 2 hr. The reaction solution was concentrated under the reduced pressure, and 100 mL of water was added to the residue. The mixture was extracted with methylene chloride, and the extract was dried over anhydrous magnesium sulfate and then concentrated to dryness to give 4.0 g of the following compound.

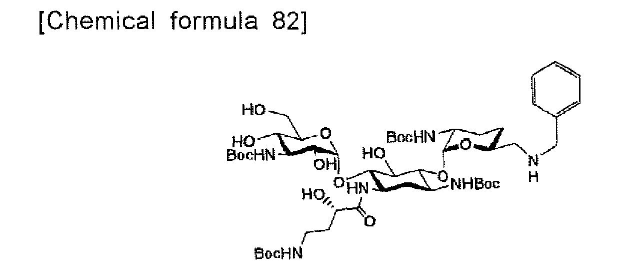

Production step 12-(e)
6.58 g of the compound produced in production step 12-(d) was used in the same manner as in production step 12-(d) except that formaldehyde was used instead of benzaldehyde, to give the following compound (6.74 g).

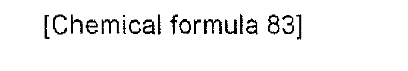

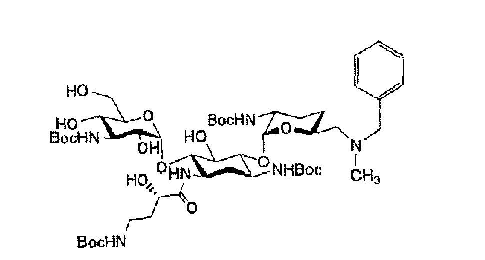

Production step 12-(f)
Water (20 mL) was added to a solution of 4 g of the compound produced in production step 12-(e) dissolved in 140 mL of tetrahydrofuran, 1 g of palladium hydroxide was added thereto, and the mixture was stirred under a hydrogen atmosphere for 3 hr. The mixture was filtered through Celite, and the filtrate was concentrated to dryness. The residue was dissolved in 100 mL of tetrahydrofuran. Triethylamine (1.2 mL) and 2.9 mL of di-t-butyl dicarbonate were added to the solution, and the mixture was stirred for 3 hr. The reaction solution was concentrated under the reduced pressure, and the residue was purified by column chromatography on silica gel (methylene chloride : methanol : aqueous ammonia = 100 : 10 : 1) to give 1.0 g of the following compound.

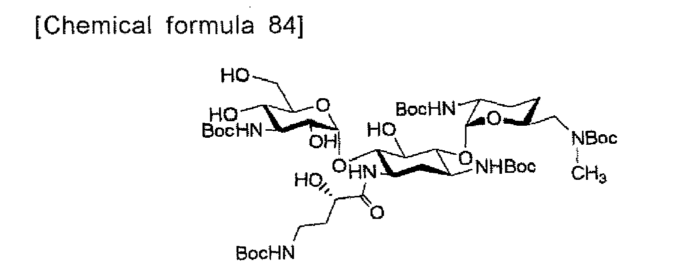

Production step 12-(g)
1 g of the compound produced in production step 12-(f) was used in the same manner as in production step 1-(b) to give the following compound (1.2 g).

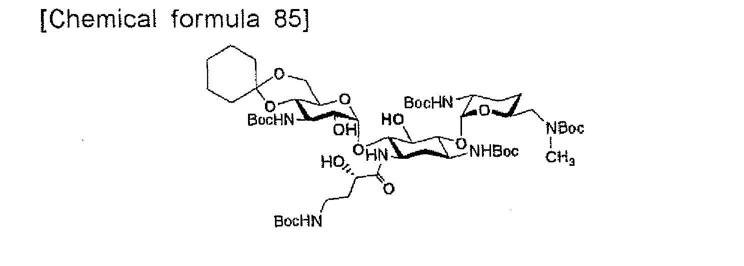

Production step 12-(h)
The compound (1.2 g) produced in production step 12-(g) was dissolved in 7 mL of pyridine, 0.25 mL of acetic anhydride was added to the solution, and a reaction was allowed to proceed for 2 hr. The reaction solution was concentrated under the reduced pressure, 30 mL of water was added to the residue, and the mixture was extracted with methylene chloride, followed by washing with a 10% potassium hydrogensulfate solution and a saturated aqueous sodium carbonate solution. The extract was then dried over anhydrous magnesium sulfate. This solution was then concentrated under the reduced pressure, and the residue was purified by column chromatography on silica gel (hexane : ethyl acetate = 1 : 2) to give 0.38 g of the following compound.

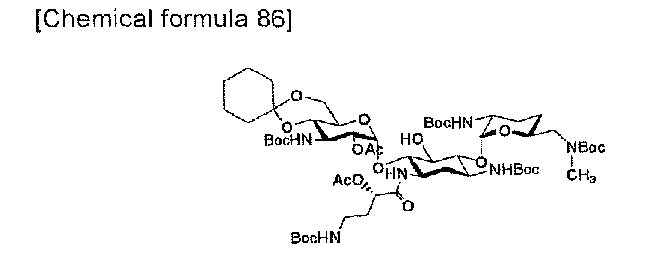

Production step 12-(i)
The compound (0.38 g) produced in production step 12-(h) was dissolved in a mixed solution composed of 10 mL of methylene chloride and 1 mL of methanol, 1 mL of a 90% aqueous trifluoroacetic acid solution was added to the solution, and the mixture was stirred for 1 hr. The mixture was then neutralized by the addition of triethylamine under an ice bath. The reaction solution was concentrated under the reduced pressure, the residue was extracted with methylene chloride, and the extract was dried over anhydrous magnesium sulfate and was concentrated to dryness to give 0.34 g of the following compound.

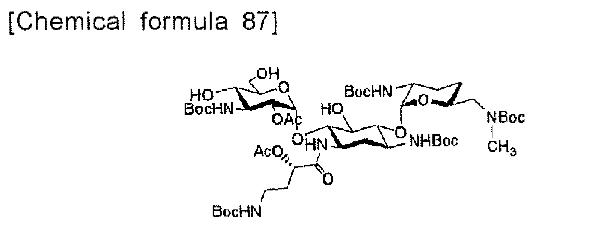

Production step 12-(i)
250 mg of the compound produced in production step 12-(i) was used in the same manner as in production step 1-(g) to give the following compound (70 mg).

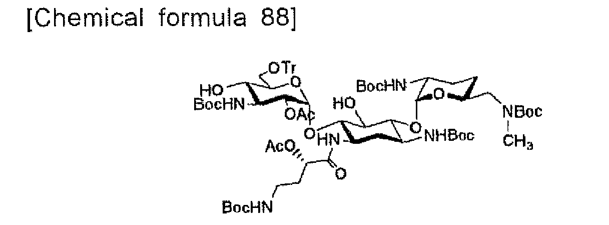

Production step 12-(k)
70 mg of the compound produced in production step 12-(j) was used in the same manner as in production step 1-(h) to give the following compound (66 mg).

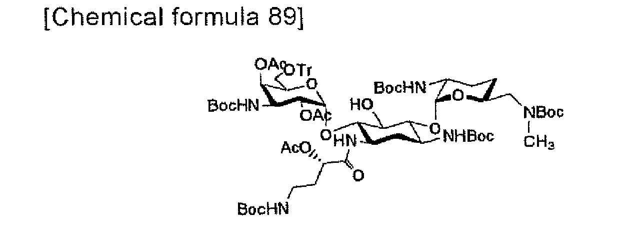

Production step 12-(I)
50 mg of the compound produced in production step 12-(k) was used in the same manner as in production step 1-(d) to give the following compound (50 mg).

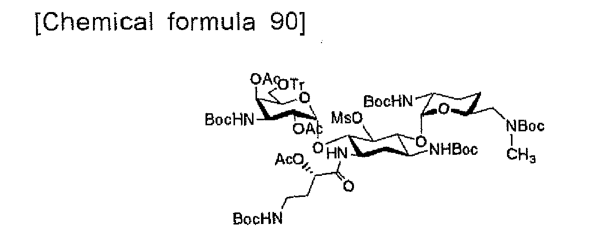

Production step 12-(m)
70 mg of the compound produced in production step 12-(I) was used in the same manner as in production step 1-(e) to give the following compound (60 mg).

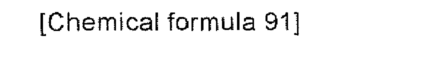

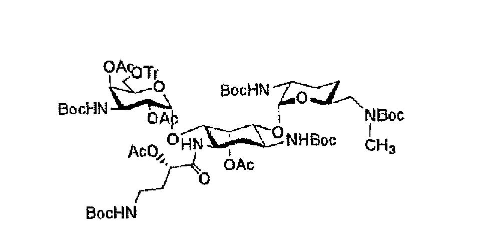

Production step 12-(n)
35 mg of the compound produced in production step 12-(m) was used in the same manner as in production steps 1-(i) and 1-(j) to give the title compound: 5,4"-diepi-6'-N-methylarbekacin (3 mg).
FABMS: m/z 567 [M+H]⁺;
¹H-NMR (D₂O+DCl) δ: 1.62 (1 H, m), 1.88 (1 H, m), 1.99 (1 H, m), 2.04 (1 H, m), 2.10 (2H, m), 3.26 (2H, m), 2.82 (3H, s), 3.16 (1H, dd, J = 9.0, 13.0 Hz), 3.25 (2H, m), 3.30 (1 H, dd, J = 2.0, 13.0 Hz), 3.59 (1 H, m), 3.62 (1 H, dd, J = 3.1, 11.2 Hz), 3.73 (1H, m), 3.80 (1H, dd, J = 8.8, 12.0 Hz), 3.87 (1 H, dd, J = 2.7, 12.0 Hz), 4.05 (2H, m), 4.22 (3H, m), 4.36 (1 H, dd, J = 3.8, 9.3 Hz), 4.41 (1 H, m), 4.80 (1 H, m), 5.26 (1H, d, J = 4.0 Hz), 5.47
(1H, d, J = 3.7 Hz).

### Example 13

### 5-Epiarbekacin

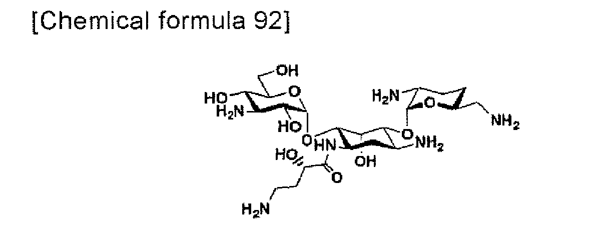

### Production step 13-(a)

14 g of the compound produced in production step 1-(a) was used in the same manner as in Production step 2-(a) to give the following compound (11.6 g). 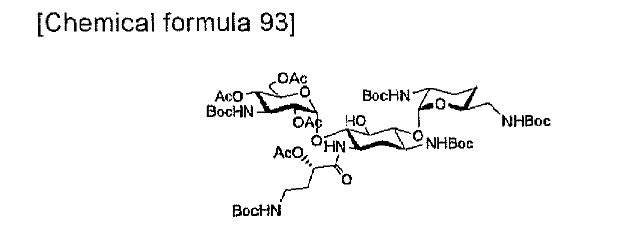 FABMS: m/z 1259 [M+K]⁺.

Production step 13-(b)
11.6 g of the compound produced in production step 13-(a) was used in the same manner as in production step 1-(d) to give the following compound (7.4 g). 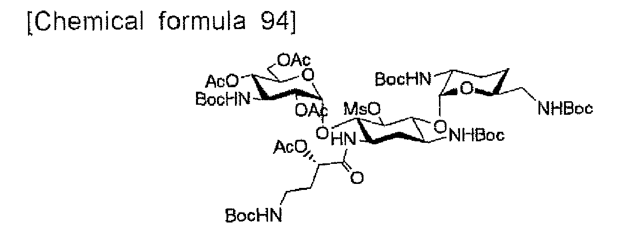 FABMS: m/z 1321 [M+Na]⁺, 1337 [M+K]⁺.

Production step 13-(c)
7.4 g of the compound produced in production step 13-(b) was used in the same manner as in production step 1-(e) to give the following compound (3.6 g). 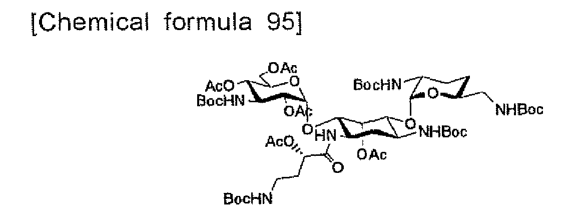 FABMS: m/z 1263 [M+H]⁺.

Production step 13-(d)
5.1 g of the compound produced in production step 13-(c) was used in the same manner as in production steps 1-(i) and 1-(j) to give the title compound: 5-epiarbekacin(1.3 g).
FABMS: m/z 553 [M+H]⁺;
¹H-NMR (D₂O+ND₃) δ: 1.27 - 1.48 (2H, m), 1.64 - 1.80 (4H, m), 1.85 - 1.95 (1 H, m), 1.98 - 2.07 (1H ,m), 2.60 - 2.86 (5H, m), 3.02 (1 H, dd, J = 10.3 Hz), 3.17 - 3.26 (2H, m), 3.39 (1 H, ddd, J = 1.2, 3.9, 10.3 Hz), 3.46 (1H, dd, J = 1.2, 10.3 Hz), 3.67 (1 H, dd, J = 6.9, 12.1 Hz), 3.77 - 3.93 (4H, m), 4.17 (1 H, dd, J = 3.7, 9.3 Hz), 4.25 (1H, dd, J = 4.4, 11.7 Hz), 4.50 (1 H, s), 4.94 (1 H, d, J = 3.2 Hz), 5.40 (1 H d, J = 3.9 Hz).

Example 14

### 5-Deoxy-5-epiacetylaminoarbekacin

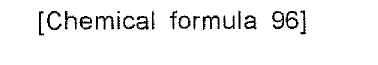

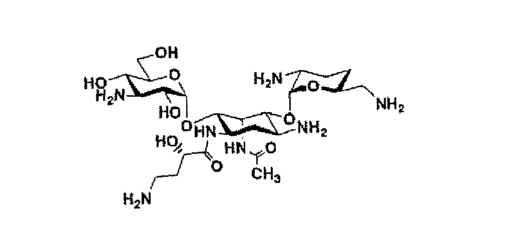

### Production step 14-(a)

12 mL of isopropyl alcohol, 60 mL of tetrahydrofuran, and 2.4 mL of triethylamine were added to a solution of 3.36 g of arbekacin in 30 mL of water. A solution of S-p-methoxybenzyloxycarbonyl-4,6-dimethyl-2-mercaptopyrimidine (10.86 g) in isopropyl alcohol/tetrahydrofuran (18 mL of isopropyl alcohol and 30 mL of tetrahydrofuran) was added dropwise thereto at room temperature, and the mixture was stirred at 60°C for 3 hr. Diethyl ether (100 mL) was added thereto, and the mixture was allowed to stand at 6°C for 14 hr. The precipitated solid was collected by filtration, was washed with diethyl ether and water, was dried under the reduced pressure to give 6.61 g of the following compound as a solid.

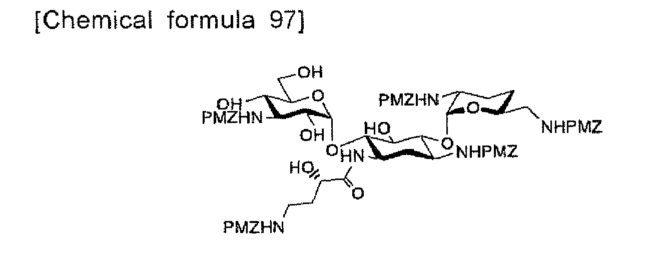

Production step 14-(b)
The compound (4.11 g) prepared in production step 14-(a) was dissolved in 50 mL of pyridine. A solution of 2.05 g of benzoyl chloride in 9 mL of methylene chloride was added to the solution under ice cooling, and the mixture was stirred under ice cooling for 0.5 hr. Thereafter, the temperature was raised to room temperature, followed by stirring for 4 hr. Water (0.1 mL) was added, and the mixture was concentrated under the reduced pressure. Ethyl acetate was added to the residue, and the mixture was washed with water, a 10% aqueous potassium hydrogensulfate solution, a saturated aqueous sodium hydrogencarbonate solution, and a saturated aqueous sodium chloride solution in that order, dried over anhydrous magnesium sulfate, and then concentrated to dryness to give 5.22 g of the following compound.

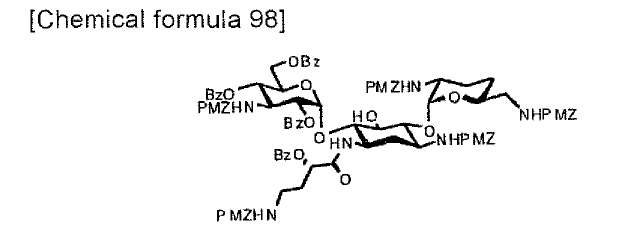

Production step 14-(c)
The compound produced in production step 14-(b) was used in the same manner as in production step 4-(a) to give the following compound.

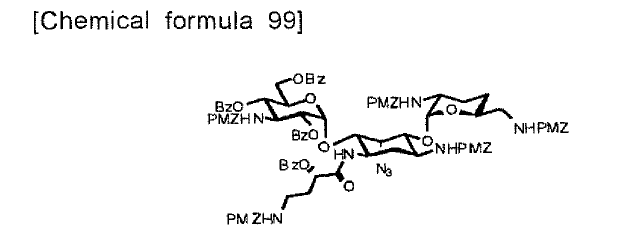

Production step 14-(d)
The compound (103 mg) produced in production step 14-(c) was dissolved in a mixed solvent composed of 2 mL of tetrahydrofuran and 0.2 mL of water, and the solution was stirred in the presence of Raney nickel at atmospheric hydrogen pressure for 3 hr. The catalyst was removed by filtration, and the filtrate was concentrated under the reduced pressure. The residue was dissolved in 2 mL of a solution of chloroform : methanol = 5 : 1. Triethylamine (0.03 mL) and 30 mg of acetic anhydride were added to the solution, and the mixture was stirred at room temperature overnight. The residue was subjected to deprotection treatment in the same manner as in production steps 1-(i) and 1-(j) to give the title compound: 5-deoxy-5-epiacetylaminoarbekacin (6 mg).
FABMS: m/z 594 [M+H]⁺.

Example 15

### 5-Deoxy-5-epimethanesulfonyloxyarbekacin

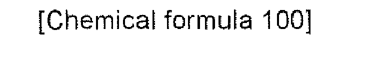

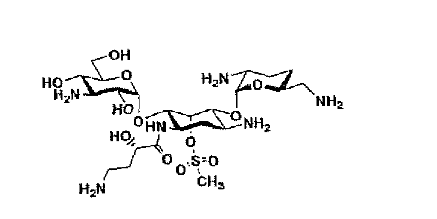

### Production step 15-(a)

The compound produced in production step 13-(c) was allowed to react in the same manner as in production step 1-(i) and was further allowed to react in the same manner as in production step 2-(a). The compound thus obtained was allowed to react in the same manner as in production step 1-(d), followed by deprotection in the same manner as in production steps 1-(i) and 1-(j) to give the title compound: 5-deoxy-5-epimethanesulfonyloxyarbekacin.
FABMS: m/z 631 [M+H]⁺;
¹H-NMR (D₂O+DCl) δ: 1.69 (1 H, m), 1.92 (3H, m), 2.15 (3H, m), 2,34 (1H, m), 3.12 (3H, m), 3.41 (2H, m), 3.47 (3H, s), 3.67 (3H, m), 3.95 (5H, m), 4.27 (1H, m), 4.35 (2H, m), 4.44 (1H, m), 5.18 (1 H, d, J = 3.6 Hz), 5.50
(1 H, brs), 5.58 (1H, brs).

Example 16

### 5-Deoxy-5-epichloroarbekacin

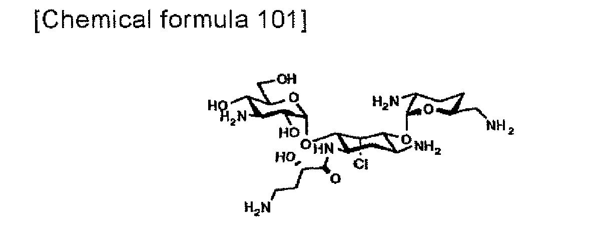

### Production step 16-(a)

The compound produced in production step 13-(b) was used in the same manner as in production step 3-(b) to give the following compound.

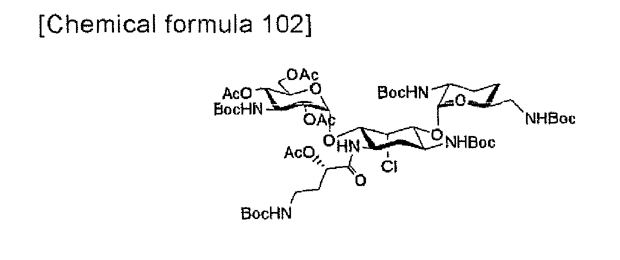

Production step 16-(b)
The compound produced in production step 15-(a) was used in the same manner as in production steps 1-(i) and 1-(j) to give the title compound: 5-deoxy-5-epichioroarbekacin.
FABMS: m/z 571 [M+H]⁺;
¹H-NMR (D₂O+ND₃) δ: 1.62 (1H, dddd, J = 3.90, 13.67, 13.67, 13.6 7 Hz), 1.85 (1H, ddd, J = 12.7, 12.7, 12.7 Hz), 1.90 - 2.10 (4H, m ), 2.17 (1 H, ddt, J = 3.42, 7.81, 14.16 Hz), 2.34 (1H, ddd, J = 4.3 9, 4.39, 12.7 Hz), 3.09 (1 H, dd, J = 7.33, 13.19 Hz), 3.17 (2H, dd, J = 6.84, 6.84 Hz), 3.27 (1H, dd, J = 2.93, 13.18 Hz), 3.38 (1H, dd, J = 10.26, 10.26 Hz), 3.58 (1H. dd, J = 9.77, 9.77 Hz), 3.58 - 3.63 (1H, m), 3.69 (1 H, dd, J = 7.33, 12.21 Hz), 3.81 (1 H, dd, J = 3.90, 11.23 Hz), 3.87 - 3.97 (3H, m), 4.05 - 4.13 (1 H, m), 4.28 ( 1H, dd, J = 3.41, 9.27 Hz), 4.30 (1 H, dd, J = 2.93, 9.77 Hz), 4.38 - 4.45 (1H, m), 4.42 (1H, dd, J = 2.93, 10.74 Hz), 5.15 (1 H, dd, J = 2.93, 2.93 Hz), 5.17 (1H, d, J = 3.91 Hz), 5.35 (1H, d, J = 3. 42 Hz).

Example 17

### 5-Deoxy-5-epiazidoarbekacin

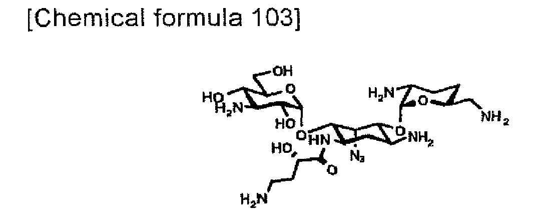

### Production step 17-(a)

The compound produced in production step 13-(b) was used in the same manner as in production step 4-(a) to give the following compound.

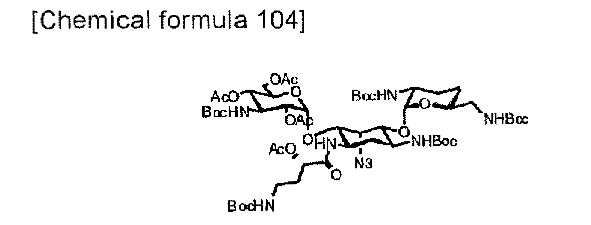

Production step 17-(b)
The compound produced in production step 17-(a) was used in the same manner as in production steps 1-(i) and 1-(j) to give the title compound: 5-deoxy-5-epiazidoarbekacin.
FABMS: 578 [M+H]⁺.

Example 18

### 5-Deoxy-5-epiaminoarbekacin

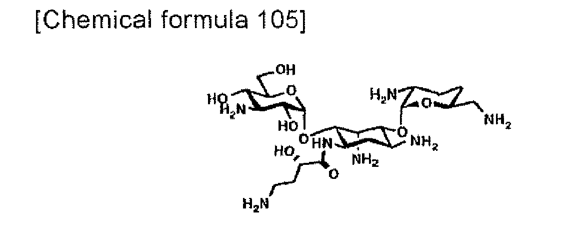

### Production step 18-(a)

The compound produced in Example 17 was used in the same manner as in Example 5 to give the title compound: 5-deoxy-5-epiaminoarbekacin.
FABMS: m/z 552 [M+H]⁺;
¹H-NMR (D₂O+ND₃) δ: 1.27 - 1.48 (1H, m), 1.33 (1H, q, J = 12.8 Hz), 1.67 - 1.80 (4H, m), 1.87 - 1.97 (1 H, m), 2.01 (1 H, dt, J = 4.7, 12.8 Hz), 2.62 - 2.87(5H, m), 3.02 (1 H, t, J = 10.1 Hz), 3.15 - 3.30 (1H, m), 3.38 (1 H, dd, J = 3.9 Hz, J = 10.1 Hz), 3.52 (1 H, dd, J = 3.1 Hz, J = 10.3 Hz), 3.73 - 3.87 (6H, m), 4.19 (1H, dd, J = 3.6, 9.5 Hz), 4.26 - 4.35 (1H, m), 4.93 (1 H, d, J = 3.4 Hz), 5.17 (1H, d, J = 3.9 Hz).

Example 19

### 5-Deoxy-5-epidimethylaminoarbekacin

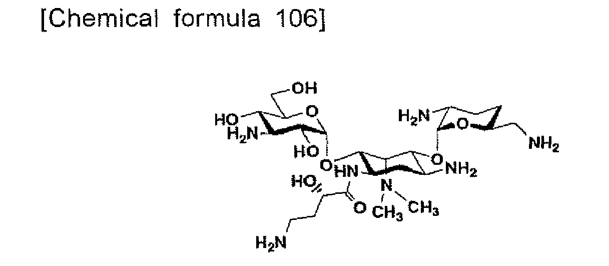

### Production step 19-(a)

288 mg of the compound produced in production step 17-(a) was used in the same reaction as in production step 1-(i) to give the following compound (250 mg).

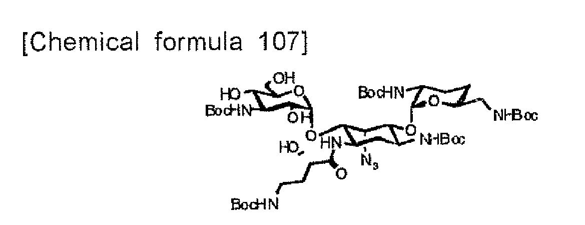

Production step 19-(b)
The compound (220 mg) produced in production step 19-(a) was dissolved in 4 mL of a solution (water : ethanol = 1 : 1). 10%Pd-C (200 mg) was added to the solution under an argon gas stream, and the mixture was stirred at room temperature overnight. The reaction solution was filtered through Celite and was purified by P-TLC (developing solvent: acetone) to give 120 mg of the following compound.

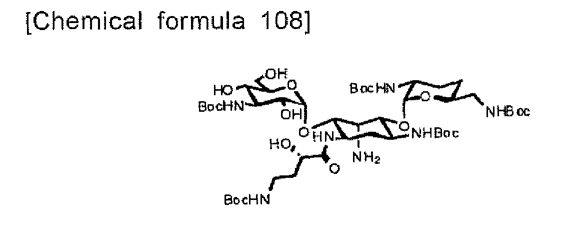

Production step 19-(c)
A reaction was carried out in the same manner as in production step 11-(b), except that 90 mg of the compound produced in production step 19-(b) was used. Further, deprotection was carried out in the same manner as in production step 1-(j) to give the title compound: 5-deoxy-5-epidimethylaminoarbekacin (8 mg).
FABMS: m/z 580 [M+H]⁺.

Example 20

### 5-Deoxy-5-epibenzoylaminoarbekacin

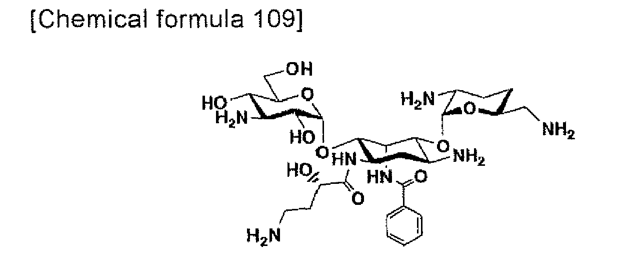

### Production step 20-(a)

A reaction was carried out in the same manner as in production step 1-(c), except that 50 mg of the compound produced in production step 19-(b) was used. Further, deprotection was carried out in the same manner as in production steps 1-(i) and 1-(j) to give the title compound: 5-deoxy-5-epibenzoylaminoarbekacin (8 mg).
FABMS: 656 [M+H]⁺.

Example 21

### 5-Deoxy-5-epibenzylaminoarbekacin

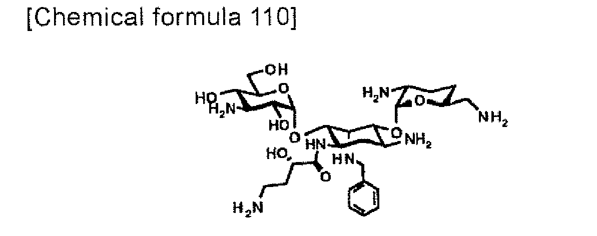

### Production step 21-(a)

This compound (100 mg) produced in production step 19-(b) was dissolved in 3 mL of N,N-dimethylformamide. Potassium carbonate (13 mg) and 0.066 mL of benzyl bromide were added to the solution, and the mixture was stirred at room temperature overnight. The reaction solution was concentrated under the reduced pressure and then extracted with methylene chloride, and the extract was washed with a saturated aqueous sodium hydrogencarbonate solution, was then dried over anhydrous magnesium sulfate and concentrated to dryness to give a compound (64 mg). This compound was used for deprotection in the same manner as in production step 1-(j) to give the title compound: 5-deoxy-5-epibenzylaminoarbekacin (30 mg).
FABMS: m/z 642 [M+H]⁺.

Example 22

### 5-Deoxy-5-epimethylaminoarbekacin

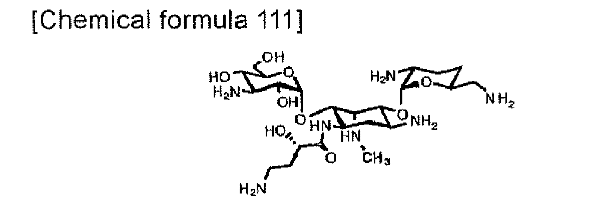

### Production step 22-(a)

500 mg of the compound produced in production step 13-(b) was used in the same manner as in production step 1-(i) to give the following compound (406 mg).

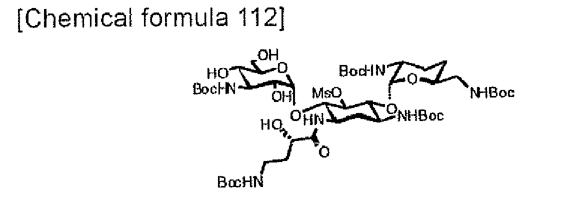

Production step 22-(b)
A methylamine-methanol solution (5.0 mL) was added to 100 mg of the compound produced in production step 22-(a), and the mixture was stirred in a sealed tube at 60°C for 4 days. The reaction solution was concentrated under the reduced pressure, and the residue was purified by column chromatography on silica gel (chloroform : methanol = 9 : 1) to give 27.0 mg of the following compound. 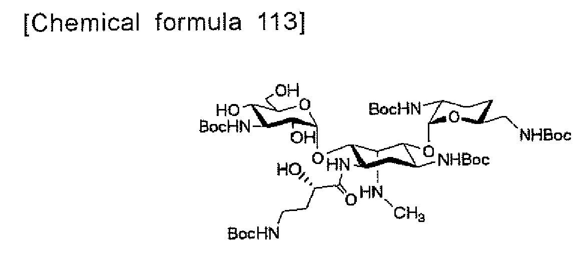 FABMS: m/z 1088 [M+Na]⁺.

Production step 22-(c)
23.0 mg of the compound produced in production step 22-(b) was used in the same manner as in production step 1-(j) to give the title compound: 5-deoxy-5-epimethylaminoarbekacin (12.0 mg).
FABMS: m/z 566 [M+H]⁺;
¹H-NMR (D₂O+ND₃) δ: 1.48 (1H, m), 1.60 (1H, m), 1.90 (4H, m), 2.09 (1H, m), 2.19 (1 H, m), 2.74 (3H, s), 2.84 (2H, m), 2.94 (2H, m), 3.01 (1 H, m), 3.15 (1 H, dd, J = 9.7, 10.5 Hz), 3.32 (1 H, m), 3.40 (1H, dd, J = 9.8, 10.0 Hz), 3.56 (2H, m), 3.72 (1H, dd, J = 3.0, 10.0 Hz), 3.88 (2H, m), 3.99 (2H, m), 4.08 (1 H, m), 4.35 (1 H, dd, J = 3.4, 9.3 Hz), 4.52 (1 H, ddd, J = 4.8, 12.0 Hz), 5.12 (1 H, d, J = 3.4 Hz), 5.20 (1 H, d, J = 4.0 Hz).

Example 23

### 5-Deoxy-5-epiallylaminoarbekacin

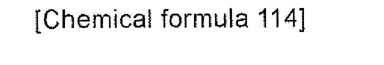

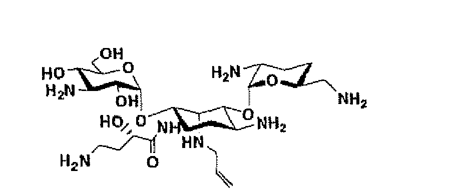

### Production step 23-(a)

100 mg of the compound produced in production step 22-(a) was used in the same manner as in production step 22-(b) except that allylamine was used instead of the methylamine-methanol solution, to give the following compound (21 mg). 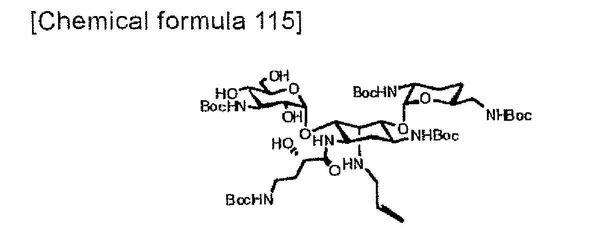 FABMS: m/z 1092 [M+H]⁺.

Production step 23-(b)
21 mg of the compound produced in production step 23-(a) was used in the same manner as in production step 1-(j) to give the title compound: 5-deoxy-5-epiallylaminoarbekacin (12.0 mg).
FABMS: m/z 592 [M+H]⁺.

Example 24

### 5-Deoxy-5-epi(2-aminoethyl)aminoarbekacin

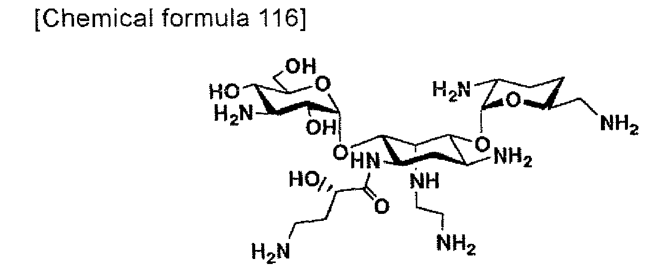

### Production step 24-(a)

100 mg of the compound produced in production step 22-(a) was used in the same manner as in production step 1-(e) except that ethylenediamine was used instead of cesium acetate, to give the following compound (21 mg). 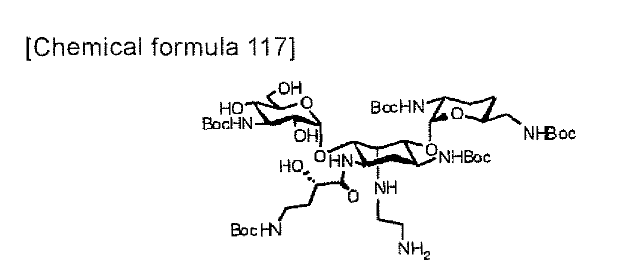 FABMS: m/z 1095 [M+H]⁺.

Production step 24-(b)
The compound produced in production step 24-(a) was used in the same manner as in production step 1-(j) to give the title compound: 5-deoxy-5-epi(2-aminoethyl)aminoarbekacin (16.0 mg).
FABMS: m/z 595 [M+H]⁺;
¹H-NMR (D₂O+ND₃) δ: 1.48 (1H, m), 1.59 (1H, m), 1.90 (4H, m), 2.08 (1 H, m), 2.18 (1 H, m), 2.83 (2H, m), 2.92 (4H, m), 3.00 (2H, m), 3.13 (1 H, dd, J = 10.2 Hz), 3.20 (1H, m), 3.35 (1H, m), 3.40 (1H, dd, J = 10.2 Hz), 3.55 (1 H, dd, J = 3.9, 10.2 Hz), 3.65 (1 H, brs), 3.71 (1 H, dd, J = 3.0, 10.1 Hz), 3.87 (1 H, m), 3.97 (2H, m), 4.06 (2H, m), 4.34 (1 H, dd, J = 3.5, 9.8 Hz), 4.59 (1 H, m), 5.10 (1 H, d, J = 3.4 Hz), 5.19 (1H, d, J = 3.9 Hz).

Example 25

### 5-Deoxy-5-epi(2-hydroxyethyl)aminoarbekacin

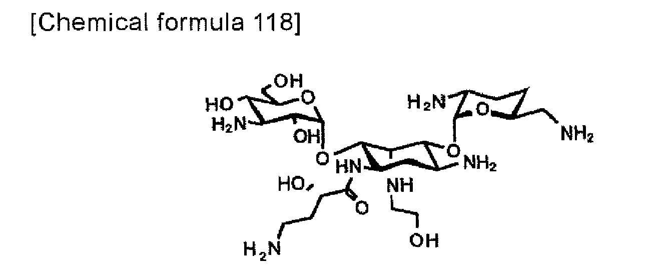

### Production step 25-(a)

100 mg of the compound produced in production step 22-(a) was used in the same manner as in production step 1-(e) except that 2-aminoethanol was used instead of cesium acetate, to give the following compound (25.0 mg). 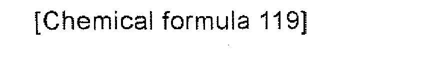 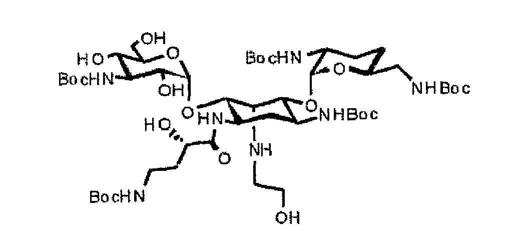 FABMS: m/z 1096 [M+H]⁺.

Production step 25-(b)
25.0 mg of the compound produced in production step 25-(a) was used in the same manner as in production step 1-(j) to give the title compound: 5-deoxy-5-epi(2-hydroxyethyl)aminoarbekacin (11.4 mg) . FABMS: m/z 596 [M+H]⁺;
¹H-NMR (D₂O+ND₃) δ: 1.26 (1H, m), 1.35 (1H, m), 1.69 (4H, m), 1.87 (1 H, m), 1.96 (1 H, m), 2.61 (2H, m), 2.70 (3H, m), 2.80 (1 H, m), 2.89 (1 H, dd, J = 9.6 Hz), 3.09 (1 H, m), 3.17 (1H. dd, J = 9.6,10.0 Hz), 3.32 (1 H, dd, J = 3.6, 9.6 Hz), 3.43 (1H, brs), 3.52 (1 H, m), 3.65 (3H, m), 3.74 (2H, m), 3.85 (2H, m), 4.12 (1 H, dd, J = 3.6, 10.0 Hz), 4.39 (1 H, m), 4.87 (1 H, d, J = 3.2 Hz), 4.97 (1 H, d, J = 3.8 Hz).

Example 26

### 5-Deoxy-5-epi(3-hydroxypropyl)aminoarbekacin

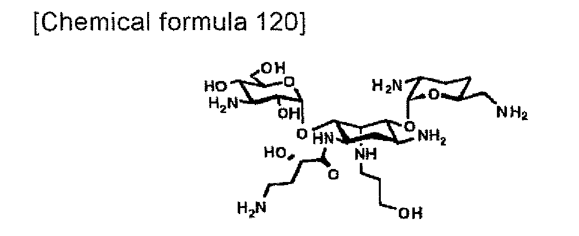

### Production step 26-(a)

100 mg of the compound produced in production step 22-(a) was used in the same manner as in production step 1-(e) except that 3-amino-1-propanol was used instead of cesium acetate, to give the following compound (43.0 mg). 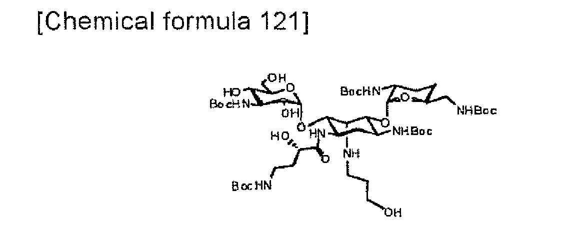 FABMS: m/z 1110 [M+H]⁺.

Production step 26-(b)
43.0 mg of the compound produced in production step 26-(a) was used in the same manner as in production step 1-(j) to give the title compound: 5-deoxy-5-epi(3-hydroxypropyl)aminoarbekacin (18.5 mg).
ESIMS: m/z 610 [M+H]⁺.

Example 27

### 5-Deoxy-5-epi(2-hydroxy-1-hydroxymethyl-ethyl)aminoarbekacin

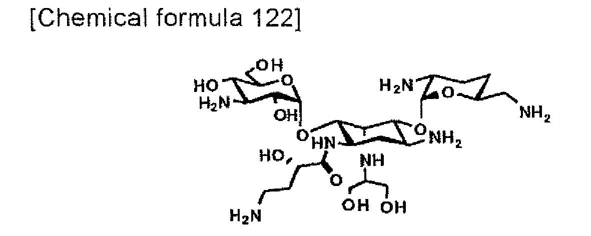

### Production step 27-(a)

100 mg of the compound produced in production step 22-(a) was used in the same manner as in production step 1-(e), except that 2-amino-1,3-propanediol was used instead of cesium acetate, to give the following compound (13.8 mg).

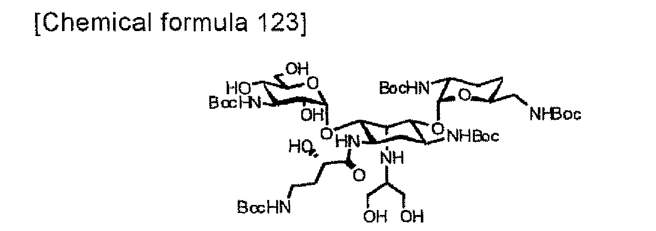

Production step 27-(b)
13.8 mg of the compound produced in production step 27-(a) was used in the same manner as in production step 1-(j) to give the title compound: 5-deoxy-5-epi(2-hydroxy-1-hydroxymethyl-ethyl)aminoarbekacin (6.0 mg).
FABMS: m/z 626 [M+H]⁺;
¹H-NMR (D₂O+ND₃) δ: 1.49 (1 H, m), 1.60 (1 H, m), 1.95 (4H, m), 2.09 (1 H, m), 2.20 (1 H, m), 2.84 (2H, m), 2.93 (2H, m), 3.03 (1 H, m), 3.11 (1 H, dd, J = 10.1 Hz), 3.34 (1 H, m), 3.43 (1 H, dd, J = 9.7, 10.1 Hz), 3.48 (1 H, m), 3.57 (1 H, dd, J = 3.8, 10.1 Hz), 3.70 - 4.09 (11H, m), 4.36 (1 H, dd, J = 3.4, 9.5 Hz), 4.81 (1 H, m), 5.17 (1 H, d, J = 3.4 Hz), 5.22 (1 H, d, J = 3.8 Hz).

Example 28

### 5-Epi-3"-N-methylarbekacin

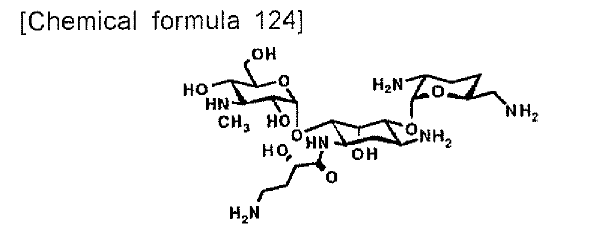

### Production step 28-(a)

3,2',6'-Tri-N-t-butoxycarbonyl-4"'-N-p-methoxybenzyloxycarbonyl-arbekacin (3.0 g) was dissolved in 30 mL of a methanol/dioxane solution (methanol : dioxane = 1 : 1), 0.84 mL of triethylamine and 0.46 mL of benzaldehyde were added to the solution, and the mixture was stirred at room temperature for 2 hr. The reaction solution was concentrated under the reduced pressure and was then washed with diisopropyl ether. The residue was dissolved in 30 mL of a methanol/dioxane solution (methanol : dioxane = 1 : 1), 113 mg of sodium borohydride was added to the solution, and the mixture was stirred at room temperature for 1 hr. The reaction solution was concentrated under the reduced pressure, the residue was extracted with methylene chloride, and the extract was dried over anhydrous magnesium sulfate and was concentrated under the reduced pressure. The residue was purified by column chromatography on silica gel (methylene chloride : methanol = 9 : 1) to give 0.87 g of the following compound. 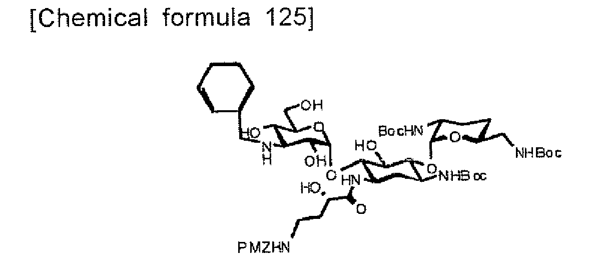 FABMS: m/z 1107 [M+H]⁺

Production step 28-(b)
1.40 g of the compound produced in production step 28-(a) was used as a starting compound in the same manner as in production step 28-(a) except that formaldehyde was used instead of benzaldehyde, to give the following compound (0.99 g). 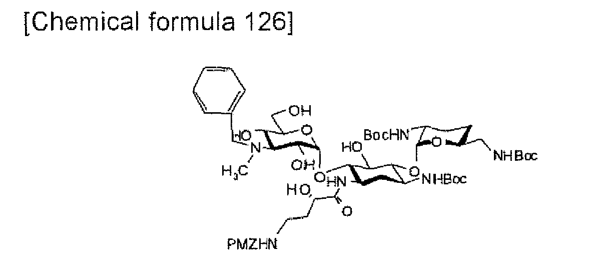 FABMS: m/z 1121 [M+H]⁺

Production step 28-(c)
370 mg of the compound produced in production step 28-(b) was used in the same manner as in production step 2-(a) to give the following compound (200 mg). 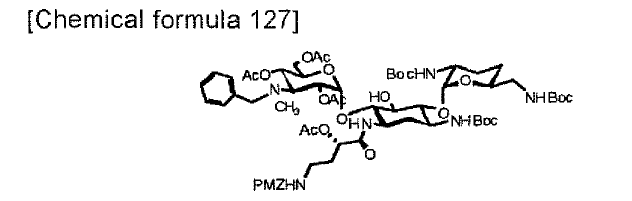 FABMS: m/z 1289 [M+H]⁺

Production step 28-(d)
200 mg of the compound produced in production step 28-(c) was used in the same manner as in production steps 1-(d) and 1-(e) to give the following compound (134 mg). 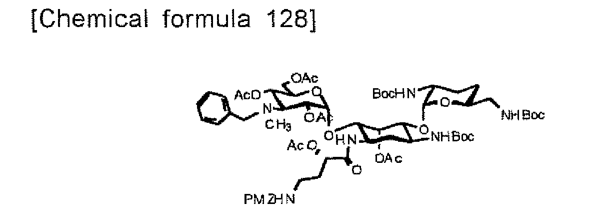 FABMS: m/z 1331 [M+H]⁺

Production step 28-(e)
130 mg of the compound produced in production step 28-(d) was used in the same manner as in production steps 1-(i) and 1-(j) to give the following compound (19 mg). 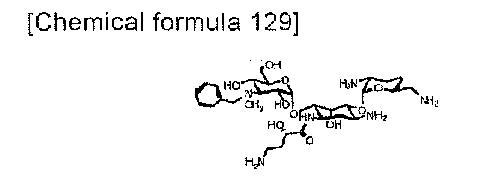 FABMS: m/z 657 [M+H]⁺

Production step 28-(f)
The compound (19.0 mg) produced in production step 28-(e) was dissolved in 5.0 mL of water, and 0.5 mL of 1 N hydrochloric acid was added to the solution. Under an argon gas stream, 5 mg of 10%Pd-C was added thereto, the air in the system was replaced by hydrogen, and the mixture was stirred at room temperature overnight. The reaction solution was filtered through Celite and was purified by CM-Sephadex(NH₄⁺) to give the title compound: 5-epi-3"-N-methylarbekacin (16.0 mg).
FABMS: 567 [M+H]⁺
¹H-NMR (D₂O+ND₃) δ: 1.40 (2H, m), 1.77 (4H, m), 1.95 (1 H, m), 2.05 (1 H, ddd, J = 4.2, 5.1, 13.2 Hz), 2.47 (3H, s), 2.70 (2H, m), 2.83 (4H, m), 3.26 (1 H, m), 3.40 (1H, dd, J = 9.8, 10.0 Hz), 3.50 (1H, dd, J = 2.2, 10.3 Hz), 3.57 (1 H, dd, J = 3.9, 10.5 Hz), 3.70 (1 H, dd, J = 7.0, 12.0 Hz), 3.85 (3H, m), 3.93 (1 H, dd, J = 2.0, 12.0 Hz), 4.23 (1 H, dd), 4.31 (1 H, m), 4.54 (1 H, dd), 4.98 (1 H, d, J = 3.6 Hz), 5.07 (1 H, d, J = 3.9 Hz).

Example 29

### 5-Epi-6'-N-methylarbekacin

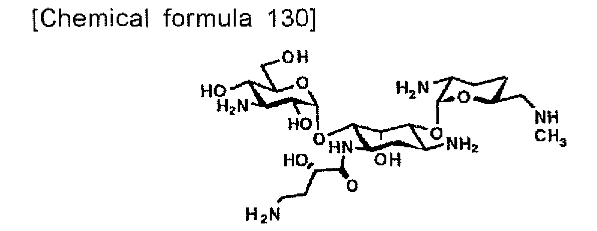

### Production step 29-(a)

The compound produced in production step 12-(f) was used in the acetylation of a hydroxyl group carried out in the same manner as in production step 2-(a). The compound thus obtained was used in the reversal reaction of the hydroxyl group at the 5-position carried out in the same manner as in production steps 1-(d) and 1-(e), followed by deprotection in the same manner as in production steps 1-(i) and 1-(j) to give the title compound: 5-epi-6'-N-methylarbekacin.
FABMS: m/z 567 [M+H]⁺

Example 30

### 6'-Aminomethyl-6'-deamino-5-epi-6'-hydroxyarbekacin

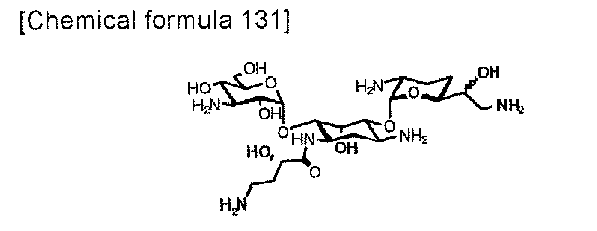

### Production step 30-(a)

A catalytic amount of p-TsOH·H₂O and 210 mg of 2,2-dimethoxypropane were added to a solution of 620 mg of the compound produced in production step 12-(b) dissolved in 10 mL of N,N-dimethylformamide, and the mixture was stirred at room temperature for 20 hr. Triethylamine was added to the reaction solution, and the mixture was concentrated under the reduced pressure. The residue was washed with isopropyl ether and was then dried under the reduced pressure to give 640 mg of the following compound as a solid.

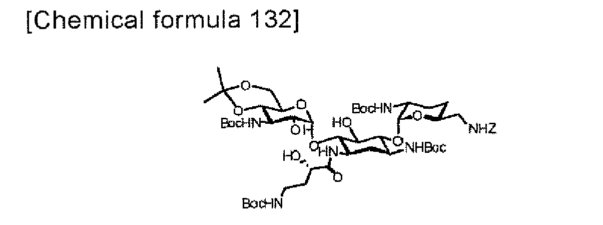

Production step 30-(b)
The compound (640 mg) produced in production step 30-(a) was dissolved in 10 mL of dioxane, 10 mL of methanol, and 8 mL of water, 170 mg of palladium hydroxide was added to the solution, and the mixture was subjected to a catalytic hydrogen reduction reaction at a hydrogen pressure of 30 Ibs for 16 hr. The reaction solution was filtered through Celite and was washed with a solution of methanol : water = 1 : 1, and the filtrate was concentrated to dryness to give 560 mg of the following compound.

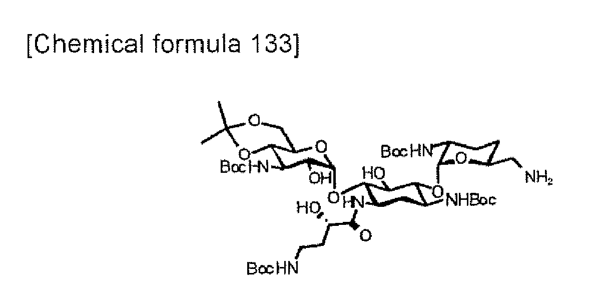

Production step 30-(c)
The compound (500 mg) produced in production step 30-(b) was dissolved in 12 mL of chloroform and 6 mL of water. Ninhydrin (440 mg) and 210 mg of sodium hydrogencarbonate were added to the solution, and the mixture was stirred at room temperature for 16 hr. Methylene chloride was added to the reaction solution. The mixture was washed with water, was dried over anhydrous magnesium sulfate, and was then concentrated under the reduced pressure. The residue was purified by column chromatography on silica gel (development system, methylene chloride : methanol = 15 : 1) to give 256 mg of the following compound.

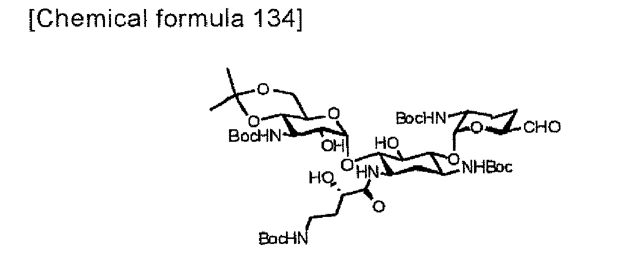

Production step 30-(d)
The compound (100 mg) produced in production step 30-(c) was dissolved in 5 mL of methylene chloride, 2 mL of tetrahydrofuran, and 1 mL of methanol. Nitromethane (60 mg), and 100 mg of a methanol solution of sodium methoxide (1 M) were added to the solution, and the mixture was stirred for 1 hr. This reaction solution was neutralized with a 1 M hydrochloric acid solution. Chloroform was added thereto, and the mixture was washed with water and was then dried over anhydrous magnesium sulfate. The solution thus obtained was concentrated under the reduced pressure, and the residue was purified by column chromatography on silica gel (development system, methylene chloride : methanol = 15 : 1) to give 82 mg of the following compound.

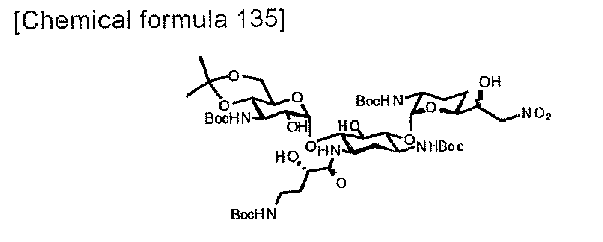

Production step 30-(e)
The compound (82 mg) produced in production step 30-(d) was dissolved in 7 mL of methanol, 3 mL of water, and 0.2 mL of acetic acid. Platinum oxide (50 mg) was added to the solution, and the mixture was subjected to a catalytic hydrogen reduction reaction at a hydrogen pressure of 40 lbs for 20 hr. The reaction solution was filtered through Celite, followed by washing with a solution of methanol : water = 1 : 1. The filtrate was then concentrated under the reduced pressure, and deprotection was carried out in the same manner as in production step 1-(j). The compound thus obtained was used for the protection of the amino group in the same manner as in production step 1-(a). Thereafter, a reaction was allowed to proceed in the same manner as in Example 13 to give the title compound: 6'-aminomethyl-6'-deamino-5-epi-6'-hydroxyarbekacin.
FABMS: m/z 583 [M+H]⁺

Example 31

### 6'-Aminomethyl-6'-deamino-5-deoxy-5-epiacetoxy-6'-hydroxy-arbekacin

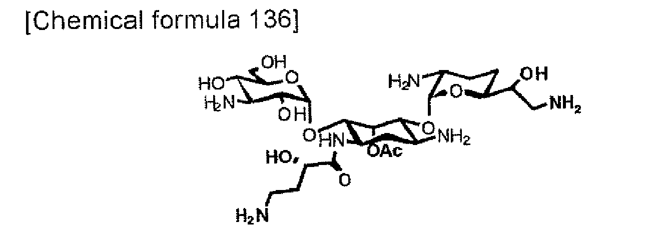

### Production step 31-(a)

A reaction was allowed to proceed in the same manner as in Example 30 to give 6'-aminomethyl-6'-deamino-5-epi-6'-hydroxyarbekacin and the title compound: 6'-aminomethyl-6'-deamino-5-deoxy-5-epiacetoxy-6'-hydroxyarbekacin.
FABMS: m/z 625 [M+H]⁺

Example 32

### 6"-Deoxy-5-epiarbekacin

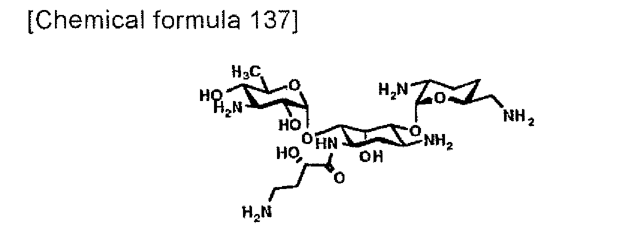

### Production step 32-(a)

The compound (1.55 g) produced in production step 1-(a) was dissolved in 20 mL of dimethylformamide. p-TsOH·H₂O (100 mg) and 2 mL of benzaldehyde dimethylacetal were added to the solution, and the mixture was stirred at 5°C for 5 hr. Triethylamine was added to the reaction solution, and the mixture was neutralized and was concentrated under the reduced pressure. Pyridine (30 mL) and 1.5 mL of acetic anhydride were added to the residue, and the mixture was stirred at room temperature for 3 days. Methanol (20 mL) was added to the reaction solution, and the mixture was concentrated under the reduced pressure. Ethyl acetate was added to the residue, and the mixture was washed with an aqueous potassium hydrogensulfate solution, an aqueous sodium hydrogencarbonate solution, and water in that order and dried over anhydrous magnesium sulfate. The solution thus obtained was concentrated under the reduced pressure, and the residue was purified by column chromatography on silica gel (development system, ethyl acetate : n-hexane = 1 : 1) to give 1.13 g of the following compound.

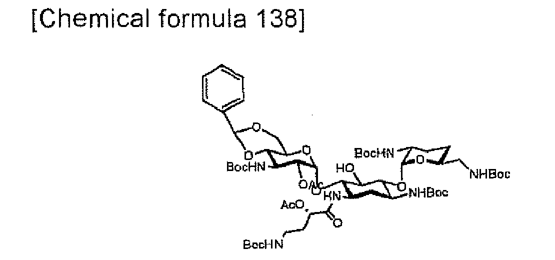

Production step 32-(b)
The compound (200 mg) produced in production step 32-(a) was dissolved in 6 mL of carbon tetrachloride, 50 mg of N-bromosuccinimide and 18 mg of barium carbonate were added to the solution, and the mixture was stirred under reflux for 3 hr. The reaction solution was concentrated under the reduced pressure, and the residue was purified by column chromatography on silica gel (development system, ethyl acetate : toluene = 1 : 1) to give 154 mg of the following compound.

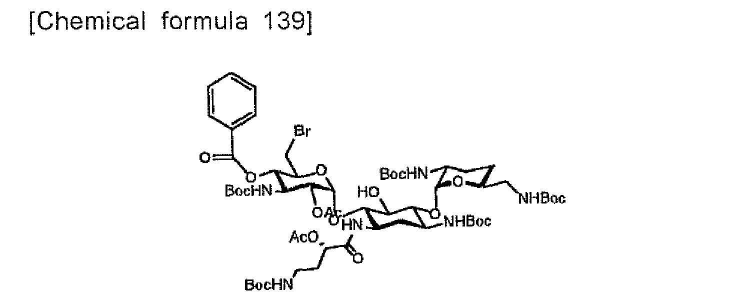

Production step 32-(c)
67 mg of the compound produced in production step 32-(b) was used in the reduction of a halogen carried out in the same manner as in production step 8-(b), to give 65 mg of the following compound.

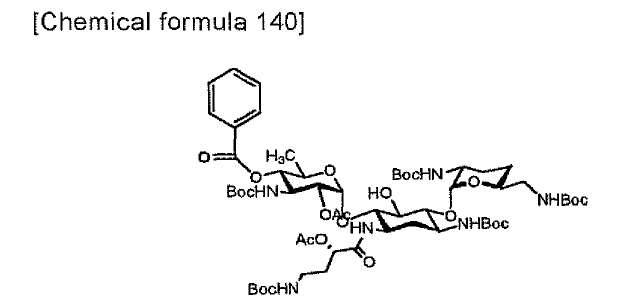

Production step 32-(d)
25 mg of the compound produced in Production step 32-(c) was used in the same manner as in production step 1-(d) to give the following compound (30 mg). 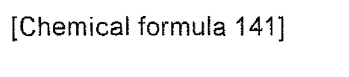 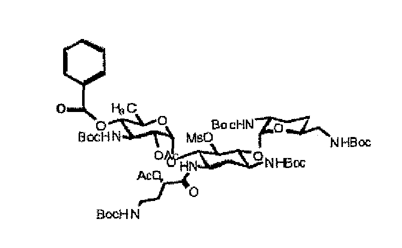 TSPMS: m/z 1303 [M+H]⁺

Production step 32-(e)
30 mg of the compound produced in production step 32-(d) was used in the same manner as in production step 1-(e). The compound thus obtained was then deprotected in the same manner as in production steps 1-(i) and 1-(j) to give the title compound: 6"-deoxy-5-epiarbekacin.
FABMS: m/z 537 [M+H]⁺

Example 33

### 6"-Aminomethyl-5-epiarbekacin

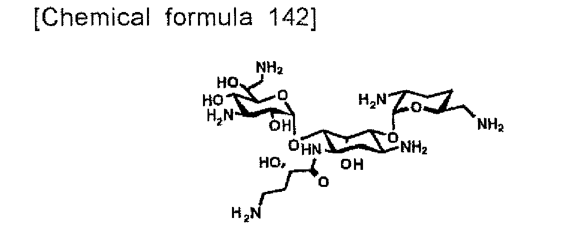

### Production step 33-(a)

The compound produced in production step 13-(c) was subjected to the deprotection of the hydroxyl group in the same manner as in production step 1-(i). 0.33 g of the compound thus obtained was used in the same manner as in production step 1-(g) to give the following compound (0.32 g).

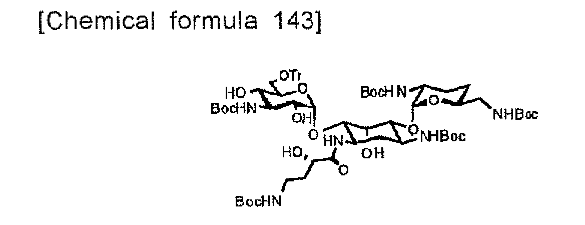

Production step 33-(b)
0.32 g of the compound produced in production step 33-(a) was used in the same manner as in production step 2-(a) to give the following compound (0.29 g).

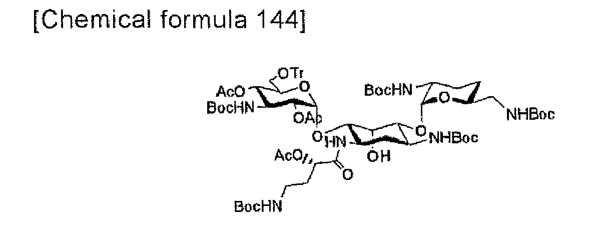

Production step 33-(c)
The compound (0.36 g) produced in production step 33-(b) was dissolved in 0.75 mL of diethyl ether and 0.75 mL of formic acid. The solution was stirred for 20 min and was then extracted with methylene chloride, followed by washing with an aqueous saturated sodium hydroxide solution. The extract was then dried over anhydrous magnesium sulfate and was then concentrated under the reduced pressure. The residue was dissolved in 12.3 mL of toluene and 1.6 mL of DMSO. Pyridine (0.085 mL), 0.027 mL of trifluoroacetic acid, and 0.26 g of 1,3-dicyclohexylcarbodiimide were added to the solution, and the mixture was stirred at room temperature overnight. The reaction solution was then extracted with ethyl acetate, and the extract was concentrated under the reduced pressure. The residue was then purified by column chromatography on silica gel (methylene chloride : methanol = 15 : 1) to give 0.05 g of the following compound.

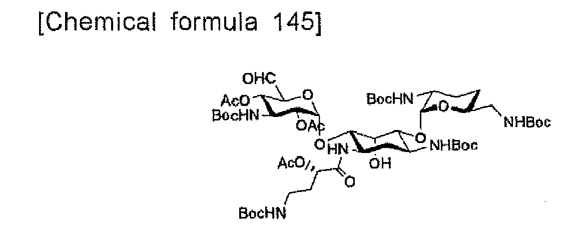

Production step 33-(d)
100 mg of the compound produced in production step 33-(c) was used in the same manner as in production step 30-(d) t o give the following compound (38 mg).

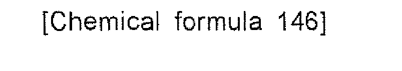

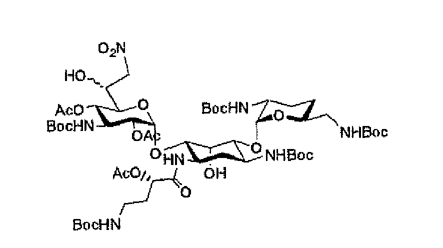

Production step 33-(e)
38 mg of the compound produced in production step 33-(d) was used in the same manner as in production step 30-(e) to give the title compound: 6"-aminomethyl-5-epiarbekacin (20 mg).
FABMS: m/z 582 [M+H]⁺.

Example 34

### 5-Methylarbekacin

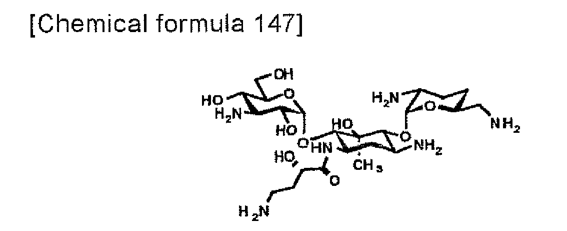

### Production step 34-(a)

The following compound (6.61 g) was produced in the same manner as in Example 14-(a).

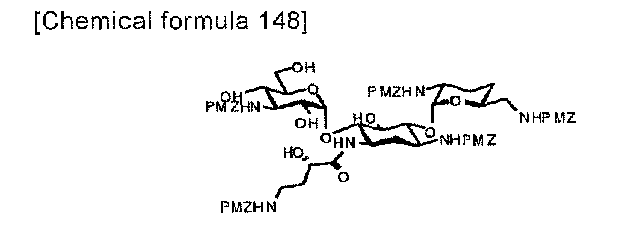

Production step 34-(b)
The compound (4.11 g) produced in production step 34-(a) was dissolved in 50 mL of pyridine, and, under ice cooling, a solution of 2.05 g of benzoyl chloride in 9 mL of methylene chloride was added to the solution. The mixture was stirred under ice cooling for 0.5 hr, and the temperature of the mixture was raised to room temperature before stirring for 4 hr. Thereafter, 0.1 mL of water was added thereto, followed by concentration under the reduced pressure. Ethyl acetate was added to the residue, and the mixture was washed with water, a 10% aqueous potassium hydrogensulfate solution, a saturated aqueous sodium hydrogencarbonate solution, and a saturated aqueous sodium chloride solution in that order, was then dried over anhydrous magnesium sulfate, and was then concentrated to dryness to give 5.22 g of the following compound.

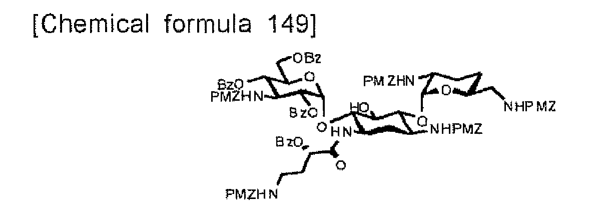

Production step 34-(c)
The compound (1.197 g) produced in production step 34-(b) was dissolved in 5 mL of dimethylsulfoxide, 1.5 mL of acetic anhydride was added to the solution, and the mixture was stirred at room temperature for 3 days. A saturated aqueous sodium hydrogencarbonate solution (1 mL) was added thereto, and the mixture was stirred for 1 hr. Ethyl acetate was added thereto, and the mixture was washed with water, a saturated aqueous sodium hydrogencarbonate solution, and a saturated aqueous sodium chloride solution in that order, was dried over anhydrous sodium sulfate, and was then concentrated to dryness to give 1.20 g of the following compound.

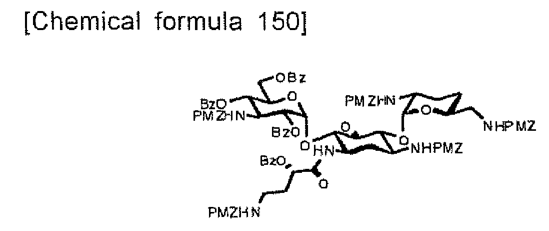

Production step 34-(d)
The compound (320 mg) produced in production step 34-(c) was dissolved in 4 mL of tetrahydrofuran, and 0.97 mL of a solution of methylmagnesium bromide (0.93 mmol/mL) in tetrahydrofuran was added to the solution under ice cooling, and the mixture was stirred under ice cooling for 3 hr. A saturated aqueous ammonium chloride solution was added to this reaction solution, and the mixture was extracted with ethyl acetate, followed by washing with saturated brine. The extract was then dried over anhydrous sodium sulfate. The solution thus obtained was concentrated under the reduced pressure, and the residue was purified by preparative TLC (development system, chloroform : methanol = 30 : 1) to give 100 mg of the following compound.

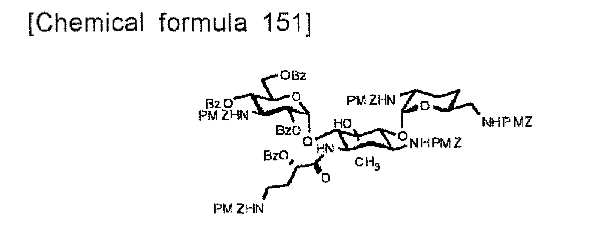

Production step 34-(e)
100 mg of the compound produced in production step 34-(d) was used in the same manner as in production steps 1-(i) and 1-(j) to give the title compound: 5-methylarbekacin (5.1 mg).
FABMS: m/z 567 [M+H]⁺;
1 H-NMR (D₂O-DCl) δ: 1.63 - 1.73 (1 H, m), 1.86 (1 H, ddd, J = 12.4, 12.4, 12.4 Hz), 1.96 - 2.03 (2H, m), 2.05 - 2.15 (2H, m), 2.21 (1 H, ddt, J = 3.7, 7.1, 14.7 Hz'), 2.31 (1H, ddd, J = 4.4, 4.4, 13.0 Hz), 3.21 (2H, d, J = 7.1, 7.1 Hz), 3.21 (1H, dd, J = 6.8, 13.1 Hz), 3.32 (1 H, dd, J = 3.5, 13.5 Hz), 3.37 (3H, s), 3.42 (1 H, dd, J = 10.5, 10.5 Hz), 3.47 - 3.56 (1H, m), 3.63 - 3.68 (1H, m), 3.75 (1 H, dd, J = 10.0, 10.0 Hz), 3.79 - 3.84 (3H, m), 3.87 (1 H, d, J = 10.8 Hz), 4.02 (1H, d, J = 10.7 Hz), 4.06 (1 H, dt, J = 3.2, 10.3 Hz), 4.10 - 4.16 (1 H, m), 4.18 - 4.25 (1 H, m), 4.31 (1 H, dd, J = 3.6, 9.3 Hz), 5.16 (1 H, d, J = 3.6 Hz), 5.83 (1 H, d, J = 3.7 Hz).

Example 35

### 5-Methoxymethylarbekacin

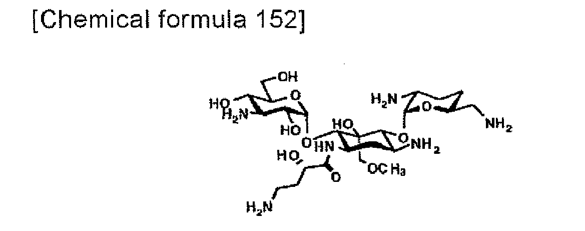

### Production step 35-(a)

The compound (60 mg) produced in production step 34-(c) was dissolved in 4 mL of methanol, 1.0 mL of a solution of diazomethane (0.6 mmol/mL) in diethyl ether was added to the solution under ice cooling, and the mixture was stirred under ice cooling for 2 hr. The reaction solution was concentrated under the reduced pressure, and the residue was purified by column chromatography on silica gel (development system, chloroform : methanol = 30 : 1) to give 50.6 mg of the following compound.

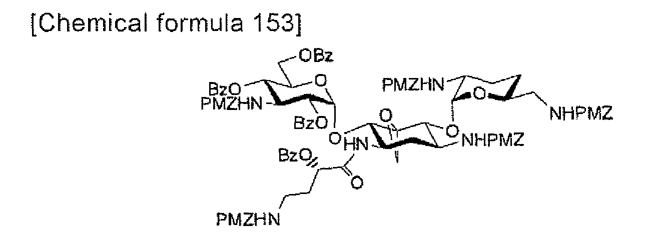

Production step 35-(b)
44 mg of the compound produced in production step 35-(a) was used in the same manner as in production steps 1-(i) and 1-(j) to give the title compound: 5-methoxymethylarbekacin (5.8 mg).
FABMS: m/z 597 [M+H]⁺;
¹H-NMR (D₂O+DCl) δ: 1.69 - 1.75 (1H, m), 1.90 (1H, ddd, J = 12.7, 12.7, 12.7 Hz), 1.99 - 2.06 (2H, m), 2.08 - 2.16 (2H, m), 2.24 (1 H, ddt, J = 3.9, 7.3, 14.4 Hz), 2.39 (1 H, ddd, J = 4.6, 4.6, 12.9 Hz), 3.20 - 3.24 (1 H, m), 3.24 (2H, dd, J = 7.4, 7.4 Hz), 3.35 (1 H, dd, J = 3.4, 13.6 Hz), 3.44 (1 H, dd, J = 10.5, 10.5 Hz), 3.50 (3H, s), 3.63 - 3.68 (1H, m), 3.77 (1H, dd, J = 10.0, 10.0 Hz), 3.82 (1H, d, J = 10.7 Hz), 3.86 (1H, dd, J = 3.7, 10.0 Hz), 3.87 (2H, d, J = 2.9 Hz), 3.87 - 3.92 (1H, m), 3.94 (1 H, d, J = 10.8 Hz), 4.02 (1H, d, J = 10.7 Hz), 4.06 (1 H, dt, J = 2.9, 10.0 Hz), 4.12 (1 H, d, J = 11.0 Hz), 4.26 (1 H, dddd, J = 3.4, 6.9, 6.9, 6.9 Hz) 4.33 (1 H, dd, J = 3.6, 6.9 Hz), 4.35-4.40 (1H, m), 5.19 (1 H, d, J = 3.6 Hz), 5.76 (1 H, d, J = 3.2 Hz).

Example 36

### 5-Vinylarbekacin

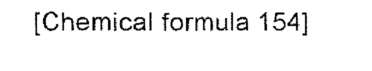

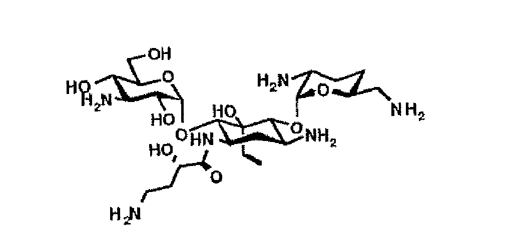

### Production step 36-(a)

The compound produced in production step 34-(c) was reacted with vinylmagnesium bromide in the same manner as in production step 34-(d), and the reaction product was then subjected to deprotection and purification in the same manner as in production steps 1-(i) and 1-(j) to give the title compound: 5-vinylarbekacin.
TSPMS: m/z 579 [M+H]⁺;
¹H-NMR (D₂O+ND₃) δ: 1.38 - 1.45 (2H, m), 1.54 - 1.63 (1H, m), 1.71 - 1.79 (3H, m), 1.89 - 1.94 (1H, m), 2.10 (1 H, ddd, J = 4.4, 4.4, 13.2 Hz), 2.64 - 2.68 (2H, m), 2.75 - 2.79 (2H, m), 2.85 (1H, ddd, J = 4.4, 4.4, 11.7 Hz), 2.88 (1H, dd, J = 10.0, 10.0 Hz), 3.01 - 3.08 (1H, m), 3.30 (1 H, dd, J = 10.0, 10.0 Hz), 3.37 (1H, dd, J = 3.6, 10.2 Hz), 3.43 (1H, d, J = 10.3 Hz), 3.70 - 3.85 (4H, m), 3.99 (1 H, dt, J = 2.9, 10.2 Hz'), 4.07 (1 H, ddd, J = 4.4, 10.8, 10.8 Hz), 4.18 (1 H, dd, J = 3.9, 9.2 Hz), 4.98 (1 H, d, J = 4.0 Hz), 5.12 (1H, d, J = 3.4 Hz), 5.53 (1H, dd, J = 1.4, 10.9 Hz), 5.58 (1H, dd, J = 1.4, 17.1 Hz), 6.09 (1 H, dd, J = 10.7, 16.8 Hz).

Example 37

### 5-Aminomethylarbekacin

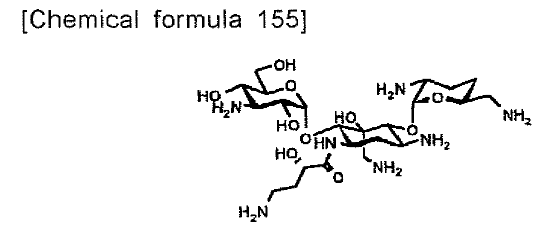

### Production step 37-(a)

The compound (50 mg) produced in production step 35-(a) was dissolved in 0.5 mL of dimethylformamide, 5.4 mg of sodium azide was added to the solution, and the mixture was stirred at 80°C for 2 hr. A saturated aqueous ammonium chloride solution was added to the reaction solution, and the mixture was extracted with ethyl acetate, washed with saturated brine, and then dried over anhydrous sodium sulfate. The solution thus obtained was concentrated under the reduced pressure, and the residue was purified by preparative TLC (development system, chloroform : methanol = 30 : 1) to give 24.3 mg of the following compound.

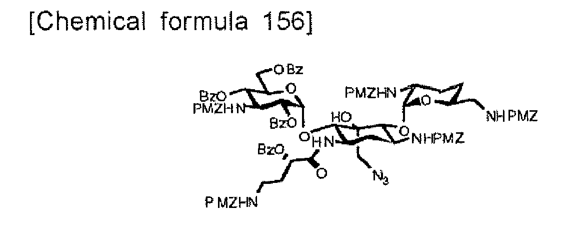

Production step 37-(b)
The following compound (5.3 mg) was produced from 24.3 mg of the compound produced in production step 37-(a) in the same manner as in production steps 1-(i) and 1-(j). 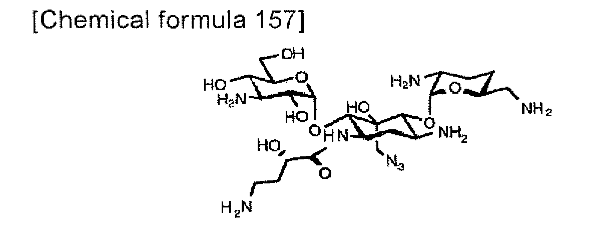 TSPMS: m/z 608 [M+H]⁺;
¹H-NMR (D₂O+DCl) δ: 1.66 - 1.77 (1 H, m), 1.92 (1 H, ddd, J = 12.4, 12.4, 12.4 Hz), 1.99 - 2.07 (2H, m), 2.08 - 2.16 (2H, m), 2.22 (1H, ddt, J = 3.9, 7.3, 14.4 Hz), 2.38 (1H, ddd, J = 4.9, 4.9, 12.9 Hz), 3.22 (2H, dd, J = 7.1, 7.1 Hz), 3.23 (1H, dd, J = 6.8, 13.9 Hz), 3.34 (1H, dd, J = 3.4, 13.4 Hz), 3.44 (1 H, dd, J = 10.5, 10.5 Hz'), 3.65 - 3.70 (1 H, m), 3.77 (1 H, dd, J = 10.0, 10.0 Hz), 3.80 - 3.85 (2H, m), 3.87 (2H, d, J = 3.4 Hz), 3.91 (1 H, d, J = 13.7 Hz), 3.96 (1H, d, J = 13.7 Hz), 4.05 (1H, d, J = 10.7 Hz), 4.08 (1 H, dt, J = 3.4, 9.8 Hz), 4.14 (1 H, d, J = 10.7 Hz), 4.21 - 4.33 (2H, m), 4.33 (1 H, dd, J = 3.7, 9.3 Hz), 5.18 (1 H, d, J = 3.6 Hz), 5.81 (1 H, d, J = 3.5 Hz).

Production step 37-(c)
The compound (5.3 mg) produced in production step 37-(b) was dissolved in 1 mL of water, 4.6 mg of 10% palladium-carbon was added to the solution, and the mixture was subjected to catalytic hydrogen reduction at room temperature under the atmospheric pressure for 3 hr. This reaction solution was filtered and was concentrated under the reduced pressure, and the residue was purified by CM-Sephadex (NH₄⁺) (development system; water : concentrated aqueous ammonia = 10 : 1) to give the title compound: 4.0 mg of 5-aminomethylarbekacin.
TSPMS: m/z 582 [M+H]⁺
¹H-NMR (D₂O+ND₃) δ: 1.70 - 1.81 (1 H, m), 1.95 - 2.10 (3H, m), 2.10 - 2.20 (2H, m), 2.25 (1 H, ddt, J = 3.7, 7.1, 14.4 Hz), 2.42 (1H, ddd, J = 4.8, 4.8, 12.9 Hz), 3.25 (2H, dd, J = 7.3, 7.3 Hz), 3.35 - 3.40 (3H, m), 3.55 (1 H, d, J = 13.9 Hz), 3.56 - 3.65 (1 H, m), 3.61 (1 H, d, J = 13.9 Hz), 3.74 - 3.77 (1 H, m), 3.78 (1 H, dd, J = 10.0, 10.0 Hz), 3.88 (1H, dd, J = 4.1, 11.8 Hz), 3.92 (1 H, dd, J = 3.9, 11.2 Hz), 3.97 (1 H, dd, J = 2.2, 12.2 Hz), 4.05 (1H, ddd, J = 2.2, 4.1, 10.0 Hz), 4.27 (1H, d, J = 11.0 Hz), 4.30 - 4.45 (4H, m), 5.29 (1 H, d, J = 4 Hz), 5.81 (1 H, d, J = 3.2 Hz).

Example 38

### 5-Methylaminomethylarbekacin

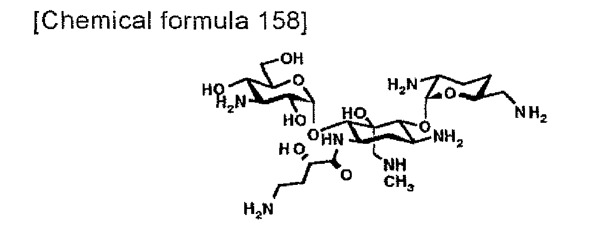

### Production step 38-(a)

The compound (150 mg) produced in production step 35-(a) was dissolved in 5 mL of a solution of methanol : methylene chloride =1:1, 0.5 mL of a 1 mol/L aqueous sodium hydroxide solution was added to the solution, and the mixture was stirred at room temperature for 4 hr. The reaction solution was neutralized with 1 mol/L hydrochloric acid and then concentrated under the reduced pressure. The residue was extracted with methylene chloride, dried over anhydrous magnesium sulfate, and then concentrated under the reduced pressure to give 110 mg of the following compound. 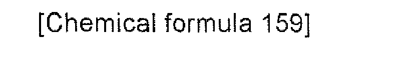 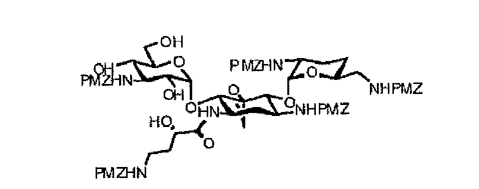 FABMS: m/z 1423 [M+K]⁺.

Production step 38-(b)
Ethanol (1 mL) and 1.1 mg of a 40% aqueous methylamine solution were added to 10 mg of the compound produced in production step 38-(a), and the mixture was stirred at 80°C for 3 hr. The reaction solution was concentrated under the reduced pressure, and the residue was deprotected and purified in the same manner as in production step 1-(j) to give the title compound: 5-methylaminomethylarbekacin (3.7 mg).
TSPMS: m/z 596 [M+H]⁺;
¹H-NMR (D₂O+ND₃), 1.38 (1 H, ddd, J = 12.7, 12.7, 12.7 Hz), 1.41 - 1.44 (1 H, m), 1.62 - 1.79 (4H, m), 1.91 - 1.93 (1 H, m), 2.22 (1 H, ddd, J = 4.7, 4.7, 13.2 Hz), 2.46 (3H, s), 2.65 - 2.70 (2H, m), 2.76 - 2.81 (2H, m), 2.90 (1H, dd, J = 10.3, 10.3 Hz), 2.93 - 3.01 (2H, m), 3.05 (1H, d, J = 13.2 Hz), 3.10 (1 H, d, J = 13.4 Hz), 3.31 (1H, dd, J = 9.8, 9.8 Hz), 3.39 (1 H, dd, J = 3.8, 10.4 Hz), 3.41 (1H, d, J = 10.3 Hz), 3.74 - 3.81 (3H, m), 3.82
- 3.92 (1 H, m), 4.04 (1 H, ddd, J = 2.6, 3.9, 10.3 Hz), 4.11 (1 H, ddd, J = 4.4, 12.2, 12.2 Hz), 4.18 (1 H, dd, J = 3.7, 9.3 Hz), 5.03 (1 H, d, J = 3.7 Hz), 5.07 (1 H, d, J = 3.4 Hz).

Example 39

### 5-(2-Hydroxyethyl)aminomethylarbekacin

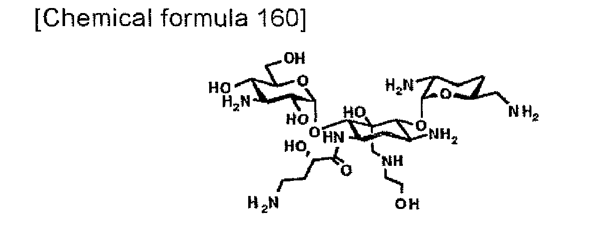

### Production step 39-(a)

The compound (12.3 mg) produced in production step 38-(a) was reacted with hydroxyethylamine in the same manner as in production step 38-(b) to give the title compound: 5-(2-hydroxyethyl)aminomethylarbekacin (4.4 mg).
TSPMS: m/z 626 [M+H]⁺;
¹H-NMR (D₂O+ND₃) δ: 1.35 (1H, ddd, J = 12.7, 12.7, 12.7 Hz), 1.37 - 1.50 (1 H, m), 1.60 - 1.85 (4H, m), 1.88 - 1.98 (1 H, m), 2.04 (1 H, ddd, J = 4.6, 4.6, 13.2 Hz), 2.62 - 2.70 (2H, m), 2.72 - 2.83 (4H, m), 2.85 - 3.00 (3H, m), 3.00 (1H, d, J = 13.0 Hz), 3.05 (1H, d, J = 13.0 Hz), 3.31 (1 H, dd, J = 9.8, 9.8 Hz), 3.38 (1 H, dd, J = 3.7, 10.5 Hz), 3.39 (1 H, d, J = 10.0 Hz), 3.65 - 3.73 (2H, m), 3.75 - 3.83 (3H, m), 3.85 - 3.95 (1 H, m), 4.02 - 4.08 (1 H, m), 4.13 (1 H, ddd, J = 3.9, 11.4, 11.4 Hz), 4.18 (1H, dd, J = 3.6, 9.3 Hz), 5.03 (1 H, d, J = 4.4 Hz), 5.04 (1 H, d, J = 4.2 Hz).

Example 40

### 5-(2-Aminoethyl)aminomethylarbekacin

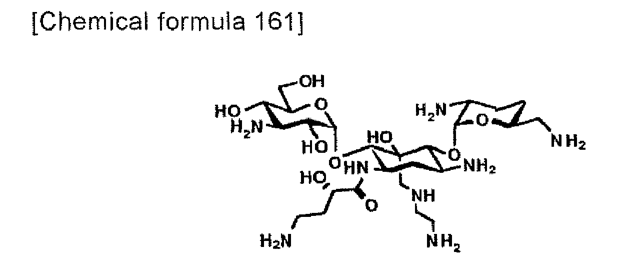

### Production step 40-(a)

The compound (15 mg) produced in production step 38-(a) was reacted with diaminoethane in the same manner as in production step 38-(b) to give the title compound: 5-(2-aminoethyl)aminomethylarbekacin (4.4 mg).
TSPMS: m/z 625 [M+H]⁺;
¹H-NMR (D₂O+ND₃) δ: 1.35 (1 H, ddd, J = 12.9, 12.9, 12.9 Hz), 1.42 (1 H, dddd, J = 4.9, 12.4, 12.4, 12.4 Hz), 1.64 (1H, dddd, J = 3.7, 12.4, 12.4, 12.4 Hz), 1.71 - 1.79(3H, m), 1.91 - 1.93 (1 H, m), 2.03 (1 H, ddd, J = 4.7, 4,7, 13.2 Hz), 2.62 - 2.82 (6H, m), 2.88 - 2.99 (3H, m), 2.99 (1 H, d, J = 13.2 Hz), 3.05 (1H, d, J = 12.9 Hz), 3.31 (1H, dd, J = 9.7, 9.7 Hz), 3.38 (1 H, dd, J = 3.7, 10.5 Hz), 3.40 (1 H, d, J = 10.2 Hz), 3.73 - 3.79 (3H, m), 3.84 - 3.92 (1 H, m), 4.02 (1 H, dt, J = 2.9, 9.5 Hz), 4.13 (1 H, ddd, J = 3.8, 11.4, 11.4 Hz), 4.18 (1H, dd, J = 3.6, 9.2 Hz), 5.04 (1 H, d, J = 3.9 Hz), 5.07 (1H, d, J = 3.2 Hz).

Example 41

### 5-(3-Aminopropyl)aminomethylarbekacin

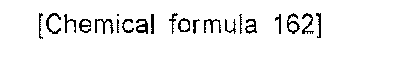

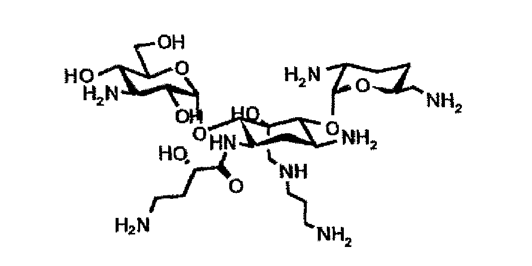

### Production step 41-(a)

The compound (30 mg) produced in production step 38-(a) was reacted with 1,3-diaminopropane in the same manner as in production step 38-(b) to give the title compound: 5-(3-aminopropyl)aminomethylarbekacin (4.0 mg).
FABMS: m/z 639 [M+H]⁺;
¹H-NMR (D₂O+ND₃) δ: 1.36 (1H, m), 1.43 (1H, m), 1.72 (5H, m), 1.94 (1H, m), 2.04 (1H, ddd, J = 4.4, 13.2 Hz), 2.68 (2H, m), 2.73 (4H, m), 2.78 (2H, m), 2.92 (2H, m), 2.99 (1 H, m), 3.04 (2H, m), 3.32 (1 H, dd, J = 10.0 Hz), 3.39 (2H, m), 3.79 (3H, m), 3.89 (1 H, m), 4.02 (1H, m), 4.12 (1H, ddd, J = 4.3, 11.4 Hz), 4.19 (1 H, dd, J = 3.6, 9.3 Hz), 5.03 (1 H, d, J =3.9 Hz), 5.05 (1 H, d, J = 3.4 Hz).

Example 42

### 5-(3-Amino-2-hydroxypropyl)aminomethylarbekacin

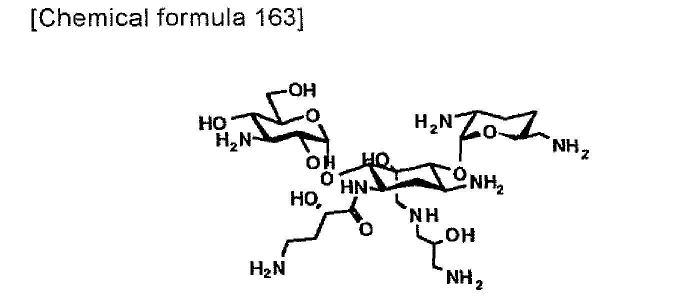

### Production step 42-(a)

The compound (30 mg) produced in production step 38-(a) was reacted with 1,3-diamino-2-hydroxypropane in the same manner as in production step 38-(b) to give the title compound: 5-(3-amino-2-hydroxypropyl)aminomethylarbekacin (10.2 mg).
TSPMS: m/z 655 [M+H]⁺;
¹H-NMR (D₂O+ND₃) δ: 1.47 (2H, m), 1.75 (1H, m), 1.83 (3H, m), 2.04 (1 H, m), 2.12 (1 H, m), 2.76 (6H, m), 2.90 (2H, m), 3.04 (4H, m), 3.16 (1 H, dd, J = 10.0, 13.0 Hz), 3.40 (1H, m), 3.47 (2H, m), 3.86 (3H, m), 3.98 (1 H, m), 4.12 (1H, m), 4.23 (1 H, dd), 4.31 (1H. m), 4.54 (1 H, dd), 4.98 (1H, d, J = 3.6 Hz), 5.07 (1 H, d, J = 3.9 Hz).

Example 43

### 5-(2-Pyridylmethyl)aminomethylarbekacin

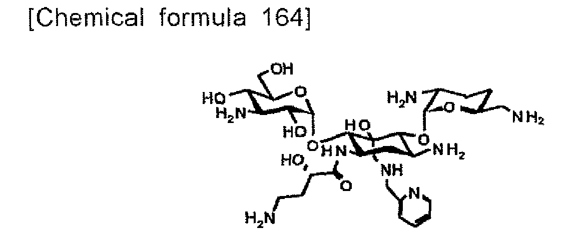

### Production step 43-(a)

The compound (20 mg) produced in production step 38-(a) was reacted with 2-(aminomethyl)pyridine in the same manner as in production step 38-(b) to give the title compound: 5-(2-pyridylmethyl)aminomethylarbekacin (10.2 mg).
TSPMS: m/z 673 [M+H]⁺.

Example 44

### 3',4'-didehydro-5-epiarbekacin

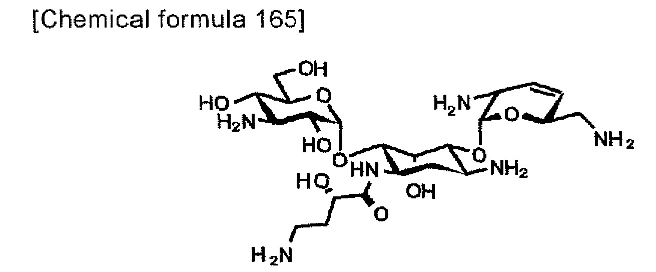

### Production step 44-(a)

3',4'-didehydroarbekacin was used in the same manner as in Example 13 to give the title compound: 3',4'-didehydro-5-epiarbekacin. FABMS: m/z 551 [M+H]⁺;
¹H-NMR (D₂O+DCl) δ: 1.77 (1H, ddd, J = 12.7, 12.7, 12.7 Hz), 1.92 - 2.01 (1 H, m), 2.19 (1H, ddt, J = 3.90, 7.32, 14.16 Hz), 2.30 (1 H, ddd, J = 4.89, 4.89, 12.69 Hz), 3.18 (2H, dd, J = 7.32, 7.32 Hz), 3.30 (1H, dd, J = 6.34, 13.67 Hz), 3.36 (1H, dd, J = 3.90, 13.67 Hz), 3.46 (1H, dd, J = 19.25, 10.25 Hz), 3.62 (1 H, dd, J = 9.77, 9.77 Hz), 3.74 (1H, dd, J = 6.83, 11.72 Hz), 3.75 - 3.79 (1H, m), 3.81 (1H, dd, J = 3.42, 10.75 Hz), 3.89 (1 H, ddd, J = 1.96, 6.84, 9.28 Hz), 3.94 (1 H, dd, J = 1.95, 11.72 Hz), 4.06 (1 H, dd, J = 2.44, 10.74 Hz), 4.19 - 4.25 (2H, m), 4.29 (1 H, dd, J = 3.91, 9.28 Hz), 4.36 (1 H, ddd, J = 4.88, 10.75, 10.75 Hz), 4.72 - 4.90 (2H, m), 5.18 (1 H, d, J = 3.91 Hz), 5.59 (1 H, d, J = 3.91 Hz), 6.04 (1 H, ddd, J = 2.93, 2.93, 10.75 Hz), 6.18 (1H, ddd, J = 2.44, 2.44, 10.25 Hz).

Example 45

### 5-Deoxy-3',4'-didehydro-5-epifluoroarbekacin

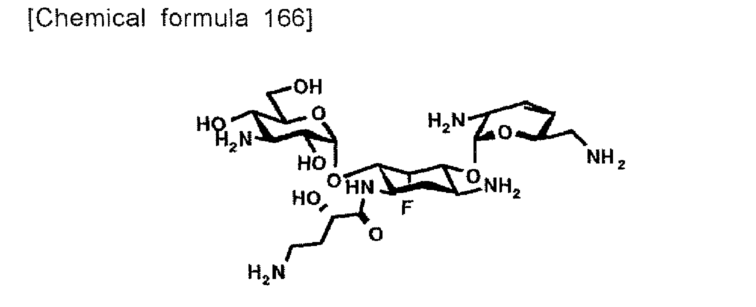

### Production step 45-(a)

The title compound: 5-deoxy-3',4'-didehydro-5-epifluoroarbekacin was produced from 3',4'-didehydroarbekacin in the same manner as in Example 13 and Production step 2-(e).
FABMS: m/z 553 [M+H]⁺.

Example 46

### 5-Deoxy-3',4'-didehydro-5-eioichloroarbekacin

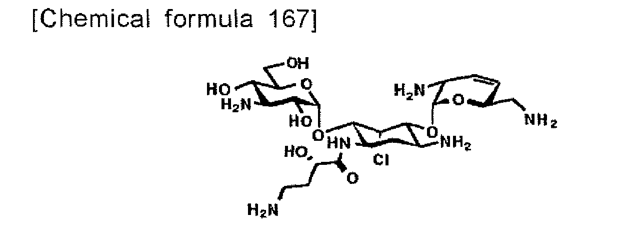

### Production step 46-(a)

The title compound: 5-deoxy-3',4'-didehydro-5-epichloroarbekacin (86 mg) was produced from 161 mg of 3',4'-didehydroarbekacin in the same manner as in Example 16.
FABMS: m/z 569 [M+H]⁺.

Example 47

### 5-Deoxy-3',4'-didehydro-5-epiazidoarbekacin

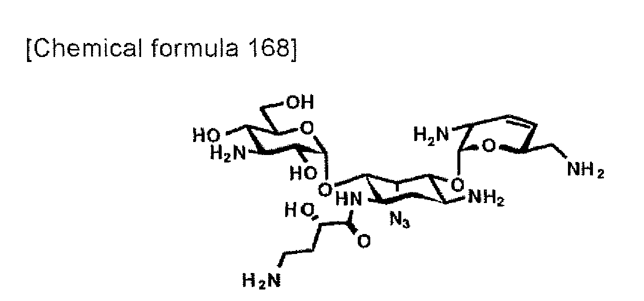

### Production step 47-(a)

The title compound: 5-deoxy-3',4'-didehydro-5-epiazidoarbekacin was produced from 3',4'-didehydroarbekacin in the same manner as in Example 17.
FABMS: m/z 576 [M+H]⁺.

Example 48

### 5-Deoxy-3',4'-didehydro-5-epiaminoarbekacin

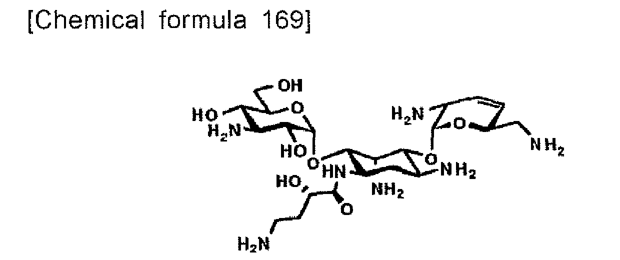

### Production step 48-(a)

The following compound (86 mg) was produced from 161 mg of 3',4'-didehydroarbekacin in the same manner as in Example 17.

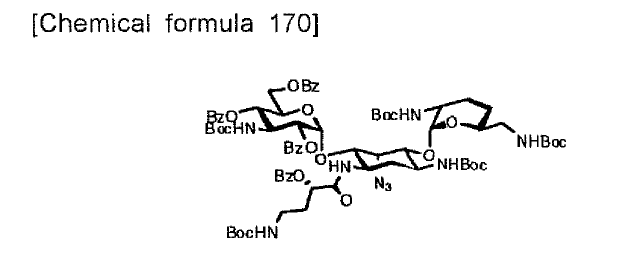

Production step 48-(b)
The compound (85 mg) produced in production step 48-(a) was dissolved in 2 mL of dimethylformamide and 6 µL of water. Tributylphosphine (0.03 mL) was added to the solution, and the mixture was stirred at room temperature for 15 hr. The reaction solution was concentrated to dryness. The residue was dissolved in ethyl acetate (10 mL), and the solution was washed with water and 10% brine in that order, dehydrated over anhydrous magnesium sulfate, and concentrated to dryness. The compound (5-epiamino form) was dissolved in 2.4 mL of methylene chloride and 1.2 mL of methanol. 0.1 mL of 0.5 M sodium methoxide was added to the solution under ice cooling, and the mixture was stirred at room temperature for 15 hr. Dry ice was added to the reaction solution, and the mixture was concentrated under the reduced pressure. The residue was washed with hexane, isopropyl ether, and water and was then dried under the reduced pressure to give 54 mg of the following compound.

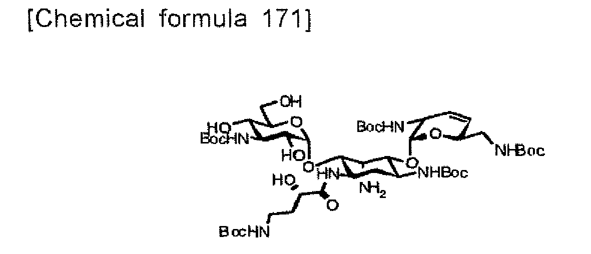

Production step 48-(c)
The title compound: 5-deoxy-3',4'-didehydro-5-epiaminoarbekacin (17 mg) was produced as a solid from 54 mg of the compound produced in production step 48-(b) in the same manner as in production step 1-(j).
FABMS: m/z 550 [M+H]⁺.

Example 49

### 1-N-[(S)-(3-Amino-2-hydroxypropanoyl)]-3',4'-didehydro-5-epidibekacin

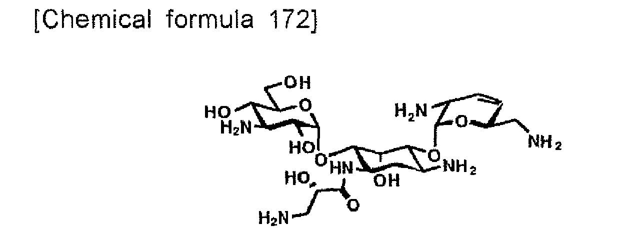

### Production step 49-(a)

2.70 g of 2',3,6'-tri-N-(t-butoxycarbonyl)-3"-N-trifluoroacetyl-3',4'-didehydrodibekacin was used in the same reaction as in production step 9-(a). The compound (3.03 g) thus obtained was used in the same manner as in production step 1-(c) to give a benzoylated compound (2.55 g). This compound was used in the same reaction as in production steps 1-(d) and (e). Further, the compound thus obtained was used in the same reaction as in production step 1-(i) to give a compound (1.77 g), which had been deprotected for benzoyl and acetyl groups. This compound (350 mg) was dissolved in 4 mL of anhydrous tetrahydrofuran. Further, under cooling, 20 mL of liquid ammonia was trapped in the reaction system, and 200 mg of metallic sodium was added thereto. After deep blue of the reaction solution was continued for 10 min, 515 mg of ammonium chloride was added to stop the reaction. Liquid ammonia was removed from the reaction system by evaporation at room temperature under a nitrogen gas stream. The reaction solution was then concentrated to dryness. The residue was extracted with chloroform : methanol = 5 : 1, followed by adjustment of pH to 6 by the addition of acetic acid. This solution was concentrated to dryness. The compound thus obtained was allowed to react in the same manner as in production step 9-(m), and deprotection was further carried out in the same manner as in production step 1-(j) to give the title compound: 1-N-[(S)-(3-amino-2-hydroxypropanoyl)]-3',4'-didehydro-5-epidibekacin (28 mg).
FABMS: m/z 537 [M+H]⁺.

Example 50

### 1-N-[(R)-(3-Amino-2-hydroxypropanoyl)]-3',4'-didehydro-5-epidibekacin

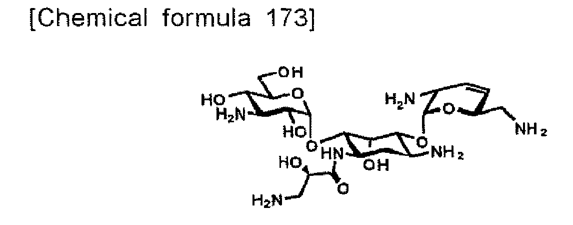

### Production step 50-(a)

The title compound: 1-N-[(R)-(3-amino-2-hydroxypropanoy 1)]-3',4'-didehydro-5-epidibekacin was produced by a reaction in the same manner as in Example 49.
FABMS: m/z 537 [M+H]⁺.

Example 51

### 3',4'-Didehydro-5,4"-diepiarbekacin

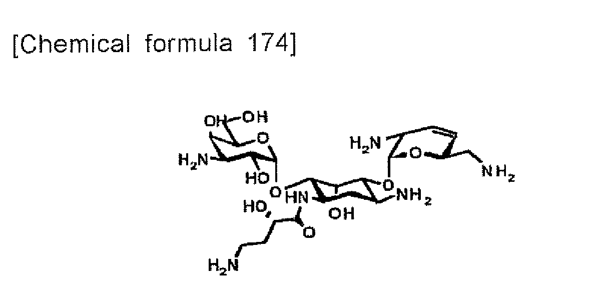

### Production step 51-(a)

The title compound: 3',4'-didehydro-5,4"-diepiarbekacin was produced from 3',4'-didehydroarbekacin in the same manner as in Example 1.
FABMS: m/z 551 [M+H]⁺;
¹H-NMR (D₂O+ND₃) δ: 1.35 (1H, m), 1.82 (1H, m), 2.02 (2H, m), 2.80 (2H, m), 2.89 (2H, m), 3.00 (1 H, dd, J = 2.9, 10.7 Hz), 3.18 (1 H, m), 3.53 (2H, m), 3.59 (1 H, dd, J = 3.9, 10.7 Hz), 3.76 (2H, m), 3.82 (1H, dd, J = 2.7, 10.7 Hz), 3.89 (1 H, brd), 4.09 (1H, m), 4.22 (1H, dd, J = 3.6, 9.3 Hz), 4.26 (1 H, m), 4.37 (1 H, m), 4.59 (1 H, m), 5.09 (1 H, d, J = 4.0 Hz), 5.16 (1 H, d, J = 4.2 Hz), 5.80 (1 H, s), 5.80 (1 H, s).

Example 52

### 5-Deoxy-3',4'-didehydro-4"-epi-5-epifluoroarbekacin

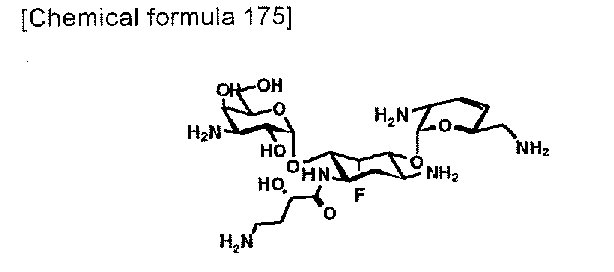

### Production step 52-(a)

The title compound: 5-deoxy-3',4'-didehydro-4"-epi-5-epifluoroarbekacin was produced from 3',4'-didehydroarbekacin in the same manner as in Example 2.
FABMS: m/z 553 [M+H]⁺;
¹H-NMR (D₂O+DCl) δ: 1.70 (1H, ddd, J = 12.7, 12.7, 12.7 Hz), 1.76 - 1.86 (1 H, m), 2.03 (1 H, ddt, J = 3.90, 7.32, 14.65 Hz), 2.20 (1 H, ddd, J = 4.39, 4.39, 13.19 Hz), 3.03 (2H, dd, J = 7.33, 7.33 Hz), 3.12 (1 H, dd, J = 6.35, 13.67 Hz), 3.22 (1 H, dd, J = 3.42, 13.67 Hz), 3.49 (1 H, dd, J = 2.93, 11.23 Hz), 3.59 - 3.66 (1 H, m), 3.63 (2H, d, J = 5.86 Hz), 3.84 (1 H, dd, J = 3.91, 10.74 Hz), 3.95 - 4.04 (3H, m), 4.07 - 4.10 (1H, m), 4.14 (1 H, dd, J = 8.91, 9.28 Hz), 4.15 - 4.23 (2H, m), 4.54 - 4.56 (1 H, m), 5.05 (1 H, d, J = 3.91 Hz), 5.48 (1H, d, J = 3.91 Hz), 5.50 (1H, d, J = 51.27 Hz), 5.86 (1 H, ddd, J = 1.95, 1.95, 11.23 Hz), 6.00 (1H, ddd, J = 1.95, 1.95, 10.74 Hz).

Example 53

### 5-Deoxy-3',4'-didehydro-4"-epi-5-epiaminoarbekacin

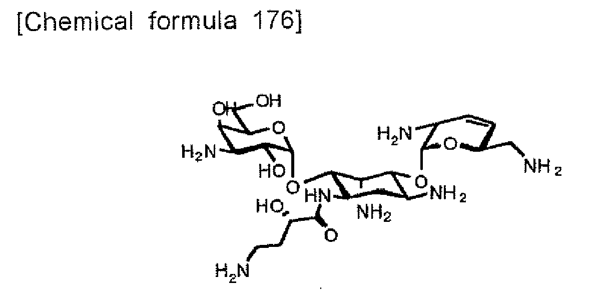

### Production step 53-(a)

The following compound was produced from 3',4'-didehy droarbekacin in the same manner as in Example 4.

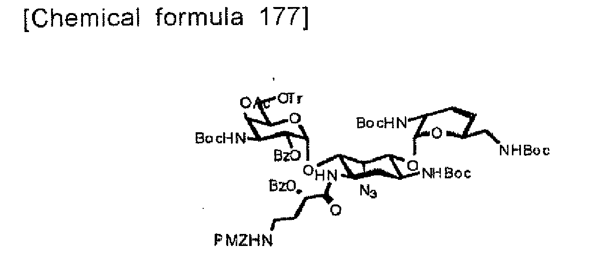

Production step 53-(b)
The title compound: 5-deoxy-3',4'-didehydro-4"-epi-5-epiaminoarbekacin (10 mg) was produced as a solid from 150 mg of the compound produced in production step 53-(a) in the same manner as in production steps 48-(b) and 48-(c).
ESIMS: m/z 572 [M+Na]⁺

Example 54

### 4"-Deoxy-3',4'-didehydro-5-epiarbekacin

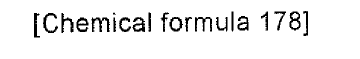

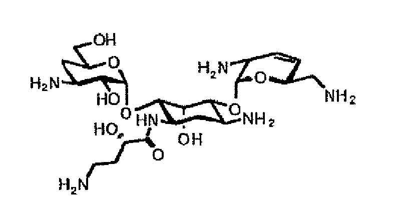

### Production step 54-(a)

1.50 g of 3,2',6',3"-tetra-N-t-butoxycarbonyl-3',4'-didehydro-4"'-p-methoxybenzyloxycarbonyl-arbekacin was used in the same reaction as in production steps 1-(b) to 1-(g)to give the following compound (0.74 g).

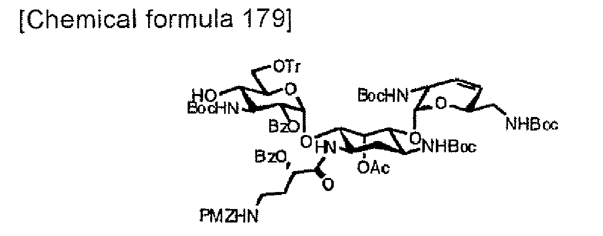

Production step 54-(b)
300 mg of the compound produced in production step 54-(a) was used in the same reation as in production steps 8-(a) and 8-(b) to give the following compound (100 mg).

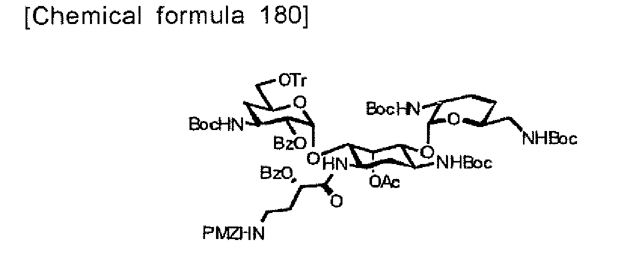

Production step 54-(c)
97 mg of the compound produced in production step 54-(b) was used for the deprotection in the same manner as in production step 1-(i) and 1-(j) to give the title compound: 4"-deoxy-3',4'-didehydro-5-epiarbekacin (20 mg).
ESIMS: m/z 557 [M+Na]⁺
¹H-NMR (D₂O+DCl) δ: 1.68 (1H, m), 1.79 (1 H, m), 2.01 (1H, m), 2.15 (1 H, m), 2.21 (1 H, m), 2.32 (1H, m), 2.32 (1 H, m), 3.21 (2H, m), 3.35 (2H, m), 3.73 (5H, m), 4.05 (1H, dd, J = 2.4, 10.7 Hz), 4.21 (2H, m), 4.25 (1H, brs), 4.32 (1 H, dd, J = 3.7, 9.3 Hz), 4.38 (1 H, m), 4.75 (1 H, brs), 5.22 (1H, J = 3.6 Hz), 5.61 (1 H, d, J = 3.9 Hz), 6.07 (1 H, m), 6.21 (1 H, m).

Example 55

### 5,6"-Dideoxy-3',4'-didehydro-6"-fluoro-5-epifluoroarbekacin

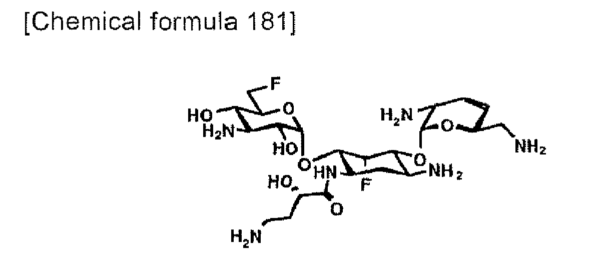

### Production step 55-(a)

2',3,6'-Tri-N-(t-butoxycarbonyl)-3"-N-trifluarnacetyl-3',4'-didehydrodibekacin (2.537 g) was reacted with (S)-4-(p-methoxybenzyloxycarbonylamino)-2-hydroxybutanoic acid in the same manner as in Example 9 to give 3.325 g of the following compound.

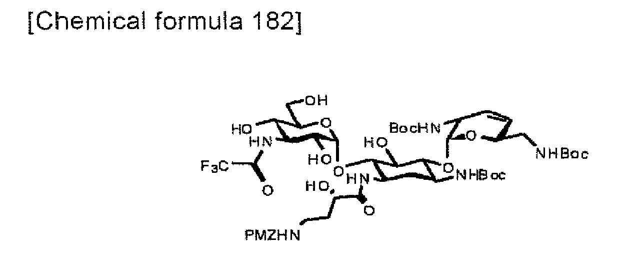

Production step 55-(b)
The following compound (1.71 g) was produced from 2.80 g of the compound produced in production step 55-(a) in the same manner as in production step 1-(g).

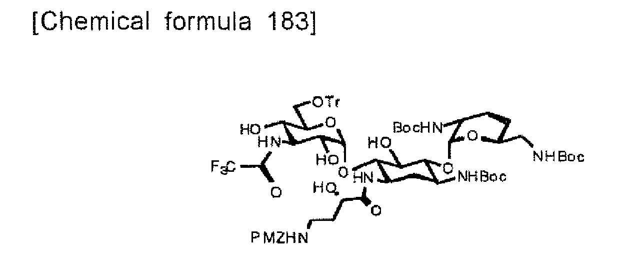

Production step 55-(c)
The following compound (1.64 g) was produced from 1.54 g of the compound produced in production step 55-(b) in the same manner as in production step 2-(a).

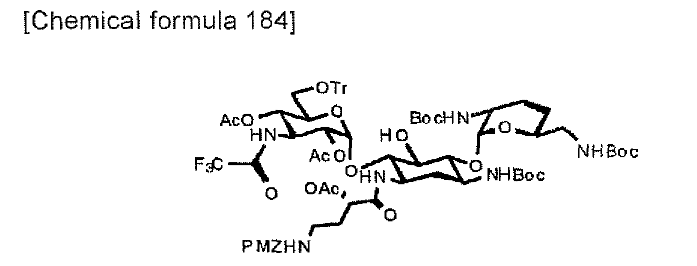

Production step 55-(d)
1.08 g of the compound produced in production step 55-(c) was used for the removal of a triphenylmethyl group in the same manner as in production step 33-(c) to give the following compound (700 mg).

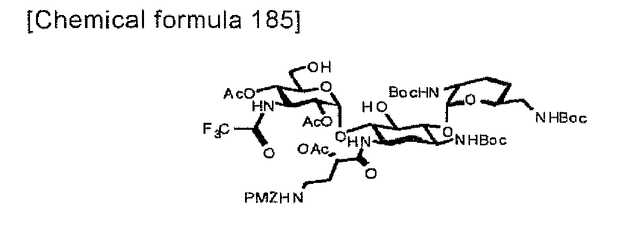

Production step 55-(e)
The following compound (180 mg) was produced from 340 mg of the compound produced in production step 55-(d) in the same manner as in production step 2-(e).

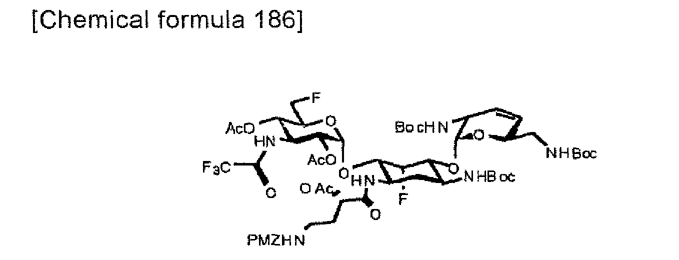

Production step 55-(f)
The title compound: 5,6"-dideoxy-3',4'-didehydro-6"-fluoro-5-epifluoroarbekacin (17 mg) was produced from 180 mg of the compound produced in production step 55-(e) in the same manner as in production steps 9-(b) and production step 1-(j).
FABMS: m/z 555 [M+H]⁺;
¹H-NMR (D₂O+DCl) δ: 1.71 (1H, ddd, J = 12.7, 12.7, 12.7 Hz), 1.77 - 1.89 (1 H, m), 2.03 (1 H, ddt, J = 3.90, 7.32, 14.65 Hz), 2.19 (1 H, ddd, J = 4.40, 4.40, 12.70 Hz), 3.02 (2H, dd, J = 7.32, 7.32 Hz), 3.14 (1 H, dd, J = 6.35, 13.67 Hz), 3.22 (1H, dd, J = 3.42, 13.67 Hz), 3.29 (1H, dd, J = 10,25, 10.25 Hz), 3.59 (1 H, dd, J = 10.26, 10.26 Hz), 3.62 - 3.65 (1 H, m), 3.69 (1 H, dd, J = 3.42, 10.75 Hz), 3.90 - 3.99 (1 H, m), 4.02 (1 H, ddd, J = 1.96, 10.74, 28.30 Hz), 4.08 - 4.10 (1 H, m), 4.14 (1H, dd, J = 3.42, 9.28 Hz), 4.17 - 4.25 (2H, m), 4.54 - 4.74 (3H, m'), 5.07 (1 H, d, J = 3.91 Hz), 5.31 (1H, d, J = 51.76 Hz), 5.50 (1H, d, J = 3.91 Hz), 5.87 (1H, ddd, J = 2.44, 2.44, 10.74 Hz), 6.01 (1H, ddd, J = 1.95, 1.95, 10.74 Hz).

Example 56

### 3"-N-Methylarbekacin

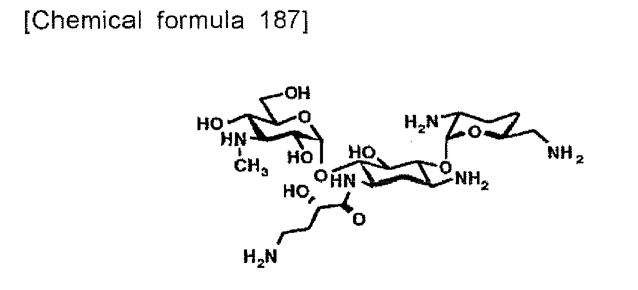

### Production step 56-(a)

4.10 g of the compound produced in production step 28-(b) was used for the Deprotection in the same manner as in production step 1-(j). A reaction was then allowed to proceed in the same manner as in production step 28-(f) to give the title compound: 3"-N-methylarbekacin (1.54 g).
FABMS: m/z 567 [M+H]⁺;
¹H-NMR (D₂O+ND₃) δ: 1.55 (2H, m), 1.76 (1H, m), 1.87 (3H, m), 2.04 (2H, m), 2.55 (3H, s), 2.78 (2H, m), 2.87 (3H, m), 2.98 (1 H, m), 3.04 (1 H, m), 3.46 (1H, dd, J = 9.3 Hz), 3.57 (1 H, dd, J = 9.7, 10.0 Hz), 3.64 (1 H, dd, J = 3.6, 10.5 Hz), 3.86 (4H, m), 3.98 (1 H, m), 4.10 (2H, m), 4.30 (1 H, dd, J = 3.6, 9.3 Hz), 5.20 (1 H, d, J = 3.6 Hz), 5.27 (1 H, d, J = 3.5 Hz).

Example 57

### 3"-N-Ethylarbekacin

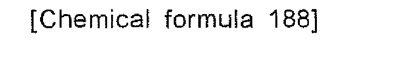

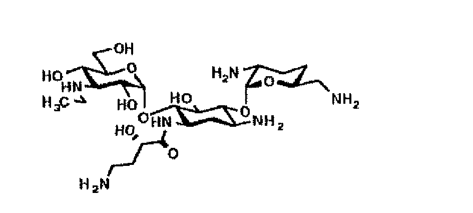

### Production step 57-(a)

The reaction was allowed to proceed in the same manner as in production step 28-(b) except acetaldehyde was used instead of formaldehyde. Further, the compound thus obtained was used in the same manner as in production step 56-(a) to give the title compound: 3"-N-ethylarbekacin.
FABMS: m/z 581 [M+H]⁺;
¹H NMR (D₂O+DCl) δ: 1.19 (3H, t, J = 7.3 Hz), 1.42 - 1.58 (1 H, m), 1.62 - 2.13 (7H, m), 2,93 - 3.46 (9H, m), 3.61 - 3.92 (8H, m), 3.94 - 4.09 (2H, m), 4.13 (1H, dd, J = 3.7, 9.3 Hz), 5.03 (1H, d, J = 3.7 Hz), 5.64 (1H, d, J = 3.6 Hz).

Example 58

### 3"-N-(2-Hydroxyethyl)arbekacin

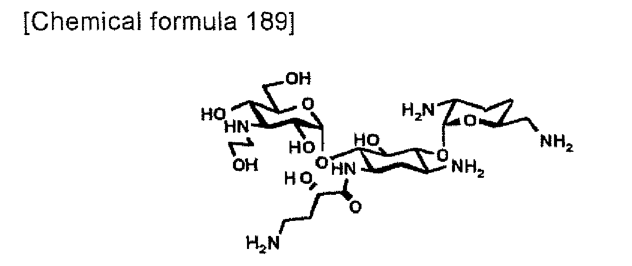

### Production step 58-(a)

3,2',6'-Tri-N-t-butoxycarbonyl-4"'-N-p-methoxybenzyloxycarbonyl-arbekacin (61 mg) was dissolved in 0.6 mL of DMF and 0.02 mL of water. Potassium carbonate (14 mg) and 42 mg of 2-(2-bromoethoxy)tetrahydro-2H-pyran were added to the solution, and the mixture was stirred at 60°C overnight. The reaction solution was concentrated under the reduced pressure. Water was added to the residue. The mixture was filtered, and the residue was then purified by preparative TLC. The compound thus obtained was treated in the same manner as in production step 1-(j) to give the title compound: 3"-N-(2-hydroxyethyl)arbekacin.
FABMS: m/z 597 [M+H]⁺.

Example 59

### 3"-N-Benzylarbekacin

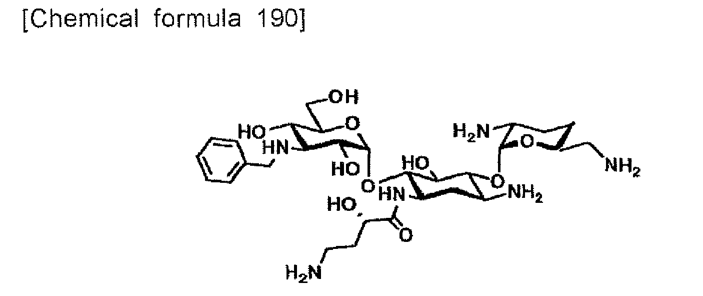

### Production step 59-(a)

85 mg of the compound produced in production step 28-(a) was used in the same manner as in production step 1-(j) to give the title compound: 3"-N-benzylarbekacin (56 mg).
TSPMS: m/z 643 [M+H]⁺;
¹H NMR (D₂O+DCl) δ: 1.58 - 1.72 (1H, m), 1.86 - 2.15 (5H, m), 2.16 - 2.33 (2H, m), 3.15 (1 H, dd, J = 7.3, 13.4 Hz), 3.21 (2H, t, J = 7.3 Hz), 3.30 (1 H, d, J = 7.3, 13.4 Hz), 3.51 - 3.64 (3H, m), 3.82 (1H, dd, J = 4.2, 12.5 Hz), 3.82 - 3.88 (1 H ,m), 3.87 (1 H, dd, J = 2.2, 11.7 Hz), 3.93 (1 H, t, J = 10.0 Hz), 3.94 (1H, t, J = 10.3 Hz), 3.97 - 4.08 (3H, m), 4.15 (1H, ddd, J = 4.6, 10.7, 12.2 Hz), 4.19 - 4.26 (1 H, m), 4.32 (1 H, dd, J = 3.8, 9.4 Hz), 4.47 (2H, q_{Ab}, J = 12.9, 16.9 Hz), 5.22 (1 H, d, J = 3.6 Hz), 5.81 (1 H, d, J = 3.7 Hz), 7.54 (5H, s).

Example 60

### 3"-N-(3-Hydroxybenzyl)arbekacin

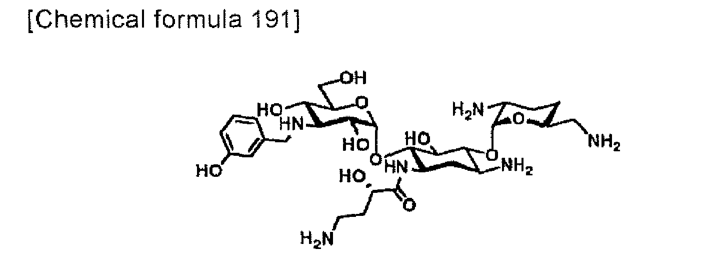

### Production step 60-(a)

A reaction was allowed to proceed in the same manner as in production step 28-(a), except that m-hydroxybenzaldehyde was used instead of benzaldehyde. The compound thus obtained was deprotected in the same manner as in production step 1-(j) to give the title compound: 3"-N-(3-hydroxybenzyl)arbekacin.
FABMS: m/z 659 [M+H]⁺;
¹H NMR (D₂O:DCl) δ: 1.36 - 1.51 (1H, m), 1.63 - 2.12 (7H, m), 2.89 - 3.12 (4H, m), 3.29 - 3.43 (3H, m), 3.57 - 4.23 (11 H, m), 4.65 (2H, s), 5.00 (1H, d, J = 3.6 Hz), 5.59 (1 H, d, J = 3.4 Hz), 6.77 - 6.89 (3H, m), 7.15 - 7.21 (1 H, m).

Example 61

### 3"-N-(4-Hydroxybenzyl)arbekacin

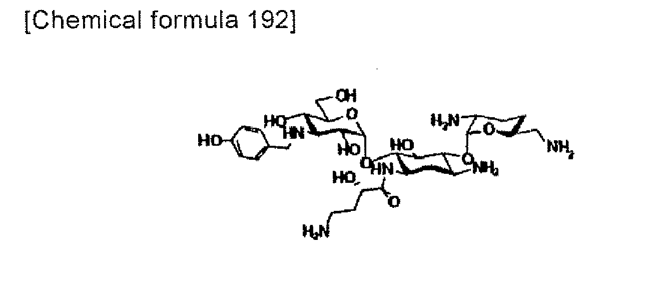

### Production step 61-(a)

A reaction was allowed to proceed in the same manner as in production step 28-(a), except that p-hydroxybenzaldehyde was used instead of benzaldehyde. The compound thus obtained was deprotected in the same manner as in production step 1-(j) to give the title compound: 3"-N-(4-hydroxybenzyl)arbekacin.
FABMS: m/z 659 [M+H]⁺;
¹H NMR (D₂O+DCl) δ: 1.35 - 1.48 (1H, m), 1.60 - 2.10 (7H, m), 2.87 - 3.09 (4H, m), 3.25 - 3.42 (3H, m), 3.54 - 4.04 (10H, m) 4.08 (1H, dd, J = 3.6, 9.5 Hz), 4.14 (2H, q_{Ab}, J = 13.2, 16.3 Hz), 4.97 (1 H, d, J = 3.6 Hz), 5.57 (1 H, d, J = 3.4 Hz), 6.74 (2H, d, J = 8.5 Hz), 7.17 (2H, d, J = 8.5 Hz).

Example 62

### 3"-N-(3-Aminobenzyl)arbekacin

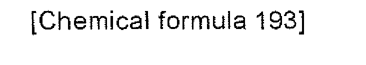

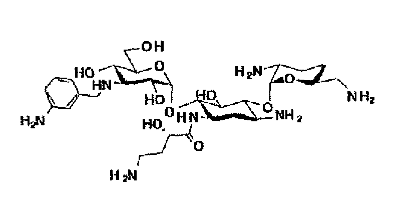

### Production step 62-(a)

A reaction was allowed to proceed in the same manner as in production step 28-(a), except that m-(t-butoxycarbonyl)aminobenzaldehyde was used instead of benzaldehyde. The compound thus obtained was deprotected in the same manner as in production step 1-(j) to give the title compound: 3"-N-(3-aminobenzyl)arbekacin.
FABMS: m/z 658 [M+H]⁺;
¹H NMR (D₂O+DCl) δ: 1.37 - 1.50 (1H, m), 1.63 - 2.11 (7H, m), 2.90 - 3.12 (4H, m), 3.30 - 3.43 (3H, m), 3.58 - 4.05 (10H, m), 4.09 - 4.14 (1 H, m), 4.32 (2H, q_{Ab}, J = 13.4, 15.6 Hz), 5.01 (1 H, d, J = 3.7Hz), 5.60 (1 H, d, J = 3.5 Hz), 7.31 - 7.48 (4H, m).

Example 63

### 3"-N-(2-Phenyletyl)arbekacin

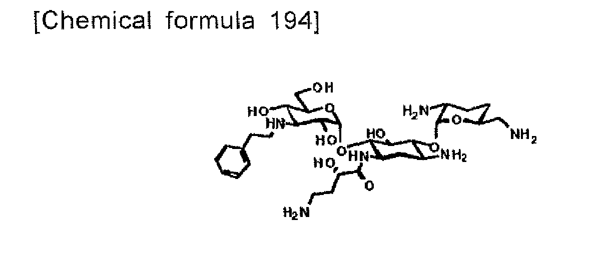

### Production step 63-(a)

A reaction was allowed to proceed in the same manner as in production step 28-(a), except that 3-phenylpropionaldehyde was used instead of benzaldehyde. The compound thus obtained was deprotected in the same manner as in production step 1-(j) to give the title compound: 3"-N-(2-phenylethyl)arbekacin.
FABMS: m/z 657 [M+H]⁺;
¹H NMR (D₂O+DCl) δ: 1.28 - 1.41 (1H, m), 1.55 - 2.02 (7H, m), 2.80 - 3.03 (6H, m), 3.21 - 3.34 (5H, m), 3.47 - 3.97 (10H, m), 4.00 (1 H, dd, J = 3.7, 9.5 Hz), 4.90 (1H, d, J = 3.7 Hz), 5.52 (1H, d, J = 3.4 Hz), 7.05 - 7.18 (5H, m).

Example 64

### 3"-N-Amidinoarbekacin

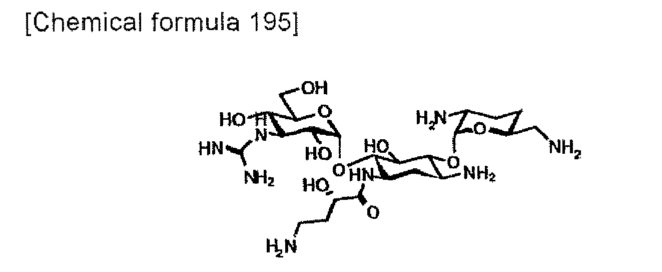

### Production step 64-(a)

3,2',6'-Tri-N-t-butoxycarbonyl-4"'-N-p-methoxybenzyloxycarbonyl-arbekacin (104 mg) was dissolved in 1 mL of N,N-dimethylformamide. A solution (0.2 mL) of 40 mg of N,N'-bis(tert-butoxycarbonyl)-S-methylisothiourea in N,N-dimethylformamide, 0.034 mL of triethylamine, and a solution of 38 mg of mercury chloride in 0.2 mL of N,N-dimethylformamide were added to the solution in that order, and the mixture was stirred at room temperature for 3 hr. The reaction solution was filtered and concentrated under the reduced pressure, and the residue was purified by column chromatography on silica gel (chloroform : methanol = 10 : 1). The compound thus obtained was deprotected in the same manner as in production step 1-(j) to give the title compound: 3"-N-amidinoarbekacin (43 mg).
FABMS: m/z 595 [M+H]⁺.

Example 65

### 3"-N-Formimidoylarbekacin

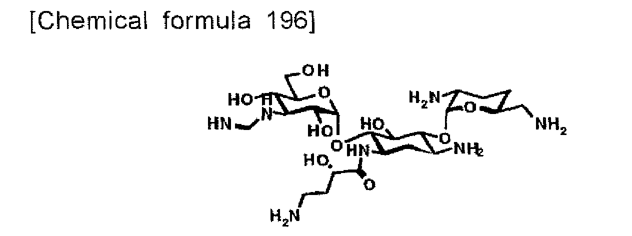

### Production step 65-(a)

3,2',6'-Tri-N-t-butoxycarbonyl-4"'-N-p-methoxybenzyloxy-carbonylarbekacin (102 mg) was dissolved in 4 mL of methanol. Ethylformimidate hydrochloride (37 mg) was added to the solution, and the mixture was stirred at room temperature for 2 days. The reaction solution was concentrated to dryness, and the residue was washed with diethyl ether. The solid thus obtained was deprotected in the same manner as in production step 1-(j) to give the title compound: 3"-N-formimidoyl arbekacin.
FABMS: m/z 580 [M+H]⁺.

Example 66

### 6'-N-(2-Hydroxybenzyl)arbekacin

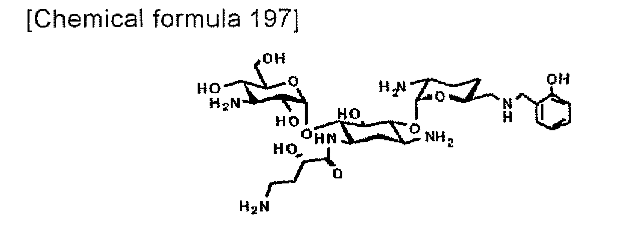

### Production step 66-(a)

= The compound (50 mg) produced in production step 12-(c) was dissolved in 1 mL of methanol. 2-Hydroxybenzaldehyde (9.6 mg) was added to the solution, and the mixture was stirred at room temperature for 2 hr. Further, 16 mg of sodium borohydride was added to this reaction solution, and the mixture was stirred for 1 hr. This reaction solution was filtered and was concentrated under the reduced pressure, and the residue was subjected to deprotection in the same manner as in production step 1-(j) to give the title compound: 6'-N-(2-hydroxybenzyl)arbekacin (27.4 mg).
FABMS: m/z 659 [M+H]⁺.

Example 67

### 6'-N,N-Bis(2-aminoethyl)arbekacin

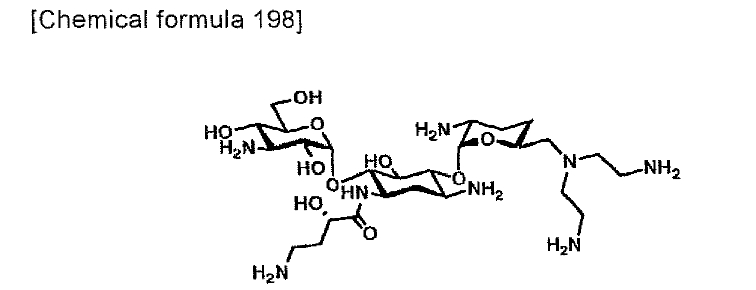

### Production step 67-(a)

A catalytic amount of p-TsOH·H₂O and 2,2-dimethoxypropane (210 mg) were added to a solution of the compound (620 mg) produced in production step 12-(b) dissolved in 10 mL of N,N-dimethylformamide, and the mixture was stirred at room temperature for 20 hr. Triethylamine was added to the reaction solution, and the mixture was concentrated under the reduced pressure. The residue was washed with isopropyl ether and dried under the reduced pressure to give 640 mg of the following compound.

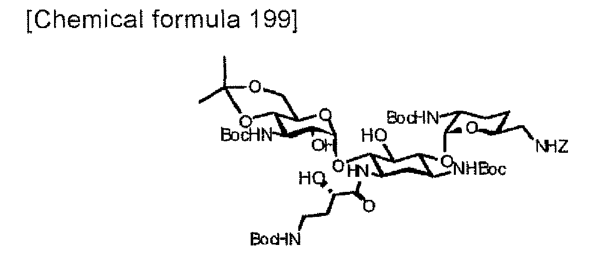

Production step 67-(b)
The compound (640 mg) produced in production step 67-(a) was dissolved in a solution of 10 mL of dioxane, 10 mL of methanol, and 8 mL of water. Palladium hydroxide (170 mg) was added to the solution, and the mixture was subjected to a catalytic hydrogen reduction reaction at a hydrogen pressure of 30 Ibs for 16 hr. This reaction solution was filtered through Celite and washd with a solution of methanol : water = 1 : 1, and the filtrate was then concentrated to dryness to give 560 mg of the following compound.

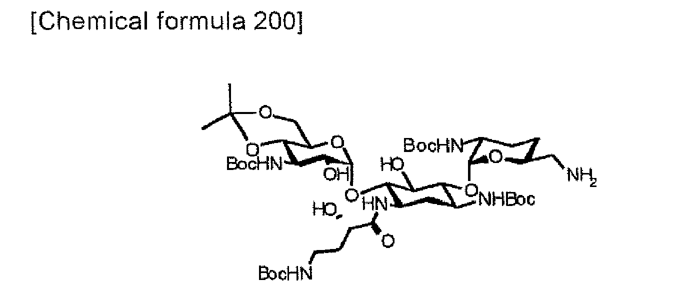

Production step 67-(c)
The compound (95 mg) produced in production step 67-(b) was dissolved in 5 mL of methanol. N-Benzyl-N-(t-butoxycarbonyl)aminoacetaldehyde (70 mg) and 14 mg of sodium cyanide borohydride were added to the solution, and the mixture was stirred at room temperature for 1 hr. The reaction solution was concentrated under the reduced pressure, and the residue was subjected to deprotection in the same manner as in production step 1-(j) to give 27 mg of the following compound.

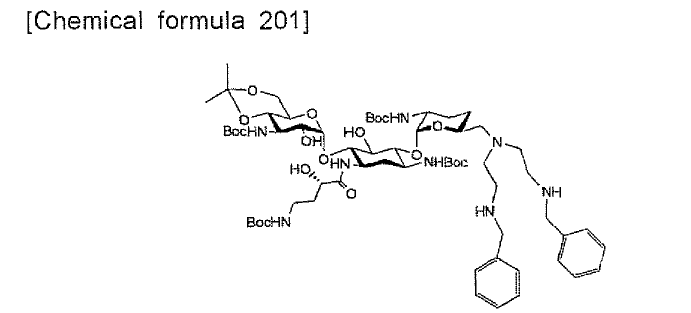

Production step 67-(d)
The compound (21 mg) produced in production step 67-(c) was dissolved in 2 mL of water and 0.3 mL of acetic acid. Palladium hydroxide (17 mg) was added to the solution, and the mixture was subjected to a catalytic hydrogen reduction reaction under a hydrogen atmosphere for 16 hr. The reaction solution was filtered through Celite, and the filtrate was concentrated to dryness. The compound thus obtained was treated in the same manner as in production step 1-(j) to give the title compound: 6'-N,N-bis(2-aminoethyl)arbekacin (13 mg).
FABMS: m/z 639 [M+H]⁺.

Example 68

### 6'-N-amidinoarbekacin

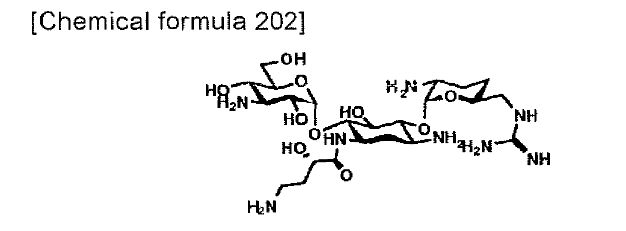

### Production step 68-(a)

The compound (109 mg) produced in production step 12-(c) was dissolved in 1 mL of N,N-dimethylformamide. To the solution, a solution (0.2 mL) of 41 mg of N,N'-bis(tert-butoxycarbonyl)-S-methylisothiourea in N,N-dimethylformamide, 0.032 mL of triethylamine, and 0.1 mL of a solution of 37 mg of mercury chloride in N,N-dimethylformamide were added in that order, and the mixture was stirred at room temperature for 3 hr. This reaction solution was filtered, was concentrated under the reduced pressure, and the residue was then purified by column chromatography on silica gel (chloroform : methanol = 10 : 1). The compound thus obtained was deprotected in the same manner as in production step 1-(j) to give the title compound: 6'-N-amidinoarbekacin (53 mg).
FABMS: m/z 595 [M+H]⁺.

Example 69

### 6'-N-Formimidoylarbekacin

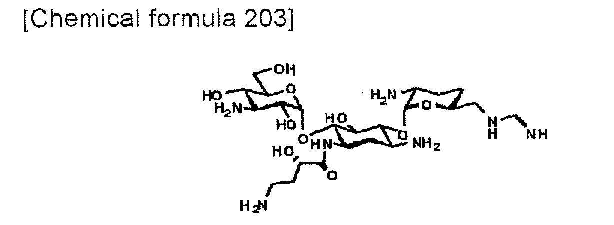

### Production step 69-(a)

The compound (196 mg) produced in production step 12-(c) was dissolved in 4 mL of methanol. Ethylformimidate hydrochloride (67 mg) was added to the solution, and the mixture was stirred at room temperature for 2 days. This reaction solution was concentrated to dryness, and the residue was washed with diethyl ether to give a solid. This solid was treated in the same manner as in production step 1-(j) to give the title compound: 6'-N-formimidoylarbekacin.
FABMS: m/z 580 [M+H]⁺.

Example 70

### 6'-Aminomethyl-6'-deamino-6'-hydroxyarbekacin

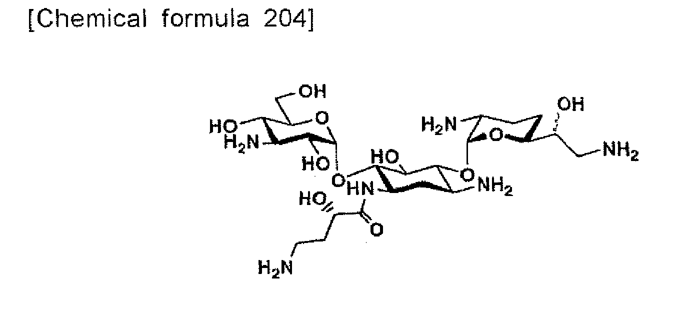

### Production step 70-(a)

The compound (82 mg) produced in production step 30-(d) was dissolved in a solution of 7 mL of methanol, 3 mL of water, and 0.2 mL of acetic acid. Platinum oxide (50 mg) was added to the solution, and the mixture was subjected to a catalytic hydrogen reduction reaction at a hydrogen pressure of 40 lbs for 20 hr, This reaction solution was filtered through Celite and was washed with a solution of methanol : water = 1 : 1, and the filtrate was concentrated under the reduced pressure. The compound thus obtained was deprotected in the same manner as in production step 1-(j) to give the title compound: 6'-aminomethyl-6'-deamino-6'-hydroxyarbekacin (31 mg).
FABMS: m/z 583 [M+H]⁺.

Example 71

### 6'-(2-Aminoethyl)-6'-deaminoarbekacin

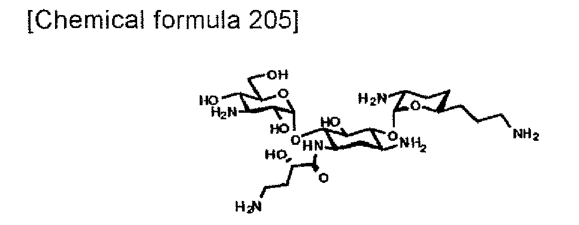

### Production step 71-(a)

The compound (75 mg) produced in production step 30-(c) was dissolved in 3 mL of chloroform. Ph₃P=CHCN (60 mg) was added to the solution, and the mixture was stirred at 40°C for 12 hr. The reaction solution was concentrated under the reduced pressure, and the residue was purified by column chromatography on silica gel (development system, methylene chloride : methanol = 25 : 1) to give 54 mg of the following compound.

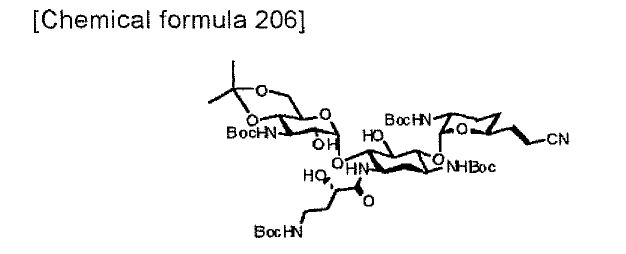

Production step 71-(b)
The compound (54 mg) produced in production step 71-(a) was dissolved in a solution of 7 mL of methanol, 3 mL of water, and 0.2 mL of acetic acid. Platinum oxide (35 mg) was added to the solution, and the mixture was subjected to a catalytic hydrogen reduction reaction at a hydrogen pressure of 40 lbs for 20 hr. The reaction solution was filtered through Celite and washed with a solution of methanol: water = 6 : 4, and the filtrate was concentrated under the reduced pressure. The product thus obtained was deprotected in the same manner as in production steps 1-(i) and (j) to give the title compound: 6'-(2-aminoethyl)-6'-deaminoarbekacin (19.8 mg).
FABMS: m/z 581 [M+H]⁺.

Example 72

### 6"-Adizo-6"-deoxyarbekacin

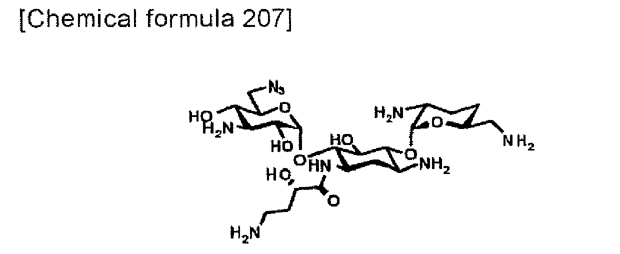

### Production step 72-(a)

The compound (100 mg) produced in production step 32-(b) was dissolved in 4 mL of dimethylformamide, 65 mg of sodium azide was added to the solution, and the mixture was stirred at 90°C for 22 hr. Insolubles were removed from the reaction solution by filtration, and the filtrate was concentrated under the reduced pressure. Ethyl acetate was added to the residue, and the mixture was washed with water and dried over anhydrous magnesium sulfate. This solution was concentrated under the reduced pressure, and the residue was purified by column chromatography on silica gel (development system, ethyl acetate : toluene = 1 : 1) to give 32 mg of the following compound.

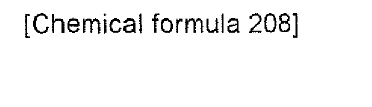

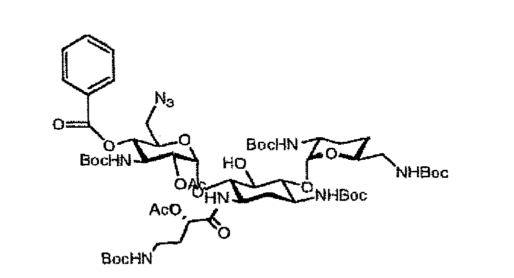

Production step 72-(b)
The compound (30 mg) produced in production step 72-(a) was dissolved in 3 mL of methanol, 0.2 mL of a 2 M aqueous sodium hydroxide solution was added to the solution, and the mixture was stirred at room temperature for 0.5 hr. This reaction solution was neutralized with 1 M hydrochloric acid and was then concentrated under the reduced pressure. The compound thus obtained was deprotected in the same manner as in production step 1-(j) to give the title compound: 6"-azido-6"-deoxyarbekacin (8 mg).
FABMS: m/z 578 [M+H]⁺.

Example 73

### 6"-Amino-6"-deoxyarbekacin

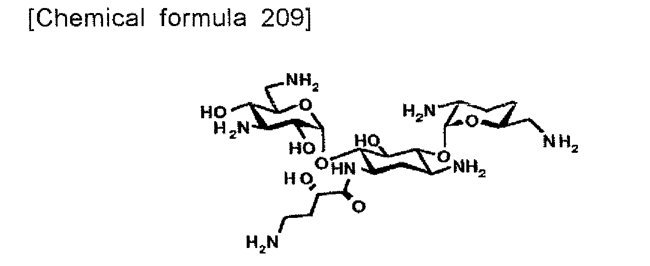

### Production step 73-(a)

The compound produced in Example 72 (6"-azido-6"-deoxyarbekacin) (25 mg) was dissolved in 2 mL of water and 0.3 mL of methanol. Palladium black (10 mg) was added to this solution, and the mixture was subjected to a catalytic hydrogen reduction reaction under a hydrogen atmosphere for 3 hr. This reaction solution was filtered through Celite, and the filtrate was concentrated to dryness. The compound thus obtained was treated in the same manner as in production step 1-(j) to give the title compound: 6"-amino-6"-deoxyarbekacin (12.6 mg).
FABMS: m/z 552 [M+H]⁺.

Example 74

### 6"-Deoxy-6"-(4-morpholinyl)arbekacin

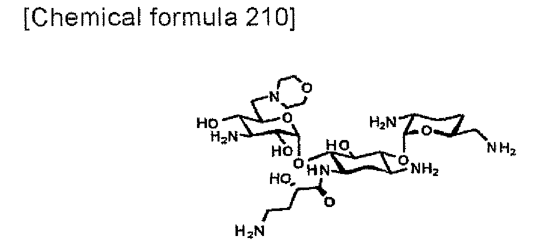

### Production step 74-(a)

The compound (1.0 g) produced in production step 1-(a) was dissolved in 20 mL of pyridine. 4-Dimethylaminopyridine (900 mg) and 560 mg of trityl chloride were added to the solution, and the mixture was stirred at 70°C for 4 hr. Acetic anhydride (0.9 mL) was added to the reaction solution, and the mixture was stirred at room temperature for 16 hr. Methanol (1 mL) was added to the reaction solution, and the mixture was concentrated under the reduced pressure. Ethyl acetate was added to the residue, and the mixture was washed with an aqueous potassium hydrogensulfate solution, an aqueous sodium hydrogencarbonate solution, and water in that order, and was then dried over anhydrous magnesium sulfate. This solution was concentrated under the reduced pressure, and the residue was purified by column chromatography on silica gel (development system, methylene chloride: methanol = 25 : 1) to give 1.24 g of the following compound.

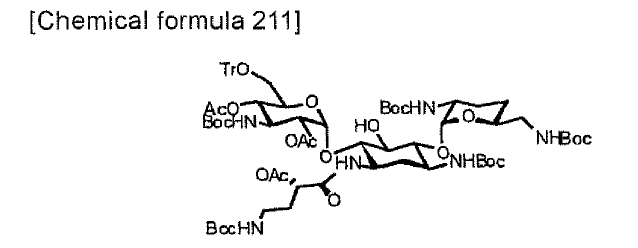

Production step 74-(b)
The compound (480 mg) produced in production step 74-(a) was dissolved in 3 mL of diethyl ether. Formic acid (3 mL) was added to the solution, and the mixture was stirred at room temperature for 20 hr. Ethyl acetate was added to the reaction solution, and the mixture was washed with an aqueous sodium hydrogencarbonate solution and water, and then dried over anhydrous magnesium sulfate. This solution was concentrated under the reduced pressure. The residue was purified by column chromatography on silica gel (development system, methylene chloride : methanol = 15 : 1) to give 200 mg of the following compound.

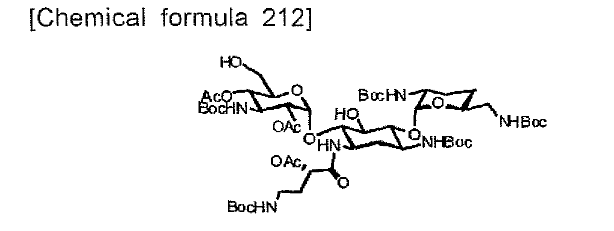

Production step 74-(c)
The compound (200 mg) produced in production step 74-(b) was dissolved in 8 mL of benzene and 1 mL of dimethylsulfoxide. 180 mg of dicyclohexylcarbodiimide, 46 mg of pyridine, and 21 mg of trifluoroacetic acid were added to the solution, and the mixture was stirred at room temperature for 16 hr. This reaction solution was filtered, and the filtrate was concentrated under the reduced pressure. Ethyl acetate was added to the residue, and the mixture was washed with water and then dried over anhydrous magnesium sulfate. This solution was concentrated under the reduced pressure, and the residue was purified by column chromatography on silica gel (development system, methylene chloride : methanol = 60 : 1) to give 200 mg of the following compound.

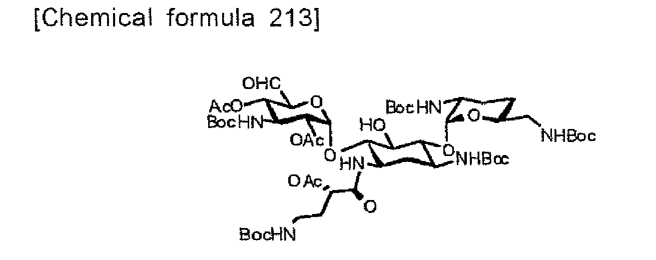

Production step 74-(d)
The compound (96 mg) produced in production step 74-(c) was dissolved in 5 mL of methanol. 230 mg of Morpholine and 18 mg of sodium cyanide borohydride were added to the solution, and the mixture was stirred at room temperature for 3 hr. Further, 0.3 mL of a 2 M aqueous sodium hydroxide solution was added to the reaction solution, and the mixture was stirred for 0.5 hr. This reaction solution was neutralized with 1 M hydrochloric acid, and then concentrated under the reduced pressure. The compound thus obtained was deprotected in the same manner as in production step 1-(j) to give the title compound: 6"-deoxy-6"-(4-morpholinyl)arbekacin (31 mg).
FABMS: m/z 622 [M+H]⁺.

Example 75

### 6"-Deoxy-6"-(2-aminoethyl)aminoarbekacin

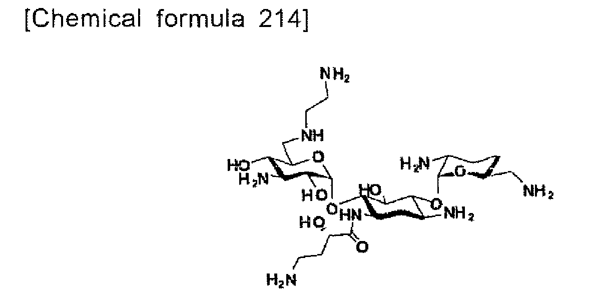

### Production step 75-(a)

The compound (70 mg) produced in production step 74-(c) was reacted with N-(tert-butoxycarbonyl)ethylenediamine in the same manner as in production step 74-(d) to give the title compound: 6" - deoxy-6"-(2-aminoethyl)aminoarbekacin (26 mg).
FABMS: m/z 595 [M+H]⁺.

Example 76

### 6"-Aminomethylarbekacin

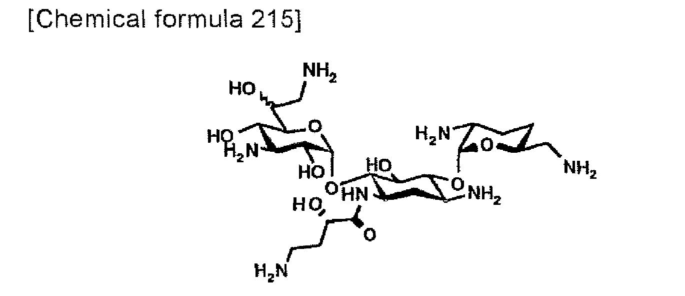

### Production step 76-(a)

0.1 mL of 1 M sodium methoxide in methanol solution was added to a solution of the compound (108 mg) produced in production step 74-(c) dissolved in 5 mL of methanol, and the mixture was stirred at room temperature for 30 min. Further, 0.55 mL of nitromethane was added to the reaction solution, and the mixture was stirred at room temperature for 4 hr. This reaction solution was neutralized with 1 M hydrochloric acid under an ice bath and concentrated to dryness. The residue was purified by column chromatography on silica gel (methylene chloride : methanol = 15 : 1) to give 43 mg of the following compound.

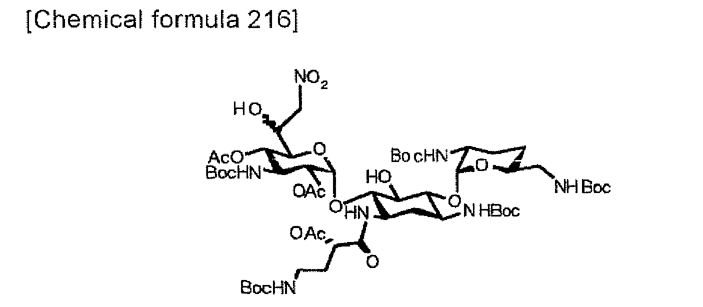

Production step 76-(b)
The compound (43 mg) produced in production step 76-(a) was dissolved in 2.5 mL of ethanol and 2.5 mL of water. Acetic acid (0.05 mL) and 20 mg of platinum oxide were added to the solution, and the mixture was subjected to a catalytic hydrogen reduction reaction under a hydrogen atmosphere. After 16 hr from the start of the reaction, 40 mg of platinum oxide was further added, and the mixture was subjected to a catalytic hydrogen reduction reaction for additional 12 hr under a hydrogen atmosphere. The reaction solution was filtered through Celite, and the filtrate was then concentrated to dryness. The residue was purified by column chromatography on silica gel (methylene chloride : methanol : aqueous ammonia = 60 : 10 : 1) to give 10 mg of the following compound.

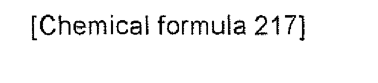

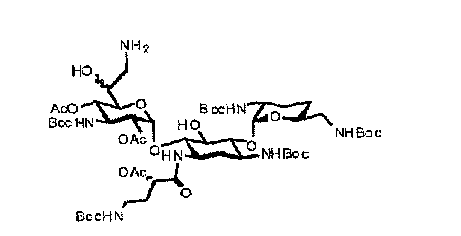

Production step 76-(c)
A 90% aqueous trifluoroacetic acid solution (1 mL) was added to the compound (10 mg) produced in production step 76-(b), and the mixture was stirred at room temperature overnight. Water (10 mL) was added to the reaction solution, and the aqueous layer was washed three times with 5 mL of diethyl ether. Further, the aqueous layer was adjusted to pH 7 by neutralization with aqueous ammonia and then purified by 20 mL of CM-Sephadex (NH₄⁺) to give the title compound: 6"-aminomethylarbekacin (0.3 g).
FABMS: m/z 582 [M+H]⁺.

Test Example 1

### Antimicrobacterial activity

The minimal inhibitory concentration (MIC, µg/mL) against MRSAs (MRSA-HR and MRSA-LR) were measured for typical comounds of novel aminoglycoside antibiotic derivatives described in the Examples (Example 1, Example 2, Example 9, Example 13, Example 18, Example 24, Example 44, Example 45, Example 48, Example 49, Example 51, Example 52, Example 53, Example 54, and Example 76) by an agar plate dilution method based on the method based on the JAPANESE SOCIETY OF CHEMOTHERAPY. As a result, the MIC value of the compounds described in the Examples was not more than 8 against MRSA-HR and not more than 4 against MRSA-LR. The same test was carried out for arbekacin described in Japanese Patent Laid-Open No. 164696/1980. The MIC value was 64 for MRSA-HR and 16 for MRSA-LR. Further, the minimal inhibitory concentration (MIC) against P. aeruginosa PAO1 was measured using the above compounds of the Examples and was found to be not more than 4.

## Claims

1. A compound represented by general formula (I) or a pharmacologically acceptable salt or solvate thereof: 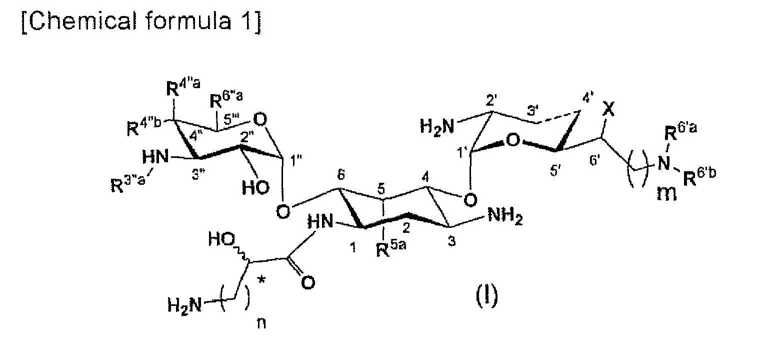 wherein
R^{4"a} and R^{4"b}, which may be the same or different, represent a hydrogen atom or hydroxyl,
R^{5a} represents a halogen atom,
hydroxyl,
amino,
azide,
C₁₋₆ alkanoyloxy,
C₁₋₆ alkylsulfonyloxy,
C₁₋₆ alkanoylamino,
arylcarbonylamino,
di-C₁₋₆ alkylamino, or,
C₁₋₆ alkylamino wherein one or more hydrogen atoms in the alkyl group are optionally substituted by hydroxyl, phenyl, vinyl, amino, or hydroxymethyl,
R^{6"a} represents C₁₋₆ alkyl wherein one or more hydrogen atoms in the alkyl group are optionally substituted by hydroxyl, a halogen atom, or amino,
R^{6'a} and R^{6'b}, which may be the same or different, represent a hydrogen atom or C₁₋₆ alkyl,
R^{3"a} represents a hydrogen atom or C₁₋₆ alkyl,
the dashed line represents a single bond or a double bond,
m represents an integer of 0 to 2,
X represents a hydrogen atom or hydroxyl,
n represents an integer of 1 to 3, and
* represents an R or S configuration, provided that
R^{5a} represents a group as defined above other than a fluorine atom when R^{4"a} represents a hydrogen atom, R^{4"b} represents hydroxyl, and the dashed line represents a single bond; and R^{5a} represents a group as defined above other than hydroxyl, amino, and azide when R^{3"a} represents a hydrogen atom, R^{4"a} represents a hydrogen atom, R^{4"b} represents hydroxyl, R^{6"2} represents hydroxymethyl, both R⁶ and R^{6'b} represent a hydrogen atom, X represents a hydrogen atom, and the dashed line represents a single bond.

2. The compound according to claim 1 or a pharmacologically acceptable salt or solvate thereof,
wherein
R^{5a} represents C₁₋₃ alkanoyloxy, C₁₋₃ alkylsulfonyloxy, C₁₋₃ alkanoylamino, phenylcarbonylamino, naphthylcarbanylamino, di-C₁₋₃ alkylamino, or C₁₋₃ alkylamino wherein one or more hydrogen atoms in the alkyl group are optionally substituted by hydroxyl, phenyl, vinyl, amino, or hydroxymethyl.

3. The compound according to claim 1 or a pharmacologically acceptable salt or solvate thereof,
wherein
R^{6"a} represents C₁₋₃ alkyl wherein one or more hydrogen atoms in the alkyl group are optionally substituted by hydroxyl, a halogen atom, or amino.

4. The compound according to claim 1 or a pharmacologically acceptable salt or solvate thereof, wherein R^{6'a} and R^{6'b}, which may be the same or different, represent a hydrogen atom or C₁₋₃ alkyl.

5. The compound according to claim 1 or a pharmacologically acceptable salt or solvate thereof, wherein R^{3"a} represents C₁₋₃ alkyl.

6. The compound according to claim 1 or a pharmacologically acceptable salt or solvate thereof,
wherein
R^{5a} represents a halogen atom, hydroxyl, amino, azide, C₁₋₃ alkanoyloxy, C₁₋₃ alkylsulfonyloxy, C₁₋₃ alkanoylamino, phenylcarbonylamino, naphthylcarbonylamino, di-C₁₋₃ alkylamino, or, C₁₋₃ alkylamino wherein one or more hydrogen atoms in the alkyl group are optionally substituted by hydroxyl, phenyl, vinyl, amino, or hydroxymethyl,
R^{6"a} represents C₁₋₃ alkyl wherein one or more hydrogen atoms in the alkyl group are optionally substituted by hydroxyl, a halogen atom, or amino,
R^{6'a} and R^{6'b}, which may be the same or different, represent a hydrogen atom or C₁₋₃ alkyl, and
R^{3"a} represents a hydrogen atom or C₁₋₃ alkyl.

7. The compound according to claim 1 or a pharmacologically acceptable salt or solvate thereof,
wherein
R^{4"a} represents a hydrogen atom or hydroxyl,
R^{4"b} represents a hydrogen atom,
R^{6"a} represents hydroxymethyl,
any one of R^{6'a} and R^{6'b} represents a hydrogen atom,
the dashed line represents a single bond,
m represents 0,
X represents a hydrogen atom, and
n represents an integer of 1 to 3.

8. The compound according to claim 1 or a pharmacologically acceptable salt or solvate thereof,
wherein
R^{5a} represents a chlorine atom, hydroxyl, amino, azide, C₁₋₆ alkanoyloxy, C₁₋₆ alkylsulfonyloxy, C₁₋₆ alkanoylamino, arylcarbonylamino, di-C₁₋₆ alkylamino, or C₁₋₆ alkylamino wherein one or more hydrogen atoms in the alkyl group are optionally substituted by hydroxyl, phenyl, vinyl, amino, or hydroxymethyl,
R^{6"a} represents hydroxymethyl,
any one of R^{6'a} and R^{6'b} represents a hydrogen atom,
the dashed line represents a single bond,
m represents 0,
X represents a hydrogen atom, and
n represents 2.

9. The compound according to claim 1 or a pharmacologically acceptable salt or solvate thereof, wherein R^{6"a} represents hydroxymethyl or fluoromethyl,
both R^{6'a} and R^{6'b} represent a hydrogen atom,
R^{3"a} represents a hydrogen atom,
the dashed line represents a double bond,
m is 0 (zero),
X represents a hydrogen atom, and
n is 1 or 2.

10. A compound represented by general formula (II) or a pharmacologically acceptable salt or solvate thereof: 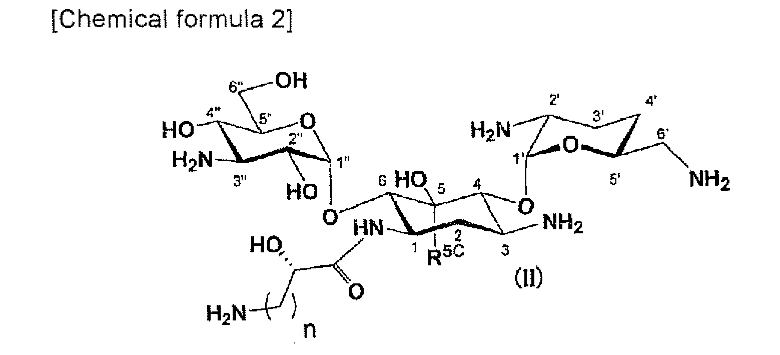 wherein
R^{5C} represents C₁₋₆ alkyl wherein one or more hydrogen atoms in the alkyl group are optionally substituted by C₁₋₆ alkoxy,
C₂₋₆ alkenyl, or,
amino C₁₋₆ alkyl wherein one or more hydrogen atoms in the amino group are optionally substituted by C₁₋₆ alkyl where one or more hydrogen atoms in the alkyl group are optionally substituted by amino, hydroxyl, or heteroaryl, and
n is an integer of 1 to 3.

11. The compound according to claim 10 or a pharmacologically acceptable salt or solvate thereof,
wherein
R^{5C} represents C₁₋₃ alkyl wherein one or more hydrogen atoms in the alkyl group are optionally substituted by C₁₋₆ alkoxy; C₂₋₄ alkenyl; or amino C₁₋₃ alkyl wherein one or more hydrogen atoms in the amino group are optionally substituted by C₁₋₆ alkyl
where one or more hydrogen atoms in the alkyl group are optionally substituted by amino, hydroxyl, or heteroaryl.

12. The compound according to claim 10 or a pharmacologically acceptable salt or solvate thereof,
wherein
R^{5C} represents C₁₋₆ alkyl wherein one or more hydrogen atoms in the alkyl group are optionally substituted by C₁₋₃ alkoxy; C₂₋₆ alkenyl; or amino C₁₋₆ alkyl wherein one or more hydrogen atoms in the amino group are optionally substituted by C₁₋₃ alkyl
where one or more hydrogen atoms in the alkyl group are optionally substituted by amino, hydroxyl, pyrrolyl, or pyridyl.

13. The compound according to claim 10 or a pharmacologically acceptable salt or solvate thereof,
wherein
R^{5c} represents C₁₋₃ alkyl wherein one or more hydrogen atoms in the alkyl group are optionally substituted by C₁₋₃ alkoxy; C₂₋₄ alkenyl; or amino C₁₋₃ alkyl wherein one or more hydrogen atoms in the amino group are optionally substituted by C₁₋₃ alkyl
where one or more hydrogen atoms in the alkyl group are optionally substituted by amino, hydroxyl, pyrrolyl, or pyridyl.

14. A compound represented by general formula (III) or a pharmacologically acceptable salt or solvate thereof: 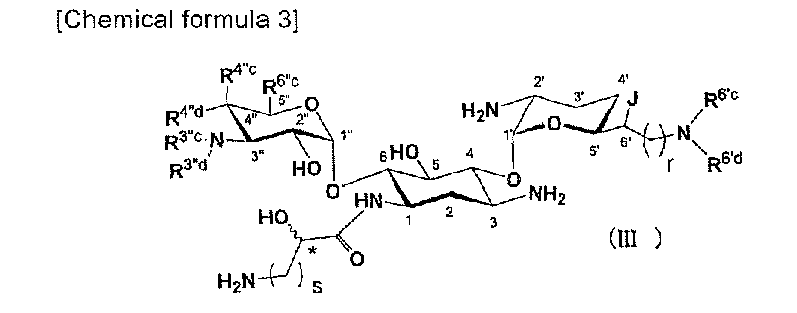 wherein
R^{4"c} represents a hydrogen atom or hydroxyl,
R^{4"d} represents a hydrogen atom or hydroxyl wherein, when
R^{4"c} represents hydroxyl, R^{4"d} represents a hydrogen atom,
R^{6"c} represents C₁₋₆ alkyl wherein one or more hydrogen atoms in the alkyl group are optionally substituted by hydroxyl, amino, or azide; or a group of the formula: 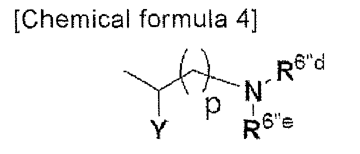 wherein R^{6"d} and R^{6"e}, which may be the same or different, represent a hydrogen atom or amino C₁₋₆ alkyl, or R^{6"d} and R^{6"e} together may represent a six-membered cyclic group containing 1 to 4 heteroatoms, Y represents a hydrogen atom or hydroxyl, and p represents an integer of 0 or 1,
R^{3"c} and R^{3"d}, which may be the same or different, represent
a hydrogen atom,
C₁₋₁₀ alkyl wherein one or more hydrogen atoms in the alkyl group are optionally substituted by hydroxyl or aryl optionally substituted by hydroxyl or amino,
formimidoyl, or
amidino,
R^{6'c} and R^{6'd}, which may be the same or different, represent
a hydrogen atom,
amino C₁₋₆ alkyl,
formimidoyl,
amidino, or
benzyl optionally substituted by hydroxyl,
r represents an integer of 0 to 2,
J represents a hydrogen atom or hydroxyl,
s represents an integer of 1 to 3, and
* represents an R or S configuration,
excluding compounds wherein
R^{4"c}, R^{3"c}, R^{3"d}, R^{6'c}, and R^{6'd} simultaneously represent a hydrogen atom, R^{4"d} represents hydroxyl, R^{6"c} represents hydroxymethyl, r represents 0, X represents a hydrogen atom, and s represents 2.

15. The compound according to claim 14 or a pharmacologically acceptable salt or solvate thereof,
wherein
R^{6"c} represents C₁₋₃ alkyl wherein one or more hydrogen atoms in the alkyl group are optionally substituted by hydroxyl, amino, or azide; or a group of the formula: 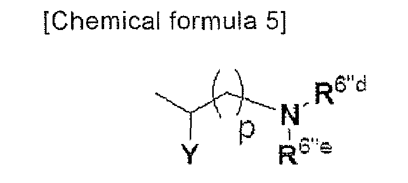 wherein R^{6"d} and R^{6"e}, which may be the same or different, represent a hydrogen atom or amino C₁₋₃ alkyl, or R^{6"d} and R^{6"e} together represent a six-membered cyclic group containing 1 to 4 heteroatoms, Y represents a hydrogen atom or hydroxyl, and p represents an integer of 0 or 1,
R^{3"c} and R^{3"d}, which may be the same or different, represent
a hydrogen atom,
C₁₋₆ alkyl wherein one or more hydrogen atoms in the alkyl group are optionally substituted by hydroxyl; phenyl optionally substituted by hydroxyl or amino; or naphthyl optionally substituted by hydroxyl or amino, formimidoyl, or
amidino,
R^{6'c} and R^{6'd}, which may be the same or different, represent
a hydrogen atom,
amino C₁₋₃ alkyl,
formimidoyl,
amidino, or
benzyl optionally substituted by hydroxyl.

16. The compound according to claim 14 or a pharmacologically acceptable salt or solvate thereof,
wherein
R^{4"c} represents a hydrogen atom, R^{4"d} represents hydroxyl, both R^{6'c} and R^{6'd} represent a hydrogen atom, both R^{3"c} and R^{3"d} represent a hydrogen atom, r represents 0, J represents a hydrogen atom, and s represents 2.

17. The compound according to claim 14 or a pharmacologically acceptable salt or solvate thereof,
wherein
R^{4"c} represents a hydrogen atom, R^{4"d} represents hydroxyl, both R^{6'c} and R^{6'd} represent a hydrogen atom, R^{6"c} represents hydroxymethyl, any one of R^{3"c} and R^{3"d} represents a hydrogen atom, r represents 0, J represents a hydrogen atom, and s represents 2.

18. The compound according to claim 14 or a pharmacologically acceptable salt or solvate thereof,
wherein
R^{4"c} represents a hydrogen atom, R^{4"d} represents hydroxyl, R^{6"c} represents hydroxymethyl, both R^{3"c} and R^{3"d} represent a hydrogen atom, and s represents 2.

19. 5,4"-Diepiarbekacin,
5-deoxy-4"-epi-5-epifluoroarbekacin,
5-deoxy-4"-epi-5-epichloroarbekacin,
5-deoxy-4"-epi-5-epiaminoarbekacin,
4"-deoxy-5-epiarbekacin,
1-N-[(S)-(3-amino-2-hydroxypropanoyl)]-5,4"-diepidibekacin,
5,4"-diepi-3"-N-methylarbekacin,
5,4"-diepi-6'-N-methylarbekacin,
5-epiarbekacin,
5-deoxy-5-epichloroarbekacin,
5-deoxy-5-epiaminoarbekacin,
5-deoxy-5-epi(2-aminoethyl)aminoarbekacin,
5-epi-3"-N-methylarbekacin,
6"-aminomethyl-5-epiarbekacin,
3',4'-didehydro-5-epiarbekacin,
5-deoxy-3',4'-didehydro-5-epifluoroarbekacin,
5-deoxy-3',4'-didehydro-5-epiaminoarbekacin,
1-N-[(S)-(3-amino-2-hydroxypropanoyl)]-3',4'-didehydro-5-epidibekacin,
3',4'-didehydro-5,4"-diepiarbekacin,
5-deoxy-3',4'-didehydro-4"-epi-5-epifluoroarbekacin,
5-deoxy-3',4'-didehydro-4"-epi-5-epiaminoarbekacin,
4"-deoxy-3',4'-didehydro-5-epiarbekacin, or
6"-aminomethylarbekacin.

20. A pharmaceutical composition comprising a compound according to any one of claims 1 to 19, or a pharmacologically acceptable salt or solvate thereof.

21. An antimicrobial agent comprising a compound according to any one of claims 1 to 19, or a pharmacologically acceptable salt or solvate thereof.

22. An anti-MRSA agent comprising a compound represented by general formula (Ia) or a pharmacologically acceptable salt or solvate thereof: 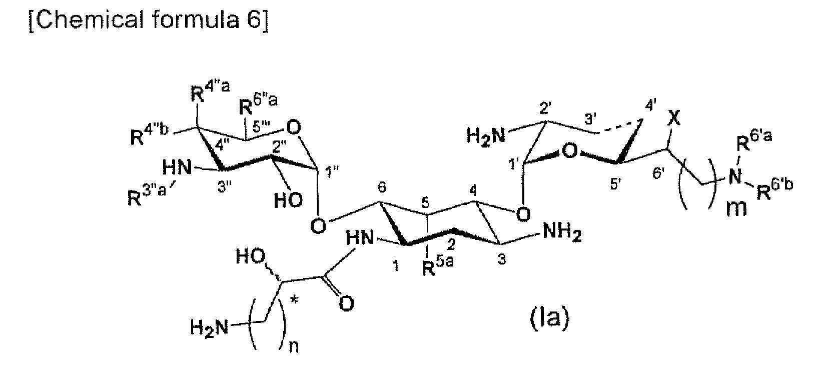 wherein
R^{4"a} and R^{4"b}, which may be the same or different, represent a hydrogen atom or hydroxyl,
R^{5a} represents a halogen atom,
hydroxyl,
amino,
azide,
C₁₋₆ alkanoyloxy,
C₁₋₆ alkylsulfonyloxy,
C₁₋₆ alkanoylamino,
arylcarbonylamino,
di-C₁₋₆ alkylamino, or
C₁₋₆ alkylamino wherein one or more hydrogen atoms in the alkyl group are optionally substituted by hydroxyl, phenyl, vinyl, amino, or hydroxymethyl,
R^{6"a} represents C₁₋₆ alkyl wherein one or more hydrogen atoms in the alkyl group are optionally substituted by hydroxyl, a halogen atom, or amino,
R^{6'a} and R^{6'b}, which may be the same or different, represent a hydrogen atom or C₁₋₆ alkyl,
R^{3"a} represents a hydrogen atom or C₁₋₆ alkyl,
the dashed line represents a single bond or a double bond,
m represents an integer of 0 to 2,
X represents a hydrogen atom or hydroxyl,
n represents an integer of 1 to 3, and
* represents an R or S configuration, provided that
R^{5a} represents a group as defined above other than a fluorine atom when R^{4"a} represents a hydrogen atom, R^{4"b} represents hydroxyl, and the dashed line represents a single bond.

23. An anti-MRSA agent comprising a compound according to any one of claims 10 to 13 or a pharmacologically acceptable salt or solvate thereof.

24. An anti-MRSA agent comprising a compound according to any one of claims 14 to 18 or a pharmacologically acceptable salt or solvate thereof.

25. Use of a compound according to any one of claims 1 to 19 or a pharmacologically acceptable salt or solvate thereof, for the manufacture of a pharmaceutical composition.

26. Use of a compound according to any one of claims 1 to 19 or a pharmacologically acceptable salt or solvate thereof, for the manufacture of an antimicrobial agent.

27. A method for treating or preventing an infectious disease, comprising the step of administering a compound according to any one of claims 1 to 19 or a pharmacologically acceptable salt or solvate thereof to an animal including a human.
